# EUROPEAN PATENT SPECIFICATION

(11) **EP 3 576 778 B1**
(45) Date of publication and mention of the grant of the patent: **21.07.2021**
(21) Application number: 18703295.8
(22) Date of filing: 01.02.2018
(51) Int. Cl.: A61K 39/00, C12N 15/10, C12Q 1/68

(54) **FUNCTIONAL SCREENING OF ANTIGENIC POLYPEPTIDES-USE FOR THE IDENTIFICATION OF ANTIGENS ELICITING A PROTECTIVE IMMUNE RESPONSE AND FOR THE SELECTION OF ANTIGENS WITH OPTIMAL PROTECTIVE ACTIVITY**
FUNKTIONELLES SCREENING VON ANTIGENEN POLYPEPTIDEN VERWENDUNG ZUR IDENTIFIZIERUNG VON ANTIGENEN ZUR ANREGUNG EINER SCHÜTZENDEN IMMUNANTWORT UND ZUR AUSWAHL VON ANTIGENEN MIT OPTIMALER SCHUTZWIRKUNG
CRIBLAGE FONCTIONNEL DE POLYPEPTIDES ANTIGÉNIQUES - UTILISATION POUR L'IDENTIFICATION D'ANTIGÈNES ÉLICITANT UNE RÉPONSE IMMUNITAIRE PROTECTRICE ET POUR LA SÉLECTION D'ANTIGÈNES PRÉSENTANT UNE ACTIVITÉ DE PROTECTION OPTIMALE

(30) Priority: 02.02.2017 EP 17305121
(43) Date of publication of application: 11.12.2019
(73) Proprietor: INSTITUT PASTEUR, 75724 Paris Cedex 15 (FR)
(72) Inventor: AMINO, Rogerio, 92220 Bagneux (FR); BEIGNON, Anne-Sophie, 75014 Paris (FR); CHARNEAU, Pierre, 75005 Paris (FR)
(74) Representative: Ernest Gutmann - Yves Plasseraud S.A.S.
(86) International application number: PCT/EP2018/052551
(87) International publication number: WO 2018/141864

(56) References cited:
- WO-A1-2011/138251
- VAN DIJK MELISSA R ET AL: "Genetically attenuated, P36p-deficient malarial sporozoites induce protective immunity and apoptosis of infected liver cells", PROCEEDINGS NATIONAL ACADEMY OF SCIENCES PNAS, NATIONAL ACADEMY OF SCIENCES, US, vol. 102, no. 34, 23 August 2005 (2005-08-23), pages 12194-12199, XP002490773, ISSN: 0027-8424, DOI: 10.1073/PNAS.0500925102

## Description

The invention is in the field of functional screening of protective antigens against *Plasmodium* pathogens in order to identify antigens suitable for the elicitation of a protective immune response in a host, and/or to optimize antigen design, in particular to define suitable antigen combinations for a protective immune response. The invention thus involves using a lentiviral vaccine platform for induction of a specific cellular or humoral response against assayed antigens on one hand, and using a fast and reproducible assay for the evaluation of the induced protective effect after challenge.

The invention relates to a method of screening antigenic polypeptides and selecting them according to their capability to elicit a protective immune response against *Plasmodium* pathogens.

Accordingly the invention is directed to a method of functionally screening *Plasmodium* antigenic polypeptides and to a method of selection of antigenic polypeptides of malaria parasite that exhibit a protective effect, especially a protective immune response in a host challenged with *Plasmodium* sporozoites. This embodiment provides an illustration of the ability of the designed method and related tools to extend identification of individual protective antigens of pathogens or cancer cells. Importantly, after identifying individual protective antigens, the invention allows testing and selecting multi-antigenic combinations with effective, preferably optimal, protective activity against pathogens or cancer cells.

Malaria is causally related to infection of a host by a parasite and has been for many decades the object of extensive researches with a view to identify agents effective in the treatment of the clinical symptoms associated with the infection and protective vaccines. In the last 15 years, malaria control measures reduced by 48% the global deaths caused by this mosquito-borne disease. Despite this significant decrease in mortality, the WHO estimated ∼215 millions of malaria clinical episodes, resulting in more than 400,000 deaths in 2015. Actual malaria control programs rely mainly on the use of insecticides and antiplasmodial medicines, but the emergence and spreading of resistant mosquitos and parasites put the efficacy of these interventions at risk¹. In this scenario, an efficient malaria vaccine could be an important additional tool to control and eventually eliminate malaria.

Since the 60's, it has been known that multiple immunizations using irradiated sporozoites can elicit sterile protection against malaria infection. However, during the last 50 years only a few protective antigens were identified, but none of them, individually or in combination, could match the robust protection induced by irradiated parasites.

The most advanced malaria vaccine, RTS,S (Mosquirix, GSK), targets the *Plasmodium falciparum* circumsporozoite protein (CSP), the major surface protein of sporozoites, the motile stage inoculated in the skin during an infective mosquito bite. This subunit vaccine reduced the clinical cases of malaria in African infants and children by 26-36%². This partial protection is mainly associated with high titers of anti-CSP antibodies³, and albeit significant, it is far from achieving the standards established by the WHO malaria vaccine road map, which preconizes the development of a vaccine with at least 75% of efficacy against clinical malaria, and ideally targeting morbidity, mortality and parasite transmission⁴.

On the other hand, live irradiated sporozoites can invade but are arrested as early liver-stages inside hepatocytes, conferring sterile immunity against a homologous sporozoite challenge⁵. Unfortunately, technical and economical impediments associated with the production, storage and delivery of these live parasites still hinder their use for mass vaccination in poor tropical countries. This sterile protection seems to be mainly dependent on CD8+ T cells, since their depletion abolishes sterile immunity in several experimental models, however, the identity of the antigens conferring such robust protection is still elusive⁶. So far, the number of known protective antigens among the thousands of possible proteins expressed by pre-erythrocytic (PE) stages, sporozoite and the ensuing liver-stage, is extremely limited, and these antigens only confer weaker protection than CSP alone or in multi-antigenic formulations in humans⁷. To date the attempts to identify new protective antigens from live attenuated sporozoites have not yielded suitable candidates, despite the screening of thousands of PE peptides, mini-genes and genes⁸⁻¹⁰.

WO 2011/138251 describes detection of development of parasitemia after inoculation of the Plasmodium sporozoites in mice using RT-PCR or by staining retrieved blood smears (paragraph [0206] in WO 2011/138251).

Despite these considerable efforts and despite significant improvement in the treatment of the infection and its outcomes, long-term protection on a large scale has not yet been successfully achieved with vaccine candidates. Being challenging in many aspects Malaria thus appears a good candidate to assess the relevancy of a new proposed method to identity protective antigens either as such or in multi-antigen combinations.

In this regard, the invention relates to a method of functionally screening immunogenic and protective properties of antigenic polypeptides, also regarded as screening protective antigenic polypeptides, of a determined *Plasmodium* pathogen comprising the steps of:
a. optionally pre-selecting candidate antigenic polypeptides for the screening by reference to a group of genes, transcripts or proteins identified for a determined *Plasmodium* pathogen wherein the antigenic polypeptides may constitute putative targets for a humoral and/or cellular immune responses.
b. providing a lentiviral vector, in particular a HIV-1 based vector, expressing one or more antigenic polypeptide(s) to be assayed for its (their) protective properties,
c. immunizing a non-human mammal in particular a non-human mammal model selected for its susceptibility to infection by the determined *Plasmodium* pathogen, with the lentiviral vector of step b. in immunization dose conditions enabling elicitation of a potent cellular and/or humoral memory response against the antigenic polypeptide(s),
d. challenging the immunized non-human mammal of step c. with the *Plasmodium* pathogen to the immunized non-human mammal of step c. and quantifying the development of the pathogen wherein the quantification of the protective response against the antigenic polypeptide comprises a step of quantifying (i) the load of the sporozoites expressing a constitutive bioluminescent marker, in the liver of the non-human mammal by bioluminescence or (ii) the growth of the *Plasmodium* pathogen expressing a constitutive fluorescent marker, by determining fluorescence on red blood cells harvested from a blood sample of the non-human mammal, especially by flow cytometry, thereby enabling functional identification of the protective response capacity of the antigenic polypeptide(s).

In the disclosure of this method the pathogen may be selected in the group of extracellular or intracellular viruses, bacteria, fungi, protozoans and worms. The pathogen may be selected among arboviruses, hemorrhagic-fever causing viruses, immunodeficiency-causing virus, Mycobacterium spp, Yersinia spp, Listeria ssp, Neisseria spp, Shigella spp, Histoplasma spp, Cryptococcus spp, kinetoplastida parasites, apicomplexan parasites, including malaria parasites, and cestode, trematode or nematode worms.

A method of the disclosure is suitable for use with antigenic polypeptide(s) which are cancer cell antigens, in particular antigens selected in the group of melanoma, breast carcinoma, B cell lymphoma, colon cancer in particular colon carcinoma, liver cancer, lung cancer, bladder cancer in particular bladder carcinoma, mastocytoma, pancreas cancer and prostate adenocarcinoma.

As example of non-human mammal, in particular animal model suitable for testing according to the methoddisclosed, rodents are provided, and in particular the following animal are disclosed, which are available to the person skilled in the art: for melanoma: B16F10 C57BL/6 mice (ATCC CRL6745), for breast carcinoma: 4T1 BALB/c mice (ATCC CRL2539), for B cell lymphoma: A20 Balb/C mice (ATCC TIB-208), for bladder cancer: MBT2 C3H/HeN mice (JCRB cell bank IFO50041), for bladder carcinoma: AY27 Fischer rats, for colon cancer: ProB BD9 rats, for colon carcinoma: CT26 BALB/c mice (ATCC CRL2638), for liver cancer: Hepa1-6 C57L mice (ATCC CRL1830), for lung cancer: LL/2 C57BL mice (ATCC CRL1642) or TC1 C57BL/6 mice (ATCC CRL2785), for mastocytoma: P815 DBA/2 mice (ATCC TIB-64), for pancreas cancer: PAN02 C57BL/6 mice, for prostate Adenocarcinoma: R3327H Cop Rat.

The method of the invention may be carried out having recourse to non-pre-selected candidate antigenic polypeptides or, alternatively or in a subsequent step, may be carried out using down-selected antigens as illustrated hereafter for malaria.

By the expression **"antigenic polypeptide",** it is intended according to the present invention a polypeptide which may be an antigen of a *Plasmodium* pathogen, in particular a native antigen of a *Plasmodium* parasite, or expression product of a gene of a *Plasmodium* parasite, in particular of *P. berghei, P. cynomolgi* or of a *Plasmodium* parasite infecting humans such as *P. falciparum, P. vivax, P. malariae, P.ovale or P. knowlesi.* The application also relates to modified version of such antigenic polypeptides designated as **"polypeptidic derivative thereof"** which can be a fragment of the native antigen of the pathogen or of the cancer cell, in particular of *Plasmodium* parasite and especially may be a truncated version of such native antigen. A derivative polypeptide has an amino acid sequence which is sufficient to provide one or several epitope(s) and which essentially keeps the immunogenic properties of the antigenic polypeptide. It may accordingly have a length of at least about 4 amino acid residues for B epitopes or at least about 8 amino acid residues for T epitopes. In a particular embodiment, the recombinant polynucleotide of the lentiviral vector encodes a fragment of an antigen of the *Plasmodium* pathogen in particular of the malaria parasite, especially a fragment which results from the deletion of contiguous amino acid residues of the full-length (i.e., native) antigen, provided it keeps the capacity of the native antigen to elicit an immune response in a host.

The polypeptidic derivative as defined hereabove should be considered an alternative to the recited antigenic polypeptide in any definitions or embodiments of the invention unless it appears irrelevant in the context of the disclosure.

The screening method of the invention is in particular intended for the identification of antigenic polypeptides or polypeptidic derivatives thereof which bear T and/or B epitopes as both cellular and humoral immune response may be involved in the protection against the *Plasmodium* pathogen, in particular against the malaria parasite, or against the condition or disease resulting from the infection.

The expressions"***T-epitope"* and "*B-epitope*"** refer to antigenic determinants that are involved respectively in the adaptive immune response driven by T cells and in the immune response driven by B cells. In particular said T-epitopes and respectively B-epitopes elicit T cell, respectively B cell immune response when delivered to the host in suitable conditions. According to a particular embodiment the antigenic polypeptides targeted according to the invention and the polypeptide derivatives of these antigenic polypeptides comprise epitope(s) mediating CD8⁺ T cell response. In a particular embodiment, alternatively or cumulatively, the antigenic polypeptides of the invention and the polypeptide derivatives of these antigenic polypeptides comprise epitope(s) mediating an antibody response.

A particular aspect of the screening according to the invention relates to the use in the designed method of means consisting in lentiviral vectors, in particular HIV-1 based vectors, that show capacity to deliver genes encoding polypeptides to the cells of the non-human mammal model and to elicit a protective immune response when the antigenic polypeptide has such capability.

Accordingly, the method of the invention comprises immunization with one or more than one dose(s) of the assayed antigenic polypeptide.

The term **"protective"** in the context of the present invention refers to the capability of the screened and selected antigens to elicit a response in the administered host reflected in any of the following outcomes (i) prevention of infection by the *Plasmodium* parasite, (ii) elimination of the *Plasmodium* parasite and preferably prevention of reintroduction (iii) prevention of the transmission from the infected host to other hosts, in particular prevention of the transmission from a human host infected by a *Plasmodium* parasite to mosquitoes and preferably prevention of parasite development in mosquitoes, (iv) prevention of clinical symptoms in at least part of the targeted hosts, in particular prevention of a clinical malaria episode in at least part of the hosts infected with *Plasmodium* parasites. When cancer cells are disclosed as the target of the response, protection encompasses (i) prevention of the onset of a tumor (ii) prevention of the growth of a tumor, (iii) prevention of vascularization of a tumor. Protection may be high or modest depending on the condition and pathogen and is generally regarded as any benefit for the patient that may impact life-threatening outcomes induced by the pathogen or the cancer cells..

In a particular embodiment that proved to allow the recovery of an increased number of protective polypeptides, the screening method encompasses administering two doses of the lentiviral vector expressing the antigenic polypeptide. Accordingly, the immunization comprises administering one dose of lentiviral vector in a priming step and one dose of lentiviral vector in a boosting step wherein in the priming and boosting steps the lentviral vectors are pseudotyped with different non cross-seroneutralizing envelope proteins and wherein the priming and the boosting doses are different, in particular the boosting dose is higher than the priming dose.

In a particular embodiment of the prime-boost regimen for the immunization, the method of screening encompasses using a dose for boosting which is higher than the dose for priming.

In a further embodiment of the screening method, the administered doses of lentiviral vector expressing the antigenic polypeptide for immunization of the non-human mammal are each in the range of 1x10⁵ to 1x10⁸ TU, in particular 1x10⁵ to 1x10⁷. The inventors have shown that the lentiviral vector may be formulated as a suspension of either concentrated or non-concentrated lentiviral vector without significantly affecting the level of the immune response.

The invention accordingly relates in particular to a method of screening wherein the non-human mammal is immunized successively with at least:
(i) lentiviral vector particles as disclosed herein which are pseudotyped with a first VSV-G envelope of the New Jersey strain;
(ii) provided separately in time from lentiviral vector particles in (i), lentiviral vector particles as disclosed herein which are pseudotyped with a different VSV-G envelope protein wherein said second envelope is obtained from a VSV of the Indiana strain. The above disclosed embodiments relating to the conditions for priming and boosting the non-human animals apply especially to this embodiment.

The screening method of the invention is defined in a particular embodiment for use with *Plasmodium* antigens and accordingly encompasses the following features.

To identify critical protective antigens, the invention provides a lentiviral-based immunization screen designed to select plasmodial-conserved antigens capable of protecting susceptible mice against a stringent sporozoite challenge. Using this functional screen the inventors identified 8 protective antigens, including the known vaccine candidates CSP and TRAP, out of 55 tested antigens. Notably, the inventors showed that a combination of 7 antigens sterile protected more than 85% (18/21) of challenged animals versus 5% (1/20) in the CSP immunized group. These findings confirm the relevance of the designed screening test and accordingly pave the way for the development of a multi-antigenic, second-generation pre-erythrocytic malaria vaccine.

The invention thus relates to a method of functionally screening immunogenic properties of antigenic polypeptides of a determined Plasmodium parasite comprising the steps of:
a. optionally pre-selecting candidate antigenic polypeptides for the screening by reference to a group of genes or transcripts identified for a determined *Plasmodium* parasite wherein the group may reflect a particular stage of the development of the parasite or a particular biological function.
b. providing a lentiviral vector, in particular a HIV-1 based vector, expressing one or more antigenic polypeptide(s) to be assayed for its immunogenic properties,
c. immunizing a non-human mammal selected for its susceptibility to infection by the determined *Plasmodium* parasite with the lentiviral vector of step b. in immunization dose conditions enabling elicitation of a potent cellular and/or humoral memory response against the antigenic polypeptide,
d. challenging the immunized non-human mammal of step c. with sporozoites of the *Plasmodium* parasite and quantifying the development of the *Plasmodium* parasite in the non-human mammal thereby enabling functional identification of the protective response capacity of the antigenic polypeptides.

In a particular embodiment of the invention, the assayed antigenic polypeptide is not a native polypeptide of the parasite but a polypeptide derivative thereof.

The expressions ***"malaria parasite*"** and *"Plasmodium parasite*» are used interchangeably in the present application. They designate every and all forms of the parasite that are associated with the various stages of the parasite cycle in the mammalian, especially human host, including in particular sporozoites, especially sporozoites inoculated in the host skin and present in the blood flow after inoculation, or sporozoites developing in the hepatocytes (liver-stages), merozoites, including especially merozoites produced in the hepatocytes and in the red-blood cells or merozoites developing inside red blood cells (blood-stages). These various forms of the parasite are characterized by multiple specific antigens many of which are well known and identified in the art and some of which are still unknown and to which no biological function has yet been assigned. The antigens can often be designated or classified in groups by reference to their expression according to the stage of the infection. *Plasmodium* parasites according to the present disclosure encompass parasites infecting human hosts and parasites infecting non-human animals especially rodents and in particular mice. Accordingly, *Plasmodium falciparum, Plasmodium vivax, Plasmodium yoelii* and *Plasmodium berghei* are particular examples of these parasites. *Plasmodium cynomolgi* is another example which is infectious for macaques, and occasionally can infect humans. Antigens of *Plasmodium* which may be screened according to the method of the invention encompass CSP, TRAP, Celtos, SPECT, ICP, and Facilysin/Bergheilysin or antigens with unknown biological functions such as Ag11-09 and Ag11-10 as disclosed herein. More generally, the method of the invention may be applied to the screening of antigens expressed on gametes, zygotes and ookinetes, antigens of liver-stages or antigens of the asexual blood-stages, as well as, antigens of sporozoïtes. Accordingly antigens that may be screened include also in particular an antigen selected among P48/P45 antigen and Pfs25.

In a particular embodiment of the invention the method of screening encompasses using antigenic polypeptides characteristic of *P. berghei,* or *P. yoelii* and a non-human mammal which is a rodent. It is in particular a mouse susceptible to said *Plasmodium* parasite, especially a C57BL/6 mouse for *P. berghei,* or a BALB/c mouse for *P. yoelii.* According to the invention, the challenging step of the non-human mammal to detect the capacity of the antigenic polypeptide to elicit a protective immune response may be performed by administration to said non-human mammal of the pathogen or the cancer cell antigen providing the antigenic polypeptide, in particular administration of *Plasmodium* sporozoites, expressing a bioluminescent or a fluorescent marker.

As an example administration of 5,000 to 10,000 *Plasmodium* sporozoites, 30 days after the last immunization step with the lentiviral vector is suitable for such challenge step.

The time period between the immunization step and the challenge may vary slightly depending on the animals, immunization conditions and assessed *Plasmodium* pathogen.

Use of a bioluminescent marker is illustrated in the Examples for the *Plasmodium* parasite and enables *in vivo* imaging of the load of parasite especially in the liver of the animal, thereby providing indirect detection of the protective immune response elicited by the lentiviral vector bearing the antigenic polypeptide. Bioluminescence detection is for example obtained when the pathogen, for example the malaria parasite, used for the challenge of the immunized non-human animals, express a constitutive bioluminescent marker, such as luciferase, in particular luciferase-expressing *Plasmodium* sporozoites. Bioluminescence may be detected by imaging an organ, such as the liver for animal challenged with *Plasmodium* sporozoites, of the tested non-human animals and quantification of the pathogen or cancer cell cells, in particular quantification of the malaria parasite may be derived therefrom.

Alternatively constitutive fluorescence may be detected when fluorescent *Plasmodium* pathogens (e.g. expressing GFP), in particular fluorescent *Plasmodium* parasites, such as GFP-expressing sporozoites, are used for challenging the immunized animals. In such a case fluorescence may be detected in biological fluids obtained from the animal or in sub-fractions thereof, such as red blood cells harvested from a blood sample previously obtained from the animal. In such a case detection may be carried out by flow cytometry and fluorescence.

Protective immunization in the non-human mammals obtained using the lentiviral vector is assayed by comparing the average pathogen load, in particular the parasite load in the liver, a few days, such as two days after challenge using bioluminescent *Plasmodium* pathogen, in particular with *Plasmodium* sporozoites, between the GFP-immunized control group and the test group. The challenge is delivered four weeks following the last immunization step.

When the effect of the lentiviral vector immunization is assessed using fluorescence, protective immunization in the non-human mammal is evaluated by comparing the average pathogen contents, in particular the average of log parasitemia with *Plasmodium* parasite, after a determined duration following challenge, such as at day five post challenge of the GFP-immunized control group and the test group. Alternatively, when development of Plasmodium parasite is assessed, individuals with a log parasitemia inferior to the average of log parasitemia of GFP-group minus 2.5 standard deviations are considered protected. When parasites are not detected in red blood cells after 10 days following the challenge, the immunized animals are considered sterile protected. The challenge is delivered four weeks after the last immunization step.

In a particular embodiment of the invention, the method of screening encompasses the use of a positive control for protection capability against the *Plasmodium* pathogen, in particular against infection by *Plasmodium* parasite or against the parasite-induced condition or disease wherein the positive control is a particular antigen of the *Plasmodium* pathogen or, such as CSP (circumsporozoite protein) or TRAP for *Plasmodium* parasite, and using a negative control for protective capability, which is GFP or a parasite antigen known to be non protective. In a particular embodiment of the invention, the method of screening encompasses a step wherein immune-sera in the sample obtained from an immunized non-human mammal are incubated with GFP-expressing *Plasmodium* sporozoites wherein a subgroup of these sporozoites is permeabilized and a subgroup of these sporozoites is not permeabilized. Such a step allows to identify the location of the target of immune reaction elicited by the antigen expressed by the lentiviral particles.

In a particular embodiment of the invention, the lentiviral vector used in the screening contains in its genome a synthetic gene for an antigenic polypeptide of the *Plasmodium* pathogen, such as an antigenic polypeptide of *Plasmodium,* the coding sequence of which is codon-optimized for the expression of the antigenic polypeptide in mammalian cells and wherein expression of the antigenic polypeptide is driven by a promoter suitable for directing gene expression in various mammalian cell types, including dendritic cells, such as a beta-2 microglobulin promoter. The presence of the codon-optimized gene enables a more efficient expression of the antigenic polypeptide in mammalian cells.

In a further embodiment of the invention, the synthetic codon-optimized gene sequence is cloned into restriction sites of a pTRIP plasmid harboring a determined promoter, such pTRIP plasmid providing the transfer vector for the preparation of the genome of the vector particles. This plasmid is hence used for the production of the lentiviral particles when it is co-transfected with other plasm ids expressing on the one hand the packaging construct for the particles and on the other hand the envelope of the particles. Plasmid pTRIP has been disclosed in the art, especially in the cited patent applications disclosing lentiviral vector preparation. In addition to the choice of the promoter driving the expression of the antigenic polypeptide, the transfer vector may comprise regulatory elements that increase the expression of the antigenic polypeptides. Such regulatory elements may be a WPRE sequence (woodchuck hepatitis virus).

In a particular embodiment of the invention, the codon-optimized sequence is a mammal-codon optimized sequence, in particular a rodent- or a mouse-codon optimized sequence.

As disclosed herein the screening method of the invention may encompass a pre-selection of the candidate antigenic polypeptides to be assayed for their capacity to elicit a protective immune response. Such pre-selection step may include a step of transcriptome and proteome profiling of the *Plasmodium* pathogen, or a step of comparative transcriptome and proteome profiling at different stages of *Plasmodium* pathogen development if this appears to be relevant.

In particular when the malaria parasite is concerned, a step of transcriptome and proteome profiling of the pre-erythrocytic stages of development is performed and compared with the transcriptome and proteome profiling of the parasite in another stage of development, in particular in the blood-stage of development of said parasite, to identify overexpressed transcripts in the pre-erythrocytic stages of the parasite. Overexpressed transcripts of the pre-erythrocytic stages are considered to be preferred candidates for screening with a view to identify vaccine candidates using antigenic polypeptides harboring ability to elicit a protective immune response in a host.

When an antigenic polypeptide of a determined *Plasmodium* species is identified as a candidate for the screening, the orthologous gene in a *Plasmodium* parasite infecting a human host, in particular *Plasmodium falciparum, Plasmodium vivax* is determined. The detailed disclosure provided in respect of screening of antigens of *Plasmodium* may be transposed to other pathogens. In such a case reporter genes suitable for use with the particular pathogens may have to be selected. The following table illustrates suitable combinations of various pathogens and reporter genes.

| | ***pathogen*** | **reporter gene** | **reference** |
|---|---|---|---|
| **parasites** | *L. major* | luciferase | Mears et al., PLoS pathogens 2015 (i) |
| | *L. amazonensis* | luciferase | Mears et al., PLoS pathogens 2015 |
| | *L. major* | eGFP | Mears et al., PLoS pathogens 2015 |
| | *L. panamensis* | eGFP | Mears et al., PLoS pathogens 2015 |
| | *L. major* | mCherry | Mears et al., PLoS pathogens 2015 |
| | *L. major* | fusion eGFP-luciferase | Mears et al., PLoS pathogens 2015 |
| | *Entomeaba histolytica* | luciferase | Andreu et al., FEMS Microbiol Rev. 2011 (ii) |
| | *Schistosoma mansoni* | wt pathogen* | Krautz-Peterson et al., FASEB J 2009 (iii) |
| | *Toxoplasma gondii* | luciferase | Andreu et al., FEMS Microbiol Rev. 2011 |
| | *Trypanosoma brucei* | luciferase | Andreu et al., FEMS Microbiol Rev. 2011 |
| | *Trypanosoma cruzi* | luciferase | Andreu et al., FEMS Microbiol Rev. 2011 |
| **viruses** | *influenza virus* | luciferase | Tran et al., JVI 2013 (iv) |
| | *influenza virus* | 'Color-flu' viruses** | Fukuyama et al, Nature Com 2015 (v) |
| | *Herpes simplex virus Type I* | luciferase | Andreu et al., FEMS Microbiol Rev. 2011 |
| | *Varicella zoster virus* | luciferase | Andreu et al., FEMS Microbiol Rev. 2011 |
| **bacteria** | *Bacillus anthracis* | luciferase | Andreu et al., FEMS Microbiol Rev. 2011 |
| | *Brucella melitensis* | luciferase | Andreu et al., FEMS Microbiol Rev. 2011 |
| | *Escherichia coli* | luciferase | Andreu et al., FEMS Microbiol Rev. 2011 |
| | *Haemophilus influenza* | luciferase | Andreu et al., FEMS Microbiol Rev. 2011 |
| | *Listeria monocytogenes* | luciferase | Andreu et al., FEMS Microbiol Rev. 2011 |
| | *Mycobacterium tuberculosis* | luciferase | Andreu et al., FEMS Microbiol Rev. 2011 |
| | *Mycobacterium tuberculosis* | wt pathogen*** | Kong et al., PNAS 2010 (vi) |
| | *Pseudomonas aeruginosa* | luciferase | Andreu et al., FEMS Microbiol Rev. 2011 |
| | *Salmonella enterica Typhimurium* | luciferase | Andreu et al., FEMS Microbiol Rev. 2011 |
| | *Staphylococcus aureus* | luciferase | Andreu et al., FEMS Microbiol Rev. 2011 |
| | *Streptococcus pneumonia* | luciferase | Andreu et al., FEMS Microbiol Rev. 2011 |
| | *Yersinia enterocolitica* | luciferase | Andreu et al., FEMS Microbiol Rev. 2011 |
| **fungi** | *Aspergillus fumigatus* | luciferase | Andreu et al., FEMS Microbiol Rev. 2011 |
| | *Candida albicans* | luciferase | Andreu et al., FEMS Microbiol Rev. 2011 |

| | | | |
|---|---|---|---|
| *The pathogen is not recombinant for a reporter gene, NIR imaging agent ProSense 680 and fluorescence molecular tomography (FMT) imaging are used. The near-infrared imaging agent ProSense 680 was injected into the vasculature of mice that were exposed to infectious schistosome larvae (cercariae). The feeding schistosomes ingest the intravenous injected imaging agent as part of their blood meal and their abundant intestinal cathepsins cleave the compound to release measurable fluorochrome in the parasite's gut. ** several fluorescent proteins of different colours ***The pathogen is not recombinant for a reporter gene, a near-infrared fluorogenic substrate which is eukaryotic cell permeable and activated by β-lactamase-producing bacteria is used. (i) http://journals.plos.org/plosntds/article?id=10.1371/journaj.pntd.0003889 (ii) https://www.ncbi.nlm.nih.gov/pmc/articles/PMC3084502/#b183 (iii) https://www.ncbi.nlm.nih.gov/pmc/articles/PMC2717771/ (iv) https://www.ncbi.nlm.nih.gov/pmc/articles/PMC3838222/ (v) http://www.nature.com/articles/ncomms7600 (vi) https://www.ncbi.nlm.nih.gov/pmc/articles/PMC2901431/ | | | |

### Detailed description of the lentiviral vectors for use according to the invention

In order to carry out the method of screening of the invention lentiviral vector are used which are lentiviral particles (i.e. vector particles), and which may be replication-incompetent lentiviral vectors, especially replication-incompetent HIV-1 based vectors characterized in that (i) they are pseudotyped with a determined heterologous viral envelope protein or viral envelope proteins originating from a RNA virus which is not HIV and (ii) they comprise in their genome at least one recombinant polynucleotide encoding at least one antigenic polypeptide (or polypeptide derivative thereof) carrying epitope(s) of an antigen of a specific group or stage of development of a *Plasmodium* parasite, in particular antigen which is a pre-erythrocytic stage antigen or a polypeptidic derivative thereof of a *Plasmodium* parasite capable of infecting a determined non-human mammalian host used for screening, and wherein said epitope(s) encompass(es) T-epitope(s).

In a particular embodiment of the invention, the encoded antigenic polypeptide, in particular of a pre-erythrocytic stage antigen of a *Plasmodium* parasite further comprises B-epitope(s).

According to the disclosure, the lentiviral vectors are either designed to express proficient (i.e., integrative-competent) or deficient (i.e., integrative-incompetent) particles.

The preparation of the lentiviral vectors is well known from the skilled person and has been extensively disclosed in the literature (confer for review Sakuma T. et al (Biochem. J. (2012) 443, 603-618). The preparation of such vectors is also illustrated herein in the Examples.

In a particular embodiment of the invention, the polynucleotide(s) encoding the antigenic polypeptides of the lentiviral vector has(have) been mammal-codon optimized (CO). Optionally the lentiviral sequences of the genome of said particles have also a mammal-codon optimized nucleotide sequence. In a particular aspect of the invention the codon optimization has been carried out for expression in mouse cells when the screening method is performed in mice.

It has been observed that codon optimized nucleotide sequences, especially when optimized for expression in mammalian cells, enable the production of higher yield of particles in such mammalian cells. Production cells are illustrated in the examples. Accordingly, when lentiviral vector particles of the invention are administered to a mammalian, higher amounts of particles are produced in said host which favour the elicitation of a strong immune response.

The lentiviral vector (or lentiviral vectors particles or lentiviral-based vector particles) defined in the present invention are pseudotyped lentiviral vectors consisting of vector particles bearing envelope protein or envelope proteins which originate from a virus different from the particular lentivirus (especially a virus different from HIV, in particular HIV-1), which provides the vector genome of the lentiviral vector particles. Accordingly, said envelope protein or envelope proteins, are "heterologous" viral envelope protein or viral envelope proteins with respect to the vector genome of the particles. In the following pages, reference will also be made to "envelope protein(s)" to encompass any type of envelope protein or envelope proteins suitable to perform the invention. When reference is made to "lentiviral" vectors (lentiviral-based vectors) in the application, it relates in particular, to HIV-based vectors and especially HIV-1-based vectors.

The lentiviral vectors suitable to perform the invention are so-called replacement vectors, meaning that the sequences of the original lentivirus encoding the lentiviral proteins are essentially deleted in the genome of the vector or, when present, are modified, and especially mutated, especially truncated, to prevent expression of biologically active lentiviral proteins, in particular, in the case of HIV, to prevent the expression by said transfer vector, of functional ENV, GAG, and POL proteins and optionally of further structural and/or accessory and/or regulatory proteins of the lentivirus, especially of HIV.The lentiviral vector may be a first-generation vector, in particular a first-generation of a HIV-based vector which is characterized in that it is obtained using separate plasmids to provide (i) the packaging construct, (ii) the envelope and (iii) the transfer vector genome. Alternatively it may be a second-generation vector, in particular a second-generation of a HIV-based vector which in addition, is devoid of viral accessory proteins (such as in the case of HIV-1, Vif, Vpu, Vpr or Nef) and therefore includes only four out of nine HIV full genes: *gag, pol, tat* and *rev.* The vector may be a third-generation vector, in particular a third-generation of a HIV-based vector which is furthermore devoid of said viral accessory proteins and also is Tat-independent; these third-generation vectors may be obtained using 4 plasmids to provide the functional elements of the vector, including one plasmid encoding the Rev protein of HIV when the vector is based on HIV-1. Such vector system comprises only three of the nine genes of HIV-1. The structure and design of such generations of HIV-based vectors is well known in the art.

The **"vector genome"** of the vector particles is a recombinant nucleic acid which also comprises the polynucleotide or transgene of interest encoding the antigenic polypeptide(s) of malaria parasite which are candidates for the screening. The lentiviral-based sequence and polynucleotide/transgene of the vector genome are borne by a plasmid vector thus giving rise to the "transfer vector" also referred to as "sequence vector". Accordingly, these expressions are used interchangeably in the present application.

The vector genome as defined herein accordingly contains, apart from the so-called recombinant polynucleotide placed under control of proper regulatory sequences for its expression, the sequences of the original lentiviral genome which are non-coding regions of said genome, and are necessary to provide recognition signals for DNA or RNA synthesis and processing (mini-viral genome). These sequences are cis-acting sequences necessary for packaging (ψ), reverse transcription (LTRs possibly mutated with respect to the original ones) and transcription and optionally integration (RRE) and furthermore for the particular purpose of the invention, they contain a functional sequence favouring nuclear import in cells and accordingly transgene transfer efficiency in said cells, which element is described as a DNA Flap element that contains or consists of the so-called central cPPT-CTS nucleotidic domain present in lentiviral genome sequences especially in HIV-1 or in some retroelements such as those of yeasts.

The structure and composition of the vector genome used to prepare the lentiviral vectors of the invention are based on the principles described in the art and on examples of such lentiviral vectors primarily disclosed in (Zennou et al, 2000; Firat H. et al, 2002; VandenDriessche T. et al). Constructs of this type have been deposited at the CNCM (Institut Pasteur, France) as will be referred to herein. In this respect reference is also made to the disclosure, including to the deposited biological material, in patent applications WO 99/55892, WO 01/27300 and WO 01/27304.

According to a particular embodiment of the invention, a vector genome may be a replacement vector in which all the viral protein coding sequences between the 2 long terminal repeats (LTRs) have been replaced by the recombinant polynucleotide encoding the polypeptide of the malaria parasite, and wherein the DNA-Flap element has been re-inserted in association with the required cis-acting sequences described herein. Further features relating to the composition of the vector genome are disclosed in relation to the preparation of the particles.

In a particular embodiment of the invention one lentiviral vector encodes one antigenic polypeptide of the *Plasmodium* parasite.

In a particular embodiment, a lentiviral vector of the invention may comprise in its genome one or more than one recombinant polynucleotide encoding at least one antigenic polypeptide carrying epitope(s) of determined group of antigens, such as antigens of a determined development stage of the parasite, in particular a pre-erythrocytic stage antigen, as disclosed herein. In particular, said vector genome comprises two polynucleotides which are consecutive or separated on the genome and which encode different polypeptides of either the same or distinct antigens of the determined group, in particular of the pre-erythrocytic stage of a *Plasmodium* parasite or different antigenic polypeptides of distinct antigens of different forms of the malaria parasite, especially antigens of the pre-erythrocytic stage and antigens of the erythrocytic stage of the parasite.

In a particular embodiment, the vector genome contains two or more recombinant polynucleotides, each of them encoding a distinct antigenic polypeptide and each polypeptide originating from a different antigen of a determined group, in particular of a determined development stage, including the pre-erythrocytic stage, of the malaria parasite.

The description made herein in respect to antigenic polypeptides similarly applies to polypeptidic derivatives thereof.

Particular features of the lentiviral vectors used in accordance with the various embodiments of the invention are also disclosed in the Examples, such features being either taken alone or in combination to produce the vectors.

According to the invention, the lentiviral vector particles are pseudotyped with a heterologous viral envelope protein or viral polyprotein of envelope originating from a RNA virus which is not the lentivirus providing the lentiviral sequences of the genome of the lentiviral particles.

As examples of typing envelope proteins for the preparation of the lentiviral vector, the invention relates to viral transmembrane glycosylated (so-called G proteins) envelope protein(s) of a Vesicular Stomatitis Virus (VSV), which is(are) for example chosen in the group of VSV-G protein(s) of the Indiana strain and VSV-G protein(s) of the New Jersey strain.

The envelope glycoprotein of the vesicular stomatitis virus (VSV-G) is a transmembrane protein that functions as the surface coat of the wild type viral particles. It is also a suitable coat protein for engineered lentiviral vectors. Presently, nine virus species are definitively classified in the VSV gender, and nineteen rhabdoviruses are provisionally classified in this gender, all showing various degrees of cross-neutralisation. When sequenced, the protein G genes indicate sequence similarities. The VSV-G protein presents a N-terminal ectodomain, a transmembrane region and a C-terminal cytoplasmic tail. It is exported to the cell surface *via* the transGolgi network (endoplasmic reticulum and Golgi apparatus).

Vesicular stomatitis Indiana virus (VSIV) and Vesicular stomatitis New Jersey virus (VSNJV) are preferred strains to pseudotype the lentiviral vectors of the invention, or to design recombinant envelope protein(s) to pseudotype the lentiviral vectors. Their VSV-G proteins are disclosed in GenBank, where several strains are presented. For VSV-G New Jersey strain reference is especially made to the sequence having accession number V01214. For VSV-G of the Indiana strain, reference is made to the sequence having accession number AAA48370.1 in Genbank corresponding to strain JO2428.

Said viral envelope protein(s) are capable of uptake by antigen presenting cells and especially by dendritic cells including by liver dendritic cells by mean of fusion and/or of endocytosis. In a particular embodiment, the efficiency of the uptake may be used as a feature to choose the envelope of a VSV for pseudotyping. In this respect the relative titer of transduction (Titer DC/Titer of other transduced cells e.g. 293T cells) may be considered as a test and envelope having a relative good ability to fuse with DC would be preferred.

Antigen Presenting Cells (APC) and especially Dentritic cells (DC) are proper target cells for pseudotyped lentiviral vectors which are used as immune compositions accordingly.

The VSV-G envelope protein(s) are expressed from a polynucleotide containing the coding sequence for said protein(s), which polynucleotide is inserted in a plasmid (designated envelope expression plasmid or pseudotyping env plasmid) used for the preparation of the lentiviral vector particles of the invention. The polynucleotide encoding the envelope protein(s) is under the control of regulatory sequences for the transcription and/or expression of the coding sequence (including optionally post-transcriptional regulatory elements (PRE), in particular such elements that may improve the antigen expression from the vector, especially a polynucleotide such as the element of the Woodchuck hepatitis virus, i.e. the WPRE sequence, obtainable from Invitrogen).

Accordingly, a nucleic acid construct is provided which comprises an internal promoter suitable for the use in mammalian cells, especially in human cells *in vivo* and the nucleic acid encoding the envelope protein under the control of said promoter. A plasmid containing this construct is used for transfection or for transduction of cells suitable for the preparation of vector particles. Promoters may in particular be selected for their properties as constitutive promoters, tissue-specific promoters, or inducible promoters. Examples of suitable promoters encompass the promoters of the following genes: MHC class1 promoter, human beta-2 microglobulin gene (β2M promoter), EF1α, human PGK, PPI (preproinsulin), thiodextrin, HLA DR invariant chain (P33), HLA DR alpha chain, Ferritin L chain or Ferritin H chain, Chymosin beta 4, Chymosin beta 10, Cystatin Ribosomal Protein L41, CMVie or chimeric promoters such as GAG(CMV early enhancer / chicken β actin) disclosed in Jones S. et al (Jones S. et al Human Gene Therapy, 20:630-640(June 2009)).

These promoters may also be used in regulatory expression sequences involved in the expression of *gag-pol* derived proteins from the encapsidation plasm ids, and/or to express the antigenic polypeptides from the transfer vector.

Alternatively, when the envelope expression plasmid is intended for expression in stable packaging cell lines, especially for stable expression as continuously expressed viral particles, the internal promoter to express the envelope protein(s) is advantageously an inducible promoter such as one disclosed in Cockrell A.S. et al. (Mol. Biotechnol. (2007) 36:184-204). As examples of such promoters, reference is made to tetracycline and ecdysone inducible promoters. The packaging cell line may be the STAR packaging cell line (ref Cockrell A.S. et al (2007), Ikedia Y. et al (2003) Nature Biotechnol. 21: 569-572) or a SODk packaging cell line, such as SODk0 derived cell lines, including SODk1 and SODk3 (ref Cockrell A.S. et al (2007), Cockrell A;S.et al (2006) Molecular Therapy, 14: 276-284, Xu K. et al. (2001) ,Kafri T. et al (1999) Journal of Virol. 73:576-584).

According to the invention, the lentiviral vector are the product recovered from co-transfection of mammalian cells, with:
- a vector plasmid comprising (i) lentiviral, especially HIV-1, cis-active sequences necessary for packaging, reverse transcription, and transcription and further comprising a functional lentiviral, especially derived from HIV-1, DNA flap element and (ii) a polynucleotide encoding an antigenic polypeptide of a malaria parasite as disclosed herein under the control of regulatory expression sequences, and optionally comprising sequences for integration into the genome of the host cell;
- an expression plasmid encoding a pseudotyping envelope derived from a RNA virus, said expression plasmid comprising a polynucleotide encoding an envelope protein or proteins for pseudotyping, wherein said envelope pseudotyping protein is advantageously from a VSV and is in particular a VSV-G of the Indianan strain or of the New Jersey strain and,
- an encapsidation plasmid, which either comprises lentiviral, especially HIV-1, *gag-pol* packaging sequences suitable for the production of integration-competent vector particles or modified *gag-pol* packaging sequences suitable for the production of integration-deficient vector particles.

The invention thus also concerns lentiviral vector particles as described above, which are the product recovered from a stable cell line transfected with:
- a vector plasmid comprising (i) lentiviral, especially HIV-1, cis-active sequences necessary for packaging, reverse transcription, and transcription and further comprising a functional lentiviral, especially HIV-1, DNA flap element and optionally comprising cis-active sequences necessary for integration, said vector plasmid further comprising (ii) a polynucleotide of a codon-optimized sequence for a determined non-human mammal of the gene encoding the antigenic polypeptide of a *Plasmodium* parasite, under the control of regulatory expression sequences, especially a promoter;
- a VSV-G envelope expression plasmid comprising a polynucleotide encoding a VSV-G envelope protein in particular VSV-G of the Indiana strain or of the New Jersey strain, wherein said polynucleotide is under the control of regulating expression sequences, in particular regulatory expression sequences comprising an inducible promoter, and;
- an encapsidation plasmid, wherein the encapsidation plasmid either comprises lentiviral, especially HIV-1, *gag-pol* coding sequences suitable for the production of integration-competent vector particles or modified *gag-pol* coding sequences suitable for the production of integration-deficient vector particles, wherein said *gag-pol* sequences are from the same lentivirus sub-family as the DNA flap element, wherein said lentiviral *gag-pol* or modified *gag-pol* sequence is under the control of regulating expression sequences.

The stable cell lines expressing the vector particles of the invention are in particular obtained by transduction of the plasmids.

The polynucleotide encodes at least one antigenic polypeptide of a malaria parasite according to any embodiment disclosed in the present specification. In particular, it encodes a polypeptide which is a truncated mammalian, especially human, codon-optimized sequence coding for such antigenic polypeptide of *Plasmodium falciparum, Plasmodium vivax, Plasmodium malariae, Plasmodium ovale, Plasmodium knowlesi or Plasmodium berghei.*

In a particular embodiment, the polynucleotide encodes two or more antigenic polypeptides of the malaria parasite which originate and/or are derived from distinct antigens of said parasite as disclosed in the various embodiments. Accordingly, the vector plasmid may comprise several expression cassettes for the expression of the various antigenic polypeptides or may comprise bicistronic or multicistronic expression cassettes where the polynucleotides encoding the various polypeptides are separated by an IRES sequence of viral origin (Internal Ribosome Entry Site), or it may encode fusion protein(s).

The internal promoter contained the vector genome and controlling the expression of the polynucleotide encoding an antigenic polypeptide of the malaria parasite (as a transgene or in an expression cassette) may be selected from the promoters of the following genes: human beta-2 microglobulin gene (β2M promoter), EF1α, human PGK, PPI (preproinsulin), thiodextrin, HLA DR invariant chain (P33), HLA DR alpha chain, Ferritin L chain or Ferritin H chain, Chymosin beta 4, Chymosin beta 10, or Cystatin Ribosomal Protein L41 CMVie or chimeric promoters such as GAG(CMV early enhancer / chicken β actin) disclosed in Jones S. et al (2009).

A promoter among the above cited internal promoters may also be selected for the expression of the envelope protein(s) and packaging (*gag-pol* derived) proteins.

Alternatively, vector particles can be produced from co-transfection of the plasmids disclosed herein, in stable packaging cell lines which thus become capable of continuously secreting vector particles. Promoters used in the regulatory expression sequences involved for the expression of the envelope protein(s) are advantageously inducible promoters.

The following particular embodiments may be carried out when preparing the lentiviral vector based on human lentivirus, and especially based on HIV-1 virus.

According to the invention, the genome of the lentiviral vector is derived from a human lentivirus, especially from the HIV lentivirus. In particular, the pseudotyped lentiviral vector is an HIV-based vector, such as an HIV-1, or HIV-2 based vector, in particular is derived from HIV-1M, for example from the BRU or LAI isolates. Alternatively, the lentiviral vector providing the necessary sequences for the vector genome may be originating from lentiviruses such as EIAV, CAEV, VISNA, FIV, BIV, SIV, HIV-2, HIV-O which are capable of transducing mammalian cells.

As stated above, when considering it apart from the recombinant polynucleotide that it finally contains, the vector genome is a replacement vector in which the nucleic acid between the 2 long terminal repeats (LTRs) in the original lentivirus genome have been restricted to cis-acting sequences for DNA or RNA synthesis and processing, including for the efficient delivery of the transgene to the nuclear of cells in the host, or at least are deleted or mutated for essential nucleic acid segments that would enable the expression of lentiviral structure proteins including biological functional GAG polyprotein and possibly POL and ENV proteins.

In a particular embodiment, the 5' LTR and 3' LTR sequences of the lentivirus are used in the vector genome, but the 3'-LTR at least is modified with respect to the 3'LTR of the original lentivirus at least in the U3 region which for example can be deleted or partially deleted for the enhancer. The 5'LTR may also be modified, especially in its promoter region where for example a Tat-independent promoter may be substituted for the U3 endogenous promoter.

In a particular embodiment the vector genome comprises one or several of the coding sequences for Vif-, Vpr, Vpu- and Nef-accessory genes (for HIV-1 lentiviral vectors).

Alternatively, these sequences can be deleted independently or each other or can be non-functional (second-generation lentiviral vector).

The vector genome of the lentiviral vector particles comprises, as an inserted cis-acting fragment, at least one polynucleotide consisting in the DNA flap element or containing such DNA flap element. Especially, the DNA flap is inserted upstream of the polynucleotide encoding the antigenic polypeptide of *Plasmodium* parasite, and is advantageously - although not necessarily - located in an approximate central position in the vector genome. A DNA flap suitable for the invention may be obtained from a lentivirus, in particular a human lentivirus especially a HIV-1 retrovirus, or from a retrovirus-like organism such as retrotransposon. It may be alternatively obtained from the CAEV (Caprine Arthritis Encephalitis Virus) virus, the EIAV (Equine Infectious Anaemia Virus) virus, the VISNA virus, the SIV (Simian Immunodeficiency Virus) virus or the FIV (Feline Immunodeficiency Virus) virus. The DNA flap may be either prepared synthetically (chemical synthesis) or by amplification of the DNA providing the DNA Flap from the appropriate source as defined above such as by Polymerase chain reaction (PCR). In a more preferred embodiment, the DNA flap is obtained from an HIV retrovirus, for example HIV-1 or HIV-2 virus including any isolate of these two types.

The DNA flap (also designated cPPT/CTS) (defined in Zennou V. et al. ref 27, 2000, Cell vol 101, 173-185 or in WO 99/55892 and WO 01/27304), is a structure which is central in the genome of some lentiviruses especially in HIV, where it gives rise to a 3-stranded DNA structure normally synthesized during especially HIV reverse transcription and which acts as a cis-determinant of HIV genome nuclear import. The DNA flap enables a central strand displacement event controlled in *cis* by the central polypurine tract (cPPT) and the central termination sequence (CTS) during reverse transcription. When inserted in lentiviral-derived vectors, the polynucleotide enabling the DNA flap to be produced during reverse-transcription, stimulates gene transfer efficiency and complements the level of nuclear import to wild-type levels (Zennou et al., Cell, 2000).

Sequences of DNA flaps have been disclosed in the prior art, especially in the above cited patent applications. These sequences are also disclosed in the sequence of SEQ ID No.1 from position 2056 to position 2179. They are preferably inserted as a fragment, optionally with additional flanking sequences, in the vector genome, in a position which is preferably near the centre of said vector genome. Alternatively they may be inserted immediately upstream from the promoter controlling the expression of the polynucleotide(s) encoding the antigenic polypeptide. Said fragments comprising the DNA flap, inserted in the vector genome may have a sequence of about 80 to about 200 bp, depending on its origin and preparation.

According to a particular embodiment, a DNA flap has a nucleotide sequence of about 90 to about 140 nucleotides.

In HIV-1, the DNA flap is a stable 99-nucleotide-long plus strand overlap. When used in the genome vector of the lentiviral vector of the invention, it may be inserted as a longer sequence, especially when it is prepared as a PCR fragment. A particular appropriate polynucleotide comprising the structure providing the DNA flap is a 124-base pair polymerase chain reaction (PCR) fragment encompassing the cPPT and CTS regions of the HIV-1 DNA (as disclosed in SEQ ID No.1)

It is specified that the DNA flap used in the genome vector and the polynucleotides of the encapsidation plasmid encoding the GAG and POL polyproteins should originate from the same lentivirus sub-family or from the same retrovirus-like organism.

Preferably, the other cis-activating sequences of the genome vector also originate from the same lentivirus or retrovirus-like organism, as the one providing the DNA flap. The vector genome may further comprise one or several unique restriction site(s) for cloning the recombinant polynucleotide.

In a preferred embodiment, in said vector genome, the 3' LTR sequence of the lentiviral vector genome is devoid of at least the activator (enhancer) and possibly the promoter of the U3 region. In another particular embodiment, the 3' LTR region is devoid of the U3 region (delta U3). In this respect, reference is made to the description in WO 01/27300 and WO 01/27304.

In a particular embodiment, in the vector genome, the U3 region of the LTR 5' is replaced by a non lentiviral U3 or by a promoter suitable to drive tat-independent primary transcription. In such a case, the vector is independent of *tat* transactivator (third generation vector).

The vector genome also comprises the psi (ψ) packaging signal. The packaging signal is derived from the N-terminal fragment of the *gag* ORF. In a particular embodiment, its sequence could be modified by frameshift mutation(s) in order to prevent any interference of a possible transcription/translation of gag peptide, with that of the transgene.

The vector genome may optionally also comprise elements selected among a splice donor site (SD), a splice acceptor site (SA) and/or a Rev-responsive element (RRE). According to a particular embodiment, the vector plasmid (or added genome vector) comprises the following cis-acting sequences for a transgenic expression cassette:
1. The LTR sequence (Long-Terminal Repeat), required for reverse transcription, the sequences required for transcription and including optionally sequences for viral DNA integration. The 3' LTR is deleted in the U3 region at least for the promoter to provide SIN vectors (Self-inactivating), without perturbing the functions necessary for gene transfer, for two major reasons: first, to avoid trans-activation of a host gene, once the DNA is integrated in the genome and secondly to allow self-inactivation of the viral *cis*-sequences after retrotranscription. Optionally, the tat-dependent U3 sequence from the 5'-LTR which drives transcription of the genome is replaced by a non endogenous promoter sequence. Thus, in target cells only sequences from the internal promoter will be transcribed (transgene).
2. The ψ region, necessary for viral RNA encapsidation.
3. The RRE sequence (REV Responsive Element) allowing export of viral messenger RNA from the nucleus to the cytosol after binding of the *Rev* protein.
4. The DNA flap element (cPPT/CTS) to facilitate nuclear import.
5. Optionally post-transcriptional elements such as the WPRE *cis*-active sequence (Woodchuck hepatitis B virus Post-Responsive Element) also added to optimize stability of mRNA (Zufferey et al., 1999), the matrix or scaffold attachment regions (SAR and MAR sequences) such as those of the immunoglobulin-kappa gene (Park F. et al Mol Ther 2001; 4: 164-173).

The lentiviral vector suitable for use in the invention is non replicative (replication-incompetent) i.e., the vector and lentiviral vector genome are regarded as suitable to alleviate concerns regarding replication competent lentiviruses and especially are not able to form new particles budding from the infected host cell after administration. This may be achieved in well known ways as the result of the absence in the lentiviral genome of the *gag, pol* or *env* genes, or their absence as "functional genes". The *gag* and *pol* genes are thus, only provided in *trans.* This can also be achieved by deleting other viral coding sequence(s) and/or cis-acting genetic elements needed for particles formation.

By ***"functional"*** it is meant a gene that is correctly transcribed, and/or correctly expressed. Thus, if present in the lentiviral vector genome of the invention in this embodiment contains sequences of the *gag, pol,* or *env* are individually either not transcribed or incompletely transcribed; the expression *"incompletely transcribed"* refers to the alteration in the transcripts gag, gag-pro or gag-pro-pol, one of these or several of these being not transcribed. Other sequences involved in lentiviral replication may also be mutated in the vector genome, in order to achieve this status. The absence of replication of the lentiviral vector should be distinguished from the replication of the lentiviral genome. Indeed, as described before, the lentiviral genome may contain an origin of replication ensuring the replication of the lentiviral vector genome without ensuring necessarily the replication of the vector particles.

In order to obtain lentiviral vectors according to the invention, the vector genome (as a vector plasmid) must be encapsidated in particles or pseudo-particles. Accordingly, lentiviral proteins, except the envelope proteins, have to be provided *in trans* to the vector genome in the producing system, especially in producing cells, together with the vector genome, having recourse to at least one encapsidation plasmid carrying the *gag* gene and either the *pol* lentiviral gene or an integrative-incompetent *pol* gene, and preferably lacking some or all of the coding sequences for *Vif-, Vpr, Vpu-* and *Nef-*accessory genes and optionally lacking *Tat* (for HIV-1 lentiviral vectors).

A further plasmid is used, which carries a polynucleotide encoding the envelope pseudotyping protein(s) selected for pseudotyping lentiviral vector particles.

In a preferred embodiment, the packaging plasmid encodes only the lentiviral proteins essential for viral particle synthesis. Accessory genes whose presence in the plasmid could raise safety concerns are accordingly removed. Accordingly, viral proteins brought in *trans* for packaging are respectively as illustrated for those originating from HIV-1:
1. GAG proteins for building of the matrix (MA, with apparent Molecular Weight p17), the capsid (CA, p24) and nucleocapsid (NC, p6).
2. POL encoded enzymes: integrase, protease and reverse transcriptase.
3. TAT and REV regulatory proteins, when TAT is necessary for the initiation of LTR-mediated transcription; TAT expression may be omitted if the U3 region of 5'LTR is substituted for a promoter driving tat-independent transcription. REV may be modified and accordingly used for example in a recombinant protein which would enable recognition of a domain replacing the RRE sequence in the vector genome, or used as a fragment enabling binding to the RRE sequence through its RBD (RNA Binding Domain).

In order to avoid any packaging of the mRNA generated from the genes contained in the packaging plasmid in the viral particles, the ψ region is removed from the packaging plasmid. A heterologous promoter is inserted in the plasmid to avoid recombination issues and a poly-A tail is added 3' from the sequences encoding the proteins. Appropriate promoters have been disclosed above.

The envelope plasmid encodes the envelope protein(s) for pseudotyping which are disclosed herein, under the control of an internal promoter, as disclosed herein.

Any or all the described plasmids for the preparation of the lentiviral vector particles suitable for use in the invention may be codon optimized (CO) in the segment encoding proteins. Codon optimization according to the invention is preferably performed to improve translation of the coding sequences contained in the plasmids, in mammalian cells, murine or especially human cells. According to the invention, codon optimization is especially suited to directly or indirectly improve the preparation of the vector particles or to improve their uptake by the cells of the host to whom they are administered, or to improve the efficiency of the transfer of the polynucleotide encoding the antigenic polypeptide of the malaria parasite (transgene) in the genome of the transduced cells of the host. Methods for optimizing codons are well known in the art and codon optimization is especially performed using available programs to that effect. Codon optimization is illustrated for the coding sequences used in the examples.

In a particular aspect of the disclosure, the pseudotyped lentiviral vector is also, or alternatively, integrative-incompetent. In such a case, the vector genome and thus the recombinant polynucleotide which it contains do not integrate into the genome of the transduced cells or in the cells of the host to whom it has been administered.

The present disclosure relates to the use of a lentiviral vector wherein the expressed integrase protein is defective and which further comprises a polynucleotide especially encoding at least one antigenic polypeptide carrying epitope(s) of a pre-erythrocytic stage antigen of a *Plasmodium* parasite, in an immunogenic composition.

By **"integration-incompetent",** it is meant that the integrase, preferably of lentiviral origin, is devoid of the capacity of integration of the lentiviral genome into the genome of the host cells i.e., an integrase protein mutated to specifically alter its integrase activity.

Integration-incompetent lentiviral vectors are obtained by modifying the *pol* gene encoding the Integrase, resulting in a mutated *pol* gene encoding an integrative deficient integrase, said modified *pol* gene being contained in the encapsidation plasmid. Such integration-incompetent lentiviral vectors have been described in patent application WO 2006/010834. Accordingly the integrase capacity of the protein is altered whereas the correct expression from the encapsidation plasmid of the GAG, PRO and POL proteins and/or the formation of the capsid and hence of the vector particles, as well as other steps of the viral cycle, preceding or subsequent to the integration step, such as the reverse transcription, the nuclear import, stay intact. An integrase is said defective when the integration that it should enable is altered in a way that an integration step takes place less than 1 over 1000, preferably less than 1 over 10000, when compared to a lentiviral vector containing a corresponding wild-type integrase.

In a particular aspect of the disclosure, the defective integrase results from a mutation of class 1, preferably amino acid substitutions (one-amino acid substitution) or short deletions fulfilling the requirements of the expression of a defective integrase. The mutation is carried out within the *pol* gene. These vectors may carry a defective integrase with the mutation D64V in the catalytic domain of the enzyme, which specifically blocks the DNA cleaving and joining reactions of the integration step. The D64V mutation decreases integration of pseudotyped HIV-1 up to 1/10,000 of wild type, but keep their ability to transduce non dividing cells, allowing efficient transgene expression.

Other mutations in the *pol* gene which are suitable to affect the integrase capacity of the integrase of HIV-1 are the following: H12N, H12C, H16C, H16V, S81 R, D41A, K42A, H51A, Q53C, D55V, D64E, D64V, E69A, K71A, E85A, E87A, D116N, D116I, D116A, N120G, N120I, N120E, E152G, E152A, D-35-E, K156E, K156A, E157A, K159E, K159A, K160A, R166A, D167A, E170A, H171A, K173A, K186Q, K186T, K188T, E198A, R199C, R199T, R199A, D202A, K211A, Q214L, Q216L, Q221 L, W235F, W235E, K236S, K236A, K246A, G247W, D253A, R262A, R263A and K264H. In a particular aspect, mutation in the *pol* gene is performed at either of the following positions D64, D116 or E152, or at several of these positions which are in the catalytic site of the protein. Any substitution at these positions is suitable, including those described above.

Another proposed substitution is the replacement of the amino acids residues RRK (positions 262 to 264) by the amino acids residues AAH.

In a particular aspect of the disclosure, when the lentiviral vector is integration-incompetent, the lentiviral genome further comprises an origin of replication (ori), whose sequence is dependent on the nature of cells where the lentiviral genome has to be expressed. Said origin of replication may be from eukaryotic origin, preferably of mammalian origin, most preferably of human origin. It may alternatively be of viral origin, especially coming from DNA circular episomic viruses, such as SV40 or RPS. It is an advantageous embodiment of the invention to have an origin or replication inserted in the lentiviral genome of the lentiviral vector of the invention. Indeed, when the lentiviral genome does not integrate into the cell host genome (because of the defective integrase), the lentiviral genome is lost in cells that undergo frequent cell divisions; this is particularly the case in immune cells, such as B or T cells. The presence of an origin of replication ensures that at least one lentiviral genome is present in each cell, even after cell division, accordingly maximazing the efficiency of the immune response.

The lentiviral vector genome of said lentiviral vectors of the invention may especially be derived from HIV-1 plasmid pTRIPΔU3.CMV-GFP deposited at the CNCM (Paris, France) on October 11, 1999 under number I-2330 (also described in WO01/27300) or variants thereof. The sequence of such variants are provided as SEQ ID NO.1 or 2.

When the vector genome is derived from these particular plasmids, a sequence of a recombinant polynucleotide encoding an antigenic polypeptide of a *Plasmodium parasite* as disclosed in the present application is inserted therein, in addition or in replacement of the GFP coding fragment. The GFP coding sequence may also be substituted by a different marker. The CMV promoter may also be substituted by another promoter, especially one of the promoters disclosed above, especially in relation to the expression of the transgene.

The WPRE sequence also contained in the particular deposited pTRIP vectors may optionally be deleted.

Vector particles may be produced after transfection of appropriate cells (such as mammalian cells or human cells, such as Human Embryonic Kidney cells illustrated by 293 T cells) by said plasmids, or by other processes. In the cells used for the expression of the lentiviral particles, all or some of the plasmids may be used to stably express their coding polynucleotides, or to transiently or semi-stably express their coding polynucleotides.

The concentration of particles produced can be determined by measuring the P24 (capsid protein for HIV-1) content of cell supernatants.

The lentiviral vector of the invention, once administered into the host, infects cells of the host, possibly specific cells, depending on the envelope proteins it was pseudotyped with. The infection leads to the release of the lentiviral vector genome into the cytoplasm of the host cell where the retrotranscription takes place. Once under a triplex form (via the DNA flap), the lentiviral vector genome is imported into the nucleus, where the polynucleotide(s) encoding polypeptide(s) of antigen(s) of the malaria parasite is (are) expressed via the cellular machinery. When non-dividing cells are transduced (such as DC), the expression may be stable. When dividing cells are transduced, such as B cells, the expression is temporary in absence of origin of replication in the lentiviral genome, because of nucleic acid dilution and cell division. The expression may be longer by providing an origin of replication ensuring a proper diffusion of the lentiviral vector genome into daughter cells after cell division. The stability and/or expression may also be increased by insertion of MAR (Matrix Associated Region) or SAR (Scaffold Associated Region) elements in the vector genome.

Indeed, these SAR or MAR regions are AT-rich sequences and enable to anchor the lentiviral genome to the matrix of the cell chromosome, thus regulating the transcription of the polynucleotide encoding at least one antigenic polypeptide, and particularly stimulating gene expression of the transgene and improving chromatin accessibility.

If the lentiviral genome is non integrative, it does not integrate into the host cell genome. Nevertheless, the at least one polypeptide encoded by the transgene is sufficiently expressed and longer enough to be processed, associated with MHC molecules and finally directed towards the cell surface. Depending on the nature of the polynucleotide(s) encoding antigenic polypeptide(s) of a malaria parasite, the at least one polypeptide epitope associated with the MHC molecule triggers a humoral or a cellular immune response.

Unless otherwise stated, or unless technically not relevant, the characteristics disclosed in the present application with respect to any of the various features, embodiments or examples of the structure or use of the lentiviral particles, especially regarding their envelope protein(s), or the recombinant polynucleotide, may be combined according to any possible combinations.

Further features and properties of the present invention, including features to be used in the embodiments described above will be described in the examples and figures which follow and may accordingly be used to characterise the invention.

### LEGENDS OF THE FIGURES

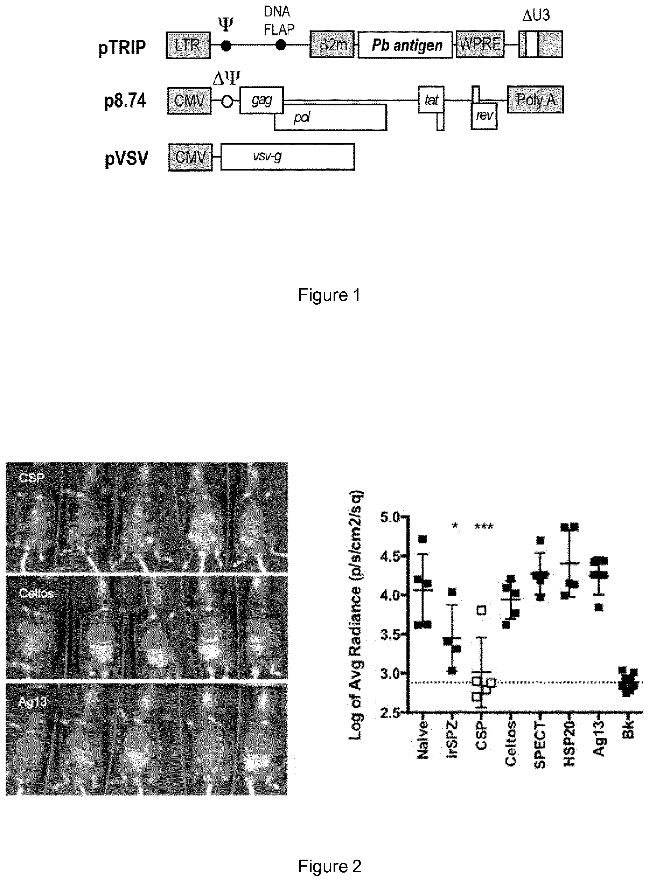

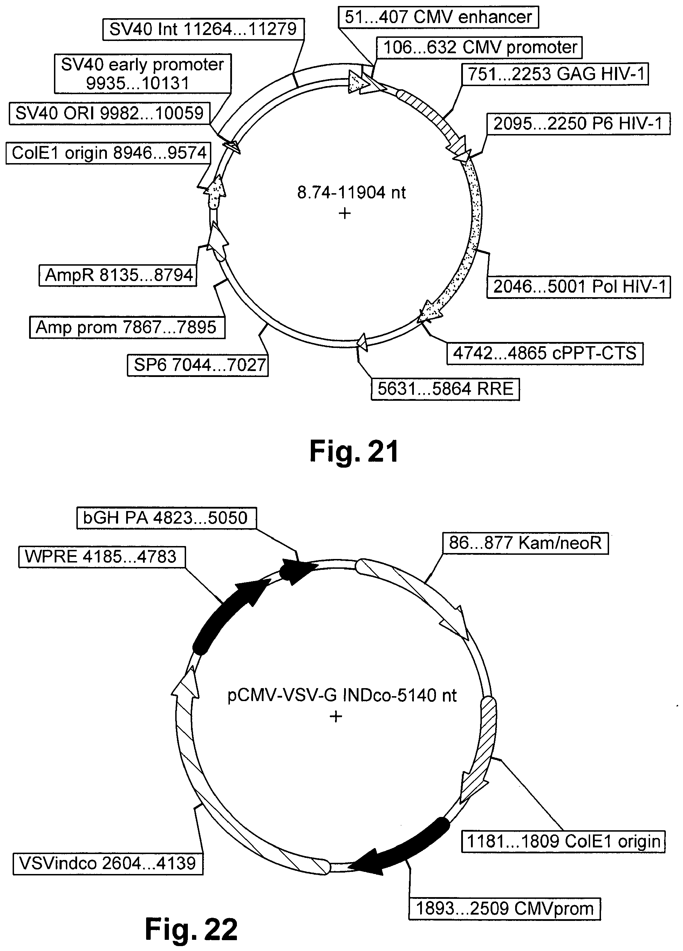

**Figure 1****.** Schema of plasmids used in the production of Lentiviral Particles.
**Figure 2****.** C57BL/6 mice (n=5) were immunized intramuscularly with 5x10⁷ TU of VSV^{IND} pseudotyped lentiviral particles coding for the antigens, CSP, Celtos SPECT, HSP20 and Ag13. As a positive control of protection, mice were immunized with 50k irradiated sporozoites via intravenous injection. Thirty days after immunization, the animals were challenged with 10,000 bioluminescent sporozoites micro-injected subcutaneously in the mice footpad. The parasite load in the liver was quantified two days later by bioluminescence as shown in the picture for CSP, Celtos and Ag13. The graph shows the quantification of the liver infection represented as the log of average radiance (squares). Dotted line represents the average of background signal (Bk) of a non-infected region. *P<0.05 and ***P<0.001 (ANOVA).

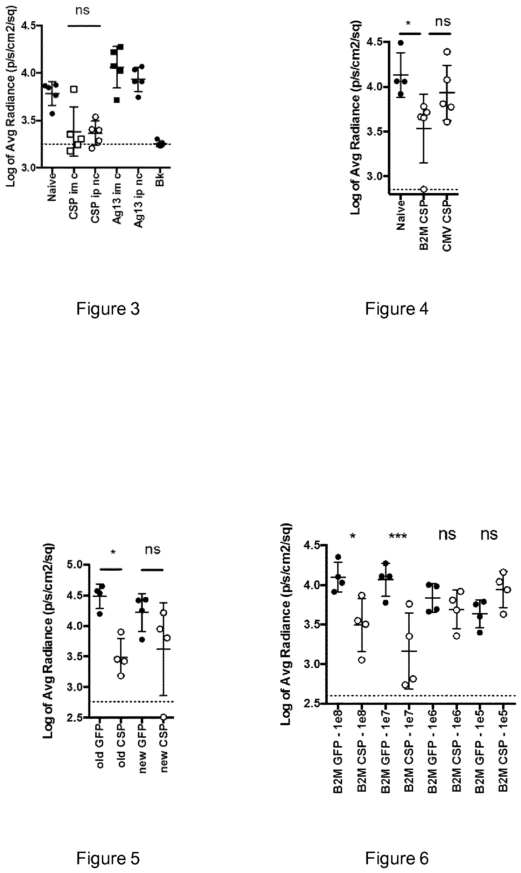

**Figure 3****.** C57BL/6 mice (n=5 per group) were immunized or not (naïve) with 5x10⁷ TU of VSV^{IND} LPs carrying Ag13 (negative control) and CSP (positive control). The groups receiving concentrated LPs were inoculated intramuscularly in the thigh muscle with 50 uL of vector (Ag13 im c and CSP im c). The groups receiving non-concentrated LPs were inoculated intraperitoneally with 700 uL of vector (Ag13 ip nc and CSP ip nc). Thirty days after immunization, the animals were challenged with 5,000 luciferase-expressing sporozoites, micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). ns, not significant (ANOVA).
**Figure 4****.** C57BL/6 mice (n=4-5 per group) were intraperitoneally immunized or not (naïve) with 1x10⁷ TU of non concentrated VSV^{IND} CSP LPs under the control of CMV or B2M promoters (CMV CSP and B2M CSP, respectively). Thirty days after immunization, the animals were challenged with 5,000 luciferase-expressing sporozoites micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). *P<0.05; ns, not significant (ANOVA).
**Figure 5.** 4 and 7 weeks-old C57BL/6 mice (n=4-per group) were acclimated for 3 weeks (old groups) and 3 days (new groups). These age-matched groups were then intraperitoneally immunized with 1x10⁷ TU of non concentrated VSV^{IND} B2M CSP or GFP LPs. Thirty days after immunization, the animals were challenged with 5,000 luciferase-expressing sporozoites micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). *P<0.05; ns, not significant (ANOVA).
**Figure 6.** 4 weeks-old C57BL/6 mice (n=4-per group) were acclimated for 3 weeks (old groups) and intraperitoneally immunized with different doses of non-concentrated VSV^{IND} B2M CSP (black) or GFP (white) LPs. Thirty days after immunization, the animals were challenged with 5,000 luciferase-expressing sporozoites micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). *P<0.05; ***P<0.001; ns, not significant (ANOVA).

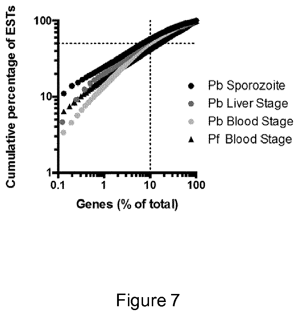

**Figure 7****.** Analysis of Sporozoite, Liver Stage and Blood Stage cDNA libraries of Plasmodium berghei (Pb) and falciparum (Pf) deposited in Plasmodb. The percentage of each expression sequence tag (EST) was normalized to the total number of ESTs and represented cumulatively. Each symbol represents one gene, ranked by EST abundance (higher to lower) and represented as % of total ESTs. Of note ∼10% of genes (most abundant) are responsible for ∼50% of total ESTs (dotted lines).

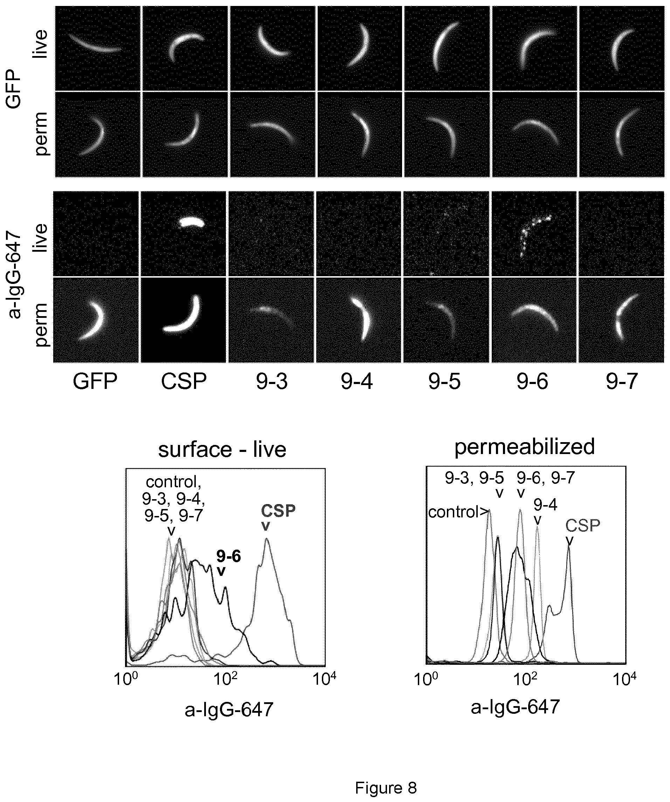

**Figure 8****. Expression and surface localization of antigens.** GFP-expressing Pb sporozoites were fixed with 2% of PFA and permeabilized with 0.1% of Triton X100 (perm) or not (live). Parasites were incubated with the indicated immune-sera (1/50) for one hour on ice, washed and revealed with goat anti-mouse secondary antibody labelled with AlexaFluor 647. Sporozoites were then analysed by cytometry as shown in the right histograms (surface, staining using live non-permeabilized SPZ; permeabilized, staining using fixed and permeabilized SPZ) or by fluorescence microscopy, as depicted in the pictures. Notice that CSP and antigen 9-6 present a surface pattern staining both by cytometry and microscopy.

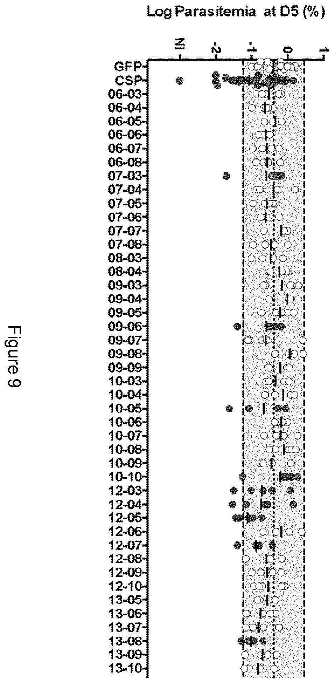

**Figure 9****. Targeted screening of protective antigens.** 4 weeks-old C57BL/6 mice (n=5 per group) were acclimated for 3 weeks and intraperitoneally immunized with a single dose of 1x10⁷ TU of non-concentrated VSV^{IND} B2M LPs. Thirty days after immunization, the animals were challenged with 5,000 GFP-expressing sporozoites micro-injected subcutaneously in the mice footpad. The parasite infection was measured by flow cytometry. The graph shows the average of the log of parasitemia (trace, individual mice represented by circles) immunized with the indicated plasmodial antigens. Bold dotted lines represent the 95% tolerance interval of GFP log normal distribution. Mice with parasitemia below the lower limit of the tolerance interval are considered protected. Top dotted line is the average of control and bottom dotted line represents non-infected (NI) mice.

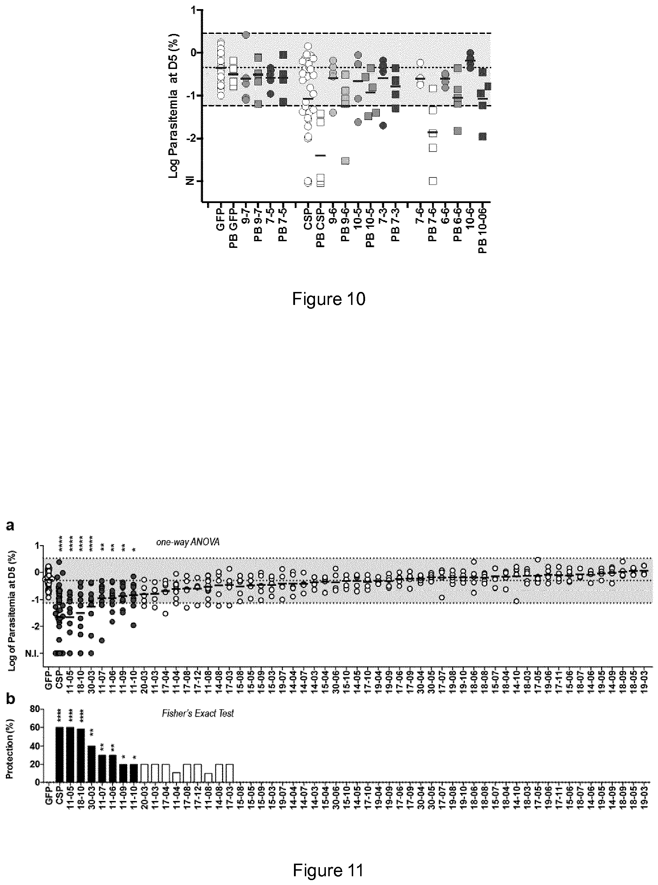

**Figure 10****. Comparison of protection induced by one or two immunization doses.** 4 weeks-old C57BL/6 mice (n=5 per group) were acclimated for 3 weeks and intraperitoneally immunized with a first dose of 5x10⁵ TU of non-concentrated VSV^{NJ} B2M LPs. Thirty days after the first immunization, the animals received a second dose of 1x10⁷ TU of non-concentrated VSV^{IND} B2M LPs. Thirty days later, mice were challenged with 5,000 GFP-expressing sporozoites micro-injected subcutaneously in the footpad. The parasite infection was measured by flow cytometry. The graph shows the log of parasitemia at day 5 post-inoculation of individual challenged mice that received two immunization doses (Squares, PB). Circles represent mice that received only one immunization dose of LPs (data from experiment shown in Fig. 9). Traces represents the average of the Log Parasitemia. Bold dotted lines represent the 95% tolerance interval of GFP log normal distribution. Mice below the lower limit of tolerance interval are considered protected. NI, non-infected mice.
**Figure 11****. Targeted Screening of Protective Antigens.** 4 weeks-old C57BL/6 mice (n=5-10 per group) were acclimated for 3 weeks and intraperitoneally immunized with a first dose of 5x10⁵ TU of non-concentrated VSV^{NJ} B2M LPs. Thirty days after the first immunization, the animals received a second dose of 1x10⁷ TU of non-concentrated VSV^{IND} B2M LPs. Third days later, mice were challenged with 5,000 GFP-expressing sporozoites micro-injected subcutaneously in the footpad. The parasite blood infection was measured by flow cytometry. (a.) The upper graph shows the log of parasitemia of individual mouse at day 5 post-infection. Traces represent the mean of the log parasitemia. The average of the GFP group (control of protection) is represented by the dotted middle line. The superior and inferior dotted lines delineate the 95% tolerance interval (grey box) of the GFP control group. The CSP group is the positive control of protection. NI (not infected = no parasitemia at day 10 post-infection, located at the limit of detection of our method of parasitemia quantification). Black circles represent antigens where there was a significant decrease in the averaged log parasitemia and therefore are considered protective (ANOVA). (b) The bottom graph represents the percentage of protected mice (% of animals below the 95% tolerance interval). Black bars represent protective antigens (Fisher's Exact test). *P<0.05, **P<0.01, ****P<0.0001.

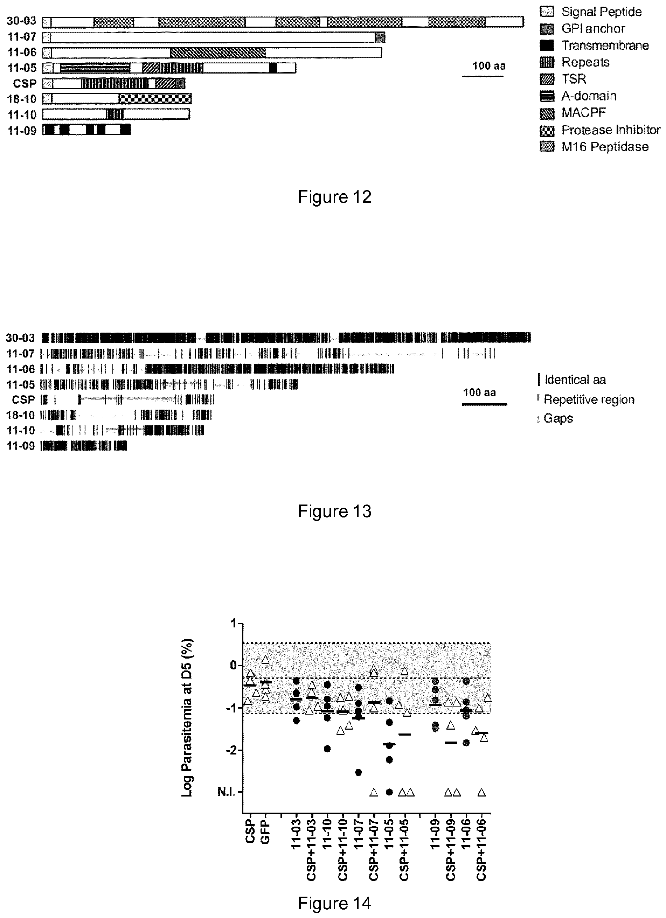

**Figure 12****. Structure of *P.berghei* protective antigens.** Conserved structural and functional domains are represented by boxes according to the code on the right. GPI (glycosylphosphatidylinositol), TSR (thrombospondin type I repeat), MACPF (membrane attack complex/perforin).
**Figure 13****. Protective antigens are conserved among plasmodial species.** Amino acid sequence of protective orthologous antigens from rodent-infecting P. *berghei,* macaque-infecting *P.cynomolgi,* and human-infecting *P.falciparum and P.vivax* parasites were aligned by **MU**ltiple **S**equence **C**omparison by **L**og- **E**xpectation (MUSCLE). Vertical black bars represent identical amino acids conserved in the four plasmodial species, short dark gray bars represent repetitive regions and short light gray bars, insertional gaps used for the alignment.
**Figure 14****. Protection induced by combination of down-selected protective antigens with a sub-optimal dose of CSP.** Mice were immunized twice, four weeks apart, with a sub-optimal dose of CSP (5x10⁵ TU of non-concentrated VSV^{NJ} B2M LP in the first immunization and 5x10⁶ TU of non-concentrated VSV^{IND} B2M LP in the second immunization, white triangle, CSP) and the usual dose of protective plasmodial antigens (CSP+11-03, +11-05, +11-06, +11-07, +11-09 and +11-10; triangles). As negative control mice were immunized with the usual, two doses of GFP. 4 weeks after the second immunization dose, animals were challenged with 5,000 sporozoites.

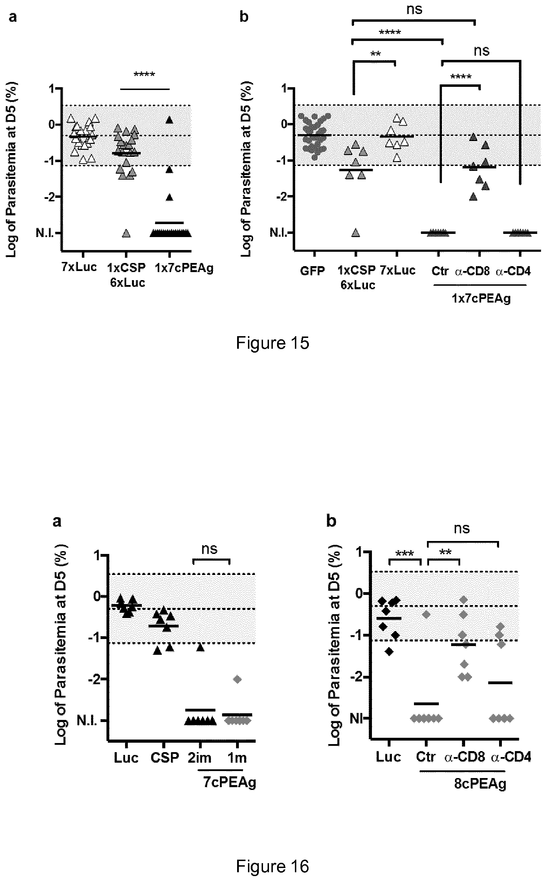

**Figure 15****. Sterile protection induced by a multigenic combination.** Mice were immunized twice, four weeks apart, with 7x the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen AL11 -luciferase (Luc, white triangles), with the individual dose of CSP plus 6x Luc (gray triangles), or with the individual doses of CSP and of 6 conserved PE antigens (11-05, 11-06, 11-07, 11-09, 11-10 and 18-10; black triangles, 7cPEAg). 4 weeks after the second immunization dose, mice were challenged with 5,000 GFP SPZs. Both graphs show the individual log of parasitemia at day 5 post-challenge. (a) The graph shows the pooled results of three independent experiments. Number of sterile protected/challenged mice: 7xLuc (0/21), 1xCSP 6xLuc (1/20) and 1x7cPEAg (18/21). (b) Three and one day before sporozoite challenge, 1x7cPEAg immunized mice were injected with 400 µg of control (Ctr), CD4-depleting (a-CD4+, clone GK1.5) and CD8-depleting (a-CD8+, clone 2.43) monoclonal antibodies. GFP data comes from experiment showed in figure 11 (gray circles). Number of sterile protected/challenged mice: 7xLuc (0/7), 1xCSP 6xLuc (1/7) and 1x7cPEAg (ctr,7/7; a-CD8, 0/7 and a-CD4, 7/7). Notice that depletion of CD8+ cells abolished sterile protection. *P<0.05, **P<0.01, ****P<0.0001 (ANOVA).
**Figure 16****. Sterile protection induced by a multigenic combination in a single immunization dose. (a)** Mice were immunized twice, four weeks apart, with 7x the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen AL11-luciferase (Luc, black triangles), with the individual dose of CSP plus 6x Luc (CSP, black triangles), or with the individual dose of CSP and of 6 conserved PE antigens (11-05, 11-06, 11-07, 11-09, 11-10 and 18-10; black triangles; 2 im 7cPEAg). Alternatively, mice were administered only with the second individual immunization dose (1x10⁷ TU) of CSP and of 6 conserved PE antigens (11-05, 11-06, 11-07, 11-09, 11-10 and 18-10; grey diamonds; 1 im 7cPEAg). 4 weeks after the second immunization dose, mice were challenged with 5,000 GFP SPZs. The graph shows the individual log of parasitemia at day 5 post-challenge. Black bars are the average of log of parasitemia. Number of sterile protected/challenged mice: Luc (0/7), CSP (0/7), 2im 7cPEAg (6/7) and 1m 7cPEAg (6/7). (b) Mice were immunized once with 9x the individual dose (1 dose = 1x10⁷ TU of non-concentrated VSV^{IND} B2M LPs) of the control antigen AL11-luciferase (Luc, black diamonds), or with the individual doses of CSP + of 7 conserved PE antigens (11-05, 11-06, 11-07, 11-09, 11-10, 18-10, 30-03A and 30-03B; grey diamonds; 8cPEAg). Three and one day before sporozoite challenge, 8cPEAg immunized mice were injected with 400 µg of control (Ctr), CD4-depleting (a-CD4+, clone GK1.5) and CD8-depleting (a-CD8+, clone 2.43) monoclonal antibodies. 4 weeks after the single immunization dose, mice were challenged with 5,000 GFP SPZs. The graph shows the individual log of parasitemia at day 5 post-challenge. Black bars are the average of log of parasitemia. Number of sterile protected/challenged mice: 7xLuc (0/7) and 8cPEAg (ctr,6/7; a-CD8, 0/7 and a-CD4, 4/7). Notice that depletion of CD8+ cells abolished protection. *P<0.05; **P<0.01; ****P<0.0001; ns, P>0.05 (ANOVA).

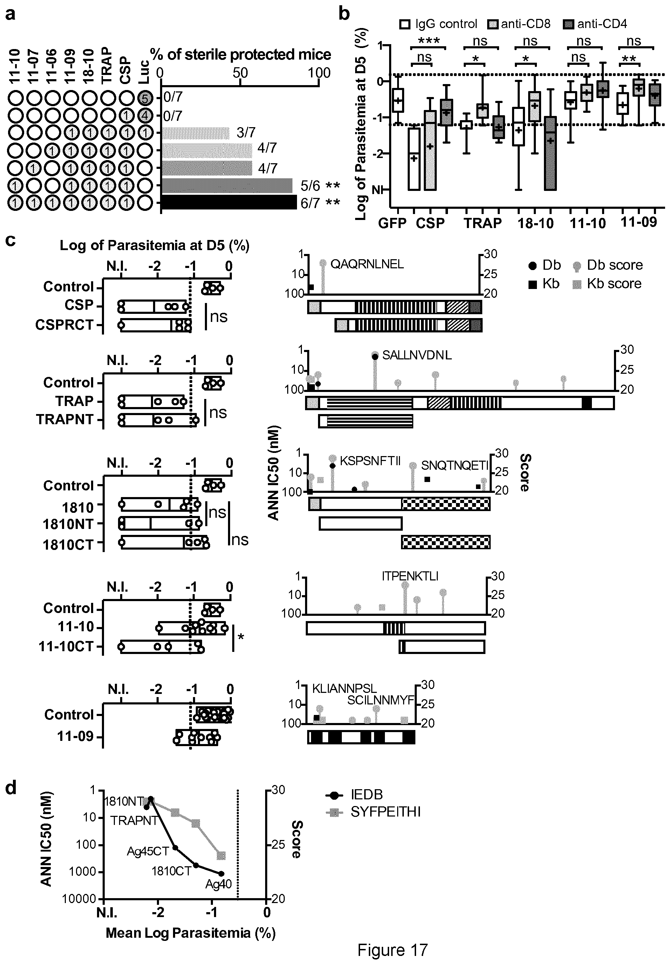

**Figure 17****. Sterile protection induced by a minimal combination of 5 PE antigens. (a)** Mice were immunized twice, four weeks apart, with the individual dose multiplied by the number indicated in the circles (1dose = 5x10⁵ TU of non-concentrated VSV^{NJ} B2M LPs in the first immunization / 1x10⁷ TU of non-concentrated VSV^{IND} B2M LPs in the second immunization). For example, for the control antigen AL11-luciferase (LUC), animals were immunized with 5x the individual dose. All groups received 5 doses, with exception of the positive control of protection that received 7 doses of LPs (7PEAg). 4 weeks after the second immunization dose, mice were challenged with 5,000 GFP SPZs. Bars represents the percentage of sterile protected mice. The numbers of sterile protected/ challenged mice are shown at the right of bars. **P<0.01 (Fisher's Exact test). **(b)** Mice were immunized twice, four weeks apart, with the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen GFP (GFP, black circles) or with the individual dose of CSP, TRAP, 18-10, 11-09 or 11-10 (grey triangles). Three and one day before sporozoite challenge, immunized mice were injected with 400 µg of control (Ctr), CD4-depleting (a-CD4+, clone GK1.5) and CD8-depleting (a-CD8+, clone 2.43) monoclonal antibodies. 4 weeks after the second immunization dose, mice were challenged with 5,000 GFP SPZs. Graphs show the average ± sd of log of parasitemia at day 5 post-challenge. *P<0.05; ns, P>0.05 (ANOVA). (c) The 5 down-selected protective antigens were split according the presence of predicted CD8 T cell epitopes and respecting conserved structural domains as depicted by the schematic representation of the antigens. The graphs above the schematic proteins represent the distribution of epitopes predicted to bind to H2Kb (8 aa) and H2Kd (9 aa) MHC class I molecules using SYFPEITHI (score) and IEDB ANN IC 50 (nM). The graphs on the left of schematic proteins represent the protection induced by these constructs, where bars are the average ± sd of log of parasitemia at day 5 post-challenge. Data shown for antigen 11-09 come from figure 11. Dotted line represents the inferior limit of the tolerance interval of the control calculated in the figure 11. *P<0.05; ns, P>0.05 (ANOVA). **(d)** Correlation of the best epitope predicted to bind to MHC class I molecules in the segments of CD8+ T cell dependent PE antigens and mean protective activity obtained from 17c. Circles show the IC50 using IEDB ANN software and squares the score values using SYFPEITHI. Dotted line shows the average of Luc control.

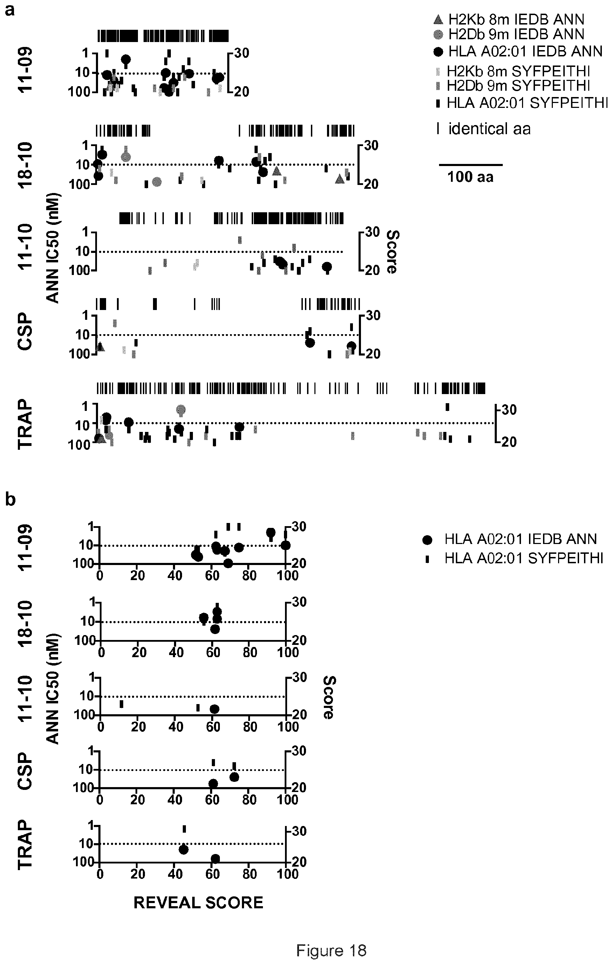

**Figure 18****. Clustering of CD8 T cell epitopes in conserved amino acid regions and binding of predicted Pf epitopes to HLA A02:01.** Amino acid sequences of protective orthologous antigens from rodent-infecting *P. berghei,* macaque-infecting *P.cynomolgi,* and human-infecting *P.falciparum and P.vivax* parasites were aligned by **MU**ltiple **S**equence **C**omparison by **L**og- **E**xpectation (MUSCLE). Vertical black bars represent identical amino acids conserved in the four plasmodial species. The graph shows the distribution of Pb epitopes predicted to bind to H2Kb (8 aa) and H2Kd (9 aa) MHC class I molecules or of Pf epitopes predicted to bind to the HLA A02:01 (9 mers) using SYFPEITHI (score) and IEDB ANN IC50 (nM). The best predicted HLA binders were tested in the assay of stabilization of MHC class I molecule in the presence of peptide and β2-microglobulin (REVEAL® Score). The score of 100 corresponds to the binding of a positive control peptide. Notice the clustering of epitopes in regions of conserved amino acids.

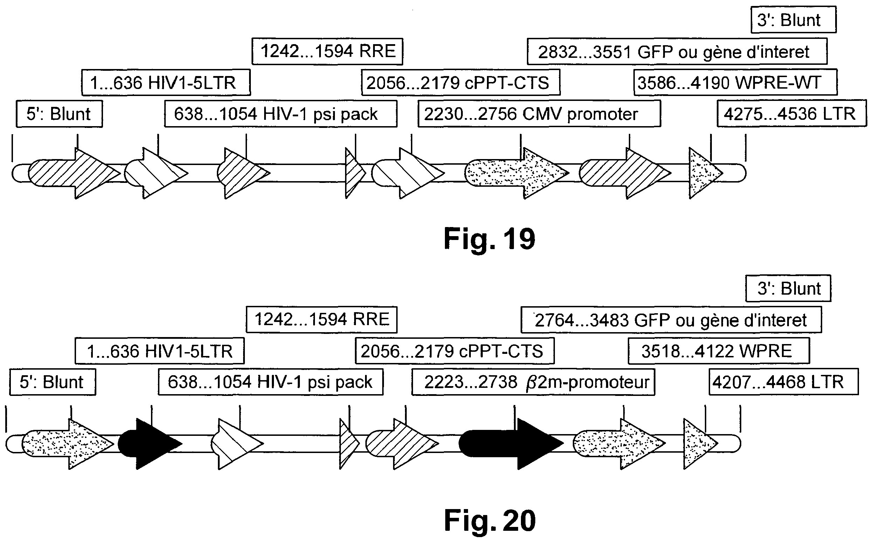

**Figure 19****. plasmid used to produce VSV-pseutdotyped lentiviral particles:** pTRIP CMV GFP
   The sequence of the plasmid is constituted by the following functional regions wherein the cis-acive lentiviral regions are derived from the HIV genome, and the promoter driving the expression of the protein (GFP) is CMV:
   The insert in the plasmid that provides the vector genome is composed as follows: LTR-Ψ- RRE- cPPT/CTS-CMV-*GFP*-WPRE-ΔU3LTR, wherein
   - LTR: is Long Terminal Repeat
   - Psi (Ψ): is Packaging signal
   - RRE: is Rev Responsive Element
   - CMV: is Immediate early CytoMegaloVirus promoter
   - cPPT: is central PolyPurine Tract, and wherein the nucleotide segment from
   - cPPT to CTS: forms the flap sequence
   - CTS: is Central Termination Sequence
   - WPRE: is Woodchuck hepatitis virus Post Regulatory Element
   The nucleotide sequence is provided as SEQ ID No. 1
**Figure 20****: alternative plasmid (to the plasmid of** **figure 16****) used to produce VSV-pseutdotyped lentiviral particles:** pTRIP B2M GFP
   The insert in the plasmid that provides the vector genome is composed as follows: LTR- Ψ- RRE- cPPT/CTS - B2M-GFP-WPRE-ΔU3LTR.
   The nucleotide sequence is provided as SEQ ID No. 2
**Figure 21****. plasmid used to produce VSV-pseutdotyped lentiviral particles:** packaging 8.74 plasmid
   The plasmid provides the required GAG and POL coding sequences of the HIV-1 lentivirus under the control of the CMV promoter.
   The nucleotide sequence is provided as SEQ ID No. 3.
**Figure 22****. plasmid used to produce VSV-pseutdotyped lentiviral particles:** encapsidation plasmid pCMV-VSV-INDco
   The envelope protein is the VSV-G of the Indiana strain and the coding sequence has been mouse-codon optimized.
   The nucleotide sequence is provided as SEQ ID No. 4.

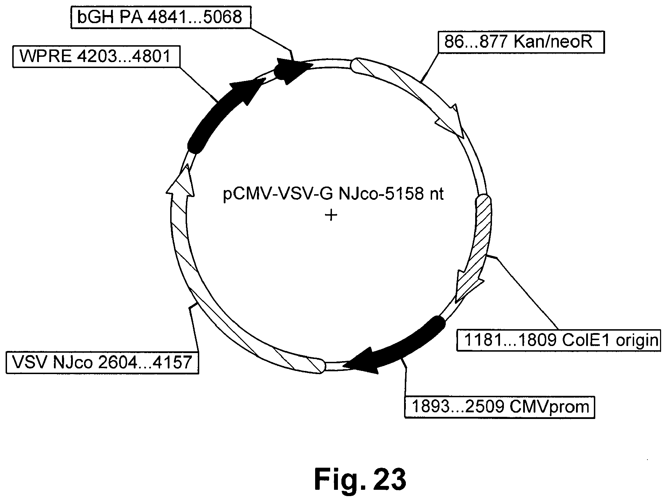

**Figure 23****. alternative plasmid (to plasmid of** **figure 19****) used to produce VSV-pseutdotyped lentiviral particles:** encapsidation plasmid pCMV-VSV-NJco
   The envelope protein is the VSV-G of the New-Jersey strain and the coding sequence has been mouse-codon optimized.

The nucleotide sequence is provided as SEQ ID No. 5.

The following table provides the list and identification of the sequences contained in the sequence listing..

| SEQ ID No. | Sequence designation | Origin | Type |
|---|---|---|---|
| 1 | pTRIP CMV GFP | | DNA |
| 2 | pTRIP B2M GFP | | DNA |
| 3 | PACKAGING 8.74 PLASMID | | DNA |
| 4 | pCMV-VSV-INDco | | DNA |
| 5 | pCMV-VSV-Njco | | DNA |
| 6 | eGFP | | DNA |
| 7 | eGFP protein | | protein |
| 8 | AL11-Luciferase | | protein |
| 9 | AL11-Luciferase | | protein |
| 10 | circumsporozoite (CS) protein (CSP) mouseCO + Kozak | P. berghei ANKA strain | DNA |
| 11 | PbCSP (mouseCO + Kozak) | P. berghei ANKA strain | protein |
| 12 | PbCSP | P. berghei ANKA strain | protein |
| 13 | PfCSP humanCO + Kozak | P falciparum 3D7 strain | DNA |
| 14 | PfCSP (humanCO + Kozak) | P falciparum 3D7 strain | protein |
| 15 | PfCSP | P falciparum | protein |
| 16 | PvCSP humanCO + Kozak | P vivax Sal-1 strain | DNA |
| 17 | PvCSP (humanCO + Kozak) | P vivax Sal-1 strain | protein |
| 18 | PvCSP | P vivax Sal-1 strain | protein |
| 19 | thrombospondin-related anonymous protein (PbTRAP) mouseCO + Kozak | P. berghei ANKA strain | DNA |
| 20 | PbTRAP (mouseCO + Kozak) | P. berghei ANKA strain | protein |
| 21 | PbTRAP | P. berghei ANKA strain | protein |
| 22 | PfTRAP humanCO + Kozak | P falciparum 3D7 strain | DNA |
| 23 | PfTRAP (humanCO + Kozak) | P falciparum 3D7 strain | protein |
| 24 | PfTRAP | P falciparum | protein |
| 25 | PvTRAPhumanCO | P vivax Sal-1 strain | DNA |
| 26 | PvTRAP | P vivax Sal-1 strain | protein |
| 27 | PvTRAP | P vivax | protein |
| 28 | inhibitor of cysteine proteases (ICP) mouseCO + Kozak | P. berghei ANKA strain | DNA |
| 29 | PblCP (mouseCO + Kozak) | P. berghei ANKA strain | protein |
| 30 | PblCP | P. berghei ANKA strain | protein |
| 31 | PflCP humanCO | P falciparum 3D7 strain | DNA |
| 32 | PflCP | P falciparum 3D7 strain | protein |
| 33 | PflCP | P falciparum | protein |
| 34 | PvlCP humanCO + Kozac | P vivax Sal-1 strain | DNA |
| 35 | PvlCP (humanCO + Kozac) | P vivax Sal-1 strain | protein |
| 36 | PvlCP | P vivax | protein |
| 37 | Bergheilysin-A-mouseCO + Kozak | P. berghei ANKA strain | DNA |
| 38 | Bergheilysin-A (1-777, mouse CO + Kozak) | P. berghei ANKA strain | protein |
| 39 | Bergheilysin entire ORF (1-1149) | P. berghei ANKA strain | protein |
| 40 | Falcilysin human CO + Kozak | P falciparum 3D7 strain | DNA |
| 41 | Falcilysin (human CO + Kozak) | P falciparum 3D7 strain | protein |
| 42 | Falcilysin | P falciparum 3D7 strain | protein |
| 43 | PvFalcilysin human CO + Kozak | P vivax Sal-1 strain | DNA |
| 44 | PvFalcilysin (humanCO + Kozak) | P vivax Sal-1 strain | protein |
| 45 | PvFalcilysin | P vivax Sal-1 strain | protein |
| 46 | Bergheilysin-B-mouseCO + Kozak + signal peptide (SP) | P. berghei ANKA strain | DNA |
| 47 | Bergheilysin-B (SP+778-1149, mouse CO + Kozak) | P. berghei ANKA strain | protein |
| 48 | perforin like protein 1 (SPECT2) mouseCO + Kozak | P. berghei ANKA strain | DNA |
| 49 | PbSPECT2 (mouseCO + Kozak) | P. berghei ANKA strain | protein |
| 50 | PbSPECT2 | P. berghei ANKA strain | protein |
| 51 | PfSPECT2 human CO + Kozak | P falciparum 3D7 strain | DNA |
| 52 | PfSPECT2 (humanCO + Kozak) | P falciparum 3D7 strain | protein |
| 53 | PfSPECT2 | P falciparum | protein |
| 54 | PvSPECT2 human CO + Kozak | P vivax Sal-1 strain | DNA |
| 55 | PvSPECT2 (human CO + Kozak) | P vivax Sal-1 strain | protein |
| 56 | PvSPECT2 | P vivax | protein |
| 57 | GPI_P113 mouseCO + Kozak | P. berghei ANKA strain | DNA |
| 58 | Pb GPI_P113 (mouseCO + Kozak) | P. berghei ANKA strain | protein |
| 59 | Pb GPI_P113 | P. berghei ANKA strain | protein |
| 60 | PfP113 human CO + Kozak | P falciparum 3D7 strain | DNA |
| 61 | PfP113 (human CO + Kozak) | P falciparum 3D7 strain | protein |
| 62 | P113 | P falciparum | protein |
| 63 | PvP113 human CO + Kozak | P vivax Sal-1 strain | DNA |
| 64 | PvP113 (human CO + Kozak) | P vivax Sal-1 strain | protein |
| 65 | P113 | P vivax | protein |
| 66 | PbAg40 mouse CO + Kozak | P. berghei ANKA strain | DNA |
| 67 | PbAg40 (mouse CO + Kozak) | P. berghei ANKA strain | protein |
| 68 | PbAg40 | P. berghei ANKA strain | protein |
| 69 | PfAg40 human CO + Kozak | P falciparum 3D7 strain | DNA |
| 70 | PfAg40 (human CO + Kozak) | P falciparum 3D7 strain | protein |
| 71 | Ag40 | P falciparum | protein |
| 72 | PvAg40 human CO + Kozak | P vivax Sal-1 strain | DNA |
| 73 | PvAg40 (human CO + Kozak) | P vivax Sal-1 strain | protein |
| 74 | PvAg40 | P vivax Sal-1 strain | protein |
| 75 | PbAg45 mouse CO + Kozak | P. berghei ANKA strain | DNA |
| 76 | PbAg45 (mouse CO + Kozak) | P. berghei ANKA strain | protein |
| 77 | PbAg45 | P. berghei ANKA strain | protein |
| 78 | PfAg45 human CO + Kozak | P falciparum 3D7 strain | DNA |
| 79 | PfAg45 (human CO + Kozak) | P falciparum 3D7 strain | protein |
| 80 | PfAg45 | P falciparum | protein |
| 81 | PvAg45 human CO + Kozak | P vivax Sal-1 strain | DNA |
| 82 | PvAg45 (human CO + Kozak) | P vivax Sal-1 strain | protein |
| 83 | PvAg45 | P vivax | protein |
| 84 | Kozak consensus sequence | | DNA |
| 85 | Kozak consensus sequence | | DNA |
| 86 | BamHI site | | DNA |
| 87 | Xhol site | | DNA |
| 88-94 | CD8 T cell epitopes | | protein |

Additional information relating to some of the sequences disclosed in the above table are provided in the table below.

| SEQ ID | GenBank | strain | pubmed |
|---|---|---|---|
| 15 | BAM84930.1 | Plasmodium falciparum isolate Pal97-042 | 23295064 |
| | | origin: Philippines | |
| | ACO49323 | Plasmodium falciparum" isolate A5 | 19460323 |
| | | origin: Thailand | |
| 18 | AAA29535.1 | P.vivax (strain Thai; isolate NYU Thai) | 2290443 |
| | | origin: Thailand | |
| 24⁽¹⁾ | EWC74605.1 | Plasmodium falciparum UGT5.1 strain | |
| | | origin: Uganda | |
| 27 | AIU97014.1 | Plasmodium vivax isolate="TMS38" | |
| | | origin: Thailand | |
| 36⁽³⁾ | KMZ87332.1 | Plasmodium vivax Brazil I strain | |
| 56 | KMZ82648.1 | Plasmodium vivax India VII | |
| 65 | KMZ78214.1 | Plasmodium vivax India VII | |
| 83 | KMZ90984.1 | Plasmodium vivax Mauritania I | |

| | | | |
|---|---|---|---|
| (1): https://www.ncbi.nlm.nih.gov/biosample/SAMN01737342 (2) : https://www.ncbi.nlm.nih.gov/biosample/SAMEA2394724 (3) : https://www.ncbi.nlm.nih.gov/biosample/SAMN00710434 | | | |

### Examples

To approach the complex problem of identifying protective antigens, the inventors devised a functional screening to identify and combine novel PE protective antigens using a rodent malaria model where mice (C57BL/6) are extremely susceptible to *Plasmodium berghei* (Pb) sporozoite infection. In this model, sterilizing protection induced by live irradiated sporozoites is mediated by antibodies and mainly by CD8 T cell responses against sporozoites and liver stages, respectively. The inventors' screening strategy was designed based on four main features: i) parameterized selection of 55 PE antigens based on abundance, orthology, predicted topology and function, ii) synthesis of codon-optimized antigens to avoid AT-rich plasmodial sequences and maximize the expression in mammalian cells, iii) immunization using HIV-based lentiviral vector that elicits strong humoral and cellular responses^{11,12}, and iv) measurement of protection after a stringent challenge of sporozoites inoculated sub-cutaneously in the immunized mice.

### 1. Setting up the parameters of the screening.

In a proof-of-concept experiment aimed at validating the viability of our strategy to screen antigens at a medium-throughput, the inventors ordered mouse-codon optimized synthtetic genes of Pb CSP (SEQ ID No. 11 and 12), a known protective antigen, and of more 4 other sporozoite antigens (Celtos, SPECT, HSP20 and Ag13), which were previously correlated with protection¹³. The synthetic plasmodial genes were cloned in to the pTRIP vector plasmid, which drives their expression in mammalian cells via the immediate-early cytomegalovirus promoter (CMV) and the post-transcriptional regulatory element of woodchuck hepatitis virus (WPRE) (Fig.1). These two elements assure a strong expression of the antigen in a wide variety of mouse cells *in vivo.* HIV-1 derived lentiviral particles were produced by transient co-transfection of HEK 293T cells with three helper plasmids encoding separate packaging functions, the pTRIP vector plasmid containing the synthetic plasmodial gene, the envelope expression plasmid encoding the glycoprotein G from the Vesicular Stomatitis Virus, Indiana (VSV^{IND}) or New Jersey (VSV^{NJ}) serotypes, and the p8.74 encapsidation plasmid (Fig. 1). This co-transfection generates integrative but replication-incompetent pseudotyped lentiviral particles capable of transducing dividing and non-dividing cells - including dendritic cells - and inducing potent cellular⁶ and humoral⁷ memory responses. The particles were collected 48 hours after co-transfection and each batch of vector were titrated in HeLa cells by quantitative PCR. This functional titration assay gives the concentration of particles capable to transfer one copy of the gene per cell and will be expressed in Transducing Units (TU)/mL. Plasmid sequences are shown in the figures and their sequences are provided in the sequence listing.

Groups of five mice were immunized with a single intra-muscular dose of 5e7 TU of ultracentrifugation-concentrated VSV^{IND} pseudotyped lentiviral particles (LPs). Thirty days after immunization, mice were challenged with 10,000 bioluminescent sporozoites inoculated sub-cutaneously in the footpad. Two days later, the parasite load in the liver was measured by bioluminescence. Surprisingly, CSP-immunization decreased 15x-fold the parasite load in the liver after a challenge using 10,000 bioluminescent sporozoites, versus a 5x-fold decrease in animals immunized intravenously with 50,000 irradiated sporozoites, our golden standard of protection (Fig. 2). This preliminary and promising result validated the high performance of the present method to functionally identify new protective antigens and showed the feasibility to scale-up our test samples.

The inventors next aimed at the transposition of these optimal experimental conditions to those of a larger screening. This transposition included the validation of the use of non-concentrated LPs, the choice of the best promoter driving the expression of the plasmodial antigens, and the dose of immunization.

The first parameter tested was the use of non-concentrated, instead of concentrated LPs, to avoid a costly and time consuming ultracentrifugation concentration step in the protocol of LP production, which requires large volumes of non-concentrated LP suspensions. Figure 3 shows that there is no significant difference between protection induced by the same dose (5x10⁷ TU) of concentrated LPs injected intramuscularly (CS im c, 50 µL) and non-concentrated LPs injected intraperitoneally (CS ip nc, 700 µL). Protection was measured by reduction in the liver infection, as assessed by bioluminescence after a challenge of 5,000 sporozoites injected subcutaneously 30 days following immunization. As negative control of protection the inventors used mice immunized with Pb Ag13, determined previously as a non-protective antigen (Fig. 1).

Next two promoters were tested to identify which one induced the best protection using the codon optimized Pb CSP. The inventors compared the use of the strong and constitutive cytomegalovirus (CMV) promoter versus a human beta-2 microglobulin (B2M) promoter, which direct gene expression in many cell types, particularly in dendritic cells. Figure 4 shows that CSP-induced protection was slightly better, although not statistically significant, using the B2M promoter at an immunization dose of 1x10⁷ TU of non-concentrated LP. Therefore the inventors further adopted this promoter in our constructs.

During this period of optimization the inventors observed some variations in the CSP-induced protection using the same stock of LPs, as can be seen in the figure 4. The inventors asked if this variability could be due to the process of mouse acclimation, including the modification of mouse microbiota. To test this hypothesis a group of mice purchased from Elevage Janvier (4 weeks-old) was reared in the animal facility for 3 weeks before immunization (group old). A second group of mice (7 weeks-old) was purchased and put in cages 3 days before the immunization (group new). Both groups were intraperitoneally immunized with 1x10⁷ TU of non-concentrated LPs. As shown in the figure 5, mouse acclimation of 3 weeks resulted in a significant and more homogeneous protection when compared to 3 days of acclimation. Consequently, the inventors adopted this period of acclimation in all our subsequent experiments.

Next, the best protective immunization dose was tested, ranging from 1x10⁸ to 1x10⁵ TU of B2M CSP non-concentrated LPs. As shown in figure 6, significant protection was observed using 10⁷ and 10⁸ TU, and the best protective activity was observed using an immunization dose of 1x10⁷ TU. In this experiment the inventors also observed a gradual loss of SPZ infectivity over time, as evidenced in the GFP groups, due to the use of a single SPZ stock to challenge all animals. To reduce the multiple shocks of temperature due to the manipulation of the stock tube, kept on ice between injections, the inventors prepared a SPZ stock for each group in the subsequent challenges and this variation disappeared.

In summary, an immunization protocol was set up based on CSP that relied on a single intraperitoneal injection of 10⁷ TU of non-concentrated VSV^{IND} B2M LP in C57BL/6 mice of 7 weeks-old, acclimated for 3 weeks in the animal facility. In the pooled data, this protocol leaded in average to a ∼5-fold decrease in the parasite liver load, as assessed by bioluminescence imaging, using a subcutaneous challenge of 5,000 luciferase-expressing SPZ.

However, this bioluminescent method of detection of parasites presents some disadvantages such as the use and associated costs of anesthesia and luminescent substrate, limited capacity of analysis of a few animals per acquisition, being time-consuming and not sensible enough to predict sterile protection. Therefore, the inventors decided to use fluorescent parasites to check protection by measuring parasitemia at day 4, 5, 6 and 10 post-inoculation by flow cytometry. The inventors analyze at least 100,000 red blood cells, which gives the sensibility to detect a parasitemia of 0.001%. At day 4 to 6, parasites grow exponentially in the blood therefore the log transform of parasitemia can be fitted using a linear regression where the slope represents the time of parasite replication per day. Consequently, the inventors use this parameter to determine if the immunization impacts the parasite growth in the blood. For quantifying protection the inventors use the log of parasitemia at day 5 post inoculation. This represents an indirect measure of liver infection and it is more robust than the measure at day 4 because more events of infected blood cells are registered. Finally the inventors defined that immunized mice are sterile protected if infected red blood cells are not detected after 10 days post inoculation. After defining the protocol of immunization and the method for the quantification of parasite infection the inventors started to screen the protective activity of down-selected antigens.

### 2. Parameterized selection of antigens

By merging proteomic and transcriptomic data using PlasmoDB (www.plasmodb.org), the inventors identified ∼9000 genes expressed in plasmodial pre-erythrocytic stages - salivary gland sporozoites and liver-stages - of three different plasmodial species, with 3654 syntenic orthologs in *Plasmodium falciparum (Pf),* the most lethal human-infecting plasmodial species. By analyzing the repertoire of pathogen transcripts, as inferred by the amount of expressed sequence tags (ESTs) obtained in cDNA libraries of different stages and species of malaria parasites, the inventors have observed that ∼50% of the total amount of ESTs are coming from only ∼10% of genes represented in these libraries, corresponding to approximately 100 genes in these libraries (Fig. 7). Therefore, by focusing on the ∼100 most abundant transcribed genes the inventors could target about 50% of the putative (to be translated) antigenic mass of a given parasite stage. Accordingly, the inventors selected ∼50 abundantly transcribed genes coding for conserved proteins with high probability of being expressed/presented on the surface of the parasite/infected cell, giving priority to candidates containing T cell epitopes predicted by IEDB MHC binding algorithm (http://tools.iedb.org/mhci/). A Kozak consensus sequence, a translational start site, was added to these down-selected genes, which were then mammalian codon-optimized and synthesized by MWG Eurofins (listed in the figures). These synthetic codon-optimized down-selected plasmodial genes were then cloned into the B2M pTRIP plasmid and produced as non-concentrated VSV^{IND} LPs.

### 3. First screening of protective antigens using a single dose of LPs

Usually, 6-10 plasmodial antigens were tested by experiment, with a negative (GFP) and positive (CSP) control of protection. After three weeks post-immunization, the immune-sera were tested on permeabilized and non-permeabilized sporozoites, allowing the determination of (i) the efficiency of the host humoral response and therefore the immunogenicity of the lentivirus-delivered antigen, and (ii) the localization of the parasite antigen (surface vs intracellular). As shown in the figure 8, where the inventors immunized mice with putative GPI-anchored antigens, surface antigens were identified by flow cytometry and immunofluorescence (CSP and 9-6). The sera of GFP and CSP group served, respectively, as positive control for intracellular and surface antigen localization.

Four weeks post-immunization the animals were challenged with 5,000 GFP-expressing sporozoites, microinjected in the footpad of immunized mice. Parasitemia was determined by flow cytometry. To define protection, parasitemia of all GFP groups (day 5 post-infection, n=35) was log transformed, pooled and the 95% tolerance interval was calculated (Fig. 9). All animals below the inferior limit of the tolerance interval, which represents a ∼8-fold decrease in parasitemia compared to the mean log of parasitemia of the GFP group, were considered protected. As positive control, 43% of animals (15/35) were protected by CSP immunization with a mean fold decrease of ∼5 fold in comparison to the GFP group. 9% of them (3/35) became sterile protected after sporozoite challenge.

In the first set of 43 antigens tested (Fig. 9), the inventors identified 9 PE antigens that protected at least one out of five immunized mice (black circles; 07-03, 09-06, 10-05, 10-10, 12-03, 12-04, 12-05, 12-07 and 13-08). Three of them were also identified as sporozoite surface antigens (09-06, 10-05 and 10-10).

To verify the robustness of our screening, the inventors selected 4 protective antigens (CSP 09-06, 10-05, 10-10 and 07-03), 4 non-protective antigens (GFP 09-07, 07-05, 07-06 06-06 and 10-06) plus GFP and CSP, and instead of only one immunization dose, the inventors administered one dose of 5x10⁵ TU of non-concentrated VSV^{NJ} B2M LPs and one month later, a second dose of 1x10⁷ TU of VSV^{IND} B2M LPs. As shown in the figure 10, the inventors observed three patterns of infection profile when the inventors compared one (circles, data from fig. 9) and two immunization doses (squares, PB). For the non-protective antigens GFP, 09-07 and 07-05, the second dose of LP did not change the profile of infection, as expected. For the protective antigens CSP (***P<0.001), 09-06, 10-05 and 07-03, the second dose of LP increased the number of protected mice and/or decreased the average parasitemia, also, as expected. Notably, the non-protective antigens 07-06, 06-06 and 10-06, as assessed by one dose of LP immunization, showed a strong protective activity, including a sterile protected mice (PB 7-6), when administered twice in mice.

These results validated some of our protective antigens detected with a single immunization dose, but also showed that some good protective antigens were not detected in our first screen, leading to the decision of repeating the screening using two immunization doses.

### 4. Second screening of protective antigens using two doses of LP

By functionally screening the protective activity of 55 down-selected plasmodial PE antigens using the protocol of two immunization doses, the inventors identified 16 antigens that protected at least one immunized mice per group. Among these 16 antigens, 7 of them (black circles/bars in the figure 11) conferred significant protection when compared to animals immunized with the GFP, both when analysing the number of protected mice (Fisher's Exact test) or the mean of the log parasitemia (ANOVA).

All of them presented a similar or an inferior protective activity when compared individually to our standard of protection, the CSP (red, Fig. 11). Five of them are molecules with assigned function (11-05, 11-06, 11-07, 30-03 and 18-10) and two are proteins with no predicted function (11-09 and 11-10). The structure of these Pb protective antigens is shown in the figure 12 and the alignment of these proteins with their respective orthologs from human-infecting parasites, *P.falciparum* (Pf) and *P.vivax* (Pv), and macaque-infecting parasite *P.cynomolgi,* is represented in the figure 13. As shown in table I, the percentage of identical amino acids between orthologs varied from 75 to 38% (Pb vs Pf), 78 to 33% (Pb vs Pv) and 79 to 26% (Pf vs Pv). The most conserved genes (>50% identity) are 30-03, 11-09, 11-10 and 11-06 orthologs. Antigens with divergent repetitive sequences are penalized in the alignment by insertional gaps, presenting less percentage of identity.

**Table I. Percent Identity Matrix created by CLUSTAL 2.1. Amino acid sequence of Pb antigens were pBlasted against Pf and Pv taxids (organism) and the best matched sequence was used to align the orthologous proteins using MUSCLE (http://www.ebi.ac.uk/Tools/msa/muscle/). The table shows the percentage of identical amino acids between species. Raw data is presented in the figures.**

| **Antigen** | **Amino acid identity (%)** | | |
|---|---|---|---|
| | Pb/Pf | Pb/Pv | Pf/Pv |
| **30-03** | 74.65 | 77.90 | 73 |
| **11-09** | 66.19 | 66.19 | 79.05 |
| **11-06** | 64.92 | 63.40 | 64.44 |
| **11-10** | 56.94 | 50.15 | 62.28 |
| **CSP** | 42.06 | 33.53 | 26.36 |
| **18-10** | 39.60 | 41.23 | 49.30 |
| **11-05** | 38.75 | 44.67 | 42.86 |
| **11-07** | 37.53 | 33.42 | 46.30 |

In a decreasing order of protection the first antigen identified is TRAP¹⁴ (SEQ ID N° 20 and 21) (thrombospondin related anonymous protein; 11-05), which validated our method of screening since immunization with TRAP is known to induce protection both in rodents¹⁵ and humans¹⁶. TRAP is a type I transmembrane protein harbouring two extracellular adhesive domains, a von Willebrand factor type A domain and a thrombospondin type 1 domain, followed by a proline-rich repetitive region. TRAP is stored in micronemal secretory vesicles and following parasite activation, the protein is translocated to the surface of sporozoites where it serves as a linker between a solid substrate and the cytoplasmic motor of sporozoites. Intriguingly, anti-TRAP antibodies do not impair parasite motility and infectivity¹⁷. CD8+ T cells seem to mediate the protection mediated by TRAP immunization^{10,15,16,18}.

The second protective antigen identified is an inhibitor of cysteine protease (ICP, 18-10)¹⁹ (SEQ ID N° 29, 30). ICP seems to be involved in the motility and infectivity capacity of sporozoites via the processing of CSP^{20,21,} as well as, in the parasite intra-hepatic development²². Although the protein does not present structural signatures of membrane localization, there is evidence that the protein is located on the surface of sporozoites^{19,20}. Opposing results are published regarding the secretion of the protein following parasite activation^{21,22}. Similarly, there are contradictory results regarding the inhibition of host cell invasion by sporozoites in vitro in the presence of anti-ICP immune sera^{20,23}.

The third protective antigen identified is a metallopeptidase (Falcilysin/Bergheilysin, 30-03)²⁴ (SEQ ID N° 38 and 39 for Falcilysin/Bergheilysin A and 47 for Falcilysin/Bergheilysin B). This protease seems to be involved in the catabolism of hemoblobin in the parasite blood stages²⁵. A H-2K^{b}-restricted CD8 T cell epitope was recently described during the parasite blood infection²⁵ suggesting that CD8 T cells could mediate the protection elicited by the antigen 30-03 during the hepatic infection.

The fourth protective antigen is a GPI-anchored protein (P113, 11-07) (SEQ ID No.57 and 58) initially described in blood stages¹⁶ and also expressed in PE stages. P113 seems to be important for liver infection, dispensable for blood infection, but its precise function is unclear¹⁷.

The fifth antigen is the pore-forming like protein SPECT2 (11-06)²⁸ (SEQ ID N° 50). This protein has a membrane attack complex/ perforin (MACPF) domain and is involved in the sporozoite cell traversal activity, being important for the progression of sporozoites in the dermis²⁹ and survival to phagocytosis in the liver³⁰.

The sixth antigen identified is a hypothetical protein that the inventors called 11-09 also called Ag40 (SEQ ID N° 67 and 68). This protein has 4-5 annotated transmembrane domains. Deletion of the gene coding for the antigen 11-09 caused impairment of Pb parasite development in the liver.

The seventh antigen is also a hypothetical protein that the inventors called 11-10 also called Ag45 (SEQ ID N° 76 and 77). This protein doesn't have annotated domains, but possesses a central region with negatively charged amino acids. Recently the 11-10 ortholog of Plasmodium yoelii, another rodent-infecting plasmodial species, was also identified as a protective antigen²¹. The deletion of the gene coding for the antigen 11-10 blocked the Pb sporozoite invasion of salivary glands and completely abolished the capacity of sporozoites to infect the liver.

To determine if CSP based protection could be additively or synergistically improved by the combination of antigens, the inventors assessed the protection elicited by a sub-optimal dose of CSP (5x10⁵ TU of VSV^{NJ}/5x10⁶ TU of VSV^{IND} B2M LPs) in the absence or presence of an usual dose of protective antigens (5x10⁵ TU of VSV^{NJ} / 1x10⁷ TU of VSV^{IND} B2M LPs). This protection induced by CSP + protective antigens was compared to the protection elicited by these antigens alone (data from Fig. 11). As negative control the inventors used animals immunized with the usual dose of GFP LPs. Figure 14 shows that 4 antigens when combined with a sub-optimal dose of CSP (CSP + 11-03, +11-10, + 11-07 and + 11-05, triangles) did not change the average of protection when compared to the protective activity elicited by these antigens administered alone. In two antigens the antigen combination (CSP+11-09 and CSP+11-06) showed a tendency of better protection (∼10 fold), but it was not statistically significant.

### 5. Identification of multi-antigenic formulations capable of sterilizing sporozoite infection via a CD8+ T cell response

Since testing all possible combinations of antigens was technically unfeasible the inventors decided to evaluate the protection elicited by the combination of these multiple protective antigens. Remarkably, two immunizations of mice with the combination of CSP and 6 of these antigens elicited **sterile protection** in the vast majority of challenged animals (7PEAg, 86-100%, Figure 15). This percentage of sterile protection was far superior to the protection conferred by CSP in the same experimental conditions (0-14%). Depletion of CD8+ cells (□-CD8) just before the challenge, but not of CD4+ cells, decreased this protection to the level of that induced by CSP, suggesting that CD8+ T cells and antibodies mediate the extra protection elicited by the addition of these 6 PE antigens.

The same protective efficacy was observed using only a single immunization for the 7PEAg or for the 7PEAg+30-03 (Figs. 16a and 16b, 8PEAg), as well as, the dependence on CD8+ T cells for the sterilizing immunity of 8PEAg (Figs. 16b). Since the antigen 30-03 is a large molecule and did not improve sterile protection when administered with the 7PEAg, the inventors excluded it from further analysis.

### 6. Design of a chimeric antigen containing the protective domains of down-selected PE antigens

To determine a minimal antigenic composition capable of eliciting this additional protective CD8+ T cell response, the inventors first identified the antigens whose protective activity was dependent on these T cells. Protection induced by two immunizations using TRAP, 18-10 and 11-09 was significantly reduced after depletion of CD8+ cells, as shown in the figure 17b. Protection induced by two immunizations using 11-10 was reduced after depletion of CD8+ cells but it was not statistically significant (Fig. 17b). Therefore the inventors grouped CSP with the CD8+ dependent protective antigens, TRAP, 18-10 and 11-09 and added separately 11-10, 11-07 and 11-06 to identify a minimal antigenic combination capable of sterile protect immunized animals like the complete combination of antigens. As shown in figure 17a, the combination of 5 antigens, CSP + TRAP, 18-10, Ag40 and Ag45 induced comparable level of sterile protection elicited by the combination of the 7PEAg.

In order to combine the protective domains of each of these 5 antigens in a single chimeric molecule and thus avoid the costs associated with the production and delivery of five different antigens, the inventors mapped the protective regions of each antigen according to the localization of predicted epitopes binding to MHC class I molecules (Fig. 17c and 18) and structural-functional conserved motifs (Fig. 12, 13, 17c and 18).

As shown in the figure 17c, all tested domains presented either a better (11-10CT) or similar protective activity when compared to the entire antigen. The level of mean protection elicited by the domains of antigens inducing protective CD8+ T cells correlated with the score (P<0.01) or affinity (P=0.01) of CD8+ T cell epitopes respectively predicted by SYFPEITHI and IEDB (Fig. 17c and 17d). Importantly, the mapping of protective domains allowed the reduction of ∼2000 basepairs in the final chimeric PE antigen construct. Due to its small size, Ag40 was not split in domains and the data presented in the figure 17c comes from the experiment showed in the figure 11.

Analysis of the distribution of epitopes of Pb antigens predicted to bind to MHC class I molecules of C57BL/6 mice (H2-K^{b}, H2-D^{b}) or of the Pf orthologs predicted to bind to HLA A02:01, a high prevalent human HLA allele, revealed that most of predicted good binders are clustering in the regions that are conserved among different plasmodial species (Pb, Pc, Pv and Pf, Fig. 18). This renders possible to use the Pb protective regions mapped in the figure 17 to select the correspondent region in the Pf orthologs. In addition, the inventors validated the binding of the best predicted Pf epitopes to the HLA A02:01 class I molecule using the REVEAL® binding assay developed by Proimmune, which allows the quantification of the binding and stabilization of the complex formed by the tested peptide, HLA A02:01 and β2-microglobulin (Fig. 18).

In summary, using a parameterized selection of antigens, a screening based on lentiviral vaccination and a direct measurement of protection in vivo against a stringent sporozoite challenge, the inventors identified 8 protective antigens, including the vaccine candidates CSP and TRAP, out of 55 tested antigens. All these 8 antigens are conserved across several plasmodial species. Remarkably, immunization using a combination of seven or eight of these antigens elicited sterile protection in the vast majority of challenged mice, either using one or two immunizations. More importantly, this protection was far superior than the one elicited by CSP, so far the best protective PE antigen. Depletion of CD8+ T cells abolished sterilizing immunity, indicating that these cells are essential for this protective phenotype, similarly to the protection conferred by irradiated sporozoites. A minimal combination of 5 of these antigens was also capable of eliciting sterile protection in most of challenged animals. Mapping of the protective domains of these 5 antigens allowed the design of a chimeric antigen containing the fused protective domains of these 5 down-selected antigens. The human-infecting parasite orthologs of these protective antigens, or of their protective domains are potential candidates for being used in the development of a malaria vaccine formulation containing multiple protective antigens or multiple protective domains fused in a single molecule.

### MATERIAL and METHODS

**Parasite strains:** Plasmodium berghei ANKA strain constitutively expressing the GFP under the control of the hsp70 promoter (Ishino et al, 2006) was used in the challenges using parasitemia, quantified by flow cytometry, as protective readout. Plasmodium berghei ANKA strain constitutively expressing a GFP-luciferase fusion under the control of the eef-1alfa promoter (Franke-Fayard et al, 2008) was used in the challenges using liver infection, assessed by bioluminescence, as protective readout. Of note, parasitemia quantified using hsp70-gfp parasites was at least 10 times more sensible than using eef-1a gfp:luc parasites due to more intense expression level of GFP.
- Ishino T, Orito Y, Chinzei Y, Yuda M (2006) A calcium-dependent protein kinase regulates Plasmodium ookinete access to the midgut epithelial cell. Mol Microbiol 59:1175-1184.
- Franke-Fayard B, Djokovic D, Dooren MW, Ramesar J, Waters AP, et al. (2008) Simple and sensitive antimalarial drug screening in vitro and in vivo using transgenic luciferase expressing Plasmodium berghei parasites. Int J Parasitol 38:1651-1662.

**Mouse strains** *:* C57BL/6 Rj and Swiss mice were purchased from Elevage Janvier (France). All experiments were approved by the Animal Care and Use Committee of Institut Pasteur (CETEA 2013-0093) and were performed in accordance with European guidelines and regulations (MESR-01324).

**Production of Lentiviral Particles stock:** Down-selected plasmodial antigens were synthesized by Eurofins MWG as mouse codon-optimized genes with the addition of a Kozak consensus sequence (GCCACCATGGCT(C) (SEQ ID No. 85 and 86), representing the first 12 nucleotides in the coding sequences of the antigenic polypeptides), encompassing the first translated ATG. This modification adds an extra alanine after the first methionine. A *Bam*HI (GGATCC - SEQ ID No.87) and *Xho* I (CTCGAG - SEQ ID No.88) restriction sites were also inserted in the 5' and 3' extremities of the construct, respectively. These synthetic codon-optimized genes were then cloned into the BamHI and *Xho* I restriction sites of the pTRIP plasmid harboring either the CMV or B2M promoter (fig 16 and 17). Lentiviral particles were produced by transient calcium co-transfection of HEK 293T cells with three helper plasmids encoding separate packaging functions, the pTRIP vector plasmid containing the synthetic plasmodial gene, the envelope expression plasmid encoding the glycoprotein G from VSV (Vesicular Stomatitis Virus, Indiana (fig 19) or New Jersey (fig 20) serotypes) and the p8.74 encapsidation plasmid (fig 18), containing the structural, accessory and regulatory genes of HIV. This co-transfection will generate integrative but replication-incompetent pseudotyped lentiviral particles. At 24 hours post-transfection, the cell culture medium was replaced by serum-free DMEM. Supernatants were collected at 48 hours post-transfection, clarified by low-speed centrifugation, and stored at -80°C. The lentiviral vector stocks were titrated by real-time PCR on cell lysates from transduced HEK 293T cells and titer were expressed as transduction unit (TU) per ml.

**Immunization protocol:** For the screening using one single dose of LPs, 4 weeks-old C57BL/6 mice (n=5 per group per experiment) were acclimated for 3 weeks and intraperitoneally immunized with a single dose of 1x10⁷ TU of non-concentrated VSV^{IND} B2M LPs. For the protocol using two immunization doses. 4 weeks-old C57BL/6 mice (n=5 per group per experiment) were acclimated for 3 weeks and intraperitoneally immunized with a first dose of 5x10⁵ TU of non-concentrated VSV^{NJ} B2M LPs. Thirty days after the first immunization, the animals received a second dose of 1x10⁷ TU of non-concentrated VSV^{IND} B2M LPs. For testing combinations of a sub-optimal dose of CSP + an optimal dose of down-selected antigens, mice were immunized twice, four weeks apart, with a sub-optimal dose of CSP (5x10⁵ TU of non-concentrated VSV^{NJ} B2M LP in the first immunization and 5x10⁶ TU of non-concentrated VSV^{IND} B2M LP in the second immunization) and the usual dose of protective plasmodial antigens (5x10⁵ TU of non-concentrated VSV^{NJ} B2M LP in the first immunization and 1x10⁷ TU of non-concentrated VSV^{IND} B2M LP in the second immunization). For testing the combination of multiple antigens, mice were immunized twice, four weeks apart, with 7x the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen Al11-luciferase (Luc), with the individual dose of CSP plus 6 doses of Luc or with the individual doses of CSP and of the 6 conserved PE antigens (11-05, 11-06, 11-07, 11-09, 11-10 and 18-10). For this experiment the inventors used ultrafiltration and lenti-X (Clontech) concentrated stocks. The average volume of injection was 500 µL of LPs diluted in DMEM.

In all cases, thirty days after last immunization, mice were challenged with 5,000 GFP-expressing sporozoites micro-injected subcutaneously in the mice footpad.

**Sporozoite challenge:** *Anopheles stephensi* (Sda500 strain) mosquitoes were reared using standard procedures. 3-5 days after emergence, mosquitoes were fed on infected Swiss mice with a parasitemia superior to 2%, and kept as described in Amino et al, 2007. Between 20 and 23 days post-feeding, the salivary glands of infected mosquitoes were dissected in PBS, collected in 20 µL of sterile PBS on ice and disrupted using an Eppendorf® pestle. The suspension of parasites were filtered through a nylon mesh of 40 um, counted using Kova™ glasstic™ slide (Hycor) and adjusted to a concentration of 5,000 or 10,000 sporozoites/ µL with sterile PBS. This suspension was divided in individual tubes, one for each group of immunized mice (n=4-7 per group), and kept on ice until the challenge. One microliter of parasite suspension was injected in the right footpad of mice using a Nanofil syringe (World Precision Instruments) with a 35 GA bevelled needle (NF35BV).

Amino R, Thiberge S, Blazquez S, Baldacci P, Renaud O, et al. (2007) Imaging malaria sporozoites in the dermis of the mammalian host. Nat Protoc 2:1705-1712.

**Measurement of Parasite Infection:** Hepatic parasite loads were quantified at ∼ 44h by bioluminescence in fur shaved mice infected with GFP LUC parasites. Infected mice were first anesthetized with isoflurane and injected subcutaneously with D-luciferin (150 mg/kg, Caliper LifeSciences). After a 5 minutes incubation allowing the distribution of the substrate in the body of the anesthetized animals, mice were transferred to the stage of an intensified charge-coupled device photon-counting video camera box where anesthesia was maintained with 2.5% isoflurane delivered via nose cones. After 5 minutes of signal acquisition controlled by the Living Image software (Xenogen Corporation), animals were returned to their cage. Automated detection of bioluminescence signals by the system resulted in the generation of bioluminescence signal maps superimposed to the gray-scale photograph of the experimental mice. These images were then quantified using the Living Image software. Briefly, regions of interest (ROI) encompassing the liver were manually defined, applied to all animals and the average radiance within these ROIs was automatically calculated. Background signal was measured in the lower region of the abdomen, and the average values of background signal obtained.
Alternatively, blood infection was assessed by flow cytometry using hsp70-GFP parasites. At day 4, 5, 6 and >10 post-challenge, a millimetric excision was performed in the tail of mice allowing the collection of a drop of blood that was readily diluted in 500 µL of PBS. This diluted blood was analyzed using a flow cytometer. 500,000 events were analyzed at day 4 post-challenge and 100,000 events in the subsequent days. Non-infected mice after day 10 were considered as sterile protected.

**Statistical analysis:** Parasitemia data from GFP immunized control were log transformed and pooled for the calculation of 95% tolerance of interval with 95% of certitude. For the immunization protocol of one dose this limit comprised the interval of the mean value ± 2.49 SD (mean=-0.3906, SD= 0.3392, n=35). Similarly, for the immunization protocol of two doses this limit comprised the interval of the mean value ± 2.51 (mean=-0.3002, SD= 0.3305, n=33). All mice with a log parasitemia inferior to the lower limit (mean - 2.5 SD) were considered as significantly different from the control mice (P<0.05), and therefore considered as protected. In the protocol using two immunization doses, the difference in the numbers of protected mice, following the definition above, between the test group and the GFP control group was assessed using the Fisher's exact test. The average of the log parasitemia of the groups with significant differences in the Fisher's Test were compared to the GFP group using one-way ANOVA (Holm-Sidak's multiple comparison test).

### References:

1. http://www.who.int/mediacentre/factsheets/fs094/en/
2. RTS,S Clinical Trials Partnership. Lancet. 2015; 4;386(9988):31-45.
3. Moorthy VS, Ballou WR. Malar J. 2009 ; 8: 312.
4. http://www.who.int/immunization/topics/malaria/vaccine_roadmap/en/
5. Seder RA, et al. Science. 2013 ; 341:1359-65.
6. Amino R, Ménard R. Nature. 2012; 484(7395):S22-3
7. Kester KE, et al. 2014. pii: S0264-410X(14)00822-6.
8. Mishra S, et al. Vaccine. 2011 ;29(43):7335-42.
9. Murphy SC, Kas A, Stone BC, Bevan MJ. Proc Natl Acad Sci U S A. 2013;110(15):6055-60.
10.Hafalla JC, et al. PLoS Pathog. 2013;9(5):e1003303.
11.Iglesias, M.C., et al. J Gene Med. 2006; 8, 265-274.
12.Firat, H., et al. J Gene Med. 2002; 4, 38-45.
13.Doolan DL, et al. Proc Natl Acad Sci U S A. 2003; 100 :9952-7.
14.Robson KJ, et al. Nature. 1988; 335(6185):79-82.
15.Khusmith S, et al. Science. 1991;252(5006):715-8.
16.Ewer KJ, et al. Nat Commun. 2013;4:2836.
17.Gantt S, et al. Infect Immun. 2000;68(6):3667-73.
18.Khusmith S, Sedegah M, Hoffman SL. Infect Immun. 1994; 62(7):2979-83.
19.LaCrue AN, et al. Mol Biochem Parasitol. 2006;148(2):199-209.
20.Rennenberg A et al. PLoS Pathog. 2010; 6(3):e1000825.
21.Boysen KE, Matuschewski K. MBio. 2013;4(6):e00874-13.
22.Lehmann C, et al. PLoS Pathog. 2014;10(8):e1004336.
23.Pei Y, et al. Cell Microbiol. 2013 Sep;15(9):1508-26.
24.Eggleson KK, Duffin KL, Goldberg DE. J Biol Chem.;274(45):32411-7.
25.Poh CM, Howland SW, Grotenbreg GM, Rénia L. Infect Immun. 2014;82(11):4854-64.
26.Gilson PR, et al. Mol Cell Proteomics. 2006;5(7):1286-99.
27.Offeddu V, Rauch M, Silvie O, Matuschewski K. Mol Biochem Parasitol. 2014;193(2):101-9.
28.Ishino T, Chinzei Y, Yuda M. Cell Microbiol. 2005;7(2):199-208.
29.Amino R, et al. Cell Host Microbe. 2008 Feb 14;3(2):88-96.
30.Tavares J, et al. J Exp Med. 2013;210(5):905-15.
31.Speake C, et al. PLoS One. 2016;11(7):e0159449.

## Claims

1. A method of functionally screening protective antigenic polypeptides of a determined *Plasmodium* pathogen comprising the steps of:
a. optionally pre-selecting candidate antigenic polypeptides for the screening by reference to a group of genes, transcripts or proteins identified for a determined *Plasmodium* pathogen wherein the antigenic polypeptides may constitute putative targets for a humoral and/or cellular immune response .
b. providing a lentiviral vector, in particular a HIV-1 based vector, expressing one or more antigenic polypeptide(s) to be assayed for its (their) immunogenic properties,
c. immunizing a non-human mammal in particular a non-human mammal model selected for its susceptibility to infection by the determined *Plasmodium* pathogen, with the lentiviral vector of step b. in immunization dose conditions enabling elicitation of a potent cellular and/or humoral memory response against the antigenic polypeptide(s),
d. challenging the immunized non-human mammal of step c. with the *Plasmodium* pathogen administered to the immunized non-human mammal of step c. and quantifying the development of the *Plasmodium* pathogen wherein the quantification of the protective response against the antigenic polypeptide comprises a step of quantifying (i) the load of the sporozoites expressing a constitutive bioluminescent marker, in the liver of the non-human mammal by bioluminescence or (ii) the growth of the *Plasmodium* pathogen expressing a constitutive fluorescent marker, by determining fluorescence on red blood cells harvested from a blood sample of the non-human mammal, especially by flow cytometry, thereby enabling functional identification of the protective response capacity of the antigenic polypeptide(s).

2. A method of functionally screening immunogenic properties of antigenic polypeptides of a Plasmodium parasite according to claim 1 comprising the steps of:
a. optionally pre-selecting candidate antigenic polypeptides for the screening by reference to a group of genes, transcripts or proteins identified for a determined Plasmodium parasite wherein the group may reflect a particular stage of the development of the parasite or a particular biological function.
b. providing a lentiviral vector, in particular a HIV-1 based vector, expressing one or more antigenic polypeptide(s) to be assayed for its protective properties,
c. immunizing a non-human mammal model selected for its susceptibility to infection by the determined Plasmodium parasite with the lentiviral vector of step b. in immunization dose conditions enabling elicitation of a potent cellular and/or humoral memory response against the antigenic polypeptide,
d. challenging the immunized non-human mammal of step c. with sporozoites of the Plasmodium parasite and quantifying the development of the Plasmodium parasite in the non-human mammal thereby enabling functional identification of the protective response capacity of the antigenic polypeptides.

3. The method according to claim 2, wherein the *Plasmodium* parasite is *P. berghei,* or *P. yoelii* and the non-human mammal model is a rodent, in particular a mouse susceptible to said *Plasmodium* parasite, especially a C57BI/6 mouse for *P. berghei,* or a BALB/c mouse for *P. yoelii.*

4. The method of any one of claims 1 to 3 wherein the immunization comprises administering one dose of lentiviral vector in a priming step and one dose of lentiviral vector in a boosting step wherein in the priming and boosting steps the lentiviral vectors are pseudotyped with different non cross-seroneutralizing envelope proteins and wherein the priming and the boosting doses are different, in particular the boosting dose is higher than the priming dose or are equal.

5. The method according to any one of claims 1 to 3, wherein the administered doses of lentiviral vector expressing the antigenic polypeptide for immunization of the non-human mammal are each in the range of 1x10⁵ to 1x10⁸ TU, in particular 1x10⁵ to 1x10⁷ and wherein the lentiviral vector is formulated as a suspension of either concentrated or non-concentrated lentiviral vector.

6. The method of any one of claims 1 to 5 wherein a positive control for protection capability against infection by *Plasmodium* parasite or against the parasite-induced condition or disease is used which is CSP, or an antigen selected among antigens on the gametes, zygotes or ookinetes of the parasite such as sexual-stage antigens, antigens of the liver-stage or antigens of the asexual blood-stage, such as antigens of sporozoites, in particular an antigen selected among P48/P45 antigen Pfs25, MSP1, CSP, TRAP, Celtos, SPECT, ICP, and Facilysin/Bergheilysin Ag11-09, Ag11-10 and TRAP and a negative control for protective capability is used, which is GFP, a known non-protective antigen or an empty equivalent vector.

7. The method according to any one of claims 1 to 6, wherein immune-sera in the sample obtained from immunized non-human mammal were incubated with GFP-expressing *Plasmodium* sporozoites wherein a subgroup of these sporozoites is permeabilized and a subgroup of these sporozoites is not permeabilized.

8. The method according to any one of claims 1 to 7, wherein the lentiviral vector contains in its genome a synthetic gene for an antigenic polypeptide of *Plasmodium* the coding sequence of which is codon-optimized for the expression of the antigenic polypeptide in mammalian cells and wherein expression of the antigenic polypeptide is driven by a promoter suitable for directing gene expression in various mammalian cell types, including dendritic cells, such as a beta-2 microglobulin promoter.

9. The method according to any one of claims 1 to 8, wherein the genome of the lentiviral vector is obtained from a transfer vector which is a pTRIP plasmid wherein a *Plasmodium* synthetic nucleic acid, in particular a mammal-codon optimized nucleic acid, encoding the antigenic polypeptide to be assayed has been cloned under control of a promoter functional in mammalian cells, in particular the human beta-2 microglobulin promoter, and optionally under the control of post-transcriptional regulatory element of the woodchuck hepatitis virus (WPRE).

10. The method according to any one of claims 2 to 9, wherein the envelope protein pseudotyping the lentiviral vector is a VSV-G envelope protein from the Indiana strain, from the New Jersey strain or from any other non-cross neutralizing strain, such as Cocal and Chandipura.

11. The method of any one of claims 1 to 10, wherein the pre-selection of candidate antigenic polypeptide includes the step of transcriptome profiling of the Plasmodium parasite for the pre-erythrocytic stage of development compared with transcriptome profiling of the Plasmodium parasite for a different stage of development, in particular the blood-stage of development of said Plasmodium parasite and identifying over expressed transcripts in the pre-erythrocytic stage parasite.

12. A method of identifying an antigenic polypeptide for the design of a vaccine candidate against malaria parasite for protection of a human host, which comprises the steps of screening antigenic polypeptides according to a method of any one of claims 1 to 11, and determining the orthologous gene in a Plasmodium parasite infecting a human host, in particular *Plasmodium falciparum, Plasmodium vivax, Plasmodium malariae, Plasmodium ovale* or *Plasmodium knowlesi.*

## Patentansprüche

1. Verfahren zum funktionellen Screening von schützenden antigenen Polypeptiden eines bestimmten *Plasmodium-*Erregers worin dieses Verfahren folgende Schritte umfasst:
a. fakultative Vorauswahl von antigenen Polypeptidkandidaten für das Screening durch Bezugnahme auf eine Gruppe von Genen, Transkriptionen oder Proteinen, die für einen bestimmten *Plasmodium-*Erreger identifiziert wurden, wobei die antigenen Polypeptide mutmaßliche Ziele für eine humorale bzw. zelluläre Immunantwort darstellen können.
b. Bereitstellen eines lentiviralen Vektors, insbesondere eines auf HIV-1 basierenden Vektors, der ein oder mehrere antigene(s) Polypeptid(e) ausdrückt, das/die auf seine (ihre) immunogenen Eigenschaften untersucht werden soll(en),
c. Immunisieren eines nicht-menschlichen Säugetiers, insbesondere eines nicht-menschlichen Säugetiertyps, der aufgrund seiner Anfälligkeit für eine Infektion durch den bestimmten *Plasmodium-*Erreger ausgewählt wurde, mit dem lentiviralen Vektor des Schrittes b. beiImmunisierungsdosisbedingungen, die das Auslösen einer starken zellulären bzw. humoralen Gedächtnisreaktion gegen das/die antigene(n) Polypeptid(e) ermöglichen,
d. Herausfordern des immunisierten nicht-menschlichen Säugetiers von Schritt c. mit dem *Plasmodium-*Erreger, das dem immunisierten nicht-menschlichen Säugetier von Schritt c verabreicht wird, und Quantifizieren der Entwicklung des *Plasmodium-*Erregers, wobei die Quantifizierung der Schutzantwort gegen das antigene Polypeptid einen Schritt umfasst, der (i) die Belastung der Sporozoiten, die einen konstitutiven Biolumineszenzmarker ausdrücken, in der Leber des nicht-menschlichen Säugetiers durch Biolumineszenz oder (ii) das Wachstum des *Plasmodium-*Erregers, der einen konstitutiven Fluoreszenzmarker ausdrückt, umfasst, wobei die Fluoreszenz an roten Blutkörperchen, die aus einer Blutprobe des nicht-menschlichen Säugetiers entnommen wurden, insbesondere durch Durchflusszytometrie erfasst wird, wodurch eine funktionelle Identifizierung der schützenden Reaktionsfähigkeit des/der antigenen Polypeptide(s) ermöglicht wird.

2. Verfahren zum funktionellen Screening immunogener Eigenschaften von antigenen Polypeptiden eines Plasmodium-Parasiten gemäß Patentanspruch 1, worin dieses Verfahren folgende Schritte umfasst:
a. fakultative Vorauswahl von antigenen Polypeptidkandidaten für das Screening durch Bezugnahme auf eine Gruppe von Genen, Transkriptionen oder Proteinen, die für einen bestimmten Plasmodium-Parasiten identifiziert wurden, wobei die Gruppe ein bestimmtes Entwicklungsstadium des Parasiten oder eine bestimmte biologische Funktion widerspiegeln kann.
b. Bereitstellen eines lentiviralen Vektors, insbesondere eines auf HIV-1 basierenden Vektors, der ein oder mehrere antigene(s) Polypeptid(e) ausdrückt, das/die auf seine (ihre) schützenden Eigenschaften untersucht werden soll(en),
c. Immunisieren eines nicht-menschlichen Säugetiertyps, der aufgrund seiner Anfälligkeit für eine Infektion durch den bestimmten Plasmodium-Parasiten ausgewählt wurde, mit dem lentiviralen Vektor des Schrittes b. bei Immunisierungsdosisbedingungen, die das Auslösen einer starken zellulären bzw. humoralen Gedächtnisreaktion gegen das/die antigene Polypeptid ermöglichen,
d. Herausfordern des immunisierten nicht-menschlichen Säugetiers von Schritt c. mit Sporozoiten des Plasmodium-Parasiten und Quantifizierung der Entwicklung des Plasmodium-Parasiten in dem nicht-menschlichen Säugetier, wodurch eine funktionelle Identifizierung der Schutzreaktionsfähigkeit der antigenen Polypeptide ermöglicht wird.

3. Verfahren gemäß Patentanspruch 2, wobei der *Plasmodium-*Parasit *Plasmodium berghei* oder *Plasmodium yoelii* ist und der nicht-menschliche Säugetiertyp ein Nagetier ist, insbesondere eine Maus, die für den *Plasmodium-*Parasiten anfällig ist, insbesondere eine C57B1/6-Maus für *Plasmodium berghei* oder eine BALB/c-Maus für *Plasmodium yoelii.*

4. Verfahren nach einem der Patentansprüche von 1 bis 3, wobei die Immunisierung die Verabreichung einer Dosis eines lentiviralen Vektors in einem anfänglichen Schritt und einer Dosis eines lentiviralen Vektors in einem steigernden Schritt umfasst wobein in den anfänglichen und steigernden Schritten die lentiviralen Vektoren mit unterschiedlichen nichtkreuzseroneutralisierenden Hüllproteinen pseudotypisiert werden, und wobei die Dosen der anfänglichen und steigernden Schritte unterschiedlich, insbesondere die steigernde Dosis höher ist als die anfängliche Dosis, oder gleich sind.

5. Verfahren nach einem der Patentansprüche von 1 bis 3, wobei die verabreichten Dosen des lentiviralen Vektors, der das antigene Polypeptid ausdrückt, zur Immunisierung des nicht-menschlichen Säugetiers jeweils im Bereich von 1 x 10⁵ bis 1 x 10⁸ TU, insbesondere 1 x 10⁵ bis 1 x 10⁷ liegen, und der lentiviraler Vektor als Suspension von entweder konzentriertem oder nicht konzentriertem lentiviralen Vektor formuliert wurde.

6. Verfahren nach einem der Patentansprüche von 1 bis 5, wobei eine Positivkontrolle für die Schutzfähigkeit gegen eine Infektion durch *Plasmodium*-Parasiten oder gegen den durch Parasiten induzierten Zustand oder die Krankheit verwendet wird, die CSP oder ein Antigen ausgewählt aus Antigenen auf den Gameten, Zygoten oder Ookineten des Parasiten ist, wie Antigene im Sexualstadium, Antigene im Leberstadium oder Antigene im asexuellen Blutstadium, wie Antigene von Sporozoiten, insbesondere ein Antigen ausgewählt aus P48/P45-Antigen Pfs25, MSP1, CSP, TRAP, Celtos, SPECT, ICP und Facilysin/Bergheilysin Ag11-09, Agl1-10 und TRAP und eine Negativkontrolle für die Schutzfähigkeit verwendet wird, die GFP ist, ein bekanntes nicht-protektives Antigen oder ein leerer äquivalenter Vektor.

7. Verfahren nach einem der Patentansprüche von 1 bis 6, wobei Immunseren der vom immunisierten nicht-menschlichen Säugetier erhaltenen Probe mit GFP-ausdrückenden *Plasmodium-*Sporozoiten inkubiert wurden, wobei eine Untergruppe dieser Sporozoiten permeabilisiert ist und eine Untergruppe dieser Sporozoiten nicht permeabilisiert ist.

8. Verfahren nach einem der Patentansprüche von 1 bis 7, wobei der lentivirale Vektor in seinem Genom ein synthetisches Gen für ein antigenes Polypeptid von *Plasmodium* enthält, dessen kodierende Sequenz für den Ausdruck des antigenen Polypeptids in Säugetierzellen Codon-optimiert ist, und wobei der Ausdruck des antigenen Polypeptids durch einen Promotor gesteuert wird, der geeignet ist, die Genexpression in verschiedenen Säugerzelltypen, einschließlich dendritischen Zellen, wie einem Beta-2-Mikroglobulin-Promotor, zu steuern.

9. Verfahren nach einem der Patentansprüche von 1 bis 8, wobei das Genom des lentiviralen Vektors von einem Transfervektor erhalten wird, der ein pTRIP-Plasmid ist, wobei eine synthetische *Plasmodium*-Nukleinsäure, insbesondere eine Säugercodon-optimierte Nukleinsäure, die durch die Kodierung des zu testenden antigenen Polypeptids unter der Kontrolle eines funktionellen Promotors in Säugerzellen, insbesondere des menschlichen Beta-2-Mikroglobulin-Promotors, und gegebenenfalls unter der Kontrolle des posttranskriptionellen regulatorischen Elements des Waldmurmeltier-Hepatitisvirus (VVPRE) kloniert wurde.

10. Verfahren nach einem der Patentansprüche von 2 bis 9, wobei das Hüllprotein, das den lentiviralen Vektor pseudotypisiert, ein VSV-G-Hüllprotein des Indiana-Stamms, des New-Jersey-Stamms oder eines anderen nicht kreuzneutralisierenden Stamms wie Cocal und Chandipura ist.

11. Verfahren nach einem der Patentansprüche von 1 bis 10, wobei die Vorauswahl des antigenen Polypeptidkandidaten den Schritt des Transkriptom-Profiling des Plasmodium-Parasiten für das prä-erythrozytäre Entwicklungsstadium im Vergleich mit dem Transkriptom-Profiling des Plasmodium-Parasiten für ein anderes Stadium der Entwicklung umfasst, insbesondere des Blutstadiums der Entwicklung des Plasmodium-Parasiten, und Identifizieren von überexprimierten Transkriptionen in dem prä-erythrozytären Parasiten.

12. Verfahren zum Identifizieren eines antigenen Polypeptids für die Entwicklung eines Impfstoffkandidaten gegen Malariaparasiten zum Schutz eines menschlichen Wirts, das die Schritte des Screenings von antigenen Polypeptiden gemäß einem Verfahren nach einem der Patentansprüche von 1 bis 11 und des Bestimmens des orthologen Gens in einem Plasmodium-Parasiten umfasst, der einem menschlichen Wirt infiziert wurde, insbesondere *Plasmodium falciparum, Plasmodium vivax, Plasmodium malariae, Plasmodium ovale* oder *Plasmodium knowlesi.*

## Revendications

1. Procédé de criblage fonctionnel de polypeptides antigéniques de protection d'un agent pathogène *Plasmodium* déterminé, comprenant les étapes :
a. de présélection éventuelle des polypeptides antigéniques candidats pour le criblage par référence à un groupe de gènes, de transcrits ou de protéines identifié(e)s pour un pathogène *Plasmodium* déterminé, dans lequel les polypeptides antigéniques peuvent constituer des cibles putatives pour une réponse immunitaire humorale et / ou cellulaire ;
b. de fourniture d'un vecteur lentiviral, en particulier, un vecteur basé sur le VIH-1, exprimant un ou plusieurs polypeptide(s) antigénique(s) dont les propriétés immunogènes doivent être testées ;
c. d'immunisation d'un mammifère non humain, en particulier, d'un modèle de mammifère non humain sélectionné pour sa sensibilité à l'infection par l'agent pathogène *Plasmodium* déterminé, avec le vecteur lentiviral de l'étape b, dans des conditions de dose d'immunisation permettant l'élicitation d'une puissante réponse mémoire cellulaire et / ou humorale contre le(s) polypeptide(s) antigénique(s) ;
d. de réalisation d'un challenge du mammifère non humain immunisé de l'étape c avec l'agent pathogène *Plasmodium* administré au mammifère non humain immunisé de l'étape c, et de quantification du développement de l'agent pathogène *Plasmodium,* dans lequel la quantification de la réponse protectrice contre le polypeptide antigénique comprend une étape de quantification (i) de la charge des sporozoïtes exprimant un marqueur bioluminescent constitutif, dans le foie du mammifère non humain par bioluminescence ou (ii) de la croissance de l'agent pathogène *Plasmodium* exprimant un marqueur fluorescent constitutif, en déterminant la fluorescence sur les globules rouges prélevés à partir d'un échantillon de sang du mammifère non humain, en particulier par cytométrie en flux, permettant ainsi une identification fonctionnelle de la capacité de réponse protectrice du ou des polypeptide(s) antigénique(s).

2. Procédé de criblage fonctionnel des propriétés immunogènes de polypeptides antigéniques d'un parasite Plasmodium selon la revendication 1, comprenant les étapes :
a. de présélection éventuelle des polypeptides antigéniques candidats pour le criblage par référence à un groupe de gènes, de transcrits ou de protéines identifié(e)s pour un parasite Plasmodium déterminé, dans lequel le groupe peut refléter un stade particulier du développement du parasite ou une fonction biologique particulière ;
b. de fourniture d'un vecteur lentiviral, en particulier un vecteur basé sur le VIH-1, exprimant un ou plusieurs polypeptide(s) antigénique(s) dont les propriétés protectrices doivent être testées ;
c. d'immunisation d'un modèle de mammifère non humain sélectionné pour sa sensibilité à l'infection par le parasite Plasmodium déterminé avec le vecteur lentiviral de l'étape b, dans des conditions de dose d'immunisation permettant l'élicitation d'une puissante réponse mémoire cellulaire et / ou humorale contre le polypeptide antigénique,
d. de réalisation d'un challenge du mammifère non humain immunisé de l'étape c. avec des sporozoïtes du parasite Plasmodium et de quantification du développement du parasite Plasmodium chez le mammifère non humain, permettant ainsi une identification fonctionnelle de la capacité de réponse protectrice des polypeptides antigéniques.

3. Procédé selon la revendication 2, dans lequel le parasite *Plasmodium* est *P*. *berghei,* ou *P*. *yoelii,* et le modèle de mammifère non humain est un rongeur, en particulier une souris sensible audit parasite *Plasmodium* , notamment une souris C57B1/6 pour *P. berghei,* ou une souris BALB/c pour *P*. *yoelii.*

4. Procédé selon l'une quelconque des revendications 1 à 3, dans lequel l'immunisation comprend l'administration d'une dose de vecteur lentiviral dans une étape d'amorçage et d'une dose de vecteur lentiviral dans une étape de renforcement, dans lequel, dans les étapes d'amorçage et de renforcement, les vecteurs lentiviraux sont pseudotypés avec différentes protéines d'enveloppe sans séroneutralisation croisée et dans lequel les doses d'amorçage et de renforcement sont différentes, en particulier la dose de renforcement est supérieure à la dose d'amorçage ou sont égales.

5. Procédé selon l'une quelconque des revendications 1 à 3, dans lequel les doses administrées de vecteur lentiviral exprimant le polypeptide antigénique pour l'immunisation du mammifère non humain sont chacune dans la gamme de 1x10⁵ à 1x10⁸ TU, en particulier de 1x10⁵ à 1x10⁷ et dans lequel le vecteur lentiviral est formulé sous la forme d'une suspension de vecteur lentiviral concentré ou non concentré.

6. Procédé selon l'une quelconque des revendications 1 à 5, dans lequel on utilise un témoin positif pour la capacité de protection contre l'infection par le parasite *Plasmodium* ou contre l'état ou la maladie induit(e) par le parasite, qui est CSP, ou un antigène choisi dans les antigènes des gamètes, des zygotes ou des ookinètes du parasite, tels que des antigènes du stade sexuel, des antigènes du stade hépatique ou antigènes du stade sanguin asexué, tels que des antigènes des sporozoïtes, en particulier un antigène choisi dans les antigènes P48/P45 Pfs25, MSP1, CSP, TRAP, Celtos, SPECT, ICP, et Facilysin/Bergheilysin Ag11-09, Agl1-10 et TRAP et un témoin négatif pour la capacité de protection est utilisé, qui est GFP, un antigène non protecteur connu ou un vecteur équivalent vide.

7. Procédé selon l'une quelconque des revendications 1 à 6, dans lequel les antisérums dans l'échantillon obtenu à partir du mammifère non humain immunisé ont été incubés avec des sporozoïtes de *Plasmodium* exprimant la GFP, dans lequel un sous-groupe de ces sporozoïtes est perméabilisé et un sous-groupe de ces sporozoïtes n'est pas perméabilisé.

8. Procédé selon l'une quelconque des revendications 1 à 7, dans lequel le vecteur lentiviral contient dans son génome un gène synthétique pour un polypeptide antigénique de *Plasmodium* dont la séquence codante est optimisée en codon pour l'expression du polypeptide antigénique dans des cellules de mammifères et dans lequel l'expression du polypeptide antigénique est dirigée par un promoteur approprié pour diriger l'expression génique dans divers types de cellules de mammifères, y compris les cellules dendritiques, tel qu'un promoteur de la bêta-2 microglobuline.

9. Procédé selon l'une quelconque des revendications 1 à 8 dans lequel le génome du vecteur lentiviral est obtenu à partir d'un vecteur de transfert qui est un plasmide pTRIP dans lequel un acide nucléique synthétique de *Plasmodium,* en particulier un acide nucléique optimisé en codon pour les mammifères, codant pour le polypeptide antigénique à tester, a été cloné sous le contrôle d'un promoteur fonctionnel dans les cellules de mammifères, en particulier le promoteur de la bêta-2 microglobuline humaine, et éventuellement sous le contrôle de l'élément régulateur post-transcriptionnel du virus de l'hépatite de la marmotte (VVPRE).

10. Procédé selon l'une quelconque des revendications 2 à 9, dans lequel la protéine d'enveloppe pseudotypant le vecteur lentiviral est une protéine d'enveloppe de VSV-G provenant de la souche Indiana, de la souche New Jersey ou de toute autre souche ne présentant pas de neutralisation croisée, telle que Cocal et Chandipura.

11. Procédé selon l'une quelconque des revendications 1 à 10, dans lequel la présélection du polypeptide antigénique candidat inclut l'étape de profilage du transcriptome du parasite Plasmodium pour le stade de développement pré-érythrocytaire comparé au profilage du transcriptome du parasite Plasmodium pour un stade de développement différent, en particulier le stade de développement sanguin dudit parasite Plasmodium, et d'identification des transcrits sur-exprimés dans le parasite de stade pré-érythrocytaire.

12. Procédé d'identification d'un polypeptide antigénique pour la conception d'un candidat vaccin contre le parasite du paludisme pour la protection d'un hôte humain, qui comprend les étapes de criblage des polypeptides antigéniques conformément à un procédé selon l'une quelconque des revendications 1 à 11, et de détermination du gène orthologue dans un parasite Plasmodium infectant un hôte humain, en particulier *Plasmodium falciparum, Plasmodium vivax, Plasmodium malariae, Plasmodium ovale* ou *Plasmodium knowlesi.*


### SEQUENCE LISTING

<110> INSTITUT PASTEUR
   CENTRE NATIONAL DE LA RECHERCHE SCIENTIFIQUE
<120> Functional screening of antigenic polypeptides - use for the identification of antigens eliciting a protective immune response and for the selection of antigens with optimal protective activity.
<130> B12095A-AD/BA
<140> PCT/EP2018/XXXXXX
   <141> 2018-02-01
<150> EP17305121.0
   <151> 2017-02-02
<160> 94
<170> PatentIn version 3.5
<210> 1
   <211> 4536
   <212> DNA
   <213> Artificial Sequence
<220>
   <223> pTRIP CMV GFP
<400> 1
   tggaagggct aattcactcc caacgaagac aagatatcct tgatctgtgg atctaccaca      60
   cacaaggcta cttccctgat tagcagaact acacaccagg gccagggatc agatatccac     120
   tgacctttgg atggtgctac aagctagtac cagttgagcc agagaagtta gaagaagcca     180
   acaaaggaga gaacaccagc ttgttacaac ctgtgagcct gcatgggatg gatgacccgg     240
   agagagaagt gttagagtgg aggtttgaca gccgcctagc atttcatcac ggtggcccga     300
   gagctgcatc cggagtactt caagaactgc tgatatcgag cttgctacaa gggactttcc     360
   gctgggggac tttccaggga ggcgtggcct gggcgggact ggggagtggc gagccctcag     420
   atcctgcata taagcagctg ctttttgcct gtactgggtc tctctggtta gaccagatct     480
   gagcctggga gctctctggc taactaggga acccactgct taagcctcaa taaagcttgc     540
   cttgagtgct tcaagtagtg tgtgcccgtc tgttgtgtga ctctggtaac tagagatccc     600
   tcagaccctt ttagtcagtg tggaaaatct ctagcagtgg cgcccgaaca gggacttgaa     660
   agcgaaaggg aaaccagagg agctctctcg acgcaggact cggcttgctg aagcgcgcac     720
   ggcaagaggc gaggggcggc gactggtgag tacgccaaaa attttgacta gcggaggcta     780
   gaaggagaga gatgggtgcg agagcgtcag tattaagcgg gggagaatta gatcgcgatg     840
   ggaaaaaatt cggttaaggc cagggggaaa gaaaaaatat aaattaaaac atatagtatg     900
   ggcaagcagg gagctagaac gattcgcagt taatcctggc ctgttagaaa catcagaagg 960
   ctgtagacaa atactgggac agctacaacc atcccttcag acaggatcag aagaacttag 1020
   atcattatat aatacagtag caaccctcta ttgtgtgcat caaaggatag agataaaaga 1080
   caccaaggaa gctttagaca agatagagga agagcaaaac aaaagtaaga ccaccgcaca 1140
   gcaagcggcc gctgatcttc agacctggag gaggagatat gagggacaat tggagaagtg 1200
   aattatataa atataaagta gtaaaaattg aaccattagg agtagcaccc accaaggcaa 1260
   agagaagagt ggtgcagaga gaaaaaagag cagtgggaat aggagctttg ttccttgggt 1320
   tcttgggagc agcaggaagc actatgggcg cagcgtcaat gacgctgacg gtacaggcca 1380
   gacaattatt gtctggtata gtgcagcagc agaacaattt gctgagggct attgaggcgc 1440
   aacagcatct gttgcaactc acagtctggg gcatcaagca gctccaggca agaatcctgg 1500
   ctgtggaaag atacctaaag gatcaacagc tcctggggat ttggggttgc tctggaaaac 1560
   tcatttgcac cactgctgtg ccttggaatg ctagttggag taataaatct ctggaacaga 1620
   tttggaatca cacgacctgg atggagtggg acagagaaat taacaattac acaagcttaa 1680
   tacactcctt aattgaagaa tcgcaaaacc agcaagaaaa gaatgaacaa gaattattgg 1740
   aattagataa atgggcaagt ttgtggaatt ggtttaacat aacaaattgg ctgtggtata 1800
   taaaattatt cataatgata gtaggaggct tggtaggttt aagaatagtt tttgctgtac 1860
   tttctatagt gaatagagtt aggcagggat attcaccatt atcgtttcag acccacctcc 1920
   caaccccgag gggacccgac aggcccgaag gaatagaaga agaaggtgga gagagagaca 1980
   gagacagatc cattcgatta gtgaacggat ctcgacggta tcgccgaatt cacaaatggc 2040
   agtattcatc cacaatttta aaagaaaagg ggggattggg gggtacagtg caggggaaag 2100
   aatagtagac ataatagcaa cagacataca aactaaagaa ttacaaaaac aaattacaaa 2160
   aattcaaaat tttcgggttt attacaggga cagcagagat ccactttggc tgatacgcgt 2220
   ggagttccgc gttacataac ttacggtaaa tggcccgcct ggctgaccgc ccaacgaccc 2280
   ccgcccattg acgtcaataa tgacgtatgt tcccatagta acgccaatag ggactttcca 2340
   ttgacgtcaa tgggtggagt atttacggta aactgcccac ttggcagtac atcaagtgta 2400
   tcatatgcca agtacgcccc ctattgacgt caatgacggt aaatggcccg cctggcatta 2460
   tgcccagtac atgaccttat gggactttcc tacttggcag tacatctacg tattagtcat 2520
   cgctattacc atggtgatgc ggttttggca gtacatcaat gggcgtggat agcggtttga 2580
   ctcacgggga tttccaagtc tccaccccat tgacgtcaat gggagtttgt tttggcacca 2640
   aaatcaacgg gactttccaa aatgtcgtaa caactccgcc ccattgacgc aaatgggcgg 2700
   taggcgtgta cggtgggagg tctatataag cagagctcgt ttagtgaacc gtcagatcgc 2760
   ctggagacgc catccacgct gttttgacct ccatagaaga caccgcgatc ggatccccac 2820
   cggtcgccac catggtgagc aagggcgagg agctgttcac cggggtggtg cccatcctgg 2880
   tcgagctgga cggcgacgta aacggccaca agttcagcgt gtccggcgag ggcgagggcg 2940
   atgccaccta cggcaagctg accctgaagt tcatctgcac caccggcaag ctgcccgtgc 3000
   cctggcccac cctcgtgacc accctgacct acggcgtgca gtgcttcagc cgctaccccg 3060
   accacatgaa gcagcacgac ttcttcaagt ccgccatgcc cgaaggctac gtccaggagc 3120
   gcaccatctt cttcaaggac gacggcaact acaagacccg cgccgaggtg aagttcgagg 3180
   gcgacaccct ggtgaaccgc atcgagctga agggcatcga cttcaaggag gacggcaaca 3240
   tcctggggca caagctggag tacaactaca acagccacaa cgtctatatc atggccgaca 3300
   agcagaagaa cggcatcaag gtgaacttca agatccgcca caacatcgag gacggcagcg 3360
   tgcagctcgc cgaccactac cagcagaaca cccccatcgg cgacggcccc gtgctgctgc 3420
   ccgacaacca ctacctgagc acccagtccg ccctgagcaa agaccccaac gagaagcgcg 3480
   atcacatggt cctgctggag ttcgtgaccg ccgccgggat cactctcggc atggacgagc 3540
   tgtacaagta aagcggccgc gactctagct cgagctcaag cttcgaattc ccgataatca 3600
   acctctggat tacaaaattt gtgaaagatt gactggtatt cttaactatg ttgctccttt 3660
   tacgctatgt ggatacgctg ctttaatgcc tttgtatcat gctattgctt cccgtatggc 3720
   tttcattttc tcctccttgt ataaatcctg gttgctgtct ctttatgagg agttgtggcc 3780
   cgttgtcagg caacgtggcg tggtgtgcac tgtgtttgct gacgcaaccc ccactggttg 3840
   gggcattgcc accacctgtc agctcctttc cgggactttc gctttccccc tccctattgc 3900
   cacggcggaa ctcatcgccg cctgccttgc ccgctgctgg acaggggctc ggctgttggg 3960
   cactgacaat tccgtggtgt tgtcggggaa gctgacgtcc tttccatggc tgctcgcctg 4020
   tgttgccacc tggattctgc gcgggacgtc cttctgctac gtcccttcgg ccctcaatcc 4080
   agcggacctt ccttcccgcg gcctgctgcc ggctctgcgg cctcttccgc gtcttcgcct 4140
   tcgccctcag acgagtcgga tctccctttg ggccgcctcc ccgcatcggg aattctgcag 4200
   tcgacggtac ctttaagacc aatgacttac aaggcagctg tagatcttag ccacttttta 4260
   aaagaaaagg ggggactgga agggctaatt cactcccaac gaagacaaga tcgtcgagag 4320
   atgctgcata taagcagctg ctttttgctt gtactgggtc tctctggtta gaccagatct 4380
   gagcctggga gctctctggc taactaggga acccactgct taagcctcaa taaagcttgc 4440
   cttgagtgct tcaagtagtg tgtgcccgtc tgttgtgtga ctctggtaac tagagatccc 4500
   tcagaccctt ttagtcagtg tggaaaatct ctagca 4536
<210> 2
   <211> 4468
   <212> DNA
   <213> Artificial Sequence
<220>
   <223> pTRIP B2M GFP
<400> 2
   tggaagggct aattcactcc caacgaagac aagatatcct tgatctgtgg atctaccaca        60
   cacaaggcta cttccctgat tagcagaact acacaccagg gccagggatc agatatccac       120
   tgacctttgg atggtgctac aagctagtac cagttgagcc agagaagtta gaagaagcca       180
   acaaaggaga gaacaccagc ttgttacaac ctgtgagcct gcatgggatg gatgacccgg       240
   agagagaagt gttagagtgg aggtttgaca gccgcctagc atttcatcac ggtggcccga       300
   gagctgcatc cggagtactt caagaactgc tgatatcgag cttgctacaa gggactttcc       360
   gctgggggac tttccaggga ggcgtggcct gggcgggact ggggagtggc gagccctcag       420
   atcctgcata taagcagctg ctttttgcct gtactgggtc tctctggtta gaccagatct       480
   gagcctggga gctctctggc taactaggga acccactgct taagcctcaa taaagcttgc       540
   cttgagtgct tcaagtagtg tgtgcccgtc tgttgtgtga ctctggtaac tagagatccc       600
   tcagaccctt ttagtcagtg tggaaaatct ctagcagtgg cgcccgaaca gggacttgaa       660
   agcgaaaggg aaaccagagg agctctctcg acgcaggact cggcttgctg aagcgcgcac       720
   ggcaagaggc gaggggcggc gactggtgag tacgccaaaa attttgacta gcggaggcta       780
   gaaggagaga gatgggtgcg agagcgtcag tattaagcgg gggagaatta gatcgcgatg       840
   ggaaaaaatt cggttaaggc cagggggaaa gaaaaaatat aaattaaaac atatagtatg       900
   ggcaagcagg gagctagaac gattcgcagt taatcctggc ctgttagaaa catcagaagg       960
   ctgtagacaa atactgggac agctacaacc atcccttcag acaggatcag aagaacttag      1020
   atcattatat aatacagtag caaccctcta ttgtgtgcat caaaggatag agataaaaga      1080
   caccaaggaa gctttagaca agatagagga agagcaaaac aaaagtaaga ccaccgcaca      1140
   gcaagcggcc gctgatcttc agacctggag gaggagatat gagggacaat tggagaagtg      1200
   aattatataa atataaagta gtaaaaattg aaccattagg agtagcaccc accaaggcaa      1260
   agagaagagt ggtgcagaga gaaaaaagag cagtgggaat aggagctttg ttccttgggt      1320
   tcttgggagc agcaggaagc actatgggcg cagcgtcaat gacgctgacg gtacaggcca      1380
   gacaattatt gtctggtata gtgcagcagc agaacaattt gctgagggct attgaggcgc      1440
   aacagcatct gttgcaactc acagtctggg gcatcaagca gctccaggca agaatcctgg      1500
   ctgtggaaag atacctaaag gatcaacagc tcctggggat ttggggttgc tctggaaaac      1560
   tcatttgcac cactgctgtg ccttggaatg ctagttggag taataaatct ctggaacaga      1620
   tttggaatca cacgacctgg atggagtggg acagagaaat taacaattac acaagcttaa      1680
   tacactcctt aattgaagaa tcgcaaaacc agcaagaaaa gaatgaacaa gaattattgg      1740
   aattagataa atgggcaagt ttgtggaatt ggtttaacat aacaaattgg ctgtggtata 1800
   taaaattatt cataatgata gtaggaggct tggtaggttt aagaatagtt tttgctgtac 1860
   tttctatagt gaatagagtt aggcagggat attcaccatt atcgtttcag acccacctcc 1920
   caaccccgag gggacccgac aggcccgaag gaatagaaga agaaggtgga gagagagaca 1980
   gagacagatc cattcgatta gtgaacggat ctcgacggta tcgccgaatt cacaaatggc 2040
   agtattcatc cacaatttta aaagaaaagg ggggattggg gggtacagtg caggggaaag 2100
   aatagtagac ataatagcaa cagacataca aactaaagaa ttacaaaaac aaattacaaa 2160
   aattcaaaat tttcgggttt attacaggga cagcagagat ccactttggc tgatacgcgc 2220
   cggaaaccct gcagggaatt ccccagctgt agttataaac agaagttctc cttctgctag 2280
   gtagcattca aagatcttaa tcttctgggt ttccgttttc tcgaatgaaa aatgcaggtc 2340
   cgagcagtta actggcgggg gcaccattag caagtcactt agcatctctg gggccagtct 2400
   gcaaagcgag ggggcagcct taatgtgcct ccagcctgaa gtcctagaat gagcgcccgg 2460
   tgtcccaagc tggggcgcgc accccagatc ggagggcgcc gatgtacaga cagcaaactc 2520
   acccagtcta gtgcatgcct tcttaaacat cacgagactc taagaaaagg aaactgaaaa 2580
   cgggaaagtc cctctctcta acctggcact gcgtcgctgg cttggagaca ggtgacggtc 2640
   cctgcgggcc ttgtcctgat tggctgggca cgcgtttaat ataagtggag gcgtcgcgct 2700
   ggcgggcatt cctgaagctg acagcattcg ggccgagcga tcggatcccc accggtcgcc 2760
   accatggtga gcaagggcga ggagctgttc accggggtgg tgcccatcct ggtcgagctg 2820
   gacggcgacg taaacggcca caagttcagc gtgtccggcg agggcgaggg cgatgccacc 2880
   tacggcaagc tgaccctgaa gttcatctgc accaccggca agctgcccgt gccctggccc 2940
   accctcgtga ccaccctgac ctacggcgtg cagtgcttca gccgctaccc cgaccacatg 3000
   aagcagcacg acttcttcaa gtccgccatg cccgaaggct acgtccagga gcgcaccatc 3060
   ttcttcaagg acgacggcaa ctacaagacc cgcgccgagg tgaagttcga gggcgacacc 3120
   ctggtgaacc gcatcgagct gaagggcatc gacttcaagg aggacggcaa catcctgggg 3180
   cacaagctgg agtacaacta caacagccac aacgtctata tcatggccga caagcagaag 3240
   aacggcatca aggtgaactt caagatccgc cacaacatcg aggacggcag cgtgcagctc 3300
   gccgaccact accagcagaa cacccccatc ggcgacggcc ccgtgctgct gcccgacaac 3360
   cactacctga gcacccagtc cgccctgagc aaagacccca acgagaagcg cgatcacatg 3420
   gtcctgctgg agttcgtgac cgccgccggg atcactctcg gcatggacga gctgtacaag 3480
   taaagcggcc gcgactctag ctcgagctca agcttcgaat tcccgataat caacctctgg 3540
   attacaaaat ttgtgaaaga ttgactggta ttcttaacta tgttgctcct tttacgctat 3600
   gtggatacgc tgctttaatg cctttgtatc atgctattgc ttcccgtatg gctttcattt 3660
   tctcctcctt gtataaatcc tggttgctgt ctctttatga ggagttgtgg cccgttgtca 3720
   ggcaacgtgg cgtggtgtgc actgtgtttg ctgacgcaac ccccactggt tggggcattg 3780
   ccaccacctg tcagctcctt tccgggactt tcgctttccc cctccctatt gccacggcgg 3840
   aactcatcgc cgcctgcctt gcccgctgct ggacaggggc tcggctgttg ggcactgaca 3900
   attccgtggt gttgtcgggg aagctgacgt cctttccatg gctgctcgcc tgtgttgcca 3960
   cctggattct gcgcgggacg tccttctgct acgtcccttc ggccctcaat ccagcggacc 4020
   ttccttcccg cggcctgctg ccggctctgc ggcctcttcc gcgtcttcgc cttcgccctc 4080
   agacgagtcg gatctccctt tgggccgcct ccccgcatcg ggaattctgc agtcgacggt 4140
   acctttaaga ccaatgactt acaaggcagc tgtagatctt agccactttt taaaagaaaa 4200
   ggggggactg gaagggctaa ttcactccca acgaagacaa gatcgtcgag agatgctgca 4260
   tataagcagc tgctttttgc ttgtactggg tctctctggt tagaccagat ctgagcctgg 4320
   gagctctctg gctaactagg gaacccactg cttaagcctc aataaagctt gccttgagtg 4380
   cttcaagtag tgtgtgcccg tctgttgtgt gactctggta actagagatc cctcagaccc 4440
   ttttagtcag tgtggaaaat ctctagca 4468
<210> 3
   <211> 11904
   <212> DNA
   <213> Artificial Sequence
<220>
   <223> PACKAGING 8.74 PLASMID
<400> 3
   gggctgcagg aattaattcg agctcgcccg acattgatta ttgactagtt attaatagta 60
   atcaattacg gggtcattag ttcatagccc atatatggag ttccgcgtta cataacttac 120
   ggtaaatggc ccgcctggct gaccgcccaa cgacccccgc ccattgacgt caataatgac 180
   gtatgttccc atagtaacgc caatagggac tttccattga cgtcaatggg tggagtattt 240
   acggtaaact gcccacttgg cagtacatca agtgtatcat atgccaagta cgccccctat 300
   tgacgtcaat gacggtaaat ggcccgcctg gcattatgcc cagtacatga ccttatggga 360
   ctttcctact tggcagtaca tctacgtatt agtcatcgct attaccatgg tgatgcggtt 420
   ttggcagtac atcaatgggc gtggatagcg gtttgactca cggggatttc caagtctcca 480
   ccccattgac gtcaatggga gtttgttttg gcaccaaaat caacgggact ttccaaaatg 540
   tcgtaacaac tccgccccat tgacgcaaat gggcggtagg cgtgtacggt gggaggtcta 600
   tataagcaga gctcgtttag tgaaccgtca gatcgcctgg agacgccatc cacgctgttt 660
   tgacctccat agaagacacc gggaccgatc cagcctccgc ggccgcgttg acgcgcacgg 720
   caagaggcga ggggcggcga ctggtgagag atgggtgcga gagcgtcagt attaagcggg 780
   ggagaattag atcgatggga aaaaattcgg ttaaggccag ggggaaagaa aaaatataaa 840
   ttaaaacata tagtatgggc aagcagggag ctagaacgat tcgcagttaa tcctggcctg 900
   ttagaaacat cagaaggctg tagacaaata ctgggacagc tacaaccatc ccttcagaca 960
   ggatcagaag aacttagatc attatataat acagtagcaa ccctctattg tgtgcatcaa 1020
   aggatagaga taaaagacac caaggaagct ttagacaaga tagaggaaga gcaaaacaaa 1080
   agtaagaaaa aagcacagca agcagcagct gacacaggac acagcaatca ggtcagccaa 1140
   aattacccta tagtgcagaa catccagggg caaatggtac atcaggccat atcacctaga 1200
   actttaaatg catgggtaaa agtagtagaa gagaaggctt tcagcccaga agtgataccc 1260
   atgttttcag cattatcaga aggagccacc ccacaagatt taaacaccat gctaaacaca 1320
   gtggggggac atcaagcagc catgcaaatg ttaaaagaga ccatcaatga ggaagctgca 1380
   gaatgggata gagtgcatcc agtgcatgca gggcctattg caccaggcca gatgagagaa 1440
   ccaaggggaa gtgacatagc aggaactact agtacccttc aggaacaaat aggatggatg 1500
   acacataatc cacctatccc agtaggagaa atctataaaa gatggataat cctgggatta 1560
   aataaaatag taagaatgta tagccctacc agcattctgg acataagaca aggaccaaag 1620
   gaacccttta gagactatgt agaccgattc tataaaactc taagagccga gcaagcttca 1680
   caagaggtaa aaaattggat gacagaaacc ttgttggtcc aaaatgcgaa cccagattgt 1740
   aagactattt taaaagcatt gggaccagga gcgacactag aagaaatgat gacagcatgt 1800
   cagggagtgg ggggacccgg ccataaagca agagttttgg ctgaagcaat gagccaagta 1860
   acaaatccag ctaccataat gatacagaaa ggcaatttta ggaaccaaag aaagactgtt 1920
   aagtgtttca attgtggcaa agaagggcac atagccaaaa attgcagggc ccctaggaaa 1980
   aagggctgtt ggaaatgtgg aaaggaagga caccaaatga aagattgtac tgagagacag 2040
   gctaattttt tagggaagat ctggccttcc cacaagggaa ggccagggaa ttttcttcag 2100
   agcagaccag agccaacagc cccaccagaa gagagcttca ggtttgggga agagacaaca 2160
   actccctctc agaagcagga gccgatagac aaggaactgt atcctttagc ttccctcaga 2220
   tcactctttg gcagcgaccc ctcgtcacaa taaagatagg ggggcaatta aaggaagctc 2280
   tattagatac aggagcagat gatacagtat tagaagaaat gaatttgcca ggaagatgga     2340
   aaccaaaaat gataggggga attggaggtt ttatcaaagt aagacagtat gatcagatac     2400
   tcatagaaat ctgcggacat aaagctatag gtacagtatt agtaggacct acacctgtca 2460
   acataattgg aagaaatctg ttgactcaga ttggctgcac tttaaatttt cccattagtc 2520
   ctattgagac tgtaccagta aaattaaagc caggaatgga tggcccaaaa gttaaacaat 2580
   ggccattgac agaagaaaaa ataaaagcat tagtagaaat ttgtacagaa atggaaaagg 2640
   aaggaaaaat ttcaaaaatt gggcctgaaa atccatacaa tactccagta tttgccataa 2700
   agaaaaaaga cagtactaaa tggagaaaat tagtagattt cagagaactt aataagagaa 2760
   ctcaagattt ctgggaagtt caattaggaa taccacatcc tgcagggtta aaacagaaaa 2820
   aatcagtaac agtactggat gtgggcgatg catatttttc agttccctta gataaagact 2880
   tcaggaagta tactgcattt accataccta gtataaacaa tgagacacca gggattagat 2940
   atcagtacaa tgtgcttcca cagggatgga aaggatcacc agcaatattc cagtgtagca 3000
   tgacaaaaat cttagagcct tttagaaaac aaaatccaga catagtcatc tatcaataca 3060
   tggatgattt gtatgtagga tctgacttag aaatagggca gcatagaaca aaaatagagg 3120
   aactgagaca acatctgttg aggtggggat ttaccacacc agacaaaaaa catcagaaag 3180
   aacctccatt cctttggatg ggttatgaac tccatcctga taaatggaca gtacagccta 3240
   tagtgctgcc agaaaaggac agctggactg tcaatgacat acagaaatta gtgggaaaat 3300
   tgaattgggc aagtcagatt tatgcaggga ttaaagtaag gcaattatgt aaacttctta 3360
   ggggaaccaa agcactaaca gaagtagtac cactaacaga agaagcagag ctagaactgg 3420
   cagaaaacag ggagattcta aaagaaccgg tacatggagt gtattatgac ccatcaaaag 3480
   acttaatagc agaaatacag aagcaggggc aaggccaatg gacatatcaa atttatcaag 3540
   agccatttaa aaatctgaaa acaggaaagt atgcaagaat gaagggtgcc cacactaatg 3600
   atgtgaaaca attaacagag gcagtacaaa aaatagccac agaaagcata gtaatatggg 3660
   gaaagactcc taaatttaaa ttacccatac aaaaggaaac atgggaagca tggtggacag 3720
   agtattggca agccacctgg attcctgagt gggagtttgt caatacccct cccttagtga 3780
   agttatggta ccagttagag aaagaaccca taataggagc agaaactttc tatgtagatg 3840
   gggcagccaa tagggaaact aaattaggaa aagcaggata tgtaactgac agaggaagac 3900
   aaaaagttgt ccccctaacg gacacaacaa atcagaagac tgagttacaa gcaattcatc 3960
   tagctttgca ggattcggga ttagaagtaa acatagtgac agactcacaa tatgcattgg 4020
   gaatcattca agcacaacca gataagagtg aatcagagtt agtcagtcaa ataatagagc 4080
   agttaataaa aaaggaaaaa gtctacctgg catgggtacc agcacacaaa ggaattggag 4140
   gaaatgaaca agtagataaa ttggtcagtg ctggaatcag gaaagtacta tttttagatg 4200
   gaatagataa ggcccaagaa gaacatgaga aatatcacag taattggaga gcaatggcta 4260
   gtgattttaa cctaccacct gtagtagcaa aagaaatagt agccagctgt gataaatgtc 4320
   agctaaaagg ggaagccatg catggacaag tagactgtag cccaggaata tggcagctag 4380
   attgtacaca tttagaagga aaagttatct tggtagcagt tcatgtagcc agtggatata 4440
   tagaagcaga agtaattcca gcagagacag ggcaagaaac agcatacttc ctcttaaaat 4500
   tagcaggaag atggccagta aaaacagtac atacagacaa tggcagcaat ttcaccagta 4560
   ctacagttaa ggccgcctgt tggtgggcgg ggatcaagca ggaatttggc attccctaca 4620
   atccccaaag tcaaggagta atagaatcta tgaataaaga attaaagaaa attataggac 4680
   aggtaagaga tcaggctgaa catcttaaga cagcagtaca aatggcagta ttcatccaca 4740
   attttaaaag aaaagggggg attggggggt acagtgcagg ggaaagaata gtagacataa 4800
   tagcaacaga catacaaact aaagaattac aaaaacaaat tacaaaaatt caaaattttc 4860
   gggtttatta cagggacagc agagatccag tttggaaagg accagcaaag ctcctctgga 4920
   aaggtgaagg ggcagtagta atacaagata atagtgacat aaaagtagtg ccaagaagaa 4980
   aagcaaagat catcagggat tatggaaaac agatggcagg tgatgattgt gtggcaagta 5040
   gacaggatga ggattaacac atggaattct gcaacaactg ctgtttatcc atttcagaat 5100
   tgggtgtcga catagcagaa taggcgttac tcgacagagg agagcaagaa atggagccag 5160
   tagatcctag actagagccc tggaagcatc caggaagtca gcctaaaact gcttgtacca 5220
   attgctattg taaaaagtgt tgctttcatt gccaagtttg tttcatgaca aaagccttag 5280
   gcatctccta tggcaggaag aagcggagac agcgacgaag agctcatcag aacagtcaga 5340
   ctcatcaagc ttctctatca aagcagtaag tagtacatgt aatgcaacct ataatagtag 5400
   caatagtagc attagtagta gcaataataa tagcaatagt tgtgtggtcc atagtaatca 5460
   tagaatatag gaaaatggcc gctgatcttc agacctggag gaggagatat gagggacaat 5520
   tggagaagtg aattatataa atataaagta gtaaaaattg aaccattagg agtagcaccc 5580
   accaaggcaa agagaagagt ggtgcagaga gaaaaaagag cagtgggaat aggagctttg 5640
   ttccttgggt tcttgggagc agcaggaagc actatgggcg cagcgtcaat gacgctgacg 5700
   gtacaggcca gacaattatt gtctggtata gtgcagcagc agaacaattt gctgagggct 5760
   attgaggcgc aacagcatct gttgcaactc acagtctggg gcatcaagca gctccaggca 5820
   agaatcctgg ctgtggaaag atacctaaag gatcaacagc tcctggggat ttggggttgc 5880
   tctggaaaac tcatttgcac cactgctgtg ccttggaatg ctagttggag taataaatct 5940
   ctggaacaga tttggaatca cacgacctgg atggagtggg acagagaaat taacaattac 6000
   acaagcttaa tacactcctt aattgaagaa tcgcaaaacc agcaagaaaa gaatgaacaa 6060
   gaattattgg aattagataa atgggcaagt ttgtggaatt ggtttaacat aacaaattgg 6120
   ctgtggtata taaaattatt cataatgata gtaggaggct tggtaggttt aagaatagtt 6180
   tttgctgtac tttctatagt gaatagagtt aggcagggat attcaccatt atcgtttcag 6240
   acccacctcc caaccccgag gggacccgac aggcccgaag gaatagaaga agaaggtgga 6300
   gagagagaca gagacagatc cattcgatta gtgaacggat ccttggcact tatctgggac 6360
   gatctgcgga gcctgtgcct cttcagctac caccgcttga gagacttact cttgattgta 6420
   acgaggattg tggaacttct gggacgcagg gggtgggaag ccctcaaata ttggtggaat 6480
   ctcctacaat attggagtca ggagctaaag aatagtgctg ttagcttgct caatgccaca 6540
   gccatagcag tagctgaggg gacagatagg gttatagaag tagtacaagg agcttgtaga 6600
   gctattcgcc acatacctag aagaataaga cagggcttgg aaaggatttt gctataagct 6660
   cgagtgacct tcagaccttg gcactggagg tggcccggca gaagcgcggc atcgtggatc 6720
   agtgctgcac cagcatctgc tctctctacc aactggagaa ctactgcaac taggcccacc 6780
   actaccctgt ccacccctct gcaatgaata aaacctttga aagagcacta caagttgtgt 6840
   gtacatgcgt gcatgtgcat atgtggtgcg gggggaacat gagtggggct ggctggagtg 6900
   gcgatgataa gctgtcaaac atgagaatta attcttgaag acgaaagggc ctcgtgatac 6960
   gcctattttt ataggttaat gtcatgataa taatggtttc ttagtctaga attaattccg 7020
   tgtattctat agtgtcacct aaatcgtatg tgtatgatac ataaggttat gtattaattg 7080
   tagccgcgtt ctaacgacaa tatgtacaag cctaattgtg tagcatctgg cttactgaag 7140
   cagaccctat catctctctc gtaaactgcc gtcagagtcg gtttggttgg acgaaccttc 7200
   tgagtttctg gtaacgccgt cccgcacccg gaaatggtca gcgaaccaat cagcagggtc 7260
   atcgctagcc agatcctcta cgccggacgc atcgtggccg gcatcaccgg cgccacaggt 7320
   gcggttgctg gcgcctatat cgccgacatc accgatgggg aagatcgggc tcgccacttc 7380
   gggctcatga gcgcttgttt cggcgtgggt atggtggcag gccccgtggc cgggggactg 7440
   ttgggcgcca tctccttgca tgcaccattc cttgcggcgg cggtgctcaa cggcctcaac 7500
   ctactactgg gctgcttcct aatgcaggag tcgcataagg gagagcgtcg aatggtgcac 7560
   tctcagtaca atctgctctg atgccgcata gttaagccag ccccgacacc cgccaacacc 7620
   cgctgacgcg ccctgacggg cttgtctgct cccggcatcc gcttacagac aagctgtgac 7680
   cgtctccggg agctgcatgt gtcagaggtt ttcaccgtca tcaccgaaac gcgcgagacg 7740
   aaagggcctc gtgatacgcc tatttttata ggttaatgtc atgataataa tggtttctta 7800
   gacgtcaggt ggcacttttc ggggaaatgt gcgcggaacc cctatttgtt tatttttcta 7860
   aatacattca aatatgtatc cgctcatgag acaataaccc tgataaatgc ttcaataata 7920
   ttgaaaaagg aagagtatga gtattcaaca tttccgtgtc gcccttattc ccttttttgc 7980
   ggcattttgc cttcctgttt ttgctcaccc agaaacgctg gtgaaagtaa aagatgctga 8040
   agatcagttg ggtgcacgag tgggttacat cgaactggat ctcaacagcg gtaagatcct 8100
   tgagagtttt cgccccgaag aacgttttcc aatgatgagc acttttaaag ttctgctatg 8160
   tggcgcggta ttatcccgta ttgacgccgg gcaagagcaa ctcggtcgcc gcatacacta 8220
   ttctcagaat gacttggttg agtactcacc agtcacagaa aagcatctta cggatggcat 8280
   gacagtaaga gaattatgca gtgctgccat aaccatgagt gataacactg cggccaactt 8340
   acttctgaca acgatcggag gaccgaagga gctaaccgct tttttgcaca acatggggga 8400
   tcatgtaact cgccttgatc gttgggaacc ggagctgaat gaagccatac caaacgacga 8460
   gcgtgacacc acgatgcctg tagcaatggc aacaacgttg cgcaaactat taactggcga 8520
   actacttact ctagcttccc ggcaacaatt aatagactgg atggaggcgg ataaagttgc 8580
   aggaccactt ctgcgctcgg cccttccggc tggctggttt attgctgata aatctggagc 8640
   cggtgagcgt gggtctcgcg gtatcattgc agcactgggg ccagatggta agccctcccg 8700
   tatcgtagtt atctacacga cggggagtca ggcaactatg gatgaacgaa atagacagat 8760
   cgctgagata ggtgcctcac tgattaagca ttggtaactg tcagaccaag tttactcata 8820
   tatactttag attgatttaa aacttcattt ttaatttaaa aggatctagg tgaagatcct 8880
   ttttgataat ctcatgacca aaatccctta acgtgagttt tcgttccact gagcgtcaga 8940
   ccccgtagaa aagatcaaag gatcttcttg agatcctttt tttctgcgcg taatctgctg 9000
   cttgcaaaca aaaaaaccac cgctaccagc ggtggtttgt ttgccggatc aagagctacc 9060
   aactcttttt ccgaaggtaa ctggcttcag cagagcgcag ataccaaata ctgttcttct 9120
   agtgtagccg tagttaggcc accacttcaa gaactctgta gcaccgccta catacctcgc 9180
   tctgctaatc ctgttaccag tggctgctgc cagtggcgat aagtcgtgtc ttaccgggtt 9240
   ggactcaaga cgatagttac cggataaggc gcagcggtcg ggctgaacgg ggggttcgtg 9300
   cacacagccc agcttggagc gaacgaccta caccgaactg agatacctac agcgtgagct 9360
   atgagaaagc gccacgcttc ccgaagggag aaaggcggac aggtatccgg taagcggcag 9420
   ggtcggaaca ggagagcgca cgagggagct tccaggggga aacgcctggt atctttatag 9480
   tcctgtcggg tttcgccacc tctgacttga gcgtcgattt ttgtgatgct cgtcaggggg 9540
   gcggagccta tggaaaaacg ccagcaacgc ggccttttta cggttcctgg ccttttgctg 9600
   gccttttgct cacatgttct ttcctgcgtt atcccctgat tctgtggata accgtattac 9660
   cgcctttgag tgagctgata ccgctcgccg cagccgaacg accgagcgca gcgagtcagt 9720
   gagcgaggaa gcggaagagc gcccaatacg caaaccgcct ctccccgcgc gttggccgat 9780
   tcattaatgc agctgtggaa tgtgtgtcag ttagggtgtg gaaagtcccc aggctcccca 9840
   gcaggcagaa gtatgcaaag catgcatctc aattagtcag caaccaggtg tggaaagtcc 9900
   ccaggctccc cagcaggcag aagtatgcaa agcatgcatc tcaattagtc agcaaccata 9960
   gtcccgcccc taactccgcc catcccgccc ctaactccgc ccagttccgc ccattctccg 10020
   ccccatggct gactaatttt ttttatttat gcagaggccg aggccgcctc ggcctctgag 10080
   ctattccaga agtagtgagg aggctttttt ggaggcctag gcttttgcaa aaagcttgga 10140
   cacaagacag gcttgcgaga tatgtttgag aataccactt tatcccgcgt cagggagagg 10200
   cagtgcgtaa aaagacgcgg actcatgtga aatactggtt tttagtgcgc cagatctcta 10260
   taatctcgcg caacctattt tcccctcgaa cactttttaa gccgtagata aacaggctgg 10320
   gacacttcac atgagcgaaa aatacatcgt cacctgggac atgttgcaga tccatgcacg 10380
   taaactcgca agccgactga tgccttctga acaatggaaa ggcattattg ccgtaagccg 10440
   tggcggtctg taccgggtgc gttactggcg cgtgaactgg gtattcgtca tgtcgatacc 10500
   gtttgtattt ccagctacga tcacgacaac cagcgcgagc ttaaagtgct gaaacgcgca 10560
   gaaggcgatg gcgaaggctt catcgttatt gatgacctgg tggataccgg tggtactgcg 10620
   gttgcgattc gtgaaatgta tccaaaagcg cactttgtca ccatcttcgc aaaaccggct 10680
   ggtcgtccgc tggttgatga ctatgttgtt gatatcccgc aagatacctg gattgaacag 10740
   ccgtgggata tgggcgtcgt attcgtcccg ccaatctccg gtcgctaatc ttttcaacgc 10800
   ctggcactgc cgggcgttgt tctttttaac ttcaggcggg ttacaatagt ttccagtaag 10860
   tattctggag gctgcatcca tgacacaggc aaacctgagc gaaaccctgt tcaaaccccg 10920
   ctttaaacat cctgaaacct cgacgctagt ccgccgcttt aatcacggcg cacaaccgcc 10980
   tgtgcagtcg gcccttgatg gtaaaaccat ccctcactgg tatcgcatga ttaaccgtct 11040
   gatgtggatc tggcgcggca ttgacccacg cgaaatcctc gacgtccagg cacgtattgt 11100
   gatgagcgat gccgaacgta ccgacgatga tttatacgat acggtgattg gctaccgtgg 11160
   cggcaactgg atttatgagt gggccccgga tctttgtgaa ggaaccttac ttctgtggtg 11220
   tgacataatt ggacaaacta cctacagaga tttaaagctc taaggtaaat ataaaatttt 11280
   taagtgtata atgtgttaaa ctactgattc taattgtttg tgtattttag attccaacct 11340
   atggaactga tgaatgggag cagtggtgga atgcctttaa tgaggaaaac ctgttttgct 11400
   cagaagaaat gccatctagt gatgatgagg ctactgctga ctctcaacat tctactcctc 11460
   caaaaaagaa gagaaaggta gaagacccca aggactttcc ttcagaattg ctaagttttt 11520
   tgagtcatgc tgtgtttagt aatagaactc ttgcttgctt tgctatttac accacaaagg 11580
   aaaaagctgc actgctatac aagaaaatta tggaaaaata ttctgtaacc tttataagta 11640
   ggcataacag ttataatcat aacatactgt tttttcttac tccacacagg catagagtgt 11700
   ctgctattaa taactatgct caaaaattgt gtacctttag ctttttaatt tgtaaagggg 11760
   ttaataagga atatttgatg tatagtgcct tgactagaga tcataatcag ccataccaca 11820
   tttgtagagg ttttacttgc tttaaaaaac ctcccacacc tccccctgaa cctgaaacat 11880
   aaaatgaatg caattgttgt tgtt 11904
<210> 4
   <211> 5140
   <212> DNA
   <213> Artificial Sequence
<220>
   <223> pCMV-VSV-INDco
<400> 4
   ctggatggct ttctcgccgc caaggatctg atggcgcagg ggatcaagct ctgatcaaga 60
   gacaggatga ggatcgtttc gcatgattga acaagatgga ttgcacgcag gttctccggc 120
   cgcttgggtg gagaggctat tcggctatga ctgggcacaa cagacaatcg gctgctctga 180
   tgccgccgtg ttccggctgt cagcgcaggg gcgcccggtt ctttttgtca agaccgacct 240
   gtccggtgcc ctgaatgaac tgcaagacga ggcagcgcgg ctatcgtggc tggccacgac 300
   gggcgttcct tgcgcagctg tgctcgacgt tgtcactgaa gcgggaaggg actggctgct 360
   attgggcgaa gtgccggggc aggatctcct gtcatctcac cttgctcctg ccgagaaagt 420
   atccatcatg gctgatgcaa tgcggcggct gcatacgctt gatccggcta cctgcccatt 480
   cgaccaccaa gcgaaacatc gcatcgagcg agcacgtact cggatggaag ccggtcttgt 540
   cgatcaggat gatctggacg aagagcatca ggggctcgcg ccagccgaac tgttcgccag 600
   gctcaaggcg agcatgcccg acggcgagga tctcgtcgtg acccatggcg atgcctgctt 660
   gccgaatatc atggtggaaa atggccgctt ttctggattc atcgactgtg gccggctggg 720
   tgtggcggac cgctatcagg acatagcgtt ggctacccgt gatattgctg aagagcttgg 780
   cggcgaatgg gctgaccgct tcctcgtgct ttacggtatc gccgctcccg attcgcagcg 840
   catcgccttc tatcgccttc ttgacgagtt cttctgaatt attaacgctt acaatttcct 900
   gatgcggtat tttctcctta cgcatctgtg cggtatttca caccgcatac aggtggcact 960
   tttcggggaa atgtgcgcgg aacccctatt tgtttatttt tctaaataca ttcaaatatg 1020
   tatccgctca tgagacaata accctgataa atgcttcaat aatagcacgt gctaaaactt 1080
   catttttaat ttaaaaggat ctaggtgaag atcctttttg ataatctcat gaccaaaatc 1140
   ccttaacgtg agttttcgtt ccactgagcg tcagaccccg tagaaaagat caaaggatct 1200
   tcttgagatc ctttttttct gcgcgtaatc tgctgcttgc aaacaaaaaa accaccgcta 1260
   ccagcggtgg tttgtttgcc ggatcaagag ctaccaactc tttttccgaa ggtaactggc 1320
   ttcagcagag cgcagatacc aaatactgtc cttctagtgt agccgtagtt aggccaccac 1380
   ttcaagaact ctgtagcacc gcctacatac ctcgctctgc taatcctgtt accagtggct 1440
   gctgccagtg gcgataagtc gtgtcttacc gggttggact caagacgata gttaccggat 1500
   aaggcgcagc ggtcgggctg aacggggggt tcgtgcacac agcccagctt ggagcgaacg 1560
   acctacaccg aactgagata cctacagcgt gagctatgag aaagcgccac gcttcccgaa 1620
   gggagaaagg cggacaggta tccggtaagc ggcagggtcg gaacaggaga gcgcacgagg 1680
   gagcttccag ggggaaacgc ctggtatctt tatagtcctg tcgggtttcg ccacctctga 1740
   cttgagcgtc gatttttgtg atgctcgtca ggggggcgga gcctatggaa aaacgccagc 1800
   aacgcggcct ttttacggtt cctgggcttt tgctggcctt ttgctcacat gttcttgact 1860
   cttcgcgatg tacgggccag atatacgcgt tgacattgat tattgactag ttattaatag 1920
   taatcaatta cggggtcatt agttcatagc ccatatatgg agttccgcgt tacataactt 1980
   acggtaaatg gcccgcctgg ctgaccgccc aacgaccccc gcccattgac gtcaataatg 2040
   acgtatgttc ccatagtaac gccaataggg actttccatt gacgtcaatg ggtggactat 2100
   ttacggtaaa ctgcccactt ggcagtacat caagtgtatc atatgccaag tacgccccct 2160
   attgacgtca atgacggtaa atggcccgcc tggcattatg cccagtacat gaccttatgg 2220
   gactttccta cttggcagta catctacgta ttagtcatcg ctattaccat ggtgatgcgg 2280
   ttttggcagt acatcaatgg gcgtggatag cggtttgact cacggggatt tccaagtctc 2340
   caccccattg acgtcaatgg gagtttgttt tggcaccaaa atcaacggga ctttccaaaa 2400
   tgtcgtaaca actccgcccc attgacgcaa atgggcggta ggcgtgtacg gtgggaggtc 2460
   tatataagca gagctctctg gctaactaga gaacccactg cttactggct tatcgaaatt 2520
   aatacgactc actataggga gacccaagct ggctagcgtt taaacttaag cttggtaccg 2580
   agctcggatc ctgatcagcc accatgaaat gcctgctcta tctggccttc ctgttcatcg 2640
   gcgtgaactg caagttcacc atcgtgttcc cccacaacca gaagggcaac tggaagaacg 2700
   tgcccagcaa ctaccactac tgccccagca gcagcgacct gaactggcac aacgacctga 2760
   tcggcaccgc cctgcaggtg aagatgccca agagccacaa ggccattcag gctgatggct 2820
   ggatgtgtca tgccagcaag tgggtgacca cctgcgactt ccggtggtac ggccccaagt 2880
   acatcaccca cagcatccgc agcttcaccc ccagcgtgga gcagtgcaaa gagagcatcg 2940
   agcagaccaa gcagggcacc tggctgaacc ccggcttccc cccccagtcc tgcggctacg 3000
   ccaccgtgac cgacgccgag gccgtgatcg tgcaggtgac cccccaccac gtgctggtcg 3060
   acgagtacac cggcgagtgg gtggacagcc agttcatcaa cggcaagtgc agcaactaca 3120
   tctgccctac cgtgcacaac agcaccacct ggcacagcga ctacaaggtg aaaggcctgt 3180
   gcgacagcaa cctgatcagc atggacatca cctttttcag cgaggacggc gagctgtcca 3240
   gcctgggcaa agagggcacc ggcttcagaa gcaactactt cgcctacgag acaggcggca 3300
   aggcctgcaa gatgcagtac tgcaagcact ggggcgtgcg gctgcctagc ggcgtgtggt 3360
   tcgagatggc cgacaaggac ctgttcgccg ctgcccggtt ccctgagtgc cccgagggca 3420
   gcagcatcag cgcccccagc cagaccagcg tggacgtgag cctgatccag gacgtggaga 3480
   gaatcctgga ctacagcctg tgccaggaaa cctggtccaa gatcagagcc ggcctgccca 3540
   tcagccccgt ggacctgagc tacctggccc ccaagaaccc cggcaccggc ccagccttca 3600
   ccatcatcaa tggcaccctg aagtacttcg agacacggta catcagagtg gacattgccg 3660
   cccctatcct gagccggatg gtgggcatga tcagcggcac caccaccgag cgggagctgt 3720
   gggacgactg ggccccctac gaggatgtgg agatcggccc caacggcgtg ctgcggacca 3780
   gcagcggcta caagttcccc ctgtacatga tcggccacgg catgctggac agcgacctgc 3840
   acctgagcag caaggcccag gtgttcgagc acccccacat ccaggacgcc gccagccagc 3900
   tgcccgacga cgagagcctg ttcttcggcg acaccggcct gagcaagaac cccatcgaac 3960
   tggtggaggg ctggttcagc agctggaaga gcagcattgc cagctttttc ttcatcatcg 4020
   gcctgatcat cgggctgttt ctggtgctga gagtgggcat ccacctgtgc atcaagctga 4080
   agcacaccaa gaagcggcag atctacaccg acatcgagat gaatcgcctg gggaagtaag 4140
   aattctgcag atatccagca cagtggcggc cgctcgagtg tacaaattcc cgataatcaa 4200
   cctctggatt acaaaatttg tgaaagattg actggtattc ttaactatgt tgctcctttt 4260
   acgctatgtg gatacgctgc tttaatgcct ttgtatcatg ctattgcttc ccgtatggct 4320
   ttcattttct cctccttgta taaatcctgg ttgctgtctc tttatgagga gttgtggccc 4380
   gttgtcaggc aacgtggcgt ggtgtgcact gtgtttgctg acgcaacccc cactggttgg 4440
   ggcattgcca ccacctgtca gctcctttcc gggactttcg ctttccccct ccctattgcc 4500
   acggcggaac tcatcgccgc ctgccttgcc cgctgctgga caggggctcg gctgttgggc 4560
   actgacaatt ccgtggtgtt gtcggggaag ctgacgtcct ttccatcgct gctcgcctgt 4620
   gttgccacct ggattctgcg cgggacgtcc ttctgctacg tcccttcggc cctcaatcca 4680
   gcggaccttc cttcccgcgg cctgctgccg gctctgcggc ctcttccgcg tcttcgcctt 4740
   cgccctcaga cgagtcggat ctccctttgg gccgcctccc cgctcgagtc tagagggccc 4800
   gtttaaaccc gctgatcagc ctcgactgtg ccttctagtt gccagccatc tgttgtttgc 4860
   ccctcccccg tgccttcctt gaccctggaa ggtgccactc ccactgtcct ttcctaataa 4920
   aatgaggaaa ttgcatcgca ttgtctgagt aggtgtcatt ctattctggg gggtggggtg 4980
   gggcaggaca gcaaggggga ggattgggaa gacaatagca ggcatgctgg ggatgcggtg 5040
   ggctctatgg cttctactgg gcggttttat ggacagcaag cgaaccggaa ttgccagctg 5100
   gggcgccctc tggtaaggtt gggaagccct gcaaagtaaa 5140
<210> 5
   <211> 5158
   <212> DNA
   <213> Artificial Sequence
<220>
   <223> pCMV-VSV-Njco
<400> 5
   ctggatggct ttctcgccgc caaggatctg atggcgcagg ggatcaagct ctgatcaaga 60
   gacaggatga ggatcgtttc gcatgattga acaagatgga ttgcacgcag gttctccggc 120
   cgcttgggtg gagaggctat tcggctatga ctgggcacaa cagacaatcg gctgctctga 180
   tgccgccgtg ttccggctgt cagcgcaggg gcgcccggtt ctttttgtca agaccgacct 240
   gtccggtgcc ctgaatgaac tgcaagacga ggcagcgcgg ctatcgtggc tggccacgac 300
   gggcgttcct tgcgcagctg tgctcgacgt tgtcactgaa gcgggaaggg actggctgct 360
   attgggcgaa gtgccggggc aggatctcct gtcatctcac cttgctcctg ccgagaaagt 420
   atccatcatg gctgatgcaa tgcggcggct gcatacgctt gatccggcta cctgcccatt 480
   cgaccaccaa gcgaaacatc gcatcgagcg agcacgtact cggatggaag ccggtcttgt 540
   cgatcaggat gatctggacg aagagcatca ggggctcgcg ccagccgaac tgttcgccag 600
   gctcaaggcg agcatgcccg acggcgagga tctcgtcgtg acccatggcg atgcctgctt 660
   gccgaatatc atggtggaaa atggccgctt ttctggattc atcgactgtg gccggctggg 720
   tgtggcggac cgctatcagg acatagcgtt ggctacccgt gatattgctg aagagcttgg 780
   cggcgaatgg gctgaccgct tcctcgtgct ttacggtatc gccgctcccg attcgcagcg 840
   catcgccttc tatcgccttc ttgacgagtt cttctgaatt attaacgctt acaatttcct 900
   gatgcggtat tttctcctta cgcatctgtg cggtatttca caccgcatac aggtggcact 960
   tttcggggaa atgtgcgcgg aacccctatt tgtttatttt tctaaataca ttcaaatatg 1020
   tatccgctca tgagacaata accctgataa atgcttcaat aatagcacgt gctaaaactt 1080
   catttttaat ttaaaaggat ctaggtgaag atcctttttg ataatctcat gaccaaaatc 1140
   ccttaacgtg agttttcgtt ccactgagcg tcagaccccg tagaaaagat caaaggatct 1200
   tcttgagatc ctttttttct gcgcgtaatc tgctgcttgc aaacaaaaaa accaccgcta 1260
   ccagcggtgg tttgtttgcc ggatcaagag ctaccaactc tttttccgaa ggtaactggc 1320
   ttcagcagag cgcagatacc aaatactgtc cttctagtgt agccgtagtt aggccaccac 1380
   ttcaagaact ctgtagcacc gcctacatac ctcgctctgc taatcctgtt accagtggct 1440
   gctgccagtg gcgataagtc gtgtcttacc gggttggact caagacgata gttaccggat 1500
   aaggcgcagc ggtcgggctg aacggggggt tcgtgcacac agcccagctt ggagcgaacg 1560
   acctacaccg aactgagata cctacagcgt gagctatgag aaagcgccac gcttcccgaa 1620
   gggagaaagg cggacaggta tccggtaagc ggcagggtcg gaacaggaga gcgcacgagg 1680
   gagcttccag ggggaaacgc ctggtatctt tatagtcctg tcgggtttcg ccacctctga 1740
   cttgagcgtc gatttttgtg atgctcgtca ggggggcgga gcctatggaa aaacgccagc 1800
   aacgcggcct ttttacggtt cctggccttt tgctggcctt ttgctcacat gttcttgact 1860
   cttcgcgatg tacgggccag atatacgcgt tgacattgat tattgactag ttattaatag 1920
   taatcaatta cggggtcatt agttcatagc ccatatatgg agttccgcgt tacataactt 1980
   acggtaaatg gcccgcctgg ctgaccgccc aacgaccccc gcccattgac gtcaataatg 2040
   acgtatgttc ccatagtaac gccaataggg actttccatt gacgtcaatg ggtggactat 2100
   ttacggtaaa ctgcccactt ggcagtacat caagtgtatc atatgccaag tacgccccct 2160
   attgacgtca atgacggtaa atggcccgcc tggcattatg cccagtacat gaccttatgg 2220
   gactttccta cttggcagta catctacgta ttagtcatcg ctattaccat ggtgatgcgg 2280
   ttttggcagt acatcaatgg gcgtggatag cggtttgact cacggggatt tccaagtctc 2340
   caccccattg acgtcaatgg gagtttgttt tggcaccaaa atcaacggga ctttccaaaa 2400
   tgtcgtaaca actccgcccc attgacgcaa atgggcggta ggcgtgtacg gtgggaggtc 2460
   tatataagca gagctctctg gctaactaga gaacccactg cttactggct tatcgaaatt 2520
   aatacgactc actataggga gacccaagct ggctagcgtt taaacttaag cttggtaccg 2580
   agctcggatc ctgatcagcc accatgctgt catatctgat ctttgccctg gctgtgagcc 2640
   caatcctcgg aaagatcgaa atcgtgttcc cacaacacac cacaggggac tggaagcgcg 2700
   tgccccacga gtacaactac tgcccgacct cagccgacaa gaatagccac ggcacgcaga 2760
   ccggcatccc tgtggagctg accatgccca aggggctcac aacgcaccaa gtcgaaggct 2820
   tcatgtgcca cagcgctctc tggatgacaa cctgcgattt tcgctggtat ggccccaagt 2880
   acatcacgca cagcatccac aatgaggaac caaccgacta ccagtgcctc gaagccatca 2940
   agtcatacaa ggatggggtg agcttcaacc ccggcttccc gccccaatca tgtggctacg 3000
   gcaccgtgac cgacgccgag gcccacatcg tgaccgtgac accccactca gtcaaggtgg 3060
   acgagtacac aggcgaatgg atcgaccccc acttcatcgg gggccgctgt aagggccaaa 3120
   tctgcgagac cgtgcacaac agcaccaagt ggtttacgtc atcagacggc gaaagcgtgt 3180
   gcagccaact gtttacgctc gtgggcggca tcttctttag cgacagcgag gagatcacca 3240
   gcatgggcct cccggagaca ggaatccgca gcaactactt tccgtacatc agcaccgagg 3300
   gaatctgtaa gatgcctttt tgccgcaagc agggatataa gctgaagaat gacctgtggt 3360
   tccagatcat ggacccggac ctggacaaga ccgtccgcga tctgccccac atcaaggact 3420
   gtgatctgtc atcaagcatc atcacccccg gagaacacgc cacggacatc agcctcatca 3480
   gcgatgtgga gcgcatcctc gactacgctc tctgccagaa cacatggagc aagatcgaaa 3540
   gcggcgaacc catcacccca gtggacctga gctatctcgg cccaaagaac cccggcgtgg 3600
   ggcccgtgtt caccatcatc aacgggagcc tgcactactt tacaagcaag tatctgcgcg 3660
   tggagctcga aagcccagtc atcccccgca tggaggggaa ggtggccggg acccgcatcg 3720
   tgcgccagct gtgggaccag tggttccctt ttggcgaggt ggaaatcggc cccaacggcg 3780
   tgctgaagac caagcaagga tataagttcc cgctgcacat catcgggacg ggcgaagtgg 3840
   acagcgatat caagatggag cgcgtggtca agcactggga gcacccacac atcgaggctg 3900
   ctcagacctt tctcaagaag gacgataccg gcgaagtcct gtattacggg gatacgggag 3960
   tgagcaagaa ccctgtggag ctggtggaag gctggttcag cggatggcgc tcaagcctga 4020
   tgggcgtgct ggccgtcatc atcggatttg tgatcctgat gttcctcatc aagctgatcg 4080
   gcgtgctgtc aagcctgttc cgccctaagc gccgcccaat ctacaagagc gacgtcgaga 4140
   tggcccactt tcgctaagaa ttctgcagat atccagcaca gtggcggccg ctcgagtgta 4200
   caaattcccg ataatcaacc tctggattac aaaatttgtg aaagattgac tggtattctt 4260
   aactatgttg ctccttttac gctatgtgga tacgctgctt taatgccttt gtatcatgct 4320
   attgcttccc gtatggcttt cattttctcc tccttgtata aatcctggtt gctgtctctt 4380
   tatgaggagt tgtggcccgt tgtcaggcaa cgtggcgtgg tgtgcactgt gtttgctgac 4440
   gcaaccccca ctggttgggg cattgccacc acctgtcagc tcctttccgg gactttcgct 4500
   ttccccctcc ctattgccac ggcggaactc atcgccgcct gccttgcccg ctgctggaca 4560
   ggggctcggc tgttgggcac tgacaattcc gtggtgttgt cggggaagct gacgtccttt 4620
   ccatcgctgc tcgcctgtgt tgccacctgg attctgcgcg ggacgtcctt ctgctacgtc 4680
   ccttcggccc tcaatccagc ggaccttcct tcccgcggcc tgctgccggc tctgcggcct 4740
   cttccgcgtc ttcgccttcg ccctcagacg agtcggatct ccctttgggc cgcctccccg 4800
   ctcgagtcta gagggcccgt ttaaacccgc tgatcagcct cgactgtgcc ttctagttgc 4860
   cagccatctg ttgtttgccc ctcccccgtg ccttccttga ccctggaagg tgccactccc 4920
   actgtccttt cctaataaaa tgaggaaatt gcatcgcatt gtctgagtag gtgtcattct 4980
   attctggggg gtggggtggg gcaggacagc aagggggagg attgggaaga caatagcagg 5040
   catgctgggg atgcggtggg ctctatggct tctactgggc ggttttatgg acagcaagcg 5100
   aaccggaatt gccagctggg gcgccctctg gtaaggttgg gaagccctgc aaagtaaa 5158
<210> 6
   <211> 720
   <212> DNA
   <213> Artificial Sequence
<220>
   <223> eGFP
<400> 6
   atggtgagca agggcgagga gctgttcacc ggggtggtgc ccatcctggt cgagctggac        60
   ggcgacgtaa acggccacaa gttcagcgtg tccggcgagg gcgagggcga tgccacctac       120
   ggcaagctga ccctgaagtt catctgcacc accggcaagc tgcccgtgcc ctggcccacc       180
   ctcgtgacca ccctgaccta cggcgtgcag tgcttcagcc gctaccccga ccacatgaag       240
   cagcacgact tcttcaagtc cgccatgccc gaaggctacg tccaggagcg caccatcttc       300
   ttcaaggacg acggcaacta caagacccgc gccgaggtga agttcgaggg cgacaccctg       360
   gtgaaccgca tcgagctgaa gggcatcgac ttcaaggagg acggcaacat cctggggcac       420
   aagctggagt acaactacaa cagccacaac gtctatatca tggccgacaa gcagaagaac       480
   ggcatcaagg tgaacttcaa gatccgccac aacatcgagg acggcagcgt gcagctcgcc       540
   gaccactacc agcagaacac ccccatcggc gacggccccg tgctgctgcc cgacaaccac       600
   tacctgagca cccagtccgc cctgagcaaa gaccccaacg agaagcgcga tcacatggtc       660
   ctgctggagt tcgtgaccgc cgccgggatc actctcggca tggacgagct gtacaagtaa       720
<210> 7
   <211> 239
   <212> PRT
   <213> Artificial Sequence
<220>
   <223> eGFP protein
<400> 7
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 8
   <211> 1725
   <212> DNA
   <213> Artificial Sequence
<220>
   <223> A111-Luciferase
<400> 8
   ggatccgcca ccatggccgc agccgcagcc gtgaagaact ggatgactca aacgctcgcc      60
   gctgcaatgg aagatgccaa aaacattaag aagggcccag cgccattcta cccactcgaa     120
   gacgggaccg ccggcgagca gctgcacaaa gccatgaagc gctacgccct ggtgcccggc     180
   accatcgcct ttaccgacgc acatatcgag gtggacatta cctacgccga gtacttcgag     240
   atgagcgttc ggctggcaga agctatgaag cgctatgggc tgaatacaaa ccatcggatc     300
   gtggtgtgca gcgagaatag cttgcagttc ttcatgcccg tgttgggtgc cctgttcatc     360
   ggtgtggctg tggccccagc taacgacatc tacaacgagc gcgagctgct gaacagcatg     420
   ggcatcagcc agcccaccgt cgtattcgtg agcaagaaag ggctgcaaaa gatcctcaac     480
   gtgcaaaaga agctaccgat catacaaaag atcatcatca tggatagcaa gaccgactac     540
   cagggcttcc aaagcatgta caccttcgtg acttcccatt tgccacccgg cttcaacgag     600
   tacgacttcg tgcccgagag cttcgaccgg gacaaaacca tcgccctgat catgaacagt     660
   agtggcagta ccggattgcc caagggcgta gccctaccgc accgcaccgc ttgtgtccga     720
   ttcagtcatg cccgcgaccc catcttcggc aaccagatca tccccgacac cgctatcctc     780
   agcgtggtgc catttcacca cggcttcggc atgttcacca cgctgggcta cttgatctgc     840
   ggctttcggg tcgtgctcat gtaccgcttc gaggaggagc tattcttgcg cagcttgcaa     900
   gactataaga ttcaatctgc cctgctggtg cccacactat ttagcttctt cgctaagagc 960
   actctcatcg acaagtacga cctaagcaac ttgcacgaga tcgccagcgg cggggcgccg 1020
   ctcagcaagg aggtaggtga ggccgtggcc aaacgcttcc acctaccagg catccgccag 1080
   ggctacggcc tgacagaaac aaccagcgcc attctgatca cccccgaagg ggacgacaag 1140
   cctggcgcag taggcaaggt ggtgcccttc ttcgaggcta aggtggtgga cttggacacc 1200
   ggtaagacac tgggtgtgaa ccagcgcggc gagctgtgcg tccgtggccc catgatcatg 1260
   agcggctacg ttaacaaccc cgaggctaca aacgctctca tcgacaagga cggctggctg 1320
   cacagcggcg acatcgccta ctgggacgag gacgagcact tcttcatcgt ggaccggctg 1380
   aagagcctga tcaaatacaa gggctaccag gtagccccag ccgaactgga gagcatcctg 1440
   ctgcaacacc ccaacatctt cgacgccggg gtcgccggcc tgcccgacga cgatgccggc 1500
   gagctgcccg ccgcagtcgt cgtgctggaa cacggtaaaa ccatgaccga gaaggagatc 1560
   gtggactatg tggccagcca ggttacaacc gccaagaagc tgcgcggtgg tgttgtgttc 1620
   gtggacgagg tgcctaaagg actgaccggc aagttggacg cccgcaagat ccgcgagatt 1680
   ctcattaagg ccaagaaggg cggcaagatc gccgtgtaac tcgag 1725
<210> 9
   <211> 568
   <212> PRT
   <213> Artificial Sequence
<220>
   <223> A111-Luciferase
<400> 9
   Met Ala Ala Ala Ala Ala Val Lys Asn Trp Met Thr Gln Thr Leu Ala
   1               5                   10                  15
   Ala Ala Met Glu Asp Ala Lys Asn Ile Lys Lys Gly Pro Ala Pro Phe
               20                  25                  30
   Tyr Pro Leu Glu Asp Gly Thr Ala Gly Glu Gln Leu His Lys Ala Met
           35                  40                  45
   Lys Arg Tyr Ala Leu Val Pro Gly Thr Ile Ala Phe Thr Asp Ala His
       50                  55                  60
   Ile Glu Val Asp Ile Thr Tyr Ala Glu Tyr Phe Glu Met Ser Val Arg
   65                  70                  75                  80
   Leu Ala Glu Ala Met Lys Arg Tyr Gly Leu Asn Thr Asn His Arg Ile
                   85                  90                  95
   Val Val Cys Ser Glu Asn Ser Leu Gln Phe Phe Met Pro Val Leu Gly
               100                 105                 110
   Ala Leu Phe Ile Gly Val Ala Val Ala Pro Ala Asn Asp Ile Tyr Asn
           115                 120                 125
   Glu Arg Glu Leu Leu Asn Ser Met Gly Ile Ser Gln Pro Thr Val Val
       130                 135                 140
   Phe Val Ser Lys Lys Gly Leu Gln Lys Ile Leu Asn Val Gln Lys Lys
   145                 150                 155                 160
   Leu Pro Ile Ile Gln Lys Ile Ile Ile Met Asp Ser Lys Thr Asp Tyr
                   165                 170                 175
   Gln Gly Phe Gln Ser Met Tyr Thr Phe Val Thr Ser His Leu Pro Pro
               180                 185                 190
   Gly Phe Asn Glu Tyr Asp Phe Val Pro Glu Ser Phe Asp Arg Asp Lys
           195                 200                 205
   Thr Ile Ala Leu Ile Met Asn Ser Ser Gly Ser Thr Gly Leu Pro Lys
       210                 215                 220
   Gly Val Ala Leu Pro His Arg Thr Ala Cys Val Arg Phe Ser His Ala
   225                 230                 235                 240
   Arg Asp Pro Ile Phe Gly Asn Gln Ile Ile Pro Asp Thr Ala Ile Leu
                   245                 250                 255
   Ser Val Val Pro Phe His His Gly Phe Gly Met Phe Thr Thr Leu Gly
               260                 265                 270
   Tyr Leu Ile Cys Gly Phe Arg Val Val Leu Met Tyr Arg Phe Glu Glu
           275                 280                 285
   Glu Leu Phe Leu Arg Ser Leu Gln Asp Tyr Lys Ile Gln Ser Ala Leu
       290                 295                 300
   Leu Val Pro Thr Leu Phe Ser Phe Phe Ala Lys Ser Thr Leu Ile Asp
   305                 310                 315                 320
   Lys Tyr Asp Leu Ser Asn Leu His Glu Ile Ala Ser Gly Gly Ala Pro
                   325                 330                 335
   Leu Ser Lys Glu Val Gly Glu Ala Val Ala Lys Arg Phe His Leu Pro
               340                 345                 350
   Gly Ile Arg Gln Gly Tyr Gly Leu Thr Glu Thr Thr Ser Ala Ile Leu
           355                 360                 365
   Ile Thr Pro Glu Gly Asp Asp Lys Pro Gly Ala Val Gly Lys Val Val
       370                 375                 380
   Pro Phe Phe Glu Ala Lys Val Val Asp Leu Asp Thr Gly Lys Thr Leu
   385                 390                 395                 400
   Gly Val Asn Gln Arg Gly Glu Leu Cys Val Arg Gly Pro Met Ile Met
                   405                 410                 415
   Ser Gly Tyr Val Asn Asn Pro Glu Ala Thr Asn Ala Leu Ile Asp Lys
               420                 425                 430
   Asp Gly Trp Leu His Ser Gly Asp Ile Ala Tyr Trp Asp Glu Asp Glu
           435                 440                 445
   His Phe Phe Ile Val Asp Arg Leu Lys Ser Leu Ile Lys Tyr Lys Gly
       450                 455                 460
   Tyr Gln Val Ala Pro Ala Glu Leu Glu Ser Ile Leu Leu Gln His Pro
   465                 470                 475                 480
   Asn Ile Phe Asp Ala Gly Val Ala Gly Leu Pro Asp Asp Asp Ala Gly
                   485                 490                 495
   Glu Leu Pro Ala Ala Val Val Val Leu Glu His Gly Lys Thr Met Thr
               500                 505                 510
   Glu Lys Glu Ile Val Asp Tyr Val Ala Ser Gln Val Thr Thr Ala Lys
           515                 520                 525
   Lys Leu Arg Gly Gly Val Val Phe Val Asp Glu Val Pro Lys Gly Leu
       530                 535                 540
   Thr Gly Lys Leu Asp Ala Arg Lys Ile Arg Glu Ile Leu Ile Lys Ala
   545                 550                 555                 560
   Lys Lys Gly Gly Lys Ile Ala Val
                   565
<210> 10
   <211> 1026
   <212> DNA
   <213> Plasmodium berghei
<220>
   <221> misc_feature
   <223> circumsporozoite (CS) protein (CSP)
<400> 10
   atggccaaga agtgtaccat actggtcgtt gcgtcacttc tgttggtcaa ttctctgctc      60
   ccaggctatg gacagaacaa atccattcag gcacagagga acctcaacga actctgctac     120
   aatgaaggga atgacaataa gctgtatcac gtgctgaatt ccaagaacgg caaaatctac     180
   aatcgcaaca cagtaaatcg gttgcttgcc gatgcacccg agggtaagaa gaatgaaaag     240
   aagaatgaga aaatcgagcg caacaacaag cttaaacagc caccgcctcc tcctaaccca     300
   aatgacccac cgccacccaa tccaaacgac ccaccgcctc ccaaccctaa cgatcctcca     360
   ccgcccaacc ctaatgaccc accacctccc aatgcaaacg atccaccccc tcctaacgct     420
   aacgaccctg ctccacccaa cgctaacgat cccgcgcccc ccaatgccaa cgaccccgca     480
   ccacctaatg ccaacgatcc cgccccgccc aatgctaatg atcctccacc acccaaccca     540
   aacgaccctg cccctcctaa tgctaacgat ccaccacctc ccaatccgaa tgatcccgct     600
   ccacctcagg ggaacaacaa ccctcagccc caacctagac cacagccgca gccccaaccc     660
   caaccccagc cccagcctca accccagccc cagccacgtc cgcagcctca gcctcaacct     720
   ggaggcaaca ataacaacaa gaataacaac aacgacgaca gctacattcc aagtgccgag     780
   aaaattctgg agtttgttaa gcagatccga gacagcataa ccgaagaatg gtcacagtgt     840
   aacgtgacgt gtggatctgg catcagagtg aggaaacgga agggttccaa taagaaagca     900
   gaggatctga ctctggagga cattgataca gagatctgca aaatggacaa atgcagctct     960
   atcttcaaca tcgtgagtaa tagcctcggg tttgtgattc tgctggtcct ggtgttcttc    1020
   aattga                                                              1026
<210> 11
   <211> 341
   <212> PRT
   <213> Plasmodium berghei
<220>
   <221> misc_feature
   <223> CSP
<400> 11
   Met Ala Lys Lys Cys Thr Ile Leu Val Val Ala Ser Leu Leu Leu Val
   1               5                   10                  15
   Asn Ser Leu Leu Pro Gly Tyr Gly Gln Asn Lys Ser Ile Gln Ala Gln
               20                  25                  30
   Arg Asn Leu Asn Glu Leu Cys Tyr Asn Glu Gly Asn Asp Asn Lys Leu
           35                  40                  45
   Tyr His Val Leu Asn Ser Lys Asn Gly Lys Ile Tyr Asn Arg Asn Thr
       50                  55                  60
   Val Asn Arg Leu Leu Ala Asp Ala Pro Glu Gly Lys Lys Asn Glu Lys
   65                  70                  75                  80
   Lys Asn Glu Lys Ile Glu Arg Asn Asn Lys Leu Lys Gln Pro Pro Pro
                   85                  90                  95
   Pro Pro Asn Pro Asn Asp Pro Pro Pro Pro Asn Pro Asn Asp Pro Pro
               100                 105                 110
   Pro Pro Asn Pro Asn Asp Pro Pro Pro Pro Asn Pro Asn Asp Pro Pro
           115                 120                 125
   Pro Pro Asn Ala Asn Asp Pro Pro Pro Pro Asn Ala Asn Asp Pro Ala
       130                 135                 140
   Pro Pro Asn Ala Asn Asp Pro Ala Pro Pro Asn Ala Asn Asp Pro Ala
   145                 150                 155                 160
   Pro Pro Asn Ala Asn Asp Pro Ala Pro Pro Asn Ala Asn Asp Pro Pro
                   165                 170                 175
   Pro Pro Asn Pro Asn Asp Pro Ala Pro Pro Asn Ala Asn Asp Pro Pro
               180                 185                 190
   Pro Pro Asn Pro Asn Asp Pro Ala Pro Pro Gln Gly Asn Asn Asn Pro
           195                 200                 205
   Gln Pro Gln Pro Arg Pro Gln Pro Gln Pro Gln Pro Gln Pro Gln Pro
       210                 215                 220
   Gln Pro Gln Pro Gln Pro Gln Pro Arg Pro Gln Pro Gln Pro Gln Pro
   225                 230                 235                 240
   Gly Gly Asn Asn Asn Asn Lys Asn Asn Asn Asn Asp Asp Ser Tyr Ile
                   245                 250                 255
   Pro Ser Ala Glu Lys Ile Leu Glu Phe Val Lys Gln Ile Arg Asp Ser
               260                 265                 270
   Ile Thr Glu Glu Trp Ser Gln Cys Asn Val Thr Cys Gly Ser Gly Ile
           275                 280                 285
   Arg Val Arg Lys Arg Lys Gly Ser Asn Lys Lys Ala Glu Asp Leu Thr
   290 295 300
   Leu Glu Asp Ile Asp Thr Glu Ile Cys Lys Met Asp Lys Cys Ser Ser
   305 310 315 320
   Ile Phe Asn Ile Val Ser Asn Ser Leu Gly Phe Val Ile Leu Leu Val
   325 330 335
   Leu Val Phe Phe Asn
   340
<210> 12
   <211> 340
   <212> PRT
   <213> Plasmodium berghei
<220>
   <221> misc_feature
   <223> CSP
<400> 12
   Met Lys Lys Cys Thr Ile Leu Val Val Ala Ser Leu Leu Leu Val Asn
   1 5 10 15
   Ser Leu Leu Pro Gly Tyr Gly Gln Asn Lys Ser Ile Gln Ala Gln Arg
   20 25 30
   Asn Leu Asn Glu Leu Cys Tyr Asn Glu Gly Asn Asp Asn Lys Leu Tyr
   35 40 45
   His Val Leu Asn Ser Lys Asn Gly Lys Ile Tyr Asn Arg Asn Thr Val
   50 55 60
   Asn Arg Leu Leu Ala Asp Ala Pro Glu Gly Lys Lys Asn Glu Lys Lys
   65 70 75 80
   Asn Glu Lys Ile Glu Arg Asn Asn Lys Leu Lys Gln Pro Pro Pro Pro
   85 90 95
   Pro Asn Pro Asn Asp Pro Pro Pro Pro Asn Pro Asn Asp Pro Pro Pro
   100 105 110
   Pro Asn Pro Asn Asp Pro Pro Pro Pro Asn Pro Asn Asp Pro Pro Pro
   115 120 125
   Pro Asn Ala Asn Asp Pro Pro Pro Pro Asn Ala Asn Asp Pro Ala Pro
   130 135 140
   Pro Asn Ala Asn Asp Pro Ala Pro Pro Asn Ala Asn Asp Pro Ala Pro
   145                 150                 155                 160
   Pro Asn Ala Asn Asp Pro Ala Pro Pro Asn Ala Asn Asp Pro Pro Pro
                   165                 170                 175
   Pro Asn Pro Asn Asp Pro Ala Pro Pro Asn Ala Asn Asp Pro Pro Pro
               180                 185                 190
   Pro Asn Pro Asn Asp Pro Ala Pro Pro Gln Gly Asn Asn Asn Pro Gln
           195                 200                 205
   Pro Gln Pro Arg Pro Gln Pro Gln Pro Gln Pro Gln Pro Gln Pro Gln
       210                 215                 220
   Pro Gln Pro Gln Pro Gln Pro Arg Pro Gln Pro Gln Pro Gln Pro Gly
   225                 230                 235                 240
   Gly Asn Asn Asn Asn Lys Asn Asn Asn Asn Asp Asp Ser Tyr Ile Pro
                   245                 250                 255
   Ser Ala Glu Lys Ile Leu Glu Phe Val Lys Gln Ile Arg Asp Ser Ile
               260                 265                 270
   Thr Glu Glu Trp Ser Gln Cys Asn Val Thr Cys Gly Ser Gly Ile Arg
           275                 280                 285
   Val Arg Lys Arg Lys Gly Ser Asn Lys Lys Ala Glu Asp Leu Thr Leu
       290                 295                 300
   Glu Asp Ile Asp Thr Glu Ile Cys Lys Met Asp Lys Cys Ser Ser Ile
   305                 310                 315                 320
   Phe Asn Ile Val Ser Asn Ser Leu Gly Phe Val Ile Leu Leu Val Leu
                   325                 330                 335
   Val Phe Phe Asn
               340
<210> 13
   <211> 1203
   <212> DNA
   <213> Artificial Sequence
<220>
   <223> PfCSPhumanCO
<400> 13
   gccaccatgg ctatgagaaa gctcgcgatc ctttccgtga gctcattcct gtttgtcgaa      60
   gccttgttcc aggagtatca gtgctatgga tcatcctcca acacaagagt gctcaacgaa     120
   ctgaactatg acaatgccgg tactaacctc tacaatgaac tggagatgaa ctactacggc     180
   aaacaggaga actggtattc cctgaagaag aattccagat cactgggcga aaacgacgat     240
   gggaataatg aggataacga gaagttgcgg aaaccaaagc acaagaagtt gaaacaaccc     300
   gccgacggaa accctgatcc taacgccaac ccaaatgtag atcccaacgc caacccaaac     360
   gtcgatccca atgccaatcc caatgttgac cccaatgcaa accctaatgc aaatcccaat     420
   gccaatccca atgcaaatcc taatgctaat ccaaacgcca accctaacgc gaaccccaac     480
   gccaatccta acgcaaatcc taacgcaaat cctaatgcca accctaatgc gaacccgaac     540
   gctaatccta acgctaatcc gaatgcaaat ccaaatgcaa atccgaacgc caatcccaac     600
   gtagacccaa atgcaaaccc gaacgctaac ccgaacgcaa acccaaacgc caatccgaac     660
   gctaatccca atgctaatcc caacgctaac cccaatgcca acccgaatgc caaccccaat     720
   gcgaatccaa atgcgaaccc aaacgccaat ccgaatgcga atcctaacgc taacccaaat     780
   gctaatccaa acgctaatcc aaatgcgaac cccaatgcga atccaaataa gaacaatcag     840
   gggaatggtc agggccataa catgcctaac gaccccaacc gaaatgtgga cgagaacgct     900
   aacgcaaaca gcgctgtaaa gaacaacaac aatgaggagc cttctgacaa gcacatcaaa     960
   gagtacctga ataaaatcca gaacagtctt tctacggaat ggtccccatg tagtgttact    1020
   tgtggcaatg ggattcaagt caggatcaaa ccaggctctg cgaataagcc taaggatgaa    1080
   ctggattatg ccaatgacat cgagaagaaa atatgcaaga tggagaagtg cagtagcgtg    1140
   ttcaatgtcg taaactcaag cataggtctg ataatggtac tgagctttct gttcctcaac    1200
   taa                                                                  1203
<210> 14
   <211> 398
   <212> PRT
   <213> Artificial Sequence
<220>
   <223> PfCSP
<400> 14
   Met Ala Met Arg Lys Leu Ala Ile Leu Ser Val Ser Ser Phe Leu Phe
   1               5                   10                  15
   Val Glu Ala Leu Phe Gln Glu Tyr Gln Cys Tyr Gly Ser Ser Ser Asn
               20                  25                  30
   Thr Arg Val Leu Asn Glu Leu Asn Tyr Asp Asn Ala Gly Thr Asn Leu
           35                  40                  45
   Tyr Asn Glu Leu Glu Met Asn Tyr Tyr Gly Lys Gln Glu Asn Trp Tyr
       50                  55                  60
   Ser Leu Lys Lys Asn Ser Arg Ser Leu Gly Glu Asn Asp Asp Gly Asn
   65                  70                  75                  80
   Asn Glu Asp Asn Glu Lys Leu Arg Lys Pro Lys His Lys Lys Leu Lys
                   85                  90                  95
   Gln Pro Ala Asp Gly Asn Pro Asp Pro Asn Ala Asn Pro Asn Val Asp
               100                 105                 110
   Pro Asn Ala Asn Pro Asn Val Asp Pro Asn Ala Asn Pro Asn Val Asp
           115                 120                 125
   Pro Asn Ala Asn Pro Asn Ala Asn Pro Asn Ala Asn Pro Asn Ala Asn
       130                 135                 140
   Pro Asn Ala Asn Pro Asn Ala Asn Pro Asn Ala Asn Pro Asn Ala Asn
   145                 150                 155                 160
   Pro Asn Ala Asn Pro Asn Ala Asn Pro Asn Ala Asn Pro Asn Ala Asn
                   165                 170                 175
   Pro Asn Ala Asn Pro Asn Ala Asn Pro Asn Ala Asn Pro Asn Ala Asn
               180                 185                 190
   Pro Asn Ala Asn Pro Asn Val Asp Pro Asn Ala Asn Pro Asn Ala Asn
           195                 200                 205
   Pro Asn Ala Asn Pro Asn Ala Asn Pro Asn Ala Asn Pro Asn Ala Asn
       210                 215                 220
   Pro Asn Ala Asn Pro Asn Ala Asn Pro Asn Ala Asn Pro Asn Ala Asn
   225                 230                 235                 240
   Pro Asn Ala Asn Pro Asn Ala Asn Pro Asn Ala Asn Pro Asn Ala Asn
                   245                 250                 255
   Pro Asn Ala Asn Pro Asn Ala Asn Pro Asn Ala Asn Pro Asn Ala Asn
               260                 265                 270
   Pro Asn Lys Asn Asn Gln Gly Asn Gly Gln Gly His Asn Met Pro Asn
           275                 280                 285
   Asp Pro Asn Arg Asn Val Asp Glu Asn Ala Asn Ala Asn Ser Ala Val
   290 295 300
   Lys Asn Asn Asn Asn Glu Glu Pro Ser Asp Lys His Ile Lys Glu Tyr
   305 310 315 320
   Leu Asn Lys Ile Gln Asn Ser Leu Ser Thr Glu Trp Ser Pro Cys Ser
   325 330 335
   Val Thr Cys Gly Asn Gly Ile Gln Val Arg Ile Lys Pro Gly Ser Ala
   340 345 350
   Asn Lys Pro Lys Asp Glu Leu Asp Tyr Ala Asn Asp Ile Glu Lys Lys
   355 360 365
   Ile Cys Lys Met Glu Lys Cys Ser Ser Val Phe Asn Val Val Asn Ser
   370 375 380
   Ser Ile Gly Leu Ile Met Val Leu Ser Phe Leu Phe Leu Asn
   385 390 395
<210> 15
   <211> 423
   <212> PRT
   <213> Artificial Sequence
<220>
   <223> CSP
<400> 15
   Met Arg Lys Leu Ala Ile Leu Ser Val Ser Ser Phe Leu Phe Val Glu
   1               5                   10                  15
   Ala Leu Phe Gln Glu Tyr Gln Cys Tyr Gly Ser Ser Ser Asn Thr Arg
               20                  25                  30
   Val Leu Asn Glu Leu Asn Tyr Asp Asn Ala Gly Thr Asn Leu Tyr Asn
           35                  40                  45
   Glu Leu Glu Met Asn Tyr Tyr Gly Lys Gln Glu Asn Trp Tyr Ser Leu
       50                  55                  60
   Lys Lys Asn Ser Arg Ser Leu Gly Glu Asn Asp Asp Gly Asn Asn Asn
   65                  70                  75                  80
   Asn Gly Asp Asn Gly Arg Glu Gly Lys Asp Glu Asp Lys Arg Asp Gly
                   85                  90                  95
   Asn Asn Glu Asp Asn Glu Lys Leu Arg Lys Pro Lys His Lys Lys Leu
               100                 105                 110
   Lys Gln Pro Gly Asp Gly Asn Pro Asp Pro Asn Ala Asn Pro Asn Val
           115                 120                 125
   Asp Pro Asn Ala Asn Pro Asn Val Asp Pro Asn Ala Asn Pro Asn Val
       130                 135                 140
   Asp Pro Asn Ala Asn Pro Asn Ala Asn Pro Asn Ala Asn Pro Asn Ala
   145                 150                 155                 160
   Asn Pro Asn Ala Asn Pro Asn Ala Asn Pro Asn Ala Asn Pro Asn Ala
                   165                 170                 175
   Asn Pro Asn Ala Asn Pro Asn Ala Asn Pro Asn Ala Asn Pro Asn Val
               180                 185                 190
   Asp Pro Asn Ala Asn Pro Asn Ala Asn Pro Asn Ala Asn Pro Asn Ala
           195                 200                 205
   Asn Pro Asn Ala Asn Pro Asn Ala Asn Pro Asn Ala Asn Pro Asn Ala
       210                 215                 220
   Asn Pro Asn Ala Asn Pro Asn Ala Asn Pro Asn Ala Asn Pro Asn Ala
   225                 230                 235                 240
   Asn Pro Asn Ala Asn Pro Asn Ala Asn Pro Asn Ala Asn Pro Asn Ala
                   245                 250                 255
   Asn Pro Asn Ala Asn Pro Asn Ala Asn Pro Asn Ala Asn Pro Asn Ala
               260                 265                 270
   Asn Pro Asn Ala Asn Pro Asn Ala Asn Pro Asn Ala Asn Pro Asn Ala
           275                 280                 285
   Asn Pro Asn Ala Asn Pro Asn Ala Asn Pro Asn Lys Asn Asn Gln Gly
       290                 295                 300
   Asn Gly Gln Gly His Asn Met Pro Asn Asp Pro Asn Arg Asn Val Asp
   305                 310                 315                 320
   Glu Asn Ala Asn Ala Asn Asn Ala Val Lys Asn Asn Asn Asn Glu Glu
                   325                 330                 335
   Pro Ser Asp Lys His Ile Thr Glu Tyr Leu Lys Lys Ile Gln Asn Ser
               340                 345                 350
   Leu Ser Thr Glu Trp Ser Pro Cys Ser Val Thr Cys Gly Asn Gly Ile
               355                 360                 365
   Gln Val Arg Ile Lys Pro Gly Ser Ala Gly Lys Ser Lys Asp Glu Leu
       370                 375                 380
   Asp Tyr Glu Asn Asp Ile Glu Lys Lys Ile Cys Lys Met Glu Lys Cys
   385                 390                 395                 400
   Ser Ser Val Phe Asn Val Val Asn Ser Ser Ile Gly Leu Ile Met Val
                   405                 410                 415
   Leu Ser Phe Leu Phe Leu Asn
               420
<210> 16
   <211> 1143
   <212> DNA
   <213> Artificial Sequence
<220>
   <223> PfCSPhumanCO
<400> 16
   gccaccatgg ctaagaactt cattctgctt gccgtaagct ctattctctt ggtagacttg      60
   tttccaacac actgtggaca taatgtagat ctcagtaaag caattaacct caatggtgtt     120
   aattttaata atgtagacgc atcctctctc ggcgcggcac acgtgggtca aagtgccagt     180
   agggggcgcg gactcggcga gaacccggac gacgaagaag gggatgcgaa aaagaagaag     240
   gatgggaaga aggcagaacc caagaatcca agggagaata agctgaagca gccaggagat     300
   agagctgacg gccagcctgc cggcgatagg gccgacggac aacccgcggg agaccgcgcg     360
   gatggccaac ctgcgggtga ccgcgctgac gggcagcccg caggagacag agccgccgga     420
   caacccgccg gcgaccgagc ggatggtcag cccgcgggcg atcgagcgga cggtcagcca     480
   gctggagatc gcgcggacgg acaacctgcg ggggacaggg ctgacggtca accagccggt      540
   gatagagcag cgggccaacc ggcaggggac cgagccgcag ggcagcccgc cggggacagg     600
   gctgacgggc agccagcggg ggatagggcc gccggtcaac ctgcaggcga tcgagctgac     660
   ggccaacccg cgggtgaccg ggcagccggc caacctgctg gggatcgagc agacggacag     720
   ccggccggag atcgggccgc tggccaaccg gctggagaca gagccgctgg tcaacctgcc     780
   ggagatcggg ccgctgggca ggccgcgggt gacagggcag ccggacaagc agcagggggg     840
   aacgccggtg gacaaggaca gaacaatgag ggtgcgaatg ccccgaatga aaagagcgtt     900
   aaagagtacc tggataaggt tagagcaacc gtaggcaccg aatggacccc ctgctccgtg     960
   acttgcgggg taggtgttcg ggtcaggcgg agggtcaatg cggcgaacaa gaaaccggaa    1020
   gatctgactt tgaacgatct tgaaacagat gtatgcacga tggacaagtg tgctggcata    1080
   tttaacgtag tcagtaactc tctcggcctt gtgatcctct tggtcttggc gctgtttaac    1140
   tag                                                                  1143
<210> 17
   <211> 378
   <212> PRT
   <213> Plasmodium vivax
<220>
   <221> misc_feature
   <223> PvCSP
<400> 17
   Met Ala Lys Asn Phe Ile Leu Leu Ala Val Ser Ser Ile Leu Leu Val
   1               5                   10                  15
   Asp Leu Phe Pro Thr His Cys Gly His Asn Val Asp Leu Ser Lys Ala
               20                  25                  30
   Ile Asn Leu Asn Gly Val Asn Phe Asn Asn Val Asp Ala Ser Ser Leu
           35                  40                  45
   Gly Ala Ala His Val Gly Gln Ser Ala Ser Arg Gly Arg Gly Leu Gly
       50                  55                  60
   Glu Asn Pro Asp Asp Glu Glu Gly Asp Ala Lys Lys Lys Lys Asp Gly
   65                  70                  75                  80
   Lys Lys Ala Glu Pro Lys Asn Pro Arg Glu Asn Lys Leu Lys Gln Pro
                   85                  90                  95
   Gly Asp Arg Ala Asp Gly Gln Pro Ala Gly Asp Arg Ala Asp Gly Gln
               100                 105                 110
   Pro Ala Gly Asp Arg Ala Asp Gly Gln Pro Ala Gly Asp Arg Ala Asp
           115                 120                 125
   Gly Gln Pro Ala Gly Asp Arg Ala Ala Gly Gln Pro Ala Gly Asp Arg
       130                 135                 140
   Ala Asp Gly Gln Pro Ala Gly Asp Arg Ala Asp Gly Gln Pro Ala Gly
   145                 150                 155                 160
   Asp Arg Ala Asp Gly Gln Pro Ala Gly Asp Arg Ala Asp Gly Gln Pro
                   165                 170                 175
   Ala Gly Asp Arg Ala Ala Gly Gln Pro Ala Gly Asp Arg Ala Ala Gly
   180 185 190
   Gln Pro Ala Gly Asp Arg Ala Asp Gly Gln Pro Ala Gly Asp Arg Ala
   195 200 205
   Ala Gly Gln Pro Ala Gly Asp Arg Ala Asp Gly Gln Pro Ala Gly Asp
   210 215 220
   Arg Ala Ala Gly Gln Pro Ala Gly Asp Arg Ala Asp Gly Gln Pro Ala
   225 230 235 240
   Gly Asp Arg Ala Ala Gly Gln Pro Ala Gly Asp Arg Ala Ala Gly Gln
   245 250 255
   Pro Ala Gly Asp Arg Ala Ala Gly Gln Ala Ala Gly Asp Arg Ala Ala
   260 265 270
   Gly Gln Ala Ala Gly Gly Asn Ala Gly Gly Gln Gly Gln Asn Asn Glu
   275 280 285
   Gly Ala Asn Ala Pro Asn Glu Lys Ser Val Lys Glu Tyr Leu Asp Lys
   290 295 300
   Val Arg Ala Thr Val Gly Thr Glu Trp Thr Pro Cys Ser Val Thr Cys
   305 310 315 320
   Gly Val Gly Val Arg Val Arg Arg Arg Val Asn Ala Ala Asn Lys Lys
   325 330 335
   Pro Glu Asp Leu Thr Leu Asn Asp Leu Glu Thr Asp Val Cys Thr Met
   340 345 350
   Asp Lys Cys Ala Gly Ile Phe Asn Val Val Ser Asn Ser Leu Gly Leu
   355 360 365
   Val Ile Leu Leu Val Leu Ala Leu Phe Asn
   370 375
<210> 18
   <211> 368
   <212> PRT
   <213> Plasmodium vivax
<220>
   <221> misc_feature
   <223> CSP
<400> 18
   Met Lys Asn Phe Ile Leu Leu Ala Val Ser Ser Ile Leu Leu Val Asp
   1               5                   10                  15
   Leu Phe Pro Thr His Cys Gly His Asn Val Asp Leu Ser Lys Ala Ile
               20                  25                  30
   Asn Leu Asn Gly Val Asn Phe Asn Asn Val Asp Ala Ser Ser Leu Gly
           35                  40                  45
   Ala Ala His Val Gly Gln Ser Ala Ser Arg Gly Arg Gly Leu Gly Glu
       50                  55                  60
   Asn Pro Asp Asp Glu Glu Gly Asp Ala Lys Lys Lys Lys Asp Gly Lys
   65                  70                  75                  80
   Lys Ala Glu Pro Lys Asn Pro Arg Glu Asn Lys Leu Lys Gln Pro Gly
                   85                  90                  95
   Asp Arg Ala Asp Gly Gln Pro Ala Gly Asp Arg Ala Asp Gly Gln Pro
               100                 105                 110
   Ala Gly Asp Arg Ala Asp Gly Gln Pro Ala Gly Asp Arg Ala Gly Gln
           115                 120                 125
   Pro Ala Gly Asp Arg Ala Asp Gly Gln Pro Ala Gly Asp Arg Ala Asp
       130                 135                 140
   Gly Gln Pro Ala Gly Asp Arg Ala Asp Gly Gln Pro Ala Gly Asp Arg
   145                 150                 155                 160
   Ala Asp Gly Gln Pro Ala Gly Asp Arg Ala Gly Gln Pro Ala Gly Asp
                   165                 170                 175
   Arg Ala Gly Gln Pro Ala Gly Asp Arg Ala Asp Gly Gln Pro Ala Gly
               180                 185                 190
   Asp Arg Ala Gly Gln Pro Ala Gly Asp Arg Ala Asp Gly Gln Pro Ala
           195                 200                 205
   Gly Asp Arg Ala Gly Gln Pro Ala Gly Asp Arg Ala Asp Gly Gln Pro
       210                 215                 220
   Ala Gly Asp Arg Ala Gly Gln Pro Ala Gly Asp Arg Ala Gly Gln Pro
   225                 230                 235                 240
   Ala Gly Asp Arg Ala Gly Gln Pro Ala Gly Asp Arg Ala Gly Gln Pro
                   245                 250                 255
   Ala Gly Asn Gly Ala Gly Gly Gln Ala Ala Gly Gly Asn Ala Gly Gly
               260                 265                 270
   Gln Gly Gln Asn Asn Glu Gly Ala Asn Ala Pro Asn Glu Lys Ser Val
           275                 280                 285
   Lys Glu Tyr Leu Asp Lys Val Arg Ala Thr Val Gly Thr Glu Trp Thr
       290                 295                 300
   Pro Cys Ser Val Thr Cys Gly Val Gly Val Arg Val Arg Arg Arg Val
   305                 310                 315                 320
   Asn Ala Ala Asn Lys Lys Pro Glu Asp Leu Thr Leu Asn Asp Leu Glu
                   325                 330                 335
   Thr Asp Val Cys Thr Met Asp Lys Cys Ala Gly Ile Phe Asn Val Val
               340                 345                 350
   Ser Asn Ser Leu Gly Leu Val Ile Leu Leu Val Leu Ala Leu Phe Asn
           355                 360                 365
<210> 19
   <211> 1842
   <212> DNA
   <213> Plasmodium berghei
<220>
   <221> misc_feature
   <223> thrombospondin-related anonymous protein (TRAP)
<400> 19
   ggatccgcca ccatggctaa gcttctgggg aacagtaaat acttctttgt ggtcctgctg 60
   ctgtgcatta gcgttttcct caacggtcag gagattctgg acgaaatcaa gtactctgag 120
   gaggtctgca acgaacaaat cgatctccac attctgctgg atgggagtgg cagcataggt 180
   cactctaact ggatcagtca cgtgataccc atgctgacaa cccttgtgga caatttgaac 240
   atcagccgcg atgagatcaa tatctccatg accttgttct ccacttatgc cagggaactt 300
   gtgagactta agagatatgg gtctacaagc aaagccagtc tgaggttcat catcgcgcaa 360
   ctccagaata actattctcc tcatggaacg acaaatctga ctagcgccct gttgaatgtg 420
   gacaatctca ttcagaagaa aatgaatcgc cctaatgcca ttcagctcgt gattatcctt 480
   actgacggca tccctaacaa tctgaagaag tccactactg ttgtcaacca gctgaagaag 540
   aaggacgtca atgtcgctat tattggtgtt ggcgccggag taaacaatat gtttaaccgt 600
   atattggtag gatgtggaaa acttgggcct tgtccctact actcttatgg ctcttgggat 660
   caagcacaaa ccatgatcaa accatttctc tcaaaggtct gtcaggaagt ggagaaagtg 720
   gcactgtgcg gtaagtggga ggagtggagt gagtgttcaa ccacttgcga caacggaacg 780
   aaaataagga agcgaaaggt tctccatccc aactgtgccg gggaaatgac agccccatgt 840
   aaagtgcggg actgtcctcc caaacctgta gcccctccgg tcattcccat caaagtccct 900
   gacgtgcctg tgaaaccagt cgaacctatt gagcccgccg agccagcaga gccagcagaa 960
   ccagcagagc ctgcagaacc cgccgaaccc gctgagcccg cggagcccgc cgaacccgct 1020
   gaaccggcag aacccgcgga accagcggag cctgcagagc cagctgagcc tgctgaaccg 1080
   gcggagcccg ctgaaccagc cgagcctgct aaaccggccg aaccggcaga gcccgctgag 1140
   cctgccgagc cagcggaacc agttaacccc gataatccta tcctgccgat caagcccgag 1200
   gagccatctg gtggagccga gccattgaat ccagaggtcg agaatccctt tatcatcccc 1260
   gacgaaccca tcgaacccat tattgcgcca ggagctgtac cggataagcc catcattcct 1320
   gaggaatcaa atgagctgcc aaacaatctt ccagagtctc cctccgatag tcaggtggag 1380
   tatcctcggc caaacgacaa tggggataac agcaacaaca caatcaattc caacaagaac 1440
   ataccaaata agcatgtgcc tcctacagac gacaacccct acaagggcca ggaagaacga 1500
   atccctaagc cgcatcggag caacgacgaa tacatttact acaataatgc taacaataac 1560
   gacaagctgg agcccgagat accctctaag gattacgagg aaaacaagag caagaaacag 1620
   agcaaaagca acaatggcta taagatcgcc ggcggcataa ttggcgggct ggctattatc 1680
   ggctgcattg gagtgggcta taacttcata gccgggtcct ccgccgccgc tatggctgga 1740
   gaggcggcac cttttgagga cgtgatggct gatgatgaga aggggatcgt ggaaaacgaa 1800
   cagttcaaac tgccagagga caatgattgg aattgactcg ag 1842
<210> 20
   <211> 607
   <212> PRT
   <213> Plasmodium berghei
<220>
   <221> misc_feature
   <223> TRAP
<400> 20
   Met Ala Lys Leu Leu Gly Asn Ser Lys Tyr Phe Phe Val Val Leu Leu
   1               5                   10                  15
   Leu Cys Ile Ser Val Phe Leu Asn Gly Gln Glu Ile Leu Asp Glu Ile
               20                  25                  30
   Lys Tyr Ser Glu Glu Val Cys Asn Glu Gln Ile Asp Leu His Ile Leu
           35                  40                  45
   Leu Asp Gly Ser Gly Ser Ile Gly His Ser Asn Trp Ile Ser His Val
       50                  55                  60
   Ile Pro Met Leu Thr Thr Leu Val Asp Asn Leu Asn Ile Ser Arg Asp
   65                  70                  75                  80
   Glu Ile Asn Ile Ser Met Thr Leu Phe Ser Thr Tyr Ala Arg Glu Leu
                   85                  90                  95
   Val Arg Leu Lys Arg Tyr Gly Ser Thr Ser Lys Ala Ser Leu Arg Phe
               100                 105                 110
   Ile Ile Ala Gln Leu Gln Asn Asn Tyr Ser Pro His Gly Thr Thr Asn
           115                 120                 125
   Leu Thr Ser Ala Leu Leu Asn Val Asp Asn Leu Ile Gln Lys Lys Met
       130                 135                 140
   Asn Arg Pro Asn Ala Ile Gln Leu Val Ile Ile Leu Thr Asp Gly Ile
   145                 150                 155                 160
   Pro Asn Asn Leu Lys Lys Ser Thr Thr Val Val Asn Gln Leu Lys Lys
                   165                 170                 175
   Lys Asp Val Asn Val Ala Ile Ile Gly Val Gly Ala Gly Val Asn Asn
               180                 185                 190
   Met Phe Asn Arg Ile Leu Val Gly Cys Gly Lys Leu Gly Pro Cys Pro
           195                 200                 205
   Tyr Tyr Ser Tyr Gly Ser Trp Asp Gln Ala Gln Thr Met Ile Lys Pro
       210                 215                 220
   Phe Leu Ser Lys Val Cys Gln Glu Val Glu Lys Val Ala Leu Cys Gly
   225                 230                 235                 240
   Lys Trp Glu Glu Trp Ser Glu Cys Ser Thr Thr Cys Asp Asn Gly Thr
                   245                 250                 255
   Lys Ile Arg Lys Arg Lys Val Leu His Pro Asn Cys Ala Gly Glu Met
               260                 265                 270
   Thr Ala Pro Cys Lys Val Arg Asp Cys Pro Pro Lys Pro Val Ala Pro
           275                 280                 285
   Pro Val Ile Pro Ile Lys Val Pro Asp Val Pro Val Lys Pro Val Glu
       290                 295                 300
   Pro Ile Glu Pro Ala Glu Pro Ala Glu Pro Ala Glu Pro Ala Glu Pro
   305                 310                 315                 320
   Ala Glu Pro Ala Glu Pro Ala Glu Pro Ala Glu Pro Ala Glu Pro Ala
                   325                 330                 335
   Glu Pro Ala Glu Pro Ala Glu Pro Ala Glu Pro Ala Glu Pro Ala Glu
               340                 345                 350
   Pro Ala Glu Pro Ala Glu Pro Ala Glu Pro Ala Glu Pro Ala Lys Pro
           355                 360                 365
   Ala Glu Pro Ala Glu Pro Ala Glu Pro Ala Glu Pro Ala Glu Pro Val
       370                 375                 380
   Asn Pro Asp Asn Pro Ile Leu Pro Ile Lys Pro Glu Glu Pro Ser Gly
   385                 390                 395                 400
   Gly Ala Glu Pro Leu Asn Pro Glu Val Glu Asn Pro Phe Ile Ile Pro
                   405                 410                 415
   Asp Glu Pro Ile Glu Pro Ile Ile Ala Pro Gly Ala Val Pro Asp Lys
               420                 425                 430
   Pro Ile Ile Pro Glu Glu Ser Asn Glu Leu Pro Asn Asn Leu Pro Glu
           435                 440                 445
   Ser Pro Ser Asp Ser Gln Val Glu Tyr Pro Arg Pro Asn Asp Asn Gly
       450                 455                 460
   Asp Asn Ser Asn Asn Thr Ile Asn Ser Asn Lys Asn Ile Pro Asn Lys
   465                 470                 475                 480
   His Val Pro Pro Thr Asp Asp Asn Pro Tyr Lys Gly Gln Glu Glu Arg
                   485                 490                 495
   Ile Pro Lys Pro His Arg Ser Asn Asp Glu Tyr Ile Tyr Tyr Asn Asn
               500                 505                 510
   Ala Asn Asn Asn Asp Lys Leu Glu Pro Glu Ile Pro Ser Lys Asp Tyr
           515                 520                 525
   Glu Glu Asn Lys Ser Lys Lys Gln Ser Lys Ser Asn Asn Gly Tyr Lys
       530                 535                 540
   Ile Ala Gly Gly Ile Ile Gly Gly Leu Ala Ile Ile Gly Cys Ile Gly
   545 550 555 560
   Val Gly Tyr Asn Phe Ile Ala Gly Ser Ser Ala Ala Ala Met Ala Gly
   565 570 575
   Glu Ala Ala Pro Phe Glu Asp Val Met Ala Asp Asp Glu Lys Gly Ile
   580 585 590
   Val Glu Asn Glu Gln Phe Lys Leu Pro Glu Asp Asn Asp Trp Asn
   595 600 605
<210> 21
   <211> 606
   <212> PRT
   <213> Plasmodium berghei
<220>
   <221> misc_feature
   <223> TRAP
<400> 21
   Met Lys Leu Leu Gly Asn Ser Lys Tyr Phe Phe Val Val Leu Leu Leu
   1 5 10 15
   Cys Ile Ser Val Phe Leu Asn Gly Gln Glu Ile Leu Asp Glu Ile Lys
   20 25 30
   Tyr Ser Glu Glu Val Cys Asn Glu Gln Ile Asp Leu His Ile Leu Leu
   35 40 45
   Asp Gly Ser Gly Ser Ile Gly His Ser Asn Trp Ile Ser His Val Ile
   50 55 60
   Pro Met Leu Thr Thr Leu Val Asp Asn Leu Asn Ile Ser Arg Asp Glu
   65 70 75 80
   Ile Asn Ile Ser Met Thr Leu Phe Ser Thr Tyr Ala Arg Glu Leu Val
   85 90 95
   Arg Leu Lys Arg Tyr Gly Ser Thr Ser Lys Ala Ser Leu Arg Phe Ile
   100 105 110
   Ile Ala Gln Leu Gln Asn Asn Tyr Ser Pro His Gly Thr Thr Asn Leu
   115 120 125
   Thr Ser Ala Leu Leu Asn Val Asp Asn Leu Ile Gln Lys Lys Met Asn
   130 135 140
   Arg Pro Asn Ala Ile Gln Leu Val Ile Ile Leu Thr Asp Gly Ile Pro
   145                 150                 155                 160
   Asn Asn Leu Lys Lys Ser Thr Thr Val Val Asn Gln Leu Lys Lys Lys
                   165                 170                 175
   Asp Val Asn Val Ala Ile Ile Gly Val Gly Ala Gly Val Asn Asn Met
               180                 185                 190
   Phe Asn Arg Ile Leu Val Gly Cys Gly Lys Leu Gly Pro Cys Pro Tyr
           195                 200                 205
   Tyr Ser Tyr Gly Ser Trp Asp Gln Ala Gln Thr Met Ile Lys Pro Phe
       210                 215                 220
   Leu Ser Lys Val Cys Gln Glu Val Glu Lys Val Ala Leu Cys Gly Lys
   225                 230                 235                 240
   Trp Glu Glu Trp Ser Glu Cys Ser Thr Thr Cys Asp Asn Gly Thr Lys
                   245                 250                 255
   Ile Arg Lys Arg Lys Val Leu His Pro Asn Cys Ala Gly Glu Met Thr
               260                 265                 270
   Ala Pro Cys Lys Val Arg Asp Cys Pro Pro Lys Pro Val Ala Pro Pro
           275                 280                 285
   Val Ile Pro Ile Lys Val Pro Asp Val Pro Val Lys Pro Val Glu Pro
       290                 295                 300
   Ile Glu Pro Ala Glu Pro Ala Glu Pro Ala Glu Pro Ala Glu Pro Ala
   305                 310                 315                 320
   Glu Pro Ala Glu Pro Ala Glu Pro Ala Glu Pro Ala Glu Pro Ala Glu
                   325                 330                 335
   Pro Ala Glu Pro Ala Glu Pro Ala Glu Pro Ala Glu Pro Ala Glu Pro
               340                 345                 350
   Ala Glu Pro Ala Glu Pro Ala Glu Pro Ala Glu Pro Ala Lys Pro Ala
           355                 360                 365
   Glu Pro Ala Glu Pro Ala Glu Pro Ala Glu Pro Ala Glu Pro Val Asn
       370                 375                 380
   Pro Asp Asn Pro Ile Leu Pro Ile Lys Pro Glu Glu Pro Ser Gly Gly
   385                 390                 395                 400
   Ala Glu Pro Leu Asn Pro Glu Val Glu Asn Pro Phe Ile Ile Pro Asp
                   405                 410                 415
   Glu Pro Ile Glu Pro Ile Ile Ala Pro Gly Ala Val Pro Asp Lys Pro
               420                 425                 430
   Ile Ile Pro Glu Glu Ser Asn Glu Leu Pro Asn Asn Leu Pro Glu Ser
           435                 440                 445
   Pro Ser Asp Ser Gln Val Glu Tyr Pro Arg Pro Asn Asp Asn Gly Asp
       450                 455                 460
   Asn Ser Asn Asn Thr Ile Asn Ser Asn Lys Asn Ile Pro Asn Lys His
   465                 470                 475                 480
   Val Pro Pro Thr Asp Asp Asn Pro Tyr Lys Gly Gln Glu Glu Arg Ile
                   485                 490                 495
   Pro Lys Pro His Arg Ser Asn Asp Glu Tyr Ile Tyr Tyr Asn Asn Ala
               500                 505                 510
   Asn Asn Asn Asp Lys Leu Glu Pro Glu Ile Pro Ser Lys Asp Tyr Glu
           515                 520                 525
   Glu Asn Lys Ser Lys Lys Gln Ser Lys Ser Asn Asn Gly Tyr Lys Ile
       530                 535                 540
   Ala Gly Gly Ile Ile Gly Gly Leu Ala Ile Ile Gly Cys Ile Gly Val
   545                 550                 555                 560
   Gly Tyr Asn Phe Ile Ala Gly Ser Ser Ala Ala Ala Met Ala Gly Glu
                   565                 570                 575
   Ala Ala Pro Phe Glu Asp Val Met Ala Asp Asp Glu Lys Gly Ile Val
               580                 585                 590
   Glu Asn Glu Gln Phe Lys Leu Pro Glu Asp Asn Asp Trp Asn
           595                 600                 605
<210> 22
   <211> 1734
   <212> DNA
   <213> Artificial Sequence
<220>
   <223> thrombospondin-related anonymous protein (TRAP)
<400> 22
   gccaccatgg ctaatcactt gggcaacgtg aagtacctgg tgatagtgtt cctgatcttc 60
   ttcgacctgt ttctggtcaa cggcagagat gtgcagaaca acatagtcga cgagattaag 120
   tacagagagg aggtgtgcaa tgacgaggtg gatctctacc tgctgatgga ctgtagcggg 180
   tcaatacgac ggcacaattg ggttaatcat gctgtcccct tggctatgaa gctgatccag 240
   cagctcaatc tgaacgataa tgccattcac ctgtatgctt ccgtgttcag caataatgca 300
   agggagatca ttcgcctcca cagtgacgct agtaagaata aggagaaagc cctgatcatc 360
   atcaaatccc tgcttagcac caaccttccc tatggcaaga ccaatctcac agacgcgctt 420
   ctgcaggtaa ggaagcatct gaacgatcgc atcaacagag agaatgcgaa tcagctggta 480
   gtaatcctga ctgatgggat tcccgattcc atccaggaca gcctgaagga atcaaggaag 540
   ctcagcgata gaggggtgaa aatcgcagtt ttcggaatcg gtcaggggat taacgtggcc 600
   tttaatcgct ttctggtggg atgtcatcca tctgatggga aatgcaatct gtatgccgat 660
   tctgcgtggg agaatgtgaa gaacgtgatt ggccccttta tgaaagccgt gtgcgtagag 720
   gtggagaaaa ccgcctcctg tggagtttgg gacgaatgga gcccctgttc cgttacttgc 780
   gggaaaggga cccgaagtcg caagagagaa atccttcacg agggttgcac ttctgaactc 840
   caagagcaat gcgaggagga aaggtgtctc cctaaacgag aaccccttga tgtgcctgac 900
   gaacctgagg acgatcaacc aagaccaagg ggagacaact tcgccgtcga gaaacccaat 960
   gagaatatca ttgacaacaa tccacaggag ccatcaccta atccagagga aggcaaaggc 1020
   gaaaacccta acggttttga cttggacgag aatccggaaa atccacccaa cccgcctaac 1080
   cctccaaatc caccaaaccc tccgaatcct cctaaccctg atatccccga gcaagagcct 1140
   aatattccag aggatagcga aaaggaggtc ccctctgacg tccctaagaa ccctgaagat 1200
   gatcgcgaag agaactttga catacccaag aaacccgaga ataaacacga taatcagaac 1260
   aatctcccga acgataaatc tgaccgctac attccataca gtccattgag cccaaaggtc 1320
   ttggataatg aacggaaaca gtccgaccct caatcacagg acaataacgg taatcgtcat 1380
   gttccgaact ccgaagatcg ggaaacaagg ccccatgggc gtaacaacga aaaccggagc 1440
   tataaccgga agcacaacaa cacacccaag cacccggaac gggaagaaca tgagaagccc 1500
   gataacaaca agaagaaggc aggctctgac aacaaataca aaatagccgg aggtattgcc 1560
   ggaggattgg cactgctggc ctgcgctggc cttgcttaca agttcgtcgt tccaggcgcc 1620
   gcaacgccct atgcaggcga accagctccc tttgacgaga cactgggaga agaggacaaa 1680
   gacctggacg agcctgaaca gttcagactg cccgaggaaa acgagtggaa ttaa 1734
<210> 23
   <211> 575
   <212> PRT
   <213> Artificial Sequence
<220>
   <223> PfTRAP
<400> 23
   Met Ala Asn His Leu Gly Asn Val Lys Tyr Leu Val Ile Val Phe Leu
   1               5                   10                  15
   Ile Phe Phe Asp Leu Phe Leu Val Asn Gly Arg Asp Val Gln Asn Asn
               20                  25                  30
   Ile Val Asp Glu Ile Lys Tyr Arg Glu Glu Val Cys Asn Asp Glu Val
           35                  40                  45
   Asp Leu Tyr Leu Leu Met Asp Cys Ser Gly Ser Ile Arg Arg His Asn
       50                  55                  60
   Trp Val Asn His Ala Val Pro Leu Ala Met Lys Leu Ile Gln Gln Leu
   65                  70                  75                  80
   Asn Leu Asn Asp Asn Ala Ile His Leu Tyr Ala Ser Val Phe Ser Asn
                   85                  90                  95
   Asn Ala Arg Glu Ile Ile Arg Leu His Ser Asp Ala Ser Lys Asn Lys
               100                 105                 110
   Glu Lys Ala Leu Ile Ile Ile Lys Ser Leu Leu Ser Thr Asn Leu Pro
           115                 120                 125
   Tyr Gly Lys Thr Asn Leu Thr Asp Ala Leu Leu Gln Val Arg Lys His
       130                 135                 140
   Leu Asn Asp Arg Ile Asn Arg Glu Asn Ala Asn Gln Leu Val Val Ile
   145                 150                 155                 160
   Leu Thr Asp Gly Ile Pro Asp Ser Ile Gln Asp Ser Leu Lys Glu Ser
                   165                 170                 175
   Arg Lys Leu Ser Asp Arg Gly Val Lys Ile Ala Val Phe Gly Ile Gly
               180                 185                 190
   Gln Gly Ile Asn Val Ala Phe Asn Arg Phe Leu Val Gly Cys His Pro
           195                 200                 205
   Ser Asp Gly Lys Cys Asn Leu Tyr Ala Asp Ser Ala Trp Glu Asn Val
       210                 215                 220
   Lys Asn Val Ile Gly Pro Phe Met Lys Ala Val Cys Val Glu Val Glu
   225                 230                 235                 240
   Lys Thr Ala Ser Cys Gly Val Trp Asp Glu Trp Ser Pro Cys Ser Val
                   245                 250                 255
   Thr Cys Gly Lys Gly Thr Arg Ser Arg Lys Arg Glu Ile Leu His Glu
               260                 265                 270
   Gly Cys Thr Ser Glu Leu Gln Glu Gln Cys Glu Glu Glu Arg Cys Leu
           275                 280                 285
   Pro Lys Arg Glu Pro Leu Asp Val Pro Asp Glu Pro Glu Asp Asp Gln
       290                 295                 300
   Pro Arg Pro Arg Gly Asp Asn Phe Ala Val Glu Lys Pro Asn Glu Asn
   305                 310                 315                 320
   Ile Ile Asp Asn Asn Pro Gln Glu Pro Ser Pro Asn Pro Glu Glu Gly
                   325                 330                 335
   Lys Gly Glu Asn Pro Asn Gly Phe Asp Leu Asp Glu Asn Pro Glu Asn
               340                 345                 350
   Pro Pro Asn Pro Pro Asn Pro Pro Asn Pro Pro Asn Pro Pro Asn Pro
           355                 360                 365
   Pro Asn Pro Asp Ile Pro Glu Gln Glu Pro Asn Ile Pro Glu Asp Ser
       370                 375                 380
   Glu Lys Glu Val Pro Ser Asp Val Pro Lys Asn Pro Glu Asp Asp Arg
   385                 390                 395                 400
   Glu Glu Asn Phe Asp Ile Pro Lys Lys Pro Glu Asn Lys His Asp Asn
                   405                 410                 415
   Gln Asn Asn Leu Pro Asn Asp Lys Ser Asp Arg Tyr Ile Pro Tyr Ser
               420                 425                 430
   Pro Leu Ser Pro Lys Val Leu Asp Asn Glu Arg Lys Gln Ser Asp Pro
           435                 440                 445
   Gln Ser Gln Asp Asn Asn Gly Asn Arg His Val Pro Asn Ser Glu Asp
       450                 455                 460
   Arg Glu Thr Arg Pro His Gly Arg Asn Asn Glu Asn Arg Ser Tyr Asn
   465                 470                 475                 480
   Arg Lys His Asn Asn Thr Pro Lys His Pro Glu Arg Glu Glu His Glu
                       485                 490                 495
   Lys Pro Asp Asn Asn Lys Lys Lys Ala Gly Ser Asp Asn Lys Tyr Lys
               500                 505                 510
   Ile Ala Gly Gly Ile Ala Gly Gly Leu Ala Leu Leu Ala Cys Ala Gly
           515                 520                 525
   Leu Ala Tyr Lys Phe Val Val Pro Gly Ala Ala Thr Pro Tyr Ala Gly
       530                 535                 540
   Glu Pro Ala Pro Phe Asp Glu Thr Leu Gly Glu Glu Asp Lys Asp Leu
   545                 550                 555                 560
   Asp Glu Pro Glu Gln Phe Arg Leu Pro Glu Glu Asn Glu Trp Asn
                   565                 570                 575
<210> 24
   <211> 560
   <212> PRT
   <213> Artificial Sequence
<220>
   <223> TRAP
<400> 24
   Met Asn His Leu Gly Asn Val Lys Tyr Leu Val Ile Val Phe Leu Ile
   1               5                   10                  15
   Phe Phe Asp Leu Phe Leu Val Asn Gly Arg Asp Val Gln Asn Asn Ile
               20                  25                  30
   Val Asp Glu Ile Lys Tyr Arg Glu Glu Val Cys Asn Asp Glu Val Asp
           35                  40                  45
   Leu Tyr Leu Leu Met Asp Cys Ser Gly Ser Ile Arg Arg His Asn Trp
       50                  55                  60
   Val Asn His Ala Val Pro Leu Ala Met Lys Leu Ile Gln Gln Leu Asn
   65                  70                  75                  80
   Leu Asn Glu Asn Ala Ile His Leu Tyr Ala Asn Val Phe Ser Asn Asn
                   85                  90                  95
   Ala Arg Glu Ile Ile Arg Leu His Ser Asp Ala Ser Lys Asn Lys Glu
               100                 105                 110
   Lys Ala Leu Ser Ile Ile Lys Ser Leu Leu Ser Thr Asn Leu Pro Phe
           115                 120                 125
   Gly Arg Thr Asn Leu Thr Asp Ala Leu Leu Gln Val Arg Lys His Leu
       130                 135                 140
   Asn Asp Arg Ile Asn Arg Glu Asn Ala Asn Gln Leu Val Val Ile Leu
   145                 150                 155                 160
   Thr Asp Gly Ile Pro Asn Ser Ile Gln Asp Ser Leu Lys Glu Ser Arg
                   165                 170                 175
   Lys Leu Ser Asp Arg Gly Val Lys Ile Ala Val Phe Gly Ile Gly Gln
               180                 185                 190
   Gly Ile Asn Val Ala Phe Asn Arg Phe Leu Val Gly Cys His Pro Ser
           195                 200                 205
   Asp Gly Lys Cys Asn Leu Tyr Ala Asp Ser Ala Trp Glu Asn Val Lys
       210                 215                 220
   Asn Val Ile Gly Pro Phe Met Lys Ala Val Cys Val Glu Val Glu Lys
   225                 230                 235                 240
   Thr Ala Ser Cys Gly Val Trp Asp Glu Trp Ser Pro Cys Ser Val Thr
                   245                 250                 255
   Cys Gly Lys Gly Thr Arg Ser Arg Lys Arg Glu Ile Leu His Glu Gly
               260                 265                 270
   Cys Thr Ser Glu Leu Gln Glu Gln Cys Glu Glu Glu Arg Cys Pro Pro
           275                 280                 285
   Lys Trp Glu Pro Leu Asp Val Pro Asp Asp Pro Glu Asp Asp Gln Pro
       290                 295                 300
   Arg Pro Arg Gly Asp Asn Ser Ser Val Gln Lys Pro Glu Glu Asn Ile
   305                 310                 315                 320
   Ile Asp Asn Asn Pro Gln Glu Pro Ser Pro Asn Pro Glu Glu Gly Lys
                   325                 330                 335
   Gly Glu Asn Pro Asn Gly Phe Asp Leu Asp Glu Asn Pro Glu Asn Pro
               340                 345                 350
   Pro Asn Pro Asp Ile Pro Gln Gln Glu Pro Asn Ile Pro Glu Asp Ser
           355                 360                 365
   Glu Lys Glu Val Pro Ser Asp Val Pro Lys Asn Pro Glu Asp Asp Arg
   370 375 380
   Glu Glu Asn Phe Asp Ile Pro Lys Lys Pro Glu Asn Lys His Asp Asn
   385 390 395 400
   Gln Asn Asn Leu Pro Asn Asp Lys Ser Asp Arg Tyr Ile Pro Tyr Ser
   405 410 415
   Pro Leu Pro Pro Asn Val Leu Asp Asn Glu Arg Lys Gln Ser Asp Pro
   420 425 430
   Gln Ser Gln Asp Asn Asn Gly Asn Arg His Val Pro Asn Ser Glu Asp
   435 440 445
   Arg Glu Thr Arg Pro His Gly Arg Asn Asn Glu Asn Arg Ser Tyr Asn
   450 455 460
   Arg Lys His Asn Asp Thr Pro Lys His Pro Glu Arg Glu Glu His Glu
   465 470 475 480
   Lys Pro Asp Asn Asn Lys Lys Lys Gly Gly Ser Ser Asp Asn Lys Tyr
   485 490 495
   Lys Ile Ala Gly Gly Ile Ala Gly Gly Leu Ala Leu Leu Ala Cys Ala
   500 505 510
   Gly Leu Ala Tyr Lys Phe Val Val Pro Gly Ala Ala Thr Pro Tyr Ala
   515 520 525
   Gly Glu Pro Ala Pro Phe Asp Glu Thr Leu Gly Glu Glu Asp Lys Asp
   530 535 540
   Leu Asp Glu Pro Glu Gln Phe Arg Leu Pro Glu Glu Asn Glu Trp Asn
   545 550 555 560
<210> 25
   <211> 1680
   <212> DNA
   <213> Artificial Sequence
<220>
   <223> PvTRAPhumanCO
<400> 25
   gccaccatgg ctaagctgct gcagaacaag tcttatctcc tggtcgtgtt cctgctttac      60
   gtatccatat ttgcacgggg agatgaaaaa gttgtagatg aggtaaagta cagcgaggag     120
   gtatgcaacg aatctgtcga tctctatctt ctcgtagatg gctctggtag tattgggtat     180
   ccgaactgga taactaaggt aattcctatg ctgaacggtc ttattaatag tttgagcctc     240
   agtcgagaca cgatcaatct ctatatgaat ctcttcggaa attacactac agaactcata     300
   cggctgggct ctgggcagag tatagataaa cgacaggctc tgtcaaaggt gactgaactt     360
   agaaagacct acacgccata tggcactact aacatgacag ccgcgcttga tgaagtccaa     420
   aagcacctta atgatagagt gaaccgagag aaagcgatac aacttgtcat attgatgacc     480
   gacggggtgc ccaactcaaa atacagggcg ctggaggtcg caaataaact gaagcaacgc     540
   aatgtttcct tggccgttat tggtgtcgga caaggaatca accatcagtt caatcggctg     600
   atcgcgggct gccgacctcg ggagccgaac tgcaaatttt actcttatgc tgattggaat     660
   gaggccgtag cacttataaa gccctttatt gctaaggtat gcacagaagt tgagcgcgtt     720
   gccaactgtg gaccctggga cccctggacc gcgtgtagcg ttacgtgcgg caggggaacg     780
   cactccaggt ctcgcccttc attgcatgaa aagtgcacta cccatatggt gtctgaatgt     840
   gaagaggggg aatgcccagt ggagcctgag ccactgccag tacctgcgcc actcccaacg     900
   gttccagaag atgtaaatcc gcgagacacg gacgacgaaa atgagaaccc gaacttcaac     960
   aagggactgg acgtgccgga tgaagatgat gacgaagtac cgcccgcgaa tgaaggtgcg    1020
   gatggcaacc cagtcgagga aaatgttttc ccgcctgctg acgatagcgt tcctgatgag    1080
   agtaacgtgc tgcctctccc tccggcagtc ccaggcgggt cctctgagga gttcccggcg    1140
   gacgttcaga acaatcccga ctctcctgaa gagctgccga tggagcaaga agtgcctcaa    1200
   gataataacg tgaatgaacc agagagatcc gatagcaacg gttatggcgt aaatgagaag    1260
   gtgataccta atcccctcga caatgagcga gacatggcca ataagaataa gaccgttcac    1320
   ccaggccgga aggacagcgc gagagatcga tatgcccgcc cccatggttc cactcatgtc    1380
   aataataata gagccaatga gaactcagac ataccaaaca acccagtacc ttctgattac    1440
   gaacagccgg aggacaaggc taaaaagtcc tccaataacg gctataaaat cgctggtgga    1500
   gtgatcgctg ggctcgcgtt ggttgggtgc gtcggtttcg cgtataactt cgtagcgggt    1560
   ggaggcgctg ctggtatggc aggcgaaccg gctccctttg acgaagctat ggccgaagac    1620
   gaaaaagacg tagccgaagc ggaccagttc aaattgcctg aggacaacga ttggaattaa    1680
<210> 26
   <211> 557
   <212> PRT
   <213> Plasmodium vivax
<220>
   <221> misc_feature
   <223> PvTRAP
<400> 26
   Met Ala Lys Leu Leu Gln Asn Lys Ser Tyr Leu Leu Val Val Phe Leu
   1 5 10 15
   Leu Tyr Val Ser Ile Phe Ala Arg Gly Asp Glu Lys Val Val Asp Glu
   20 25 30
   Val Lys Tyr Ser Glu Glu Val Cys Asn Glu Ser Val Asp Leu Tyr Leu
   35 40 45
   Leu Val Asp Gly Ser Gly Ser Ile Gly Tyr Pro Asn Trp Ile Thr Lys
   50 55 60
   Val Ile Pro Met Leu Asn Gly Leu Ile Asn Ser Leu Ser Leu Ser Arg
   65 70 75 80
   Asp Thr Ile Asn Leu Tyr Met Asn Leu Phe Gly Asn Tyr Thr Thr Glu
   85 90 95
   Leu Ile Arg Leu Gly Ser Gly Gln Ser Ile Asp Lys Arg Gln Ala Leu
   100 105 110
   Ser Lys Val Thr Glu Leu Arg Lys Thr Tyr Thr Pro Tyr Gly Thr Thr
   115 120 125
   Asn Met Thr Ala Ala Leu Asp Glu Val Gln Lys His Leu Asn Asp Arg
   130 135 140
   Val Asn Arg Glu Lys Ala Ile Gln Leu Val Ile Leu Met Thr Asp Gly
   145 150 155 160
   Val Pro Asn Ser Lys Tyr Arg Ala Leu Glu Val Ala Asn Lys Leu Lys
   165 170 175
   Gln Arg Asn Val Ser Leu Ala Val Ile Gly Val Gly Gln Gly Ile Asn
   180 185 190
   His Gln Phe Asn Arg Leu Ile Ala Gly Cys Arg Pro Arg Glu Pro Asn
   195 200 205
   Cys Lys Phe Tyr Ser Tyr Ala Asp Trp Asn Glu Ala Val Ala Leu Ile
   210 215 220
   Lys Pro Phe Ile Ala Lys Val Cys Thr Glu Val Glu Arg Val Ala Asn
   225 230 235 240
   Cys Gly Pro Trp Asp Pro Trp Thr Ala Cys Ser Val Thr Cys Gly Arg
   245 250 255
   Gly Thr His Ser Arg Ser Arg Pro Ser Leu His Glu Lys Cys Thr Thr
               260                 265                 270
   His Met Val Ser Glu Cys Glu Glu Gly Glu Cys Pro Val Glu Pro Glu
           275                 280                 285
   Pro Leu Pro Val Pro Ala Pro Leu Pro Thr Val Pro Glu Asp Val Asn
       290                 295                 300
   Pro Arg Asp Thr Asp Asp Glu Asn Glu Asn Pro Asn Phe Asn Lys Gly
   305                 310                 315                 320
   Leu Asp Val Pro Asp Glu Asp Asp Asp Glu Val Pro Pro Ala Asn Glu
                   325                 330                 335
   Gly Ala Asp Gly Asn Pro Val Glu Glu Asn Val Phe Pro Pro Ala Asp
               340                 345                 350
   Asp Ser Val Pro Asp Glu Ser Asn Val Leu Pro Leu Pro Pro Ala Val
           355                 360                 365
   Pro Gly Gly Ser Ser Glu Glu Phe Pro Ala Asp Val Gln Asn Asn Pro
       370                 375                 380
   Asp Ser Pro Glu Glu Leu Pro Met Glu Gln Glu Val Pro Gln Asp Asn
   385                 390                 395                 400
   Asn Val Asn Glu Pro Glu Arg Ser Asp Ser Asn Gly Tyr Gly Val Asn
                   405                 410                 415
   Glu Lys Val Ile Pro Asn Pro Leu Asp Asn Glu Arg Asp Met Ala Asn
               420                 425                 430
   Lys Asn Lys Thr Val His Pro Gly Arg Lys Asp Ser Ala Arg Asp Arg
           435                 440                 445
   Tyr Ala Arg Pro His Gly Ser Thr His Val Asn Asn Asn Arg Ala Asn
       450                 455                 460
   Glu Asn Ser Asp Ile Pro Asn Asn Pro Val Pro Ser Asp Tyr Glu Gln
   465                 470                 475                 480
   Pro Glu Asp Lys Ala Lys Lys Ser Ser Asn Asn Gly Tyr Lys Ile Ala
                   485                 490                 495
   Gly Gly Val Ile Ala Gly Leu Ala Leu Val Gly Cys Val Gly Phe Ala
               500                 505                 510
   Tyr Asn Phe Val Ala Gly Gly Gly Ala Ala Gly Met Ala Gly Glu Pro
   515 520 525
   Ala Pro Phe Asp Glu Ala Met Ala Glu Asp Glu Lys Asp Val Ala Glu
   530 535 540
   Ala Asp Gln Phe Lys Leu Pro Glu Asp Asn Asp Trp Asn
   545 550 555
<210> 27
   <211> 556
   <212> PRT
   <213> Plasmodium vivax
<220>
   <221> misc_feature
   <223> TRAP
<400> 27
   Met Lys Leu Leu Gln Asn Lys Ser Tyr Leu Leu Val Val Phe Leu Leu
   1 5 10 15
   Tyr Val Ser Ile Phe Ala Arg Gly Asp Glu Lys Val Val Asp Glu Val
   20 25 30
   Lys Tyr Ser Glu Glu Val Cys Asn Glu Ser Val Asp Leu Tyr Leu Leu
   35 40 45
   Val Asp Gly Ser Gly Ser Ile Gly Tyr Pro Asn Trp Ile Thr Lys Val
   50 55 60
   Ile Pro Met Leu Asn Gly Leu Ile Asn Ser Leu Ser Leu Ser Arg Asp
   65 70 75 80
   Thr Ile Asn Leu Tyr Met Asn Leu Phe Gly Asn Tyr Thr Thr Glu Leu
   85 90 95
   Ile Arg Leu Gly Ser Gly Gln Ser Ile Asp Lys Arg Gln Ala Leu Ser
   100 105 110
   Lys Val Thr Glu Leu Arg Lys Ser Tyr Ser Pro Tyr Gly Thr Thr Asn
   115 120 125
   Met Thr Ala Ala Leu Asp Glu Val Gln Lys His Leu Asn Asp Arg Val
   130 135 140
   Asn Arg Glu Lys Ala Ile Gln Leu Val Ile Leu Met Thr Asp Gly Ile
   145 150 155 160
   Pro Asn Ser Lys Tyr Thr Ala Leu Glu Val Ala Lys Lys Leu Lys Gln
                   165                 170                 175
   Arg Asn Val Ser Leu Ala Val Ile Gly Ile Gly Gln Gly Ile Asn His
               180                 185                 190
   Gln Phe Asn Arg Leu Ile Ala Gly Cys Arg Pro Arg Glu Ser Asn Cys
           195                 200                 205
   Lys Phe Tyr Ser Tyr Ala Asp Trp Asn Glu Ala Val Ala Leu Ile Lys
       210                 215                 220
   Pro Phe Ile Ala Lys Val Cys Thr Glu Val Glu Arg Val Ala Asn Cys
   225                 230                 235                 240
   Gly Pro Trp Asp Pro Trp Thr Ala Cys Ser Val Thr Cys Gly Arg Gly
                   245                 250                 255
   Thr His Ser Arg Ser Arg Pro Ser Leu His Glu Gly Cys Thr Thr His
               260                 265                 270
   Met Val Ser Glu Cys Glu Glu Gly Glu Cys Pro Val Glu Pro Glu Pro
           275                 280                 285
   Leu Pro Val Pro Ala Pro Leu Pro Thr Val Pro Glu Asp Val Asn Pro
       290                 295                 300
   Arg Asp Thr Asp Asp Glu Asn Glu Asn Pro Asn Phe Asn Lys Gly Leu
   305                 310                 315                 320
   Asp Val Pro Glu Glu Asp Asp Asp Glu Val Pro Pro Ala Asn Glu Arg
                   325                 330                 335
   Ala Asp Gly Asn Pro Val Glu Glu Asn Val Phe Pro Pro Ala Asp Asp
               340                 345                 350
   Ser Val Pro Asp Glu Ser Asn Val Leu Pro Leu Pro Pro Ala Val Pro
           355                 360                 365
   Gly Gly Ser Ser Glu Glu Phe Pro Ala Asp Val Gln Asn Asn Pro Asp
       370                 375                 380
   Ser Pro Glu Glu Leu Pro Met Glu Gln Glu Val Pro Gln Asp Asn Asn
   385                 390                 395                 400
   Val Asn Glu Pro Glu Arg Ser Asp Ser Asn Gly Tyr Gly Val Asn Glu
   405 410 415
   Lys Val Ile Pro Asn Pro Leu Asp Asn Glu Arg Asp Met Ala Asn Lys
   420 425 430
   Asn Lys Thr Val His Pro Asp Arg Lys Asp Ser Ala Arg Asp Arg Tyr
   435 440 445
   Ala Arg Pro His Gly Ser Thr His Val Asn Asn Asn Arg Ala Asn Glu
   450 455 460
   Asn Ser Asp Ile Pro Asn Asn Pro Val Pro Ser Asp Tyr Glu Gln Pro
   465 470 475 480
   Glu Asp Lys Ala Lys Lys Ser Ser Asn Asn Gly Tyr Lys Ile Ala Gly
   485 490 495
   Gly Val Ile Ala Gly Leu Ala Leu Val Gly Cys Val Gly Phe Ala Tyr
   500 505 510
   Asn Phe Val Ala Gly Gly Gly Ala Ala Gly Met Ala Gly Glu Pro Ala
   515 520 525
   Pro Phe Asp Glu Ala Met Ala Glu Asp Glu Lys Asp Val Ala Glu Ala
   530 535 540
   Asp Gln Phe Lys Leu Pro Glu Asp Asn Asp Trp Asn
   545 550 555
<210> 28
   <211> 1086
   <212> DNA
   <213> Artificial Sequence
<220>
   <223> inhibitor of cysteine proteases (ICP)
<400> 28
   ggatccgcca ccatggctaa atccattacc ttctttgtgt tcaacatttg cagcattttg      60
   gctctgctga gtcactgtga ggacaatgac atctacagct tcgacattgt caatgagaca     120
   aattggctga agatcgccaa gaacatcttc aaaggcaagt ctcctagcaa tttcacgatc     180
   ataccgttta acaataccgg tagttctaac gataacgagt caaacaagga ggaatcagta     240
   ctgctgatca gaaagaagat caaaagcaac aagaatcacg atagttccat cattagtggt     300
   gacactgtta acggggacat tagtgacctg aattatacgg ctagcaactt ttccgataac     360
   tctgaggaca tagaagataa ccagaaatat cccacaacca gctacaatag tttcaaccat     420
   ctcaattcca atatcgcctt taacgaagag tccgaataca ttgagattaa tagcgagtct     480
   gacttggaga acaagatcaa ggacatcaac atcaaatcca atcttgagga aaacaatacc     540
   atgaacgaat ccggcaaagt ggatagcaag tatgagctca ctggggacga gaaatgtggt     600
   aaaagcctga agctcggcaa catcagcaat cagacaaacc aggaaaccat aacccaaagc     660
   ctgtcagttg gagagattct gtgcattgac ctcgaaggga atgcaggaac aggctatctg     720
   tgggtgttgc tgggcataca caaggatgag ccaatcataa accccgagaa cttcccaacc     780
   aaactgacaa agaagtcttt cttttccgag gaaataagtg tgactcagcc aaagaagtac     840
   aagatcgatg agcatgattc ttcaaagaat gtgaatcgcg aaatcgaaag ccctgaacag     900
   aaggagtccg actcaaagcc caagaaacct cagatgcaac ttcttggagg accagatcgg     960
   atgaggtcag tcatcaaagg acacaaacct ggcaaatatt acattgtgta ctcttactat    1020
   cgaccgtttt ctcccacttc tggggcgaac actaaaatca tttacgtcac agtacagtga    1080
   ctcgag                                                                1086
<210> 29
   <211> 355
   <212> PRT
   <213> Plasmodium berghei
<220>
   <221> misc_feature
   <223> ICP
<400> 29
   Met Ala Lys Ser Ile Thr Phe Phe Val Phe Asn Ile Cys Ser Ile Leu
   1               5                   10                  15
   Ala Leu Leu Ser His Cys Glu Asp Asn Asp Ile Tyr Ser Phe Asp Ile
               20                  25                  30
   Val Asn Glu Thr Asn Trp Leu Lys Ile Ala Lys Asn Ile Phe Lys Gly
           35                  40                  45
   Lys Ser Pro Ser Asn Phe Thr Ile Ile Pro Phe Asn Asn Thr Gly Ser
       50                  55                  60
   Ser Asn Asp Asn Glu Ser Asn Lys Glu Glu Ser Val Leu Leu Ile Arg
   65                  70                  75                  80
   Lys Lys Ile Lys Ser Asn Lys Asn His Asp Ser Ser Ile Ile Ser Gly
                   85                  90                  95
   Asp Thr Val Asn Gly Asp Ile Ser Asp Leu Asn Tyr Thr Ala Ser Asn
               100                 105                 110
   Phe Ser Asp Asn Ser Glu Asp Ile Glu Asp Asn Gln Lys Tyr Pro Thr
           115                 120                 125
   Thr Ser Tyr Asn Ser Phe Asn His Leu Asn Ser Asn Ile Ala Phe Asn
       130                 135                 140
   Glu Glu Ser Glu Tyr Ile Glu Ile Asn Ser Glu Ser Asp Leu Glu Asn
   145                 150                 155                 160
   Lys Ile Lys Asp Ile Asn Ile Lys Ser Asn Leu Glu Glu Asn Asn Thr
                   165                 170                 175
   Met Asn Glu Ser Gly Lys Val Asp Ser Lys Tyr Glu Leu Thr Gly Asp
               180                 185                 190
   Glu Lys Cys Gly Lys Ser Leu Lys Leu Gly Asn Ile Ser Asn Gln Thr
           195                 200                 205
   Asn Gln Glu Thr Ile Thr Gln Ser Leu Ser Val Gly Glu Ile Leu Cys
       210                 215                 220
   Ile Asp Leu Glu Gly Asn Ala Gly Thr Gly Tyr Leu Trp Val Leu Leu
   225                 230                 235                 240
   Gly Ile His Lys Asp Glu Pro Ile Ile Asn Pro Glu Asn Phe Pro Thr
                   245                 250                 255
   Lys Leu Thr Lys Lys Ser Phe Phe Ser Glu Glu Ile Ser Val Thr Gln
               260                 265                 270
   Pro Lys Lys Tyr Lys Ile Asp Glu His Asp Ser Ser Lys Asn Val Asn
           275                 280                 285
   Arg Glu Ile Glu Ser Pro Glu Gln Lys Glu Ser Asp Ser Lys Pro Lys
       290                 295                 300
   Lys Pro Gln Met Gln Leu Leu Gly Gly Pro Asp Arg Met Arg Ser Val
   305                 310                 315                 320
   Ile Lys Gly His Lys Pro Gly Lys Tyr Tyr Ile Val Tyr Ser Tyr Tyr
                   325                 330                 335
   Arg Pro Phe Ser Pro Thr Ser Gly Ala Asn Thr Lys Ile Ile Tyr Val
               340                 345                 350
   Thr Val Gln
           355
<210> 30
   <211> 354
   <212> PRT
   <213> Plasmodium berghei
<220>
   <221> misc_feature
   <223> ICP
<400> 30
   Met Lys Ser Ile Thr Phe Phe Val Phe Asn Ile Cys Ser Ile Leu Ala
   1               5                   10                  15
   Leu Leu Ser His Cys Glu Asp Asn Asp Ile Tyr Ser Phe Asp Ile Val
               20                  25                  30
   Asn Glu Thr Asn Trp Leu Lys Ile Ala Lys Asn Ile Phe Lys Gly Lys
           35                  40                  45
   Ser Pro Ser Asn Phe Thr Ile Ile Pro Phe Asn Asn Thr Gly Ser Ser
       50                  55                  60
   Asn Asp Asn Glu Ser Asn Lys Glu Glu Ser Val Leu Leu Ile Arg Lys
   65                  70                  75                  80
   Lys Ile Lys Ser Asn Lys Asn His Asp Ser Ser Ile Ile Ser Gly Asp
                   85                  90                  95
   Thr Val Asn Gly Asp Ile Ser Asp Leu Asn Tyr Thr Ala Ser Asn Phe
               100                 105                 110
   Ser Asp Asn Ser Glu Asp Ile Glu Asp Asn Gln Lys Tyr Pro Thr Thr
           115                 120                 125
   Ser Tyr Asn Ser Phe Asn His Leu Asn Ser Asn Ile Ala Phe Asn Glu
       130                 135                 140
   Glu Ser Glu Tyr Ile Glu Ile Asn Ser Glu Ser Asp Leu Glu Asn Lys
   145                 150                 155                 160
   Ile Lys Asp Ile Asn Ile Lys Ser Asn Leu Glu Glu Asn Asn Thr Met
                   165                 170                 175
   Asn Glu Ser Gly Lys Val Asp Ser Lys Tyr Glu Leu Thr Gly Asp Glu
               180                 185                 190
   Lys Cys Gly Lys Ser Leu Lys Leu Gly Asn Ile Ser Asn Gln Thr Asn
                   195                 200                 205
   Gln Glu Thr Ile Thr Gln Ser Leu Ser Val Gly Glu Ile Leu Cys Ile
       210                 215                 220
   Asp Leu Glu Gly Asn Ala Gly Thr Gly Tyr Leu Trp Val Leu Leu Gly
   225                 230                 235                 240
   Ile His Lys Asp Glu Pro Ile Ile Asn Pro Glu Asn Phe Pro Thr Lys
                   245                 250                 255
   Leu Thr Lys Lys Ser Phe Phe Ser Glu Glu Ile Ser Val Thr Gln Pro
               260                 265                 270
   Lys Lys Tyr Lys Ile Asp Glu His Asp Ser Ser Lys Asn Val Asn Arg
           275                 280                 285
   Glu Ile Glu Ser Pro Glu Gln Lys Glu Ser Asp Ser Lys Pro Lys Lys
       290                 295                 300
   Pro Gln Met Gln Leu Leu Gly Gly Pro Asp Arg Met Arg Ser Val Ile
   305                 310                 315                 320
   Lys Gly His Lys Pro Gly Lys Tyr Tyr Ile Val Tyr Ser Tyr Tyr Arg
                   325                 330                 335
   Pro Phe Ser Pro Thr Ser Gly Ala Asn Thr Lys Ile Ile Tyr Val Thr
               340                 345                 350
   Val Gln
<210> 31
   <211> 1251
   <212> DNA
   <213> Artificial Sequence
<220>
   <223> PfICPhumanCO
<400> 31
   gccaccatgg ctaatctgtt ggtgttcttt tgcttctttc tgttgtcttg catcgtacac      60
   ctctctcgtt gttctgacaa caattcatat agctttgaga tcgtgaatcg gagcacatgg     120
   ctcaacattg ctgaacggat attcaaaggc aatgcaccat tcaactttac tattataccg     180
   tacaactatg tgaacaatag tactgaagag aataataaca aagactctgt ccttctgata     240
   agcaagaatc tgaagaattc ctcaaaccca gtggacgaaa ataaccacat cattgactca     300
   accaagaaga acacgtccaa taacaacaat aataacagca atatcgtcgg aatatacgaa     360
   agtcaggtac atgaggagaa gattaaagag gacaatacaa gacaggataa tatcaacaag     420
   aaggaaaacg agataatcaa caataaccat cagatccctg tgtccaacat cttttcagag     480
   aacattgaca acaacaagaa ctacattgag agcaactaca agagcaccta taacaataat     540
   cccgagttga ttcatagcac agatttcatt ggcagtaata acaaccacac tttcaatttc     600
   ctgtctcgct ataacaactc agtgctgaac aacatgcaag ggaataccaa agttccaggg     660
   aatgttcccg aactgaaagc ccgcattttc tccgaggaag aaaacaccga agtcgaaagc     720
   gccgaaaaca atcacactaa cagtctgaat cctaacgagt cttgtgatca aatcatcaaa     780
   ctgggcgata tcattaacag cgtcaatgag aagatcatca gcatcaatag tacggtgaat     840
   aacgtgctct gcataaatct ggattccgtc aatggcaatg gcttcgtttg gacccttctt     900
   ggggtacaca agaagaaacc cctgattgac ccctccaatt ttcccactaa aagggtgact     960
   cagtcctacg tttcacctga catttccgtt acaaaccctg tgccaattcc aaagaacagc    1020
   aacaccaaca aagatgacag catcaataat aaacaggatg gttcccagaa taacacaaca    1080
   acgaatcact ttccgaagcc tagagagcaa ctcgtgggtg gatcttctat gctgatcagt    1140
   aagatcaaac cccataaacc cggaaagtat ttcatcgtgt atagctacta cagaccattt    1200
   gaccctacaa gggataccaa cacccgaatt gtggagctga atgtccagta a             1251
<210> 32
   <211> 414
   <212> PRT
   <213> Plasmodium falciparum
<220>
   <221> misc_feature
   <223> PfICP
<400> 32
   Met Ala Asn Leu Leu Val Phe Phe Cys Phe Phe Leu Leu Ser Cys Ile
   1               5                   10                  15
   Val His Leu Ser Arg Cys Ser Asp Asn Asn Ser Tyr Ser Phe Glu Ile
               20                  25                  30
   Val Asn Arg Ser Thr Trp Leu Asn Ile Ala Glu Arg Ile Phe Lys Gly
           35                  40                  45
   Asn Ala Pro Phe Asn Phe Thr Ile Ile Pro Tyr Asn Tyr Val Asn Asn
       50                  55                  60
   Ser Thr Glu Glu Asn Asn Asn Lys Asp Ser Val Leu Leu Ile Ser Lys
   65                  70                  75                  80
   Asn Leu Lys Asn Ser Ser Asn Pro Val Asp Glu Asn Asn His Ile Ile
   85 90 95
   Asp Ser Thr Lys Lys Asn Thr Ser Asn Asn Asn Asn Asn Asn Ser Asn
   100 105 110
   Ile Val Gly Ile Tyr Glu Ser Gln Val His Glu Glu Lys Ile Lys Glu
   115 120 125
   Asp Asn Thr Arg Gln Asp Asn Ile Asn Lys Lys Glu Asn Glu Ile Ile
   130 135 140
   Asn Asn Asn His Gln Ile Pro Val Ser Asn Ile Phe Ser Glu Asn Ile
   145 150 155 160
   Asp Asn Asn Lys Asn Tyr Ile Glu Ser Asn Tyr Lys Ser Thr Tyr Asn
   165 170 175
   Asn Asn Pro Glu Leu Ile His Ser Thr Asp Phe Ile Gly Ser Asn Asn
   180 185 190
   Asn His Thr Phe Asn Phe Leu Ser Arg Tyr Asn Asn Ser Val Leu Asn
   195 200 205
   Asn Met Gln Gly Asn Thr Lys Val Pro Gly Asn Val Pro Glu Leu Lys
   210 215 220
   Ala Arg Ile Phe Ser Glu Glu Glu Asn Thr Glu Val Glu Ser Ala Glu
   225 230 235 240
   Asn Asn His Thr Asn Ser Leu Asn Pro Asn Glu Ser Cys Asp Gln Ile
   245 250 255
   Ile Lys Leu Gly Asp Ile Ile Asn Ser Val Asn Glu Lys Ile Ile Ser
   260 265 270
   Ile Asn Ser Thr Val Asn Asn Val Leu Cys Ile Asn Leu Asp Ser Val
   275 280 285
   Asn Gly Asn Gly Phe Val Trp Thr Leu Leu Gly Val His Lys Lys Lys
   290 295 300
   Pro Leu Ile Asp Pro Ser Asn Phe Pro Thr Lys Arg Val Thr Gln Ser
   305 310 315 320
   Tyr Val Ser Pro Asp Ile Ser Val Thr Asn Pro Val Pro Ile Pro Lys
   325 330 335
   Asn Ser Asn Thr Asn Lys Asp Asp Ser Ile Asn Asn Lys Gln Asp Gly
   340 345 350
   Ser Gln Asn Asn Thr Thr Thr Asn His Phe Pro Lys Pro Arg Glu Gln
   355 360 365
   Leu Val Gly Gly Ser Ser Met Leu Ile Ser Lys Ile Lys Pro His Lys
   370 375 380
   Pro Gly Lys Tyr Phe Ile Val Tyr Ser Tyr Tyr Arg Pro Phe Asp Pro
   385 390 395 400
   Thr Arg Asp Thr Asn Thr Arg Ile Val Glu Leu Asn Val Gln
   405 410
<210> 33
   <211> 413
   <212> PRT
   <213> Plasmodium falciparum
<220>
   <221> misc_feature
   <223> ICP
<400> 33
   Met Asn Leu Leu Val Phe Phe Cys Phe Phe Leu Leu Ser Cys Ile Val
   1 5 10 15
   His Leu Ser Arg Cys Ser Asp Asn Asn Ser Tyr Ser Phe Glu Ile Val
   20 25 30
   Asn Arg Ser Thr Trp Leu Asn Ile Ala Glu Arg Ile Phe Lys Gly Asn
   35 40 45
   Ala Pro Phe Asn Phe Thr Ile Ile Pro Tyr Asn Tyr Val Asn Asn Ser
   50 55 60
   Thr Glu Glu Asn Asn Asn Lys Asp Ser Val Leu Leu Ile Ser Lys Asn
   65 70 75 80
   Leu Lys Asn Ser Ser Asn Pro Val Asp Glu Asn Asn His Ile Ile Asp
   85 90 95
   Ser Thr Lys Lys Asn Thr Ser Asn Asn Asn Asn Asn Asn Ser Asn Ile
   100 105 110
   Val Gly Ile Tyr Glu Ser Gln Val His Glu Glu Lys Ile Lys Glu Asp
   115 120 125
   Asn Thr Arg Gln Asp Asn Ile Asn Lys Lys Glu Asn Glu Ile Ile Asn
   130 135 140
   Asn Asn His Gln Ile Pro Val Ser Asn Ile Phe Ser Glu Asn Ile Asp
   145 150 155 160
   Asn Asn Lys Asn Tyr Ile Glu Ser Asn Tyr Lys Ser Thr Tyr Asn Asn
   165 170 175
   Asn Pro Glu Leu Ile His Ser Thr Asp Phe Ile Gly Ser Asn Asn Asn
   180 185 190
   His Thr Phe Asn Phe Leu Ser Arg Tyr Asn Asn Ser Val Leu Asn Asn
   195 200 205
   Met Gln Gly Asn Thr Lys Val Pro Gly Asn Val Pro Glu Leu Lys Ala
   210 215 220
   Arg Ile Phe Ser Glu Glu Glu Asn Thr Glu Val Glu Ser Ala Glu Asn
   225 230 235 240
   Asn His Thr Asn Ser Leu Asn Pro Asn Glu Ser Cys Asp Gln Ile Ile
   245 250 255
   Lys Leu Gly Asp Ile Ile Asn Ser Val Asn Glu Lys Ile Ile Ser Ile
   260 265 270
   Asn Ser Thr Val Asn Asn Val Leu Cys Ile Asn Leu Asp Ser Val Asn
   275 280 285
   Gly Asn Gly Phe Val Trp Thr Leu Leu Gly Val His Lys Lys Lys Pro
   290 295 300
   Leu Ile Asp Pro Ser Asn Phe Pro Thr Lys Arg Val Thr Gln Ser Tyr
   305 310 315 320
   Val Ser Pro Asp Ile Ser Val Thr Asn Pro Val Pro Ile Pro Lys Asn
   325 330 335
   Ser Asn Thr Asn Lys Asp Asp Ser Ile Asn Asn Lys Gln Asp Gly Ser
   340 345 350
   Gln Asn Asn Thr Thr Thr Asn His Phe Pro Lys Pro Arg Glu Gln Leu
   355 360 365
   Val Gly Gly Ser Ser Met Leu Ile Ser Lys Ile Lys Pro His Lys Pro
   370 375 380
   Gly Lys Tyr Phe Ile Val Tyr Ser Tyr Tyr Arg Pro Phe Asp Pro Thr
   385 390 395 400
   Arg Asp Thr Asn Thr Arg Ile Val Glu Leu Asn Val Gln
   405 410
<210> 34
   <211> 1104
   <212> DNA
   <213> Artificial Sequence
<220>
   <223> PvICPhumanCO
<400> 34
   gccaccatgg ctaagttgtc tagcctgttc tgcctggttg tgtgttctag tgttgcccac 60
   ctctcttcct gtagtgatca aaacacttat agttttgata ttgttaatcg aaacacttgg 120
   tacagcatag ccaagaaaat ctttcaaggc acgaccccct gtaatttcac tgtaatccct 180
   agttcctatg tcaacaactc tgacggagtg tctacgagtg atgattccgt actgctcatc 240
   cgcaaaaagc tcaaggatcc gagtgaagct ggccttgacg gatcttcagt ttctggttca 300
   tccagttctg gaaacagtca ctccggttct gcaccttgtt gtgataaggg tacccccgct 360
   aaagaggcag agctgaaatt ttctacaaag tttgagggcg atgactatgc taagctgaga 420
   gattctctga gccttataga caagtcactc cgagaagagt caagctcaga ggaggacagt 480
   aagatggaag atagtcaggt cggtgaagta actcatgaag agactatcac ctacaacatg 540
   cccgaagaat atatgcccca gaacatttcc gaggtattga tcggtgccgc tgaagaggat 600
   aggacatacg cgttgaaggg ggacgagccg tgtgatgtgt acttgaaact tggcgagata 660
   atcaatggaa ctaatgaaaa gactatcgag tattctctcc aaaaaaataa gatactgtgc 720
   gttcaactcg aagcaattgg gggaaatgga tacctctggg ctctcctcgg cgtacacaaa 780
   gaaaaacccc aaatcaaccc agaggagttt ccacgaaaaa agatcacaaa atcttttttc 840
   accaatgaga tatccgtcac gcagcctaaa gcagtgcaaa agaacaaatc taataatgga 900
   ggtgagagca gttcaaactc acctggctat gggaaacccc ccgcaagcga acagctcggg 960
   ggatttgtgg gtggcacatc catgcttcag agtatagtaa aggctcataa agagggcacc 1020
   tttttcgtag tttatagcta ctaccgcccc ttcgacccta ccgccaacgc caacacgaag 1080
   atactcaaac ttacggtttc atag 1104
<210> 35
   <211> 365
   <212> PRT
   <213> Artificial Sequence
<220>
   <223> PVICP humanco + kozac
<220>
   <221> misc_feature
   <223> PvICP
<400> 35
   Met Ala Lys Leu Ser Ser Leu Phe Cys Leu Val Val Cys Ser Ser Val
   1               5                   10                  15
   Ala His Leu Ser Ser Cys Ser Asp Gln Asn Thr Tyr Ser Phe Asp Ile
               20                  25                  30
   Val Asn Arg Asn Thr Trp Tyr Ser Ile Ala Lys Lys Ile Phe Gln Gly
           35                  40                  45
   Thr Thr Pro Cys Asn Phe Thr Val Ile Pro Ser Ser Tyr Val Asn Asn
       50                  55                  60
   Ser Asp Gly Val Ser Thr Ser Asp Asp Ser Val Leu Leu Ile Arg Lys
   65                  70                  75                  80
   Lys Leu Lys Asp Pro Ser Glu Ala Gly Leu Asp Gly Ser Ser Val Ser
                   85                  90                  95
   Gly Ser Ser Ser Ser Gly Asn Ser His Ser Gly Ser Ala Pro Cys Cys
               100                 105                 110
   Asp Lys Gly Thr Pro Ala Lys Glu Ala Glu Leu Lys Phe Ser Thr Lys
           115                 120                 125
   Phe Glu Gly Asp Asp Tyr Ala Lys Leu Arg Asp Ser Leu Ser Leu Ile
       130                 135                 140
   Asp Lys Ser Leu Arg Glu Glu Ser Ser Ser Glu Glu Asp Ser Lys Met
   145                 150                 155                 160
   Glu Asp Ser Gln Val Gly Glu Val Thr His Glu Glu Thr Ile Thr Tyr
                   165                 170                 175
   Asn Met Pro Glu Glu Tyr Met Pro Gln Asn Ile Ser Glu Val Leu Ile
               180                 185                 190
   Gly Ala Ala Glu Glu Asp Arg Thr Tyr Ala Leu Lys Gly Asp Glu Pro
           195                 200                 205
   Cys Asp Val Tyr Leu Lys Leu Gly Glu Ile Ile Asn Gly Thr Asn Glu
   210 215 220
   Lys Thr Ile Glu Tyr Ser Leu Gln Lys Asn Lys Ile Leu Cys Val Gln
   225 230 235 240
   Leu Glu Ala Ile Gly Gly Asn Gly Tyr Leu Trp Ala Leu Leu Gly Val
   245 250 255
   His Lys Glu Lys Pro Gln Ile Asn Pro Glu Glu Phe Pro Arg Lys Lys
   260 265 270
   Ile Thr Lys Ser Phe Phe Thr Asn Glu Ile Ser Val Thr Gln Pro Lys
   275 280 285
   Ala Val Gln Lys Asn Lys Ser Asn Asn Gly Gly Glu Ser Ser Ser Asn
   290 295 300
   Ser Pro Gly Tyr Gly Lys Pro Pro Ala Ser Glu Gln Leu Gly Gly Phe
   305 310 315 320
   Val Gly Gly Thr Ser Met Leu Gln Ser Ile Val Lys Ala His Lys Glu
   325 330 335
   Gly Thr Phe Phe Val Val Tyr Ser Tyr Tyr Arg Pro Phe Asp Pro Thr
   340 345 350
   Ala Asn Ala Asn Thr Lys Ile Leu Lys Leu Thr Val Ser
   355 360 365
<210> 36
   <211> 359
   <212> PRT
   <213> Plasmodium vivax
<220>
   <221> misc_feature
   <223> ICP
<400> 36
   Met Lys Leu Ser Ser Leu Phe Cys Leu Val Val Cys Ser Ser Val Ala
   1 5 10 15
   His Leu Ser Ser Cys Ser Asp Gln Asn Thr Tyr Ser Phe Asp Ile Val
   20 25 30
   Asn Arg Asn Thr Trp Tyr Ser Ile Ala Lys Lys Ile Phe Gln Gly Thr
   35 40 45
   Thr Pro Cys Asn Phe Thr Val Ile Pro Ser Ser Tyr Val Asn Asn Ser
       50                  55                  60
   Asp Gly Val Ser Thr Ser Asp Asp Ser Val Leu Leu Ile Arg Lys Lys
   65                  70                  75                  80
   Leu Lys Asp Pro Ser Glu Ala Gly Leu Asp Gly Ser Ser Val Ser Gly
                   85                  90                  95
   Asn Ser His Ser Gly Ser Ala Pro Cys Cys Asp Lys Gly Thr Pro Ala
               100                 105                 110
   Lys Glu Ala Glu Leu Asn Phe Ser Thr Lys Phe Glu Gly Asp Asp Tyr
           115                 120                 125
   Ala Lys Leu Arg Asp Ser Leu Ser Leu Ile Asp Lys Ser Leu Arg Glu
       130                 135                 140
   Glu Ser Ser Ser Glu Glu Asp Ser Lys Met Glu Asp Ser Gln Val Gly
   145                 150                 155                 160
   Glu Val Thr His Glu Glu Thr Ile Thr Tyr Asn Met Pro Glu Glu Tyr
                   165                 170                 175
   Met Pro Gln Asn Ile Ser Glu Val Leu Ile Gly Ala Ala Glu Glu Asp
               180                 185                 190
   Arg Thr Tyr Ala Leu Lys Gly Asp Glu Pro Cys Asp Val Tyr Leu Lys
           195                 200                 205
   Leu Gly Glu Ile Ile Asn Gly Thr Asn Glu Lys Thr Ile Glu Tyr Ser
       210                 215                 220
   Leu Gln Lys Asn Lys Ile Leu Cys Val Gln Leu Glu Ala Ile Gly Gly
   225                 230                 235                 240
   Asn Gly Tyr Leu Trp Ala Leu Leu Gly Val His Lys Glu Lys Pro Gln
                   245                 250                 255
   Ile Asn Pro Glu Glu Phe Pro Arg Lys Lys Ile Thr Lys Ser Phe Phe
               260                 265                 270
   Thr Asn Glu Ile Ser Val Thr Gln Pro Lys Ala Val Gln Lys Asn Lys
           275                 280                 285
   Ser Asn Asn Gly Gly Glu Ser Ser Ser Asn Ser Pro Gly Tyr Gly Lys
       290                 295                 300
   Pro Pro Ala Ser Glu Gln Leu Gly Gly Phe Val Gly Gly Thr Ser Met
   305                 310                 315                 320
   Leu Gln Ser Ile Val Lys Ala His Lys Glu Gly Thr Phe Phe Val Val
                   325                 330                 335
   Tyr Ser Tyr Tyr Arg Pro Phe Asp Pro Thr Ala Asn Ala Asn Thr Lys
               340                 345                 350
   Ile Leu Lys Leu Thr Val Ser
           355
<210> 37
   <211> 2355
   <212> DNA
   <213> Artificial Sequence
<220>
   <223> Falcilysin, Bergheilysin-A-CO
<400> 37
   ggatccgcca ccatggctaa attgatgaag gttttgggtt acatcaacat tattaccaac      60
   tgcgtgaatg gaatcctctg caaaggtgac aagaaaaggt actccatctt caccaataat     120
   tacatctaca gcatatcaac cctcaataat tatagctttg ctgcaacaat gaacaaaatg     180
   cccgcttggg ttaacgagaa atgtccagaa cacaaaagct acgacatcgt tgagaagcgc     240
   tacaatgaga acctcaatct cacgtacaca gtctatgagc acaagaaggc caagactcag     300
   gtcatagcac tggggtctaa cgatcctctc gacgctgagc aagcatttgg cttctacgtg     360
   aaaaccctga cgcattcaga taaaggcatt ccgcacatac tggaacacac tgtcctgagt     420
   ggctctaaga atttcaacta caaggactca atggggcttt tggagaaagg caccctgaac     480
   acacacctga atgcctacac cttcaatgac aggaccatct acatggccgg gagtatgaac     540
   aatagggatt tctttaacat tatggccgtc tacatggata gcgtgttcca gcctaacgta     600
   ctggaaaaca aattcatctt ccagacagag ggatggacct atgaggtaga gaagctgaag     660
   gaggaggaga agaacctcga cattcccaag attaaggact acaaggtgtc ttttaacgga     720
   atcgtgtata atgagatgaa gggtgcgttt agcaatcctc tgcaggacct gtattatgaa     780
   gtgatgagaa acatgttccc cgacaacgta cacagtaaca tctccggagg agatcctaaa     840
   gaaataccaa atctgtcata tgaagagttt aaggagtttt actacaagaa ttacaatccg     900
   aagaaaatca aagtgttctt tttctccaaa aataatccga cagagctgct caactttgtg     960
   gacaactatc tctgtcagct ggacttcacg aaatatcggg atgatgctgt ggaacatgtc    1020
   aattaccaag aataccgcaa aggcccattc tatattaaga agaaattcgc tgatcactca    1080
   gaagagaaag aaaatcttgc ctccgtgagt tggcttctga atcccaagaa acacaagaat 1140
   tccgatactg atctctctct ggagtctcct acagactatt tcgccttgct catcatcaat 1200
   aatctgctta ctcataccag tgagagcgtc ctgtacaaag ccctgataga atccggattg 1260
   gggaatagta ttgtagatcg agggctgaat gattccctgg ttcagtatgt gttcagcatc 1320
   ggcctgaaag gcataaaaga gaagaacgag aagaacatct ccctggacaa ggtccactac 1380
   gaggtggaaa agatcgttct tgaggcactg aaaaaagtgg tcaaagaagg tttcaataag 1440
   tcagcagttg aggcagccat taacaatatt gagttcgtcc tgaaagaagc caatctcaag 1500
   atctccaaat ctatagactt tgtgtttgaa atggccagca gactgaacta tggcaaagat 1560
   ccactgctga tctttgagtt tgaaaagcat ctcaacgtag tgaaggacaa gatcaagaac 1620
   gagcctaaat acctggagaa gtatgtggag aaacatcttc tgaacaatga tcatcgagtc 1680
   gttattctgc tggaagggga tgaaaactat ggcaccgaac aggagaaact ggaaaaggac 1740
   atgctgaaga agcggattga aagcttcact gagaaagaaa aggagaatat tatcacagac 1800
   ttcgaaaatc ttacgaagta caagaacact gaggaatctc ccgaacatct ggacaagttt 1860
   cccatcatta gcattagcga cctcaatgga aagactttgg agatccccgt gaaccctttc 1920
   tttaccaacc tgaacaacga gaacaacatg cagcactata atgagacaaa gaacaaccaa 1980
   accctggtca aagaaaacat ggaccgtttc attaacaaat acattctcaa caaggatgga 2040
   aacgataaga acgacagcaa gaacgcggat gtgccaatgc tgatttatga aatcccaaca 2100
   tctggcatat tgtatctgca gtttatcttc tctctggata accttacact ggaagaattg 2160
   tcctacctga atctgtttaa aagcttgata ctggagaaca agactaacaa gagatcaagt 2220
   gaagagtttg tgatcttgcg ggagaagaat attgggaata tgatgaccaa tgttgctctt 2280
   cttagcacat ccgatcgcct caatgtgact gacaaatata atgcgaaagg tttctttaac 2340
   tttgagtaac tcgag 2355
<210> 38
   <211> 778
   <212> PRT
   <213> Plasmodium berghei
<220>
   <221> misc_feature
   <223> Falcilysin, Bergheilysin-A
<400> 38
   Met Ala Lys Leu Met Lys Val Leu Gly Tyr Ile Asn Ile Ile Thr Asn
   1 5 10 15
   Cys Val Asn Gly Ile Leu Cys Lys Gly Asp Lys Lys Arg Tyr Ser Ile
               20                  25                  30
   Phe Thr Asn Asn Tyr Ile Tyr Ser Ile Ser Thr Leu Asn Asn Tyr Ser
           35                  40                  45
   Phe Ala Ala Thr Met Asn Lys Met Pro Ala Trp Val Asn Glu Lys Cys
       50                  55                  60
   Pro Glu His Lys Ser Tyr Asp Ile Val Glu Lys Arg Tyr Asn Glu Asn
   65                  70                  75                  80
   Leu Asn Leu Thr Tyr Thr Val Tyr Glu His Lys Lys Ala Lys Thr Gln
                   85                  90                  95
   Val Ile Ala Leu Gly Ser Asn Asp Pro Leu Asp Ala Glu Gln Ala Phe
               100                 105                 110
   Gly Phe Tyr Val Lys Thr Leu Thr His Ser Asp Lys Gly Ile Pro His
           115                 120                 125
   Ile Leu Glu His Thr Val Leu Ser Gly Ser Lys Asn Phe Asn Tyr Lys
       130                 135                 140
   Asp Ser Met Gly Leu Leu Glu Lys Gly Thr Leu Asn Thr His Leu Asn
   145                 150                 155                 160
   Ala Tyr Thr Phe Asn Asp Arg Thr Ile Tyr Met Ala Gly Ser Met Asn
                   165                 170                 175
   Asn Arg Asp Phe Phe Asn Ile Met Ala Val Tyr Met Asp Ser Val Phe
               180                 185                 190
   Gln Pro Asn Val Leu Glu Asn Lys Phe Ile Phe Gln Thr Glu Gly Trp
           195                 200                 205
   Thr Tyr Glu Val Glu Lys Leu Lys Glu Glu Glu Lys Asn Leu Asp Ile
       210                 215                 220
   Pro Lys Ile Lys Asp Tyr Lys Val Ser Phe Asn Gly Ile Val Tyr Asn
   225                 230                 235                 240
   Glu Met Lys Gly Ala Phe Ser Asn Pro Leu Gln Asp Leu Tyr Tyr Glu
                   245                 250                 255
   Val Met Arg Asn Met Phe Pro Asp Asn Val His Ser Asn Ile Ser Gly
               260                 265                 270
   Gly Asp Pro Lys Glu Ile Pro Asn Leu Ser Tyr Glu Glu Phe Lys Glu
           275                 280                 285
   Phe Tyr Tyr Lys Asn Tyr Asn Pro Lys Lys Ile Lys Val Phe Phe Phe
       290                 295                 300
   Ser Lys Asn Asn Pro Thr Glu Leu Leu Asn Phe Val Asp Asn Tyr Leu
   305                 310                 315                 320
   Cys Gln Leu Asp Phe Thr Lys Tyr Arg Asp Asp Ala Val Glu His Val
                   325                 330                 335
   Asn Tyr Gln Glu Tyr Arg Lys Gly Pro Phe Tyr Ile Lys Lys Lys Phe
               340                 345                 350
   Ala Asp His Ser Glu Glu Lys Glu Asn Leu Ala Ser Val Ser Trp Leu
           355                 360                 365
   Leu Asn Pro Lys Lys His Lys Asn Ser Asp Thr Asp Leu Ser Leu Glu
       370                 375                 380
   Ser Pro Thr Asp Tyr Phe Ala Leu Leu Ile Ile Asn Asn Leu Leu Thr
   385                 390                 395                 400
   His Thr Ser Glu Ser Val Leu Tyr Lys Ala Leu Ile Glu Ser Gly Leu
                   405                 410                 415
   Gly Asn Ser Ile Val Asp Arg Gly Leu Asn Asp Ser Leu Val Gln Tyr
               420                 425                 430
   Val Phe Ser Ile Gly Leu Lys Gly Ile Lys Glu Lys Asn Glu Lys Asn
           435                 440                 445
   Ile Ser Leu Asp Lys Val His Tyr Glu Val Glu Lys Ile Val Leu Glu
       450                 455                 460
   Ala Leu Lys Lys Val Val Lys Glu Gly Phe Asn Lys Ser Ala Val Glu
   465                 470                 475                 480
   Ala Ala Ile Asn Asn Ile Glu Phe Val Leu Lys Glu Ala Asn Leu Lys
                   485                 490                 495
   Ile Ser Lys Ser Ile Asp Phe Val Phe Glu Met Ala Ser Arg Leu Asn
               500                 505                 510
   Tyr Gly Lys Asp Pro Leu Leu Ile Phe Glu Phe Glu Lys His Leu Asn
           515                 520                 525
   Val Val Lys Asp Lys Ile Lys Asn Glu Pro Lys Tyr Leu Glu Lys Tyr
   530 535 540
   Val Glu Lys His Leu Leu Asn Asn Asp His Arg Val Val Ile Leu Leu
   545 550 555 560
   Glu Gly Asp Glu Asn Tyr Gly Thr Glu Gln Glu Lys Leu Glu Lys Asp
   565 570 575
   Met Leu Lys Lys Arg Ile Glu Ser Phe Thr Glu Lys Glu Lys Glu Asn
   580 585 590
   Ile Ile Thr Asp Phe Glu Asn Leu Thr Lys Tyr Lys Asn Thr Glu Glu
   595 600 605
   Ser Pro Glu His Leu Asp Lys Phe Pro Ile Ile Ser Ile Ser Asp Leu
   610 615 620
   Asn Gly Lys Thr Leu Glu Ile Pro Val Asn Pro Phe Phe Thr Asn Leu
   625 630 635 640
   Asn Asn Glu Asn Asn Met Gln His Tyr Asn Glu Thr Lys Asn Asn Gln
   645 650 655
   Thr Leu Val Lys Glu Asn Met Asp Arg Phe Ile Asn Lys Tyr Ile Leu
   660 665 670
   Asn Lys Asp Gly Asn Asp Lys Asn Asp Ser Lys Asn Ala Asp Val Pro
   675 680 685
   Met Leu Ile Tyr Glu Ile Pro Thr Ser Gly Ile Leu Tyr Leu Gln Phe
   690 695 700
   Ile Phe Ser Leu Asp Asn Leu Thr Leu Glu Glu Leu Ser Tyr Leu Asn
   705 710 715 720
   Leu Phe Lys Ser Leu Ile Leu Glu Asn Lys Thr Asn Lys Arg Ser Ser
   725 730 735
   Glu Glu Phe Val Ile Leu Arg Glu Lys Asn Ile Gly Asn Met Met Thr
   740 745 750
   Asn Val Ala Leu Leu Ser Thr Ser Asp Arg Leu Asn Val Thr Asp Lys
   755 760 765
   Tyr Asn Ala Lys Gly Phe Phe Asn Phe Glu
   770 775
<210> 39
   <211> 1149
   <212> PRT
   <213> Plasmodium berghei
<220>
   <221> misc_feature
   <223> Falcilysin, Bergheilysin-A
<400> 39
   Met Lys Leu Met Lys Val Leu Gly Tyr Ile Asn Ile Ile Thr Asn Cys
   1               5                   10                  15
   Val Asn Gly Ile Leu Cys Lys Gly Asp Lys Lys Arg Tyr Ser Ile Phe
               20                  25                  30
   Thr Asn Asn Tyr Ile Tyr Ser Ile Ser Thr Leu Asn Asn Tyr Ser Phe
           35                  40                  45
   Ala Ala Thr Met Asn Lys Met Pro Ala Trp Val Asn Glu Lys Cys Pro
       50                  55                  60
   Glu His Lys Ser Tyr Asp Ile Val Glu Lys Arg Tyr Asn Glu Asn Leu
   65                  70                  75                  80
   Asn Leu Thr Tyr Thr Val Tyr Glu His Lys Lys Ala Lys Thr Gln Val
                   85                  90                  95
   Ile Ala Leu Gly Ser Asn Asp Pro Leu Asp Ala Glu Gln Ala Phe Gly
               100                 105                 110
   Phe Tyr Val Lys Thr Leu Thr His Ser Asp Lys Gly Ile Pro His Ile
           115                 120                 125
   Leu Glu His Thr Val Leu Ser Gly Ser Lys Asn Phe Asn Tyr Lys Asp
       130                 135                 140
   Ser Met Gly Leu Leu Glu Lys Gly Thr Leu Asn Thr His Leu Asn Ala
   145                 150                 155                 160
   Tyr Thr Phe Asn Asp Arg Thr Ile Tyr Met Ala Gly Ser Met Asn Asn
                   165                 170                 175
   Arg Asp Phe Phe Asn Ile Met Ala Val Tyr Met Asp Ser Val Phe Gln
               180                 185                 190
   Pro Asn Val Leu Glu Asn Lys Phe Ile Phe Gln Thr Glu Gly Trp Thr
           195                 200                 205
   Tyr Glu Val Glu Lys Leu Lys Glu Glu Glu Lys Asn Leu Asp Ile Pro
       210                 215                 220
   Lys Ile Lys Asp Tyr Lys Val Ser Phe Asn Gly Ile Val Tyr Asn Glu
   225                 230                 235                 240
   Met Lys Gly Ala Phe Ser Asn Pro Leu Gln Asp Leu Tyr Tyr Glu Val
                   245                 250                 255
   Met Arg Asn Met Phe Pro Asp Asn Val His Ser Asn Ile Ser Gly Gly
               260                 265                 270
   Asp Pro Lys Glu Ile Pro Asn Leu Ser Tyr Glu Glu Phe Lys Glu Phe
           275                 280                 285
   Tyr Tyr Lys Asn Tyr Asn Pro Lys Lys Ile Lys Val Phe Phe Phe Ser
       290                 295                 300
   Lys Asn Asn Pro Thr Glu Leu Leu Asn Phe Val Asp Asn Tyr Leu Cys
   305                 310                 315                 320
   Gln Leu Asp Phe Thr Lys Tyr Arg Asp Asp Ala Val Glu His Val Asn
                   325                 330                 335
   Tyr Gln Glu Tyr Arg Lys Gly Pro Phe Tyr Ile Lys Lys Lys Phe Ala
               340                 345                 350
   Asp His Ser Glu Glu Lys Glu Asn Leu Ala Ser Val Ser Trp Leu Leu
           355                 360                 365
   Asn Pro Lys Lys His Lys Asn Ser Asp Thr Asp Leu Ser Leu Glu Ser
       370                 375                 380
   Pro Thr Asp Tyr Phe Ala Leu Leu Ile Ile Asn Asn Leu Leu Thr His
   385                 390                 395                 400
   Thr Ser Glu Ser Val Leu Tyr Lys Ala Leu Ile Glu Ser Gly Leu Gly
                   405                 410                 415
   Asn Ser Ile Val Asp Arg Gly Leu Asn Asp Ser Leu Val Gln Tyr Val
               420                 425                 430
   Phe Ser Ile Gly Leu Lys Gly Ile Lys Glu Lys Asn Glu Lys Asn Ile
           435                 440                 445
   Ser Leu Asp Lys Val His Tyr Glu Val Glu Lys Ile Val Leu Glu Ala
       450                 455                 460
   Leu Lys Lys Val Val Lys Glu Gly Phe Asn Lys Ser Ala Val Glu Ala
   465                 470                 475                 480
   Ala Ile Asn Asn Ile Glu Phe Val Leu Lys Glu Ala Asn Leu Lys Ile
                   485                 490                 495
   Ser Lys Ser Ile Asp Phe Val Phe Glu Met Ala Ser Arg Leu Asn Tyr
               500                 505                 510
   Gly Lys Asp Pro Leu Leu Ile Phe Glu Phe Glu Lys His Leu Asn Val
           515                 520                 525
   Val Lys Asp Lys Ile Lys Asn Glu Pro Lys Tyr Leu Glu Lys Tyr Val
       530                 535                 540
   Glu Lys His Leu Leu Asn Asn Asp His Arg Val Val Ile Leu Leu Glu
   545                 550                 555                 560
   Gly Asp Glu Asn Tyr Gly Thr Glu Gln Glu Lys Leu Glu Lys Asp Met
                   565                 570                 575
   Leu Lys Lys Arg Ile Glu Ser Phe Thr Glu Lys Glu Lys Glu Asn Ile
               580                 585                 590
   Ile Thr Asp Phe Glu Asn Leu Thr Lys Tyr Lys Asn Thr Glu Glu Ser
           595                 600                 605
   Pro Glu His Leu Asp Lys Phe Pro Ile Ile Ser Ile Ser Asp Leu Asn
       610                 615                 620
   Gly Lys Thr Leu Glu Ile Pro Val Asn Pro Phe Phe Thr Asn Leu Asn
   625                 630                 635                 640
   Asn Glu Asn Asn Met Gln His Tyr Asn Glu Thr Lys Asn Asn Gln Thr
                   645                 650                 655
   Leu Val Lys Glu Asn Met Asp Arg Phe Ile Asn Lys Tyr Ile Leu Asn
               660                 665                 670
   Lys Asp Gly Asn Asp Lys Asn Asp Ser Lys Asn Ala Asp Val Pro Met
           675                 680                 685
   Leu Ile Tyr Glu Ile Pro Thr Ser Gly Ile Leu Tyr Leu Gln Phe Ile
       690                 695                 700
   Phe Ser Leu Asp Asn Leu Thr Leu Glu Glu Leu Ser Tyr Leu Asn Leu
   705                 710                 715                 720
   Phe Lys Ser Leu Ile Leu Glu Asn Lys Thr Asn Lys Arg Ser Ser Glu
                   725                 730                 735
   Glu Phe Val Ile Leu Arg Glu Lys Asn Ile Gly Asn Met Met Thr Asn
               740                 745                 750
   Val Ala Leu Leu Ser Thr Ser Asp Arg Leu Asn Val Thr Asp Lys Tyr
           755                 760                 765
   Asn Ala Lys Gly Phe Phe Asn Phe Glu Met His Met Leu Ser His Lys
       770                 775                 780
   Cys Asn Asp Ala Leu Glu Ile Ala Leu Glu Ala Leu Lys Glu Ser Asp
   785                 790                 795                 800
   Phe Ser Asn Lys Lys Lys Val Ile Glu Ile Leu Lys Arg Lys Ile Asn
                   805                 810                 815
   Gly Met Lys Thr Thr Phe Ala Ser Lys Gly His Ser Ile Leu Ile Lys
               820                 825                 830
   Tyr Val Lys Ser Arg Ile Asn Ser Lys Tyr Tyr Ala Tyr Asp Leu Ile
           835                 840                 845
   His Gly Tyr Asp Asn Tyr Leu Lys Leu Gln Glu Gln Leu Lys Leu Ala
       850                 855                 860
   Glu Thr Asn Tyr Glu Ser Leu Glu Ala Ile Leu Asn Arg Ile Arg Lys
   865                 870                 875                 880
   Lys Ile Phe Lys Arg Asn Asn Leu Ile Met Asn Val Thr Val Asp Pro
                   885                 890                 895
   Gly Thr Ile Asp Gln Leu Phe Ala Lys Ser Lys Asn Ser Phe Asn Asn
               900                 905                 910
   Leu Leu Ser Tyr Phe Asp Glu Asn Glu Ser Tyr Cys Ser Lys Asn Asp
           915                 920                 925
   Ser Phe Asn Lys Val Val Gly Trp Asn Lys Glu Ile Gln Glu Lys Lys
       930                 935                 940
   Leu Leu Glu Gly Glu Glu Val Lys Lys Glu Leu Leu Val Val Pro Thr
   945                 950                 955                 960
   Phe Val Asn Ser Val Ser Met Ser Gly Val Leu Phe Asn Lys Gly Glu
   965 970 975
   Tyr Leu Asp Pro Ser Phe Thr Val Ile Val Ala Ala Leu Lys Asn Ser
   980 985 990
   Tyr Leu Trp Glu Thr Val Arg Gly Leu Asn Gly Ala Tyr Gly Val Phe
   995 1000 1005
   Ala Asp Ile Glu Tyr Asp Gly Thr Val Val Phe Leu Ser Ala Arg
   1010 1015 1020
   Asp Pro Asn Leu Glu Lys Thr Leu Gln Thr Phe Arg Glu Ala Ala
   1025 1030 1035
   Gln Gly Leu Arg Lys Met Ala Asp Val Met Thr Lys Asn Asp Leu
   1040 1045 1050
   Leu Arg Tyr Ile Ile Asn Ala Ile Gly Thr Ile Asp Arg Pro Arg
   1055 1060 1065
   Arg Gly Val Glu Leu Ser Lys Leu Ser Phe Ser Arg Ile Ile Ser
   1070 1075 1080
   Asn Glu Thr Glu Gln Asp Arg Ile Glu Phe Arg Asn Arg Val Met
   1085 1090 1095
   Asn Thr Lys Lys Glu Asp Phe Tyr Lys Phe Ala Asp Leu Leu Glu
   1100 1105 1110
   Lys Lys Val Lys Glu Phe Glu Lys Asn Val Val Ile Ile Thr Ser
   1115 1120 1125
   Lys Glu Lys Ala Asn Glu Tyr Ile Asn Asn Val Asp Asn Asp Phe
   1130 1135 1140
   Lys Lys Ile Leu Ile Glu
   1145
<210> 40
   <211> 3591
   <212> DNA
   <213> Artificial Sequence
<220>
   <223> PfFalcilysin Human CO
<400> 40
   gccaccatgg ctaatcttac caaacttatg aaagtaatag gatatataaa cattataact 60
   aattgtgtac aatcttttac taacagagcg gacaagaaga ggtacaatgt ttttgcaaag 120
   tctttcatca atacgataaa cacgaacctg tacaccttca aagcggtgat gtcaaaaacg 180
   cctgaatgga tccatgagaa atctcccaaa cacaacagtt atgacataat tgagaaacgc 240
   tataatgaag agttcaaaat gacatacacg gtttatcaac ataaaaaagc aaagacgcag 300
   gtaatttcac tcggtacgaa cgacccactt gatgtcgaac aggcctttgc cttctatgta 360
   aagactctga cccactctgg gaaggggatt ccccacatcc tcgaacacag tgtcctctca 420
   gggagtaaaa attacaacta caaaaattcc attggactcc ttgagaaggg aactctgcac 480
   acccacctta acgcatacac cttcaatgac cgaactgtgt acatggccgg ttctatgaat 540
   aacaaagact tcttcaatat aatgggcgtc tatatggaca gtgtcttcca acctaatgta 600
   ctggaaaaca aatacatatt cgagacggag ggatggactt acgaggtgga aaagctgaaa 660
   gaggatgaga agggaaaagc tgagattcca cagatgaaag attataaagt atctttcaac 720
   ggaattgttt ataatgaaat gaagggtgcc ttgtcttccc cgttggaaga tctttaccat 780
   gaagagatga agtatatgtt cccagacaac gtccactcta ataacagtgg cggagaccca 840
   aaagagatca caaacttgac ctatgaggag ttcaaggagt tctattacaa aaactataat 900
   ccaaaaaaag ttaaagtgtt cttcttttca aagaacaacc caacggagct tctcaatttc 960
   gtagaccagt acctcggaca gctggactac agcaaatacc gcgacgatgc tgttgaaagt 1020
   gttgaatacc aaacgtataa aaaaggacct ttctacatta aaaaaaagta tggggaccat 1080
   agcgaagaga aggagaatct tgtttccgta gcgtggcttc tgaaccccaa ggttgacaag 1140
   actaacaatc ataataataa ccatagtaat aaccaatcta gcgaaaataa tggttactcc 1200
   aacggctccc actctagcga tttgtccttg gagaatccca cggactattt cgtgctcttg 1260
   attatcaata acctccttat acatacccca gaaagcgtcc tgtacaaggc cctcacagat 1320
   tgcgggctgg gaaataatgt aattgatagg ggtttgaatg attcacttgt ccaatacatt 1380
   ttcagtattg ggctgaaggg aatcaaacgc aacaatgaaa aaattaaaaa cttcgataaa 1440
   gtgcactatg aagtagaaga cgtaattatg aatgctctca aaaaagtggt caaggaggga 1500
   ttcaataaat ccgccgtaga agccagcata aataatatcg agtttatcct gaaagaagcc 1560
   aatttgaaaa cttcaaaatc tatagatttc gtttttgaga tgacttccaa gctcaattat 1620
   aatagggatc cactgctgat cttcgagttt gagaaatatc ttaatattgt gaagaataag 1680
   attaagaacg aacctatgta tttggagaag tttgttgaaa aacacttcat caacaacgca 1740
   catcggtcag tgatccttct tgagggggac gagaactatg cacaggaaca ggaaaacctt 1800
   gagaaacaag aactgaagaa acgcatagag aacttcaatg agcaggagaa agagcaagtc 1860
   attaagaact tcgaggagct gtccaagtac aagaacgcgg aagagagccc ggaacacttg 1920
   aacaagtttc caataatctc catttccgac ctcaataaga aaacactgga agtcccagtt 1980
   aacgtctact tcacgaacat caacgaaaac aacaatataa tggagacata taacaagctg 2040
   aagacaaatg agcacatgct taaggacaac atggacgtgt ttctcaaaaa atacgttctc 2100
   aaaaacgata agcacaacac caacaataat aacaacaata ataataatat ggactactct 2160
   ttcaccgaaa ctaaatatga aggaaatgtg ccaatccttg tgtacgaaat gccgacgact 2220
   ggaatagtct atttgcagtt cgttttctcc ctcgatcacc tgaccgtaga cgagctcgcc 2280
   tatttgaatt tgttcaagac acttatcttg gagaacaaga caaacaaacg ctccagtgag 2340
   gatttcgtca ttttgagaga aaaaaatatt gggtcaatgt cagcgaatgt ggcgctctac 2400
   agcaaggacg accacctgaa cgtaaccgac aagtataacg cgcaagcact cttcaatctg 2460
   gaaatgcacg tactttctca taaatgcaac gatgcgctga acattgccct tgaagctgtt 2520
   aaagaatctg acttcagtaa taaaaaaaag gtcatagata tccttaagag gaagattaat 2580
   ggaatgaaaa ctacgttctc agaaaaagga tatgctatac tgatgaaata cgttaaagcc 2640
   catcttaata gcaagcatta cgcccataac ataatttatg ggtacgaaaa ctatttgaag 2700
   ctgcaagagc aattggagct tgcagaaaac gattttaaaa cattggagaa tattttggtg 2760
   agaataagaa acaaaatctt taacaaaaaa aacttgatgg tcagcgtgac gtccgactat 2820
   ggtgctctca aacacctgtt tgtgaatagc aacgaatcct tgaaaaacct tgtaagttac 2880
   ttcgaggaaa acgataagta tatcaacgat atgcagaata aagtgaatga ccctaccgtc 2940
   atggggtgga atgaagaaat caagtctaaa aagctgttcg acgaggagaa ggtgaaaaag 3000
   gagttctttg tcttgccgac ctttgtcaac agcgtctcaa tgagtggtat cctgttcaag 3060
   ccgggcgaat atctcgaccc gagtttcact gtcatcgttg cggccctgaa aaatagctat 3120
   ttgtgggaca cagttcgagg acttaacggg gcttacggtg tattcgctga tatcgaatac 3180
   gatggctctg tagtatttct ttcagctcga gaccccaatc tcgaaaagac cctcgccact 3240
   ttccgagaat ctgctaaagg attgcgcaaa atggctgaca ccatgacaga gaacgatttg 3300
   cttcggtata ttattaacac aattggcacg atcgacaagc ctcgacgagg gatagaactt 3360
   tctaagctgt cattccttag acttatttcc aacgagtcag agcaagatcg ggtggagttc 3420
   cggaaacgga taatgaacac aaaaaaggaa gatttctata aattcgcaga tcttcttgag 3480
   agcaaagtaa atgaattcga aaaaaatatc gttatcataa caacaaaaga aaaggccaac 3540
   gaatatatag caaacgtaga tggcgagttt aagaaagtct tgatcgagtg a 3591
<210> 41
   <211> 1194
   <212> PRT
   <213> Plasmodium falciparum
<220>
   <221> misc_feature
   <223> PfFalcilysin
<400> 41
   Met Ala Asn Leu Thr Lys Leu Met Lys Val Ile Gly Tyr Ile Asn Ile
   1               5                   10                  15
   Ile Thr Asn Cys Val Gln Ser Phe Thr Asn Arg Ala Asp Lys Lys Arg
               20                  25                  30
   Tyr Asn Val Phe Ala Lys Ser Phe Ile Asn Thr Ile Asn Thr Asn Leu
           35                  40                  45
   Tyr Thr Phe Lys Ala Val Met Ser Lys Thr Pro Glu Trp Ile His Glu
       50                  55                  60
   Lys Ser Pro Lys His Asn Ser Tyr Asp Ile Ile Glu Lys Arg Tyr Asn
   65                  70                  75                  80
   Glu Glu Phe Lys Met Thr Tyr Thr Val Tyr Gln His Lys Lys Ala Lys
                   85                  90                  95
   Thr Gln Val Ile Ser Leu Gly Thr Asn Asp Pro Leu Asp Val Glu Gln
               100                 105                 110
   Ala Phe Ala Phe Tyr Val Lys Thr Leu Thr His Ser Gly Lys Gly Ile
           115                 120                 125
   Pro His Ile Leu Glu His Ser Val Leu Ser Gly Ser Lys Asn Tyr Asn
       130                 135                 140
   Tyr Lys Asn Ser Ile Gly Leu Leu Glu Lys Gly Thr Leu His Thr His
   145                 150                 155                 160
   Leu Asn Ala Tyr Thr Phe Asn Asp Arg Thr Val Tyr Met Ala Gly Ser
                   165                 170                 175
   Met Asn Asn Lys Asp Phe Phe Asn Ile Met Gly Val Tyr Met Asp Ser
               180                 185                 190
   Val Phe Gln Pro Asn Val Leu Glu Asn Lys Tyr Ile Phe Glu Thr Glu
           195                 200                 205
   Gly Trp Thr Tyr Glu Val Glu Lys Leu Lys Glu Asp Glu Lys Gly Lys
       210                 215                 220
   Ala Glu Ile Pro Gln Met Lys Asp Tyr Lys Val Ser Phe Asn Gly Ile
   225                 230                 235                 240
   Val Tyr Asn Glu Met Lys Gly Ala Leu Ser Ser Pro Leu Glu Asp Leu
                   245                 250                 255
   Tyr His Glu Glu Met Lys Tyr Met Phe Pro Asp Asn Val His Ser Asn
               260                 265                 270
   Asn Ser Gly Gly Asp Pro Lys Glu Ile Thr Asn Leu Thr Tyr Glu Glu
           275                 280                 285
   Phe Lys Glu Phe Tyr Tyr Lys Asn Tyr Asn Pro Lys Lys Val Lys Val
       290                 295                 300
   Phe Phe Phe Ser Lys Asn Asn Pro Thr Glu Leu Leu Asn Phe Val Asp
   305                 310                 315                 320
   Gln Tyr Leu Gly Gln Leu Asp Tyr Ser Lys Tyr Arg Asp Asp Ala Val
                   325                 330                 335
   Glu Ser Val Glu Tyr Gln Thr Tyr Lys Lys Gly Pro Phe Tyr Ile Lys
               340                 345                 350
   Lys Lys Tyr Gly Asp His Ser Glu Glu Lys Glu Asn Leu Val Ser Val
           355                 360                 365
   Ala Trp Leu Leu Asn Pro Lys Val Asp Lys Thr Asn Asn His Asn Asn
       370                 375                 380
   Asn His Ser Asn Asn Gln Ser Ser Glu Asn Asn Gly Tyr Ser Asn Gly
   385                 390                 395                 400
   Ser His Ser Ser Asp Leu Ser Leu Glu Asn Pro Thr Asp Tyr Phe Val
                   405                 410                 415
   Leu Leu Ile Ile Asn Asn Leu Leu Ile His Thr Pro Glu Ser Val Leu
               420                 425                 430
   Tyr Lys Ala Leu Thr Asp Cys Gly Leu Gly Asn Asn Val Ile Asp Arg
           435                 440                 445
   Gly Leu Asn Asp Ser Leu Val Gln Tyr Ile Phe Ser Ile Gly Leu Lys
       450                 455                 460
   Gly Ile Lys Arg Asn Asn Glu Lys Ile Lys Asn Phe Asp Lys Val His
   465                 470                 475                 480
   Tyr Glu Val Glu Asp Val Ile Met Asn Ala Leu Lys Lys Val Val Lys
                   485                 490                 495
   Glu Gly Phe Asn Lys Ser Ala Val Glu Ala Ser Ile Asn Asn Ile Glu
               500                 505                 510
   Phe Ile Leu Lys Glu Ala Asn Leu Lys Thr Ser Lys Ser Ile Asp Phe
           515                 520                 525
   Val Phe Glu Met Thr Ser Lys Leu Asn Tyr Asn Arg Asp Pro Leu Leu
       530                 535                 540
   Ile Phe Glu Phe Glu Lys Tyr Leu Asn Ile Val Lys Asn Lys Ile Lys
   545                 550                 555                 560
   Asn Glu Pro Met Tyr Leu Glu Lys Phe Val Glu Lys His Phe Ile Asn
                   565                 570                 575
   Asn Ala His Arg Ser Val Ile Leu Leu Glu Gly Asp Glu Asn Tyr Ala
               580                 585                 590
   Gln Glu Gln Glu Asn Leu Glu Lys Gln Glu Leu Lys Lys Arg Ile Glu
           595                 600                 605
   Asn Phe Asn Glu Gln Glu Lys Glu Gln Val Ile Lys Asn Phe Glu Glu
       610                 615                 620
   Leu Ser Lys Tyr Lys Asn Ala Glu Glu Ser Pro Glu His Leu Asn Lys
   625                 630                 635                 640
   Phe Pro Ile Ile Ser Ile Ser Asp Leu Asn Lys Lys Thr Leu Glu Val
                   645                 650                 655
   Pro Val Asn Val Tyr Phe Thr Asn Ile Asn Glu Asn Asn Asn Ile Met
               660                 665                 670
   Glu Thr Tyr Asn Lys Leu Lys Thr Asn Glu His Met Leu Lys Asp Asn
           675                 680                 685
   Met Asp Val Phe Leu Lys Lys Tyr Val Leu Lys Asn Asp Lys His Asn
       690                 695                 700
   Thr Asn Asn Asn Asn Asn Asn Asn Asn Asn Met Asp Tyr Ser Phe Thr
   705                 710                 715                 720
   Glu Thr Lys Tyr Glu Gly Asn Val Pro Ile Leu Val Tyr Glu Met Pro
                   725                 730                 735
   Thr Thr Gly Ile Val Tyr Leu Gln Phe Val Phe Ser Leu Asp His Leu
               740                 745                 750
   Thr Val Asp Glu Leu Ala Tyr Leu Asn Leu Phe Lys Thr Leu Ile Leu
           755                 760                 765
   Glu Asn Lys Thr Asn Lys Arg Ser Ser Glu Asp Phe Val Ile Leu Arg
       770                 775                 780
   Glu Lys Asn Ile Gly Ser Met Ser Ala Asn Val Ala Leu Tyr Ser Lys
   785                 790                 795                 800
   Asp Asp His Leu Asn Val Thr Asp Lys Tyr Asn Ala Gln Ala Leu Phe
                   805                 810                 815
   Asn Leu Glu Met His Val Leu Ser His Lys Cys Asn Asp Ala Leu Asn
               820                 825                 830
   Ile Ala Leu Glu Ala Val Lys Glu Ser Asp Phe Ser Asn Lys Lys Lys
           835                 840                 845
   Val Ile Asp Ile Leu Lys Arg Lys Ile Asn Gly Met Lys Thr Thr Phe
       850                 855                 860
   Ser Glu Lys Gly Tyr Ala Ile Leu Met Lys Tyr Val Lys Ala His Leu
   865                 870                 875                 880
   Asn Ser Lys His Tyr Ala His Asn Ile Ile Tyr Gly Tyr Glu Asn Tyr
                   885                 890                 895
   Leu Lys Leu Gln Glu Gln Leu Glu Leu Ala Glu Asn Asp Phe Lys Thr
               900                 905                 910
   Leu Glu Asn Ile Leu Val Arg Ile Arg Asn Lys Ile Phe Asn Lys Lys
           915                 920                 925
   Asn Leu Met Val Ser Val Thr Ser Asp Tyr Gly Ala Leu Lys His Leu
       930                 935                 940
   Phe Val Asn Ser Asn Glu Ser Leu Lys Asn Leu Val Ser Tyr Phe Glu
   945                 950                 955                 960
   Glu Asn Asp Lys Tyr Ile Asn Asp Met Gln Asn Lys Val Asn Asp Pro
                   965                 970                 975
   Thr Val Met Gly Trp Asn Glu Glu Ile Lys Ser Lys Lys Leu Phe Asp
   980 985 990
   Glu Glu Lys Val Lys Lys Glu Phe Phe Val Leu Pro Thr Phe Val Asn
   995 1000 1005
   Ser Val Ser Met Ser Gly Ile Leu Phe Lys Pro Gly Glu Tyr Leu
   1010 1015 1020
   Asp Pro Ser Phe Thr Val Ile Val Ala Ala Leu Lys Asn Ser Tyr
   1025 1030 1035
   Leu Trp Asp Thr Val Arg Gly Leu Asn Gly Ala Tyr Gly Val Phe
   1040 1045 1050
   Ala Asp Ile Glu Tyr Asp Gly Ser Val Val Phe Leu Ser Ala Arg
   1055 1060 1065
   Asp Pro Asn Leu Glu Lys Thr Leu Ala Thr Phe Arg Glu Ser Ala
   1070 1075 1080
   Lys Gly Leu Arg Lys Met Ala Asp Thr Met Thr Glu Asn Asp Leu
   1085 1090 1095
   Leu Arg Tyr Ile Ile Asn Thr Ile Gly Thr Ile Asp Lys Pro Arg
   1100 1105 1110
   Arg Gly Ile Glu Leu Ser Lys Leu Ser Phe Leu Arg Leu Ile Ser
   1115 1120 1125
   Asn Glu Ser Glu Gln Asp Arg Val Glu Phe Arg Lys Arg Ile Met
   1130 1135 1140
   Asn Thr Lys Lys Glu Asp Phe Tyr Lys Phe Ala Asp Leu Leu Glu
   1145 1150 1155
   Ser Lys Val Asn Glu Phe Glu Lys Asn Ile Val Ile Ile Thr Thr
   1160 1165 1170
   Lys Glu Lys Ala Asn Glu Tyr Ile Ala Asn Val Asp Gly Glu Phe
   1175 1180 1185
   Lys Lys Val Leu Ile Glu
   1190
<210> 42
   <211> 1193
   <212> PRT
   <213> Plasmodium falciparum
<220>
   <221> misc_feature
   <223> Falcilysin
<400> 42
   Met Asn Leu Thr Lys Leu Met Lys Val Ile Gly Tyr Ile Asn Ile Ile
   1               5                   10                  15
   Thr Asn Cys Val Gln Ser Phe Thr Asn Arg Ala Asp Lys Lys Arg Tyr
               20                  25                  30
   Asn Val Phe Ala Lys Ser Phe Ile Asn Thr Ile Asn Thr Asn Leu Tyr
           35                  40                  45
   Thr Phe Lys Ala Val Met Ser Lys Thr Pro Glu Trp Ile His Glu Lys
       50                  55                  60
   Ser Pro Lys His Asn Ser Tyr Asp Ile Ile Glu Lys Arg Tyr Asn Glu
   65                  70                  75                  80
   Glu Phe Lys Met Thr Tyr Thr Val Tyr Gln His Lys Lys Ala Lys Thr
                   85                  90                  95
   Gln Val Ile Ser Leu Gly Thr Asn Asp Pro Leu Asp Val Glu Gln Ala
               100                 105                 110
   Phe Ala Phe Tyr Val Lys Thr Leu Thr His Ser Gly Lys Gly Ile Pro
           115                 120                 125
   His Ile Leu Glu His Ser Val Leu Ser Gly Ser Lys Asn Tyr Asn Tyr
       130                 135                 140
   Lys Asn Ser Ile Gly Leu Leu Glu Lys Gly Thr Leu His Thr His Leu
   145                 150                 155                 160
   Asn Ala Tyr Thr Phe Asn Asp Arg Thr Val Tyr Met Ala Gly Ser Met
                   165                 170                 175
   Asn Asn Lys Asp Phe Phe Asn Ile Met Gly Val Tyr Met Asp Ser Val
               180                 185                 190
   Phe Gln Pro Asn Val Leu Glu Asn Lys Tyr Ile Phe Glu Thr Glu Gly
           195                 200                 205
   Trp Thr Tyr Glu Val Glu Lys Leu Lys Glu Asp Glu Lys Gly Lys Ala
       210                 215                 220
   Glu Ile Pro Gln Met Lys Asp Tyr Lys Val Ser Phe Asn Gly Ile Val
   225 230 235 240
   Tyr Asn Glu Met Lys Gly Ala Leu Ser Ser Pro Leu Glu Asp Leu Tyr
   245 250 255
   His Glu Glu Met Lys Tyr Met Phe Pro Asp Asn Val His Ser Asn Asn
   260 265 270
   Ser Gly Gly Asp Pro Lys Glu Ile Thr Asn Leu Thr Tyr Glu Glu Phe
   275 280 285
   Lys Glu Phe Tyr Tyr Lys Asn Tyr Asn Pro Lys Lys Val Lys Val Phe
   290 295 300
   Phe Phe Ser Lys Asn Asn Pro Thr Glu Leu Leu Asn Phe Val Asp Gln
   305 310 315 320
   Tyr Leu Gly Gln Leu Asp Tyr Ser Lys Tyr Arg Asp Asp Ala Val Glu
   325 330 335
   Ser Val Glu Tyr Gln Thr Tyr Lys Lys Gly Pro Phe Tyr Ile Lys Lys
   340 345 350
   Lys Tyr Gly Asp His Ser Glu Glu Lys Glu Asn Leu Val Ser Val Ala
   355 360 365
   Trp Leu Leu Asn Pro Lys Val Asp Lys Thr Asn Asn His Asn Asn Asn
   370 375 380
   His Ser Asn Asn Gln Ser Ser Glu Asn Asn Gly Tyr Ser Asn Gly Ser
   385 390 395 400
   His Ser Ser Asp Leu Ser Leu Glu Asn Pro Thr Asp Tyr Phe Val Leu
   405 410 415
   Leu Ile Ile Asn Asn Leu Leu Ile His Thr Pro Glu Ser Val Leu Tyr
   420 425 430
   Lys Ala Leu Thr Asp Cys Gly Leu Gly Asn Asn Val Ile Asp Arg Gly
   435 440 445
   Leu Asn Asp Ser Leu Val Gln Tyr Ile Phe Ser Ile Gly Leu Lys Gly
   450 455 460
   Ile Lys Arg Asn Asn Glu Lys Ile Lys Asn Phe Asp Lys Val His Tyr
   465 470 475 480
   Glu Val Glu Asp Val Ile Met Asn Ala Leu Lys Lys Val Val Lys Glu
   485 490 495
   Gly Phe Asn Lys Ser Ala Val Glu Ala Ser Ile Asn Asn Ile Glu Phe
   500 505 510
   Ile Leu Lys Glu Ala Asn Leu Lys Thr Ser Lys Ser Ile Asp Phe Val
   515 520 525
   Phe Glu Met Thr Ser Lys Leu Asn Tyr Asn Arg Asp Pro Leu Leu Ile
   530 535 540
   Phe Glu Phe Glu Lys Tyr Leu Asn Ile Val Lys Asn Lys Ile Lys Asn
   545 550 555 560
   Glu Pro Met Tyr Leu Glu Lys Phe Val Glu Lys His Phe Ile Asn Asn
   565 570 575
   Ala His Arg Ser Val Ile Leu Leu Glu Gly Asp Glu Asn Tyr Ala Gln
   580 585 590
   Glu Gln Glu Asn Leu Glu Lys Gln Glu Leu Lys Lys Arg Ile Glu Asn
   595 600 605
   Phe Asn Glu Gln Glu Lys Glu Gln Val Ile Lys Asn Phe Glu Glu Leu
   610 615 620
   Ser Lys Tyr Lys Asn Ala Glu Glu Ser Pro Glu His Leu Asn Lys Phe
   625 630 635 640
   Pro Ile Ile Ser Ile Ser Asp Leu Asn Lys Lys Thr Leu Glu Val Pro
   645 650 655
   Val Asn Val Tyr Phe Thr Asn Ile Asn Glu Asn Asn Asn Ile Met Glu
   660 665 670
   Thr Tyr Asn Lys Leu Lys Thr Asn Glu His Met Leu Lys Asp Asn Met
   675 680 685
   Asp Val Phe Leu Lys Lys Tyr Val Leu Lys Asn Asp Lys His Asn Thr
   690 695 700
   Asn Asn Asn Asn Asn Asn Asn Asn Asn Met Asp Tyr Ser Phe Thr Glu
   705 710 715 720
   Thr Lys Tyr Glu Gly Asn Val Pro Ile Leu Val Tyr Glu Met Pro Thr
   725 730 735
   Thr Gly Ile Val Tyr Leu Gln Phe Val Phe Ser Leu Asp His Leu Thr
   740 745 750
   Val Asp Glu Leu Ala Tyr Leu Asn Leu Phe Lys Thr Leu Ile Leu Glu
   755 760 765
   Asn Lys Thr Asn Lys Arg Ser Ser Glu Asp Phe Val Ile Leu Arg Glu
   770 775 780
   Lys Asn Ile Gly Ser Met Ser Ala Asn Val Ala Leu Tyr Ser Lys Asp
   785 790 795 800
   Asp His Leu Asn Val Thr Asp Lys Tyr Asn Ala Gln Ala Leu Phe Asn
   805 810 815
   Leu Glu Met His Val Leu Ser His Lys Cys Asn Asp Ala Leu Asn Ile
   820 825 830
   Ala Leu Glu Ala Val Lys Glu Ser Asp Phe Ser Asn Lys Lys Lys Val
   835 840 845
   Ile Asp Ile Leu Lys Arg Lys Ile Asn Gly Met Lys Thr Thr Phe Ser
   850 855 860
   Glu Lys Gly Tyr Ala Ile Leu Met Lys Tyr Val Lys Ala His Leu Asn
   865 870 875 880
   Ser Lys His Tyr Ala His Asn Ile Ile Tyr Gly Tyr Glu Asn Tyr Leu
   885 890 895
   Lys Leu Gln Glu Gln Leu Glu Leu Ala Glu Asn Asp Phe Lys Thr Leu
   900 905 910
   Glu Asn Ile Leu Val Arg Ile Arg Asn Lys Ile Phe Asn Lys Lys Asn
   915 920 925
   Leu Met Val Ser Val Thr Ser Asp Tyr Gly Ala Leu Lys His Leu Phe
   930 935 940
   Val Asn Ser Asn Glu Ser Leu Lys Asn Leu Val Ser Tyr Phe Glu Glu
   945 950 955 960
   Asn Asp Lys Tyr Ile Asn Asp Met Gln Asn Lys Val Asn Asp Pro Thr
   965 970 975
   Val Met Gly Trp Asn Glu Glu Ile Lys Ser Lys Lys Leu Phe Asp Glu
   980 985 990
   Glu Lys Val Lys Lys Glu Phe Phe Val Leu Pro Thr Phe Val Asn Ser
   995 1000 1005
   Val Ser Met Ser Gly Ile Leu Phe Lys Pro Gly Glu Tyr Leu Asp
   1010 1015 1020
   Pro Ser Phe Thr Val Ile Val Ala Ala Leu Lys Asn Ser Tyr Leu
   1025 1030 1035
   Trp Asp Thr Val Arg Gly Leu Asn Gly Ala Tyr Gly Val Phe Ala
   1040 1045 1050
   Asp Ile Glu Tyr Asp Gly Ser Val Val Phe Leu Ser Ala Arg Asp
   1055 1060 1065
   Pro Asn Leu Glu Lys Thr Leu Ala Thr Phe Arg Glu Ser Ala Lys
   1070 1075 1080
   Gly Leu Arg Lys Met Ala Asp Thr Met Thr Glu Asn Asp Leu Leu
   1085 1090 1095
   Arg Tyr Ile Ile Asn Thr Ile Gly Thr Ile Asp Lys Pro Arg Arg
   1100 1105 1110
   Gly Ile Glu Leu Ser Lys Leu Ser Phe Leu Arg Leu Ile Ser Asn
   1115 1120 1125
   Glu Ser Glu Gln Asp Arg Val Glu Phe Arg Lys Arg Ile Met Asn
   1130 1135 1140
   Thr Lys Lys Glu Asp Phe Tyr Lys Phe Ala Asp Leu Leu Glu Ser
   1145 1150 1155
   Lys Val Asn Glu Phe Glu Lys Asn Ile Val Ile Ile Thr Thr Lys
   1160 1165 1170
   Glu Lys Ala Asn Glu Tyr Ile Ala Asn Val Asp Gly Glu Phe Lys
   1175 1180 1185
   Lys Val Leu Ile Glu
   1190
<210> 43
   <211> 3471
   <212> DNA
   <213> Artificial Sequence
<220>
   <223> PvFalcilysin Human CO
<400> 43
   gccaccatgg ctaaactgat gagggtcttt ggttatctta acataattac taactgcgtt 60
   aacggacttc tctgtaaagc ggagaaacgc aaatataacg tctttacgaa cagctttatc 120
   tattctatct ctacaacaaa tctgtactcc ttcacggcca tgatgaacaa atcacccgag 180
   tgggtgcagg aaaagtgtcc cgagcacaag tcttataaca tccttgaaaa gcggttttct 240
   gataaattcc aaatgacgta tacagtgtac gagcacaaaa aagcgaaaac ccaagtcatt 300
   gccctcggca gcaatgaccc tctggacgtt gagcagactt ttgcctttta cgtaaagacc 360
   ctgacgaact caggtaaggg tattcctcac atactcgagc acacagtttt gagcgggagc 420
   aaaaatttca attacaaaga tagcatggga ctcctggaaa agggaacact taacacccat 480
   ttgaatgcgt atactttcaa cgacaggact gtgtatatgg cggggtctat gaataataga 540
   gactttttca acataatggc ggtgtatatg gatagcgtgt ttcaaccgaa tgttcttgaa 600
   aataagttta tcttccaaac ggaaggatgg acctacgaag tggaaaagtt gaaagatgag 660
   gagaagaacg ctgatgtacc gaaaattaaa gattataagg tttcctacaa tggtatagtc 720
   tattccgaaa tgaaagggag cttctcctct cccctgcaat atctttatta tttgatcatg 780
   aaaaacatct tccctgacaa cgtccattct aacataagtg gaggggaccc taaggagatt 840
   ccaacgctca cgtatgagga attcaaagag ttctactata agaattacaa tcctaaaaaa 900
   attaaagtca ttttcttcag caaaaacaat cctacagagt tgttgaattt cgtcgacgat 960
   tatttgaacc agctcgactt tactaaatac agagacgatg cagtagaaaa cgttaattat 1020
   caggagtaca agaaggggcc attctatgtt aagaagaaat ttgcggatca cagtgaggaa 1080
   aaggaaaatc ttgtttccat ttcatggctt ctcaacccga aaaaaaatga cctcctggat 1140
   gttgatctta gccttgaaag cccccaagac tacttcgctt tgttgattat taacaacctt 1200
   ctgacgcaca ccaccgaatc tgtgctttac aaggcgctta ttgactgcgg tttgggtaac 1260
   accgttatcg atacaggcct tgacgacagc ctggtacagt tcatattttc tataggtctg 1320
   aagggtatca aggaaaagaa tgaaaagaac gtatcattgg acgttgtcca ttatgaagtg 1380
   gaaaaggtgg ttctcaaagc gctccaaaaa gtagtggatg aaggcttcaa caaatcagcg 1440
   gtcgaggcca gcattaacaa catagaattc gttttgaaag aagccaatct taaaacgtcc 1500
   aaaagcgtgg actacatatt tgaaatggct tccaggctga attataatag agaccctctg 1560
   ctgatctttg aattcgagaa gcacttgaac gtcgttaaag ataagatcaa gaatgaacca 1620
   aagtatttgg agaaattcat tgagaaacac tttataaaca ataaccatcg ggctgtcatc 1680
   ttgatggagg gtgatgaaaa ctatgggaaa gaacaggagg atttggaaaa ggagactttg 1740
   aaaaagaaga tcgaatcact cacggaaaaa gaacgggacg acataatagt tgatttcgag      1800
   aacctgacaa agtataagaa catggtcgag agccccgaac atttggataa ttttcccatc      1860
   atctctatca gcgatctgaa caaagaaacg ctggaaatcc ctgctaatgc atatttcacg      1920
   tccacggcag aggaaaataa tatggaaaag tacaataaag tgaaggcaag cgaggatgtt      1980
   atgaagaaaa acatggacca cctcatcgac aaatatgttc tgaaaggagc gcaaggaggc      2040
   gctgctaccg acggtgcagc caaacagggt gattccagcg atggtgaaat ccccatgctc      2100
   gtttacgaga tgcctaccag tggaatcttg tatctgcaat ttatctttaa cctcgatcat      2160
   ctgagcctgg aggaaatgtc atatctcaat ctctttaaga tgctcatcct tgagaacaaa      2220
   actatgaaac gctcctctga ggagtttgtc attttgaggg aaaagaatat cggcaacata      2280
   atggctaacg tcgccttgta tagtatcagc gatcacctca aggtcacttc taaatacaac      2340
   gctcacggtc tgttcaattt tgaaatgcat gtattgagtc acaagtgcaa tgagtctttg      2400
   gaaattgcct tggaagctct caaggactct gattttagca acaagaagaa aatagtggag      2460
   atcctgaaac ggaagatcaa cggcatgaag acggtcttct cttctaaagg ctactcactt      2520
   ctcctcaagt atgtaaagtc acaaatgaat gcaaaatact acgctcatga tttggttttt      2580
   ggatacggca actatttgaa gttgcaagaa cagctcaagc tcgccgaaag tgactttcca      2640
   cagttcgagc agattctcaa cagaatccga aataagattt tcactaagaa gaatctgctg      2700
   atcagcgtga ccagtgacgc tgcagcgttg gatcaactgt ttgtgcatag caaggaatcc      2760
   ctgaagaacc ttcttgggta tttcgaagag aatgatgcca agtctggaga ggctgagacc      2820
   atagggtgga atgaggagat taaacaatca aaagtgatcg aaaaggaaca aaagaagaag      2880
   gaattctttg taataccaac atttgtcaat gcagtatcaa tggctggaat gttgtttaat      2940
   gagaaggagt tcctggatcc gtctttcata gttatcgtgg ccgcattgaa aaactcatac      3000
   ctttgggaga ccgtgagggg actcaacggg gcatatggag tttttgctga tatagaatac      3060
   gatggtgccg tcgtgttctt gtcagcccgc gatccgaatc tggaaaagac gctgcaaacg      3120
   ttcaaggagt ctgctcaagg gttgagaaag atggcggata caatgaccaa aaatgacctt      3180
   cggcgctaca taatcaatgc gatcggcaat attgataagc cgagacgggg tgttgagctc      3240
   tcaaagctct cacttttgag aattatatct aatgaaacca aacaggaccg aatcgacttt      3300
   cgcaagagga ttatggagac gacgaaggaa gacttctata aattcgctga cttgcttgaa      3360
   aagaagattg cagaatttga aaagaatatt gttattatca catctaagga aaaagcctca      3420
   gaatacagca ctaacgtaga ccaggatttt aagcagattc atatcgagta g              3471
<210> 44
   <211> 1154
   <212> PRT
   <213> Plasmodium vivax
<220>
   <221> misc_feature
   <223> PvFalcilysin
<400> 44
   Met Ala Lys Leu Met Arg Val Phe Gly Tyr Leu Asn Ile Ile Thr Asn
   1               5                   10                  15
   Cys Val Asn Gly Leu Leu Cys Lys Ala Glu Lys Arg Lys Tyr Asn Val
               20                  25                  30
   Phe Thr Asn Ser Phe Ile Tyr Ser Ile Ser Thr Thr Asn Leu Tyr Ser
           35                  40                  45
   Phe Thr Ala Met Met Asn Lys Ser Pro Glu Trp Val Gln Glu Lys Cys
       50                  55                  60
   Pro Glu His Lys Ser Tyr Asn Ile Leu Glu Lys Arg Phe Ser Asp Lys
   65                  70                  75                  80
   Phe Gln Met Thr Tyr Thr Val Tyr Glu His Lys Lys Ala Lys Thr Gln
                   85                  90                  95
   Val Ile Ala Leu Gly Ser Asn Asp Pro Leu Asp Val Glu Gln Thr Phe
               100                 105                 110
   Ala Phe Tyr Val Lys Thr Leu Thr Asn Ser Gly Lys Gly Ile Pro His
           115                 120                 125
   Ile Leu Glu His Thr Val Leu Ser Gly Ser Lys Asn Phe Asn Tyr Lys
       130                 135                 140
   Asp Ser Met Gly Leu Leu Glu Lys Gly Thr Leu Asn Thr His Leu Asn
   145                 150                 155                 160
   Ala Tyr Thr Phe Asn Asp Arg Thr Val Tyr Met Ala Gly Ser Met Asn
                   165                 170                 175
   Asn Arg Asp Phe Phe Asn Ile Met Ala Val Tyr Met Asp Ser Val Phe
               180                 185                 190
   Gln Pro Asn Val Leu Glu Asn Lys Phe Ile Phe Gln Thr Glu Gly Trp
           195                 200                 205
   Thr Tyr Glu Val Glu Lys Leu Lys Asp Glu Glu Lys Asn Ala Asp Val
       210                 215                 220
   Pro Lys Ile Lys Asp Tyr Lys Val Ser Tyr Asn Gly Ile Val Tyr Ser
   225                 230                 235                 240
   Glu Met Lys Gly Ser Phe Ser Ser Pro Leu Gln Tyr Leu Tyr Tyr Leu
                   245                 250                 255
   Ile Met Lys Asn Ile Phe Pro Asp Asn Val His Ser Asn Ile Ser Gly
               260                 265                 270
   Gly Asp Pro Lys Glu Ile Pro Thr Leu Thr Tyr Glu Glu Phe Lys Glu
           275                 280                 285
   Phe Tyr Tyr Lys Asn Tyr Asn Pro Lys Lys Ile Lys Val Ile Phe Phe
       290                 295                 300
   Ser Lys Asn Asn Pro Thr Glu Leu Leu Asn Phe Val Asp Asp Tyr Leu
   305                 310                 315                 320
   Asn Gln Leu Asp Phe Thr Lys Tyr Arg Asp Asp Ala Val Glu Asn Val
                   325                 330                 335
   Asn Tyr Gln Glu Tyr Lys Lys Gly Pro Phe Tyr Val Lys Lys Lys Phe
               340                 345                 350
   Ala Asp His Ser Glu Glu Lys Glu Asn Leu Val Ser Ile Ser Trp Leu
           355                 360                 365
   Leu Asn Pro Lys Lys Asn Asp Leu Leu Asp Val Asp Leu Ser Leu Glu
       370                 375                 380
   Ser Pro Gln Asp Tyr Phe Ala Leu Leu Ile Ile Asn Asn Leu Leu Thr
   385                 390                 395                 400
   His Thr Thr Glu Ser Val Leu Tyr Lys Ala Leu Ile Asp Cys Gly Leu
                   405                 410                 415
   Gly Asn Thr Val Ile Asp Thr Gly Leu Asp Asp Ser Leu Val Gln Phe
               420                 425                 430
   Ile Phe Ser Ile Gly Leu Lys Gly Ile Lys Glu Lys Asn Glu Lys Asn
           435                 440                 445
   Val Ser Leu Asp Val Val His Tyr Glu Val Glu Lys Val Val Leu Lys
       450                 455                 460
   Ala Leu Gln Lys Val Val Asp Glu Gly Phe Asn Lys Ser Ala Val Glu
   465 470 475 480
   Ala Ser Ile Asn Asn Ile Glu Phe Val Leu Lys Glu Ala Asn Leu Lys
   485 490 495
   Thr Ser Lys Ser Val Asp Tyr Ile Phe Glu Met Ala Ser Arg Leu Asn
   500 505 510
   Tyr Asn Arg Asp Pro Leu Leu Ile Phe Glu Phe Glu Lys His Leu Asn
   515 520 525
   Val Val Lys Asp Lys Ile Lys Asn Glu Pro Lys Tyr Leu Glu Lys Phe
   530 535 540
   Ile Glu Lys His Phe Ile Asn Asn Asn His Arg Ala Val Ile Leu Met
   545 550 555 560
   Glu Gly Asp Glu Asn Tyr Gly Lys Glu Gln Glu Asp Leu Glu Lys Glu
   565 570 575
   Thr Leu Lys Lys Lys Ile Glu Ser Leu Thr Glu Lys Glu Arg Asp Asp
   580 585 590
   Ile Ile Val Asp Phe Glu Asn Leu Thr Lys Tyr Lys Asn Met Val Glu
   595 600 605
   Ser Pro Glu His Leu Asp Asn Phe Pro Ile Ile Ser Ile Ser Asp Leu
   610 615 620
   Asn Lys Glu Thr Leu Glu Ile Pro Ala Asn Ala Tyr Phe Thr Ser Thr
   625 630 635 640
   Ala Glu Glu Asn Asn Met Glu Lys Tyr Asn Lys Val Lys Ala Ser Glu
   645 650 655
   Asp Val Met Lys Lys Asn Met Asp His Leu Ile Asp Lys Tyr Val Leu
   660 665 670
   Lys Gly Ala Gln Gly Gly Ala Ala Thr Asp Gly Ala Ala Lys Gln Gly
   675 680 685
   Asp Ser Ser Asp Gly Glu Ile Pro Met Leu Val Tyr Glu Met Pro Thr
   690 695 700
   Ser Gly Ile Leu Tyr Leu Gln Phe Ile Phe Asn Leu Asp His Leu Ser
   705 710 715 720
   Leu Glu Glu Met Ser Tyr Leu Asn Leu Phe Lys Met Leu Ile Leu Glu
   725 730 735
   Asn Lys Thr Met Lys Arg Ser Ser Glu Glu Phe Val Ile Leu Arg Glu
   740 745 750
   Lys Asn Ile Gly Asn Ile Met Ala Asn Val Ala Leu Tyr Ser Ile Ser
   755 760 765
   Asp His Leu Lys Val Thr Ser Lys Tyr Asn Ala His Gly Leu Phe Asn
   770 775 780
   Phe Glu Met His Val Leu Ser His Lys Cys Asn Glu Ser Leu Glu Ile
   785 790 795 800
   Ala Leu Glu Ala Leu Lys Asp Ser Asp Phe Ser Asn Lys Lys Lys Ile
   805 810 815
   Val Glu Ile Leu Lys Arg Lys Ile Asn Gly Met Lys Thr Val Phe Ser
   820 825 830
   Ser Lys Gly Tyr Ser Leu Leu Leu Lys Tyr Val Lys Ser Gln Met Asn
   835 840 845
   Ala Lys Tyr Tyr Ala His Asp Leu Val Phe Gly Tyr Gly Asn Tyr Leu
   850 855 860
   Lys Leu Gln Glu Gln Leu Lys Leu Ala Glu Ser Asp Phe Pro Gln Phe
   865 870 875 880
   Glu Gln Ile Leu Asn Arg Ile Arg Asn Lys Ile Phe Thr Lys Lys Asn
   885 890 895
   Leu Leu Ile Ser Val Thr Ser Asp Ala Ala Ala Leu Asp Gln Leu Phe
   900 905 910
   Val His Ser Lys Glu Ser Leu Lys Asn Leu Leu Gly Tyr Phe Glu Glu
   915 920 925
   Asn Asp Ala Lys Ser Gly Glu Ala Glu Thr Ile Gly Trp Asn Glu Glu
   930 935 940
   Ile Lys Gln Ser Lys Val Ile Glu Lys Glu Gln Lys Lys Lys Glu Phe
   945 950 955 960
   Phe Val Ile Pro Thr Phe Val Asn Ala Val Ser Met Ala Gly Met Leu
   965 970 975
   Phe Asn Glu Lys Glu Phe Leu Asp Pro Ser Phe Ile Val Ile Val Ala
   980 985 990
   Ala Leu Lys Asn Ser Tyr Leu Trp Glu Thr Val Arg Gly Leu Asn Gly
   995 1000 1005
   Ala Tyr Gly Val Phe Ala Asp Ile Glu Tyr Asp Gly Ala Val Val
   1010 1015 1020
   Phe Leu Ser Ala Arg Asp Pro Asn Leu Glu Lys Thr Leu Gln Thr
   1025 1030 1035
   Phe Lys Glu Ser Ala Gln Gly Leu Arg Lys Met Ala Asp Thr Met
   1040 1045 1050
   Thr Lys Asn Asp Leu Arg Arg Tyr Ile Ile Asn Ala Ile Gly Asn
   1055 1060 1065
   Ile Asp Lys Pro Arg Arg Gly Val Glu Leu Ser Lys Leu Ser Leu
   1070 1075 1080
   Leu Arg Ile Ile Ser Asn Glu Thr Lys Gln Asp Arg Ile Asp Phe
   1085 1090 1095
   Arg Lys Arg Ile Met Glu Thr Thr Lys Glu Asp Phe Tyr Lys Phe
   1100 1105 1110
   Ala Asp Leu Leu Glu Lys Lys Ile Ala Glu Phe Glu Lys Asn Ile
   1115 1120 1125
   Val Ile Ile Thr Ser Lys Glu Lys Ala Ser Glu Tyr Ser Thr Asn
   1130 1135 1140
   Val Asp Gln Asp Phe Lys Gln Ile His Ile Glu
   1145 1150
<210> 45
   <211> 1153
   <212> PRT
   <213> Plasmodium vivax
<220>
   <221> misc_feature
   <223> Falcilysin
<400> 45
   Met Lys Leu Met Arg Val Phe Gly Tyr Leu Asn Ile Ile Thr Asn Cys
   1 5 10 15
   Val Asn Gly Leu Leu Cys Lys Ala Glu Lys Arg Lys Tyr Asn Val Phe
               20                  25                  30
   Thr Asn Ser Phe Ile Tyr Ser Ile Ser Thr Thr Asn Leu Tyr Ser Phe
           35                  40                  45
   Thr Ala Met Met Asn Lys Ser Pro Glu Trp Val Gln Glu Lys Cys Pro
       50                  55                  60
   Glu His Lys Ser Tyr Asn Ile Leu Glu Lys Arg Phe Ser Asp Lys Phe
   65                  70                  75                  80
   Gln Met Thr Tyr Thr Val Tyr Glu His Lys Lys Ala Lys Thr Gln Val
                   85                  90                  95
   Ile Ala Leu Gly Ser Asn Asp Pro Leu Asp Val Glu Gln Thr Phe Ala
               100                 105                 110
   Phe Tyr Val Lys Thr Leu Thr Asn Ser Gly Lys Gly Ile Pro His Ile
           115                 120                 125
   Leu Glu His Thr Val Leu Ser Gly Ser Lys Asn Phe Asn Tyr Lys Asp
       130                 135                 140
   Ser Met Gly Leu Leu Glu Lys Gly Thr Leu Asn Thr His Leu Asn Ala
   145                 150                 155                 160
   Tyr Thr Phe Asn Asp Arg Thr Val Tyr Met Ala Gly Ser Met Asn Asn
                   165                 170                 175
   Arg Asp Phe Phe Asn Ile Met Ala Val Tyr Met Asp Ser Val Phe Gln
               180                 185                 190
   Pro Asn Val Leu Glu Asn Lys Phe Ile Phe Gln Thr Glu Gly Trp Thr
           195                 200                 205
   Tyr Glu Val Glu Lys Leu Lys Asp Glu Glu Lys Asn Ala Asp Val Pro
       210                 215                 220
   Lys Ile Lys Asp Tyr Lys Val Ser Tyr Asn Gly Ile Val Tyr Ser Glu
   225                 230                 235                 240
   Met Lys Gly Ser Phe Ser Ser Pro Leu Gln Tyr Leu Tyr Tyr Leu Ile
                   245                 250                 255
   Met Lys Asn Ile Phe Pro Asp Asn Val His Ser Asn Ile Ser Gly Gly
               260                 265                 270
   Asp Pro Lys Glu Ile Pro Thr Leu Thr Tyr Glu Glu Phe Lys Glu Phe
           275                 280                 285
   Tyr Tyr Lys Asn Tyr Asn Pro Lys Lys Ile Lys Val Ile Phe Phe Ser
       290                 295                 300
   Lys Asn Asn Pro Thr Glu Leu Leu Asn Phe Val Asp Asp Tyr Leu Asn
   305                 310                 315                 320
   Gln Leu Asp Phe Thr Lys Tyr Arg Asp Asp Ala Val Glu Asn Val Asn
                   325                 330                 335
   Tyr Gln Glu Tyr Lys Lys Gly Pro Phe Tyr Val Lys Lys Lys Phe Ala
               340                 345                 350
   Asp His Ser Glu Glu Lys Glu Asn Leu Val Ser Ile Ser Trp Leu Leu
           355                 360                 365
   Asn Pro Lys Lys Asn Asp Leu Leu Asp Val Asp Leu Ser Leu Glu Ser
       370                 375                 380
   Pro Gln Asp Tyr Phe Ala Leu Leu Ile Ile Asn Asn Leu Leu Thr His
   385                 390                 395                 400
   Thr Thr Glu Ser Val Leu Tyr Lys Ala Leu Ile Asp Cys Gly Leu Gly
                   405                 410                 415
   Asn Thr Val Ile Asp Thr Gly Leu Asp Asp Ser Leu Val Gln Phe Ile
               420                 425                 430
   Phe Ser Ile Gly Leu Lys Gly Ile Lys Glu Lys Asn Glu Lys Asn Val
           435                 440                 445
   Ser Leu Asp Val Val His Tyr Glu Val Glu Lys Val Val Leu Lys Ala
       450                 455                 460
   Leu Gln Lys Val Val Asp Glu Gly Phe Asn Lys Ser Ala Val Glu Ala
   465                 470                 475                 480
   Ser Ile Asn Asn Ile Glu Phe Val Leu Lys Glu Ala Asn Leu Lys Thr
                   485                 490                 495
   Ser Lys Ser Val Asp Tyr Ile Phe Glu Met Ala Ser Arg Leu Asn Tyr
               500                 505                 510
   Asn Arg Asp Pro Leu Leu Ile Phe Glu Phe Glu Lys His Leu Asn Val
           515                 520                 525
   Val Lys Asp Lys Ile Lys Asn Glu Pro Lys Tyr Leu Glu Lys Phe Ile
       530                 535                 540
   Glu Lys His Phe Ile Asn Asn Asn His Arg Ala Val Ile Leu Met Glu
   545                 550                 555                 560
   Gly Asp Glu Asn Tyr Gly Lys Glu Gln Glu Asp Leu Glu Lys Glu Thr
                   565                 570                 575
   Leu Lys Lys Lys Ile Glu Ser Leu Thr Glu Lys Glu Arg Asp Asp Ile
               580                 585                 590
   Ile Val Asp Phe Glu Asn Leu Thr Lys Tyr Lys Asn Met Val Glu Ser
           595                 600                 605
   Pro Glu His Leu Asp Asn Phe Pro Ile Ile Ser Ile Ser Asp Leu Asn
       610                 615                 620
   Lys Glu Thr Leu Glu Ile Pro Ala Asn Ala Tyr Phe Thr Ser Thr Ala
   625                 630                 635                 640
   Glu Glu Asn Asn Met Glu Lys Tyr Asn Lys Val Lys Ala Ser Glu Asp
                   645                 650                 655
   Val Met Lys Lys Asn Met Asp His Leu Ile Asp Lys Tyr Val Leu Lys
               660                 665                 670
   Gly Ala Gln Gly Gly Ala Ala Thr Asp Gly Ala Ala Lys Gln Gly Asp
           675                 680                 685
   Ser Ser Asp Gly Glu Ile Pro Met Leu Val Tyr Glu Met Pro Thr Ser
       690                 695                 700
   Gly Ile Leu Tyr Leu Gln Phe Ile Phe Asn Leu Asp His Leu Ser Leu
   705                 710                 715                 720
   Glu Glu Met Ser Tyr Leu Asn Leu Phe Lys Met Leu Ile Leu Glu Asn
                   725                 730                 735
   Lys Thr Met Lys Arg Ser Ser Glu Glu Phe Val Ile Leu Arg Glu Lys
               740                 745                 750
   Asn Ile Gly Asn Ile Met Ala Asn Val Ala Leu Tyr Ser Ile Ser Asp
           755                 760                 765
   His Leu Lys Val Thr Ser Lys Tyr Asn Ala His Gly Leu Phe Asn Phe
   770 775 780
   Glu Met His Val Leu Ser His Lys Cys Asn Glu Ser Leu Glu Ile Ala
   785 790 795 800
   Leu Glu Ala Leu Lys Asp Ser Asp Phe Ser Asn Lys Lys Lys Ile Val
   805 810 815
   Glu Ile Leu Lys Arg Lys Ile Asn Gly Met Lys Thr Val Phe Ser Ser
   820 825 830
   Lys Gly Tyr Ser Leu Leu Leu Lys Tyr Val Lys Ser Gln Met Asn Ala
   835 840 845
   Lys Tyr Tyr Ala His Asp Leu Val Phe Gly Tyr Gly Asn Tyr Leu Lys
   850 855 860
   Leu Gln Glu Gln Leu Lys Leu Ala Glu Ser Asp Phe Pro Gln Phe Glu
   865 870 875 880
   Gln Ile Leu Asn Arg Ile Arg Asn Lys Ile Phe Thr Lys Lys Asn Leu
   885 890 895
   Leu Ile Ser Val Thr Ser Asp Ala Ala Ala Leu Asp Gln Leu Phe Val
   900 905 910
   His Ser Lys Glu Ser Leu Lys Asn Leu Leu Gly Tyr Phe Glu Glu Asn
   915 920 925
   Asp Ala Lys Ser Gly Glu Ala Glu Thr Ile Gly Trp Asn Glu Glu Ile
   930 935 940
   Lys Gln Ser Lys Val Ile Glu Lys Glu Gln Lys Lys Lys Glu Phe Phe
   945 950 955 960
   Val Ile Pro Thr Phe Val Asn Ala Val Ser Met Ala Gly Met Leu Phe
   965 970 975
   Asn Glu Lys Glu Phe Leu Asp Pro Ser Phe Ile Val Ile Val Ala Ala
   980 985 990
   Leu Lys Asn Ser Tyr Leu Trp Glu Thr Val Arg Gly Leu Asn Gly Ala
   995 1000 1005
   Tyr Gly Val Phe Ala Asp Ile Glu Tyr Asp Gly Ala Val Val Phe
   1010 1015 1020
   Leu Ser  Ala Arg Asp Pro Asn  Leu Glu Lys Thr Leu  Gln Thr Phe
       1025                 1030                 1035
   Lys Glu  Ser Ala Gln Gly Leu  Arg Lys Met Ala Asp  Thr Met Thr
       1040                 1045                 1050
   Lys Asn  Asp Leu Arg Arg Tyr  Ile Ile Asn Ala Ile  Gly Asn Ile
       1055                 1060                 1065
   Asp Lys  Pro Arg Arg Gly Val  Glu Leu Ser Lys Leu  Ser Leu Leu
       1070                 1075                 1080
   Arg Ile  Ile Ser Asn Glu Thr  Lys Gln Asp Arg Ile  Asp Phe Arg
       1085                 1090                 1095
   Lys Arg  Ile Met Glu Thr Thr  Lys Glu Asp Phe Tyr  Lys Phe Ala
       1100                 1105                 1110
   Asp Leu  Leu Glu Lys Lys Ile  Ala Glu Phe Glu Lys  Asn Ile Val
       1115                 1120                 1125
   Ile Ile  Thr Ser Lys Glu Lys  Ala Ser Glu Tyr Ser  Thr Asn Val
       1130                 1135                 1140
   Asp Gln  Asp Phe Lys Gln Ile  His Ile Glu
       1145                 1150
<210> 46
   <211> 1227
   <212> DNA
   <213> Artificial Sequence
<220>
   <223> Falcilysin, Bergheilysin-B-CO
<400> 46
   ggatccgcca ccatggctaa actcatgaag gtcttggggt atatcaacat catcacgaat      60
   tgcgtgaatg gcatactgtg taaaggcgat aagaagcgat acatgcacat gctctcccat     120
   aagtgtaacg acgctctgga gattgcgctg gaagccctga aagaaagcga tttcagcaat     180
   aagaagaaag tgattgaaat cctgaagagg aaaatcaatg gcatgaaaac cacgtttgcc     240
   agcaaaggtc actcaatact gattaagtac gtgaagtctc ggattaatag caaatattac     300
   gcttatgacc tgattcatgg gtatgacaac tatctgaagc tccaggaaca gcttaaactg     360
   gcggagacaa actacgagag tctggaagcc atacttaatc gcatcaggaa gaagatcttt     420
   aagcggaaca atctgatcat gaacgtgact gtcgatccag gaactattga tcagctgttt     480
   gccaaatcca agaatagctt caacaacctt ctgtcatact ttgacgagaa cgagagctac 540
   tgctctaaga atgactcctt caacaaagtg gtaggctgga acaaggagat tcaggagaag 600
   aagctgcttg aaggagagga ggtcaagaaa gagctcttgg tcgtacccac atttgtgaac 660
   agtgtctcta tgagtggagt gctgttcaac aaaggcgaat atcttgaccc ctcattcacc 720
   gtaatcgttg cagccttgaa aaactcatac ctttgggaga cagttcgagg actgaatgga 780
   gcttatgggg tgtttgccga catagagtat gacggtactg tcgttttcct gagtgcaaga 840
   gatccgaatc tggaaaagac tctccaaacc tttcgtgaag cagcacaagg tctgcgtaaa 900
   atggctgatg tgatgacaaa gaatgatctt ctgcggtata tcattaacgc tattgggacc 960
   atcgatagac ctcgcagagg cgttgagttg tccaagctgt ccttctctcg cataataagc 1020
   aacgaaaccg aacaggacag gattgaattc aggaaccggg tgatgaatac caagaaagag 1080
   gatttctaca aatttgcgga tttgctcgaa aagaaagtca aggaatttga gaagaatgtg 1140
   gtgatcatca catctaagga gaaagccaac gagtacatca acaatgttga caatgacttc 1200
   aagaagatcc tcattgagta actcgag 1227
<210> 47
   <211> 402
   <212> PRT
   <213> Plasmodium Berghei
<220>
   <221> misc_feature
   <223> Falcilysin, Bergheilysin-B
<400> 47
   Met Ala Lys Leu Met Lys Val Leu Gly Tyr Ile Asn Ile Ile Thr Asn
   1 5 10 15
   Cys Val Asn Gly Ile Leu Cys Lys Gly Asp Lys Lys Arg Tyr Met His
   20 25 30
   Met Leu Ser His Lys Cys Asn Asp Ala Leu Glu Ile Ala Leu Glu Ala
   35 40 45
   Leu Lys Glu Ser Asp Phe Ser Asn Lys Lys Lys Val Ile Glu Ile Leu
   50 55 60
   Lys Arg Lys Ile Asn Gly Met Lys Thr Thr Phe Ala Ser Lys Gly His
   65 70 75 80
   Ser Ile Leu Ile Lys Tyr Val Lys Ser Arg Ile Asn Ser Lys Tyr Tyr
   85 90 95
   Ala Tyr Asp Leu Ile His Gly Tyr Asp Asn Tyr Leu Lys Leu Gln Glu
   100 105 110
   Gln Leu Lys Leu Ala Glu Thr Asn Tyr Glu Ser Leu Glu Ala Ile Leu
   115 120 125
   Asn Arg Ile Arg Lys Lys Ile Phe Lys Arg Asn Asn Leu Ile Met Asn
   130 135 140
   Val Thr Val Asp Pro Gly Thr Ile Asp Gln Leu Phe Ala Lys Ser Lys
   145 150 155 160
   Asn Ser Phe Asn Asn Leu Leu Ser Tyr Phe Asp Glu Asn Glu Ser Tyr
   165 170 175
   Cys Ser Lys Asn Asp Ser Phe Asn Lys Val Val Gly Trp Asn Lys Glu
   180 185 190
   Ile Gln Glu Lys Lys Leu Leu Glu Gly Glu Glu Val Lys Lys Glu Leu
   195 200 205
   Leu Val Val Pro Thr Phe Val Asn Ser Val Ser Met Ser Gly Val Leu
   210 215 220
   Phe Asn Lys Gly Glu Tyr Leu Asp Pro Ser Phe Thr Val Ile Val Ala
   225 230 235 240
   Ala Leu Lys Asn Ser Tyr Leu Trp Glu Thr Val Arg Gly Leu Asn Gly
   245 250 255
   Ala Tyr Gly Val Phe Ala Asp Ile Glu Tyr Asp Gly Thr Val Val Phe
   260 265 270
   Leu Ser Ala Arg Asp Pro Asn Leu Glu Lys Thr Leu Gln Thr Phe Arg
   275 280 285
   Glu Ala Ala Gln Gly Leu Arg Lys Met Ala Asp Val Met Thr Lys Asn
   290 295 300
   Asp Leu Leu Arg Tyr Ile Ile Asn Ala Ile Gly Thr Ile Asp Arg Pro
   305 310 315 320
   Arg Arg Gly Val Glu Leu Ser Lys Leu Ser Phe Ser Arg Ile Ile Ser
   325 330 335
   Asn Glu Thr Glu Gln Asp Arg Ile Glu Phe Arg Asn Arg Val Met Asn
   340 345 350
   Thr Lys Lys Glu Asp Phe Tyr Lys Phe Ala Asp Leu Leu Glu Lys Lys
   355 360 365
   Val Lys Glu Phe Glu Lys Asn Val Val Ile Ile Thr Ser Lys Glu Lys
   370 375 380
   Ala Asn Glu Tyr Ile Asn Asn Val Asp Asn Asp Phe Lys Lys Ile Leu
   385 390 395 400
   Ile Glu
<210> 48
   <211> 2454
   <212> DNA
   <213> Plasmodium Berghei
<220>
   <221> misc_feature
   <223> perforin like protein 1 (SPECT2)
<400> 48
   ggatccgcca ccatggctaa gatgcggaac attaagaaat cacttccggt gctcttcatt 60
   ctcttgtgca tttatcagca gtactttata aactctctcc gtatttccgt ccgcaacaat 120
   aagaaccaca gggatgaaaa caaattcaac aagaatatgg agcttggtac gatggagaaa 180
   cccatcaaca ttctttgcaa tgacgtcagc tgtaacacag agaataacat ttctttcgta 240
   aaccagaaga aaaaggaaat agatagtgac agcgacctct ataacatgtt cgatgacgac 300
   gcctctacct ccgctggtga tgacgaagat gactatgacg actacacaga tgataagaac 360
   gctgagatca aagatgaaga gcaaaatgag cacatcgaca agatcgacca gaagaaggat 420
   aagaaacgca cattctctat caataagcag gaggaagaaa tcaacgaaaa taaaaacaaa 480
   acggagaaat tcttcaagaa atacaagttt aacgacgcca actcagaagg ggacgacgat 540
   gagtcagaca ccgatgacga gaatttggac aactccacgg agaacagcta cgaggaaaac 600
   aagaatcccg agaacgttat cgacaagcat atggccgtat ttcctgggct ctattttgtg 660
   gggatcggct acgatattct gttcgggaat cctctcggag aaaccgattc tctgagcgac 720
   cctggttata gagcacagat ttacctgttg aattgggaat tctcaaacca tggcatcgcc 780
   aacgatctgc acacgctcca accaattaat gcctggattc ggaaagagaa tgcatgctca 840
   cgagttgaat ccatcaacga atgttcctct gttagtgagt atacaaagaa tctgtcagtg 900
   gacgtaagtg tatcaggcag ctatatgggc ttcggcagtt tctccgctag cactgggtac 960
   aagaaattca ttaacgagat aagcaagaga acatccaaga cctactttat aaagagcaac 1020
   tgcatcaagt ataccatcgg acttccacct tatgtgccat gggagcatac cactgcttac 1080
   atgaatgcgg tgaatattct gccaaaggaa tttaccggcc tggatggaga cagcgaatgc 1140
   acaccggatg tttacgagca gaagaagatg actaaacagt gtaagaacgt gcaactgtgg 1200
   attcagttct ttaagaccta tggtacacac ataatcgtgg aggctcaatt gggagggaaa 1260
   ataaccaaaa tcatcaatgt ctctaacaca agcgtgaacc agatgaagaa ggatggcgtc 1320
   agtgtgaaag cccagatcca ggctcagttt ggttttgcaa gtgtgggcgg tagtacaagc 1380
   gtgtcaagtg acaatagcac taagaacgac aatagcagct acgacatgtc tgagaaactc 1440
   gtggttatcg gagggaatcc tataaaagac gtcaccaaag aggagaatct gtacgaatgg 1500
   agcaaaacag tgtcctctaa ccccatgcct atccacatca agctgctgcc aatctataag 1560
   tccttcgata gtgaggaact gaaagagtct tacgagaaag cggttctcta ctacaccagg 1620
   ctttatggca gctctcccca cggaactatt cagaaagatg agaacgacat catcaaaatc 1680
   ttgacggcca gtaccaccat cactaaaatt ggtgctccac ccataacagc ggaatgtcca 1740
   cataatcaag tggtgctgtt tgggtatgtc ctgaagcaga acttctggga caacacctcc 1800
   aacctgaagg gatacgacat tgagatctgt gaggctggac tgaatagttg cacgtccaaa 1860
   cagggaagta caaacaagta cgatgtgagc tatctgtaca ttgaatgtgg cacacaggca 1920
   atgtcattct ccgatcaagt cataaccgca tccaacacta cttacaatac catcaagtgt 1980
   cccaatgact acactattat ctttggattc gggttttcct ctagctccgg taagggagtt 2040
   tctgccatgc acacccacat tacatcctgt agacccggca tgaaaagctg ctccctgaat 2100
   atgggcaaca gcaatgacaa gaactacatg tacctggtgt gcgtcgatgc cacaatctgg 2160
   tctggcatta atgagctgac tattgtggcc aaagatgatt ttcacggcgc agtgaatagg 2220
   tctaagcagt tcaatgatgg cgaattggta ctgtcctgtc aggaaaatgg cactatcctg 2280
   acagggttca ctggggagac tcatacctct agcccgtatg tcaagagccc ttttagcaag 2340
   tgtcttaaat cactgaagag ctgctcagtc catgggagtg gacaatctat cggatatacc 2400
   aactataagt cactgttttc catcatactg tgcaagaacg gtgagtgact cgag 2454
<210> 49
   <211> 811
   <212> PRT
   <213> Plasmodium Berghei
<220>
   <221> misc_feature
   <223> perforin like protein 1 (SPECT2)
<400> 49
   Met Ala Lys Met Arg Asn Ile Lys Lys Ser Leu Pro Val Leu Phe Ile
   1               5                   10                  15
   Leu Leu Cys Ile Tyr Gln Gln Tyr Phe Ile Asn Ser Leu Arg Ile Ser
               20                  25                  30
   Val Arg Asn Asn Lys Asn His Arg Asp Glu Asn Lys Phe Asn Lys Asn
           35                  40                  45
   Met Glu Leu Gly Thr Met Glu Lys Pro Ile Asn Ile Leu Cys Asn Asp
       50                  55                  60
   Val Ser Cys Asn Thr Glu Asn Asn Ile Ser Phe Val Asn Gln Lys Lys
   65                  70                  75                  80
   Lys Glu Ile Asp Ser Asp Ser Asp Leu Tyr Asn Met Phe Asp Asp Asp
                   85                  90                  95
   Ala Ser Thr Ser Ala Gly Asp Asp Glu Asp Asp Tyr Asp Asp Tyr Thr
               100                 105                 110
   Asp Asp Lys Asn Ala Glu Ile Lys Asp Glu Glu Gln Asn Glu His Ile
           115                 120                 125
   Asp Lys Ile Asp Gln Lys Lys Asp Lys Lys Arg Thr Phe Ser Ile Asn
       130                 135                 140
   Lys Gln Glu Glu Glu Ile Asn Glu Asn Lys Asn Lys Thr Glu Lys Phe
   145                 150                 155                 160
   Phe Lys Lys Tyr Lys Phe Asn Asp Ala Asn Ser Glu Gly Asp Asp Asp
                   165                 170                 175
   Glu Ser Asp Thr Asp Asp Glu Asn Leu Asp Asn Ser Thr Glu Asn Ser
               180                 185                 190
   Tyr Glu Glu Asn Lys Asn Pro Glu Asn Val Ile Asp Lys His Met Ala
           195                 200                 205
   Val Phe Pro Gly Leu Tyr Phe Val Gly Ile Gly Tyr Asp Ile Leu Phe
       210                 215                 220
   Gly Asn Pro Leu Gly Glu Thr Asp Ser Leu Ser Asp Pro Gly Tyr Arg
   225                 230                 235                 240
   Ala Gln Ile Tyr Leu Leu Asn Trp Glu Phe Ser Asn His Gly Ile Ala
                   245                 250                 255
   Asn Asp Leu His Thr Leu Gln Pro Ile Asn Ala Trp Ile Arg Lys Glu
               260                 265                 270
   Asn Ala Cys Ser Arg Val Glu Ser Ile Asn Glu Cys Ser Ser Val Ser
   275 280 285
   Glu Tyr Thr Lys Asn Leu Ser Val Asp Val Ser Val Ser Gly Ser Tyr
   290 295 300
   Met Gly Phe Gly Ser Phe Ser Ala Ser Thr Gly Tyr Lys Lys Phe Ile
   305 310 315 320
   Asn Glu Ile Ser Lys Arg Thr Ser Lys Thr Tyr Phe Ile Lys Ser Asn
   325 330 335
   Cys Ile Lys Tyr Thr Ile Gly Leu Pro Pro Tyr Val Pro Trp Glu His
   340 345 350
   Thr Thr Ala Tyr Met Asn Ala Val Asn Ile Leu Pro Lys Glu Phe Thr
   355 360 365
   Gly Leu Asp Gly Asp Ser Glu Cys Thr Pro Asp Val Tyr Glu Gln Lys
   370 375 380
   Lys Met Thr Lys Gln Cys Lys Asn Val Gln Leu Trp Ile Gln Phe Phe
   385 390 395 400
   Lys Thr Tyr Gly Thr His Ile Ile Val Glu Ala Gln Leu Gly Gly Lys
   405 410 415
   Ile Thr Lys Ile Ile Asn Val Ser Asn Thr Ser Val Asn Gln Met Lys
   420 425 430
   Lys Asp Gly Val Ser Val Lys Ala Gln Ile Gln Ala Gln Phe Gly Phe
   435 440 445
   Ala Ser Val Gly Gly Ser Thr Ser Val Ser Ser Asp Asn Ser Thr Lys
   450 455 460
   Asn Asp Asn Ser Ser Tyr Asp Met Ser Glu Lys Leu Val Val Ile Gly
   465 470 475 480
   Gly Asn Pro Ile Lys Asp Val Thr Lys Glu Glu Asn Leu Tyr Glu Trp
   485 490 495
   Ser Lys Thr Val Ser Ser Asn Pro Met Pro Ile His Ile Lys Leu Leu
   500 505 510
   Pro Ile Tyr Lys Ser Phe Asp Ser Glu Glu Leu Lys Glu Ser Tyr Glu
   515 520 525
   Lys Ala Val Leu Tyr Tyr Thr Arg Leu Tyr Gly Ser Ser Pro His Gly
   530 535 540
   Thr Ile Gln Lys Asp Glu Asn Asp Ile Ile Lys Ile Leu Thr Ala Ser
   545 550 555 560
   Thr Thr Ile Thr Lys Ile Gly Ala Pro Pro Ile Thr Ala Glu Cys Pro
   565 570 575
   His Asn Gln Val Val Leu Phe Gly Tyr Val Leu Lys Gln Asn Phe Trp
   580 585 590
   Asp Asn Thr Ser Asn Leu Lys Gly Tyr Asp Ile Glu Ile Cys Glu Ala
   595 600 605
   Gly Leu Asn Ser Cys Thr Ser Lys Gln Gly Ser Thr Asn Lys Tyr Asp
   610 615 620
   Val Ser Tyr Leu Tyr Ile Glu Cys Gly Thr Gln Ala Met Ser Phe Ser
   625 630 635 640
   Asp Gln Val Ile Thr Ala Ser Asn Thr Thr Tyr Asn Thr Ile Lys Cys
   645 650 655
   Pro Asn Asp Tyr Thr Ile Ile Phe Gly Phe Gly Phe Ser Ser Ser Ser
   660 665 670
   Gly Lys Gly Val Ser Ala Met His Thr His Ile Thr Ser Cys Arg Pro
   675 680 685
   Gly Met Lys Ser Cys Ser Leu Asn Met Gly Asn Ser Asn Asp Lys Asn
   690 695 700
   Tyr Met Tyr Leu Val Cys Val Asp Ala Thr Ile Trp Ser Gly Ile Asn
   705 710 715 720
   Glu Leu Thr Ile Val Ala Lys Asp Asp Phe His Gly Ala Val Asn Arg
   725 730 735
   Ser Lys Gln Phe Asn Asp Gly Glu Leu Val Leu Ser Cys Gln Glu Asn
   740 745 750
   Gly Thr Ile Leu Thr Gly Phe Thr Gly Glu Thr His Thr Ser Ser Pro
   755 760 765
   Tyr Val Lys Ser Pro Phe Ser Lys Cys Leu Lys Ser Leu Lys Ser Cys
   770 775 780
   Ser Val His Gly Ser Gly Gln Ser Ile Gly Tyr Thr Asn Tyr Lys Ser
   785 790 795 800
   Leu Phe Ser Ile Ile Leu Cys Lys Asn Gly Glu
   805 810
<210> 50
   <211> 810
   <212> PRT
   <213> Plasmodium berghei
<220>
   <221> misc_feature
   <223> SPECT2
<400> 50
   Met Lys Met Arg Asn Ile Lys Lys Ser Leu Pro Val Leu Phe Ile Leu
   1 5 10 15
   Leu Cys Ile Tyr Gln Gln Tyr Phe Ile Asn Ser Leu Arg Ile Ser Val
   20 25 30
   Arg Asn Asn Lys Asn His Arg Asp Glu Asn Lys Phe Asn Lys Asn Met
   35 40 45
   Glu Leu Gly Thr Met Glu Lys Pro Ile Asn Ile Leu Cys Asn Asp Val
   50 55 60
   Ser Cys Asn Thr Glu Asn Asn Ile Ser Phe Val Asn Gln Lys Lys Lys
   65 70 75 80
   Glu Ile Asp Ser Asp Ser Asp Leu Tyr Asn Met Phe Asp Asp Asp Ala
   85 90 95
   Ser Thr Ser Ala Gly Asp Asp Glu Asp Asp Tyr Asp Asp Tyr Thr Asp
   100 105 110
   Asp Lys Asn Ala Glu Ile Lys Asp Glu Glu Gln Asn Glu His Ile Asp
   115 120 125
   Lys Ile Asp Gln Lys Lys Asp Lys Lys Arg Thr Phe Ser Ile Asn Lys
   130 135 140
   Gln Glu Glu Glu Ile Asn Glu Asn Lys Asn Lys Thr Glu Lys Phe Phe
   145 150 155 160
   Lys Lys Tyr Lys Phe Asn Asp Ala Asn Ser Glu Gly Asp Asp Asp Glu
                   165                 170                 175
   Ser Asp Thr Asp Asp Glu Asn Leu Asp Asn Ser Thr Glu Asn Ser Tyr
               180                 185                 190
   Glu Glu Asn Lys Asn Pro Glu Asn Val Ile Asp Lys His Met Ala Val
           195                 200                 205
   Phe Pro Gly Leu Tyr Phe Val Gly Ile Gly Tyr Asp Ile Leu Phe Gly
       210                 215                 220
   Asn Pro Leu Gly Glu Thr Asp Ser Leu Ser Asp Pro Gly Tyr Arg Ala
   225                 230                 235                 240
   Gln Ile Tyr Leu Leu Asn Trp Glu Phe Ser Asn His Gly Ile Ala Asn
                   245                 250                 255
   Asp Leu His Thr Leu Gln Pro Ile Asn Ala Trp Ile Arg Lys Glu Asn
               260                 265                 270
   Ala Cys Ser Arg Val Glu Ser Ile Asn Glu Cys Ser Ser Val Ser Glu
           275                 280                 285
   Tyr Thr Lys Asn Leu Ser Val Asp Val Ser Val Ser Gly Ser Tyr Met
       290                 295                 300
   Gly Phe Gly Ser Phe Ser Ala Ser Thr Gly Tyr Lys Lys Phe Ile Asn
   305                 310                 315                 320
   Glu Ile Ser Lys Arg Thr Ser Lys Thr Tyr Phe Ile Lys Ser Asn Cys
                   325                 330                 335
   Ile Lys Tyr Thr Ile Gly Leu Pro Pro Tyr Val Pro Trp Glu His Thr
               340                 345                 350
   Thr Ala Tyr Met Asn Ala Val Asn Ile Leu Pro Lys Glu Phe Thr Gly
           355                 360                 365
   Leu Asp Gly Asp Ser Glu Cys Thr Pro Asp Val Tyr Glu Gln Lys Lys
       370                 375                 380
   Met Thr Lys Gln Cys Lys Asn Val Gln Leu Trp Ile Gln Phe Phe Lys
   385                 390                 395                 400
   Thr Tyr Gly Thr His Ile Ile Val Glu Ala Gln Leu Gly Gly Lys Ile
                   405                 410                 415
   Thr Lys Ile Ile Asn Val Ser Asn Thr Ser Val Asn Gln Met Lys Lys
   420 425 430
   Asp Gly Val Ser Val Lys Ala Gln Ile Gln Ala Gln Phe Gly Phe Ala
   435 440 445
   Ser Val Gly Gly Ser Thr Ser Val Ser Ser Asp Asn Ser Thr Lys Asn
   450 455 460
   Asp Asn Ser Ser Tyr Asp Met Ser Glu Lys Leu Val Val Ile Gly Gly
   465 470 475 480
   Asn Pro Ile Lys Asp Val Thr Lys Glu Glu Asn Leu Tyr Glu Trp Ser
   485 490 495
   Lys Thr Val Ser Ser Asn Pro Met Pro Ile His Ile Lys Leu Leu Pro
   500 505 510
   Ile Tyr Lys Ser Phe Asp Ser Glu Glu Leu Lys Glu Ser Tyr Glu Lys
   515 520 525
   Ala Val Leu Tyr Tyr Thr Arg Leu Tyr Gly Ser Ser Pro His Gly Thr
   530 535 540
   Ile Gln Lys Asp Glu Asn Asp Ile Ile Lys Ile Leu Thr Ala Ser Thr
   545 550 555 560
   Thr Ile Thr Lys Ile Gly Ala Pro Pro Ile Thr Ala Glu Cys Pro His
   565 570 575
   Asn Gln Val Val Leu Phe Gly Tyr Val Leu Lys Gln Asn Phe Trp Asp
   580 585 590
   Asn Thr Ser Asn Leu Lys Gly Tyr Asp Ile Glu Ile Cys Glu Ala Gly
   595 600 605
   Leu Asn Ser Cys Thr Ser Lys Gln Gly Ser Thr Asn Lys Tyr Asp Val
   610 615 620
   Ser Tyr Leu Tyr Ile Glu Cys Gly Thr Gln Ala Met Ser Phe Ser Asp
   625 630 635 640
   Gln Val Ile Thr Ala Ser Asn Thr Thr Tyr Asn Thr Ile Lys Cys Pro
   645 650 655
   Asn Asp Tyr Thr Ile Ile Phe Gly Phe Gly Phe Ser Ser Ser Ser Gly
                   660                 665                 670
   Lys Gly Val Ser Ala Met His Thr His Ile Thr Ser Cys Arg Pro Gly
           675                 680                 685
   Met Lys Ser Cys Ser Leu Asn Met Gly Asn Ser Asn Asp Lys Asn Tyr
       690                 695                 700
   Met Tyr Leu Val Cys Val Asp Ala Thr Ile Trp Ser Gly Ile Asn Glu
   705                 710                 715                 720
   Leu Thr Ile Val Ala Lys Asp Asp Phe His Gly Ala Val Asn Arg Ser
                   725                 730                 735
   Lys Gln Phe Asn Asp Gly Glu Leu Val Leu Ser Cys Gln Glu Asn Gly
               740                 745                 750
   Thr Ile Leu Thr Gly Phe Thr Gly Glu Thr His Thr Ser Ser Pro Tyr
           755                 760                 765
   Val Lys Ser Pro Phe Ser Lys Cys Leu Lys Ser Leu Lys Ser Cys Ser
       770                 775                 780
   Val His Gly Ser Gly Gln Ser Ile Gly Tyr Thr Asn Tyr Lys Ser Leu
   785                 790                 795                 800
   Phe Ser Ile Ile Leu Cys Lys Asn Gly Glu
                   805                 810
<210> 51
   <211> 2538
   <212> DNA
   <213> Artificial Sequence
<220>
   <223> PfSPECT2 human CO
<400> 51
   gccaccatgg ctaagctgag aatcctcaag aagcactatt atgtcgtgtt tatccttctg      60
   tatctgtatg acatcagctg cttcaagtgc attcggctga acaaccgttc catctacaag     120
   aacaagtaca agaataacgt gcacatagga accaatgaga atatcaggag tattgagaag     180
   tatagcaacg tgctgtgtaa cagcatcttg tgtaagaacg ataaaatctc tagcttcatt     240
   aaccagagga agaatgtgga tgacgacgat gagtctgaga acgatgacat gtacgagagc     300
   accacagctg gctcttctag tgaaacggac aacgagagcg atgaagaaga gaatgacagc     360
   agtgacaaca ataatagcga tgaggaacag atagagaact ccaacaacaa taattctgac     420
   gaggaacaga atgactcctc ttccaacgac aataatgatg aggagaatga ggaacaggac     480
   gacgtcatgg acaatgacca aaacgataag aagatcaagc atagcttcaa tctcgccaac 540
   gagagtaaac acactaaaga ggaacgagtg aaagaagaga aaaagctgaa gatctatgac 600
   ttcataaacg acaaggagaa aagacttaac tttaatggcg atcagaaaga tgaagataac 660
   gaggagaacg atgataaaga tgagaacacg cttgagaatc ggaatatcat ctccaaacac 720
   acttcagtgt ttcctggcct gtacttcatc gggattgggt ataacctcct cttcgggaac 780
   cccttgggtg aagctgattc ccttatcgat ccaggttatc gggcgcaaat ttacctgatg 840
   gaatgggctc tcagcaagga aggcattgcc aacgacctga gcactctgca acccgtgaat 900
   ggatggatac gaaaggagaa tgcctgctcc agagttgaat ctattacaga atgcagctct 960
   atatctgact acaccaaatc cctgtcagcg gaggcaaagg ttagtggctc ttattgggga 1020
   atcgcctcct tctcagcatc caccgggtat agctcttttc tccacgaggt gacaaaacgc 1080
   agcaagaaaa ccttcctcgt gaaatccaac tgtgtgaaat acactatcgg gcttcctccc 1140
   tatattccct gggacaagac cacggcctac aagaatgccg taaatgaact gccagctgta 1200
   tttaccggtt tggataaaga atccgaatgt ccctctgatg tgtacgaaga gaacaagaca 1260
   aaatcaaact gcgagaacgt gagtctgtgg atgaagttct tcgacattta tggcactcac 1320
   atcatttatg aaagtcaatt gggaggaaag ataacaaaga ttatcaatgt ttccacctca 1380
   agtattgagc agatgaagaa aaatggagtc tcagtcaaag cgaaaattca agcacagttt 1440
   gggtttggtt cagccggtgg ctcaaccgac gtgaatagca gtaactcctc cgcaaatgat 1500
   gagcagtctt acgacatgaa tgaacagctg atcgtcatag gagggaatcc gatcaaagac 1560
   gtcaccaagg aggagaatct gtttgagtgg tctaaaactg tgacaaacca tcctatgccg 1620
   atcaacatta aactgactcc cattagcgac agttttgact cagacgacct gaaagaatcc 1680
   tatgataaag ccatcatcta ctattctcgc ctgtacggac tgtcccctca tgacacaatg 1740
   cagaaggatg acaaggatat tatcaagatc ctgaccaacg ctgatacggt taccaagaac 1800
   tcagctcctc caatcaacgc tcagtgtcct catgggaaag tcgttatgtt cggattcagc 1860
   ctgaagcaga atttctggga caacaccaac gcactcaaag gatacaacat cgaagtctgc 1920
   gaagcaggga gcaattcttg cactagcaaa caagggagca gcaataaata cgatacatct 1980
   tacctttaca tggaatgtgg cgatcagcca ttgcccttta gcgagcaggt gattagcgag 2040
   agtacaagta cctataatac cgtgaaatgt ccgaatgatt actccattct cctgggcttc 2100
   ggaatatcaa gtagctcagg gaggataaat agcgctgaat atgtctacag cacaccatgt 2160
   attcccggca tgaagtcctg cagcctcaat atgaataatg acaaccagaa gtcatacatc 2220
   tacgtgctgt gtgtagatac tacgatctgg agtggcgtga acaacctgag cctggttgcc 2280
   cttgatggcg cacatggtaa ggtaaaccgc agtaagaagt actccgacgg tgaactggtt 2340
   ggcacctgtc cactggacgg cacagtcctg actggattta aggttgagtt tcacacttca     2400
   tctccatatg tgcagacacc tttcgagaaa tgcgccaaaa gcttgaaagc ctgctccgta     2460
   catggctccg gtcacgccat tggcattcag aactttaagt cactgttcat atacatgttg     2520
   tgcaagaaca ataagtaa                                                   2538
<210> 52
   <211> 843
   <212> PRT
   <213> Plasmodium falciparum
<220>
   <221> misc_feature
   <223> PfSPECT2
<400> 52
   Met Ala Lys Leu Arg Ile Leu Lys Lys His Tyr Tyr Val Val Phe Ile
   1               5                   10                  15
   Leu Leu Tyr Leu Tyr Asp Ile Ser Cys Phe Lys Cys Ile Arg Leu Asn
               20                  25                  30
   Asn Arg Ser Ile Tyr Lys Asn Lys Tyr Lys Asn Asn Val His Ile Gly
           35                  40                  45
   Thr Asn Glu Asn Ile Arg Ser Ile Glu Lys Tyr Ser Asn Val Leu Cys
       50                  55                  60
   Asn Ser Ile Leu Cys Lys Asn Asp Lys Ile Ser Ser Phe Ile Asn Gln
   65                  70                  75                  80
   Arg Lys Asn Val Asp Asp Asp Asp Glu Ser Glu Asn Asp Asp Met Tyr
                   85                  90                  95
   Glu Ser Thr Thr Ala Gly Ser Ser Ser Glu Thr Asp Asn Glu Ser Asp
               100                 105                 110
   Glu Glu Glu Asn Asp Ser Ser Asp Asn Asn Asn Ser Asp Glu Glu Gln
           115                 120                 125
   Ile Glu Asn Ser Asn Asn Asn Asn Ser Asp Glu Glu Gln Asn Asp Ser
       130                 135                 140
   Ser Ser Asn Asp Asn Asn Asp Glu Glu Asn Glu Glu Gln Asp Asp Val
   145                 150                 155                 160
   Met Asp Asn Asp Gln Asn Asp Lys Lys Ile Lys His Ser Phe Asn Leu
                   165                 170                 175
   Ala Asn Glu Ser Lys His Thr Lys Glu Glu Arg Val Lys Glu Glu Lys
               180                 185                 190
   Lys Leu Lys Ile Tyr Asp Phe Ile Asn Asp Lys Glu Lys Arg Leu Asn
           195                 200                 205
   Phe Asn Gly Asp Gln Lys Asp Glu Asp Asn Glu Glu Asn Asp Asp Lys
       210                 215                 220
   Asp Glu Asn Thr Leu Glu Asn Arg Asn Ile Ile Ser Lys His Thr Ser
   225                 230                 235                 240
   Val Phe Pro Gly Leu Tyr Phe Ile Gly Ile Gly Tyr Asn Leu Leu Phe
                   245                 250                 255
   Gly Asn Pro Leu Gly Glu Ala Asp Ser Leu Ile Asp Pro Gly Tyr Arg
               260                 265                 270
   Ala Gln Ile Tyr Leu Met Glu Trp Ala Leu Ser Lys Glu Gly Ile Ala
           275                 280                 285
   Asn Asp Leu Ser Thr Leu Gln Pro Val Asn Gly Trp Ile Arg Lys Glu
       290                 295                 300
   Asn Ala Cys Ser Arg Val Glu Ser Ile Thr Glu Cys Ser Ser Ile Ser
   305                 310                 315                 320
   Asp Tyr Thr Lys Ser Leu Ser Ala Glu Ala Lys Val Ser Gly Ser Tyr
                   325                 330                 335
   Trp Gly Ile Ala Ser Phe Ser Ala Ser Thr Gly Tyr Ser Ser Phe Leu
               340                 345                 350
   His Glu Val Thr Lys Arg Ser Lys Lys Thr Phe Leu Val Lys Ser Asn
           355                 360                 365
   Cys Val Lys Tyr Thr Ile Gly Leu Pro Pro Tyr Ile Pro Trp Asp Lys
       370                 375                 380
   Thr Thr Ala Tyr Lys Asn Ala Val Asn Glu Leu Pro Ala Val Phe Thr
   385                 390                 395                 400
   Gly Leu Asp Lys Glu Ser Glu Cys Pro Ser Asp Val Tyr Glu Glu Asn
                   405                 410                 415
   Lys Thr Lys Ser Asn Cys Glu Asn Val Ser Leu Trp Met Lys Phe Phe
   420 425 430
   Asp Ile Tyr Gly Thr His Ile Ile Tyr Glu Ser Gln Leu Gly Gly Lys
   435 440 445
   Ile Thr Lys Ile Ile Asn Val Ser Thr Ser Ser Ile Glu Gln Met Lys
   450 455 460
   Lys Asn Gly Val Ser Val Lys Ala Lys Ile Gln Ala Gln Phe Gly Phe
   465 470 475 480
   Gly Ser Ala Gly Gly Ser Thr Asp Val Asn Ser Ser Asn Ser Ser Ala
   485 490 495
   Asn Asp Glu Gln Ser Tyr Asp Met Asn Glu Gln Leu Ile Val Ile Gly
   500 505 510
   Gly Asn Pro Ile Lys Asp Val Thr Lys Glu Glu Asn Leu Phe Glu Trp
   515 520 525
   Ser Lys Thr Val Thr Asn His Pro Met Pro Ile Asn Ile Lys Leu Thr
   530 535 540
   Pro Ile Ser Asp Ser Phe Asp Ser Asp Asp Leu Lys Glu Ser Tyr Asp
   545 550 555 560
   Lys Ala Ile Ile Tyr Tyr Ser Arg Leu Tyr Gly Leu Ser Pro His Asp
   565 570 575
   Thr Met Gln Lys Asp Asp Lys Asp Ile Ile Lys Ile Leu Thr Asn Ala
   580 585 590
   Asp Thr Val Thr Lys Asn Ser Ala Pro Pro Ile Asn Ala Gln Cys Pro
   595 600 605
   His Gly Lys Val Val Met Phe Gly Phe Ser Leu Lys Gln Asn Phe Trp
   610 615 620
   Asp Asn Thr Asn Ala Leu Lys Gly Tyr Asn Ile Glu Val Cys Glu Ala
   625 630 635 640
   Gly Ser Asn Ser Cys Thr Ser Lys Gln Gly Ser Ser Asn Lys Tyr Asp
   645 650 655
   Thr Ser Tyr Leu Tyr Met Glu Cys Gly Asp Gln Pro Leu Pro Phe Ser
   660 665 670
   Glu Gln Val Ile Ser Glu Ser Thr Ser Thr Tyr Asn Thr Val Lys Cys
               675                 680                 685
   Pro Asn Asp Tyr Ser Ile Leu Leu Gly Phe Gly Ile Ser Ser Ser Ser
       690                 695                 700
   Gly Arg Ile Asn Ser Ala Glu Tyr Val Tyr Ser Thr Pro Cys Ile Pro
   705                 710                 715                 720
   Gly Met Lys Ser Cys Ser Leu Asn Met Asn Asn Asp Asn Gln Lys Ser
                   725                 730                 735
   Tyr Ile Tyr Val Leu Cys Val Asp Thr Thr Ile Trp Ser Gly Val Asn
               740                 745                 750
   Asn Leu Ser Leu Val Ala Leu Asp Gly Ala His Gly Lys Val Asn Arg
           755                 760                 765
   Ser Lys Lys Tyr Ser Asp Gly Glu Leu Val Gly Thr Cys Pro Leu Asp
       770                 775                 780
   Gly Thr Val Leu Thr Gly Phe Lys Val Glu Phe His Thr Ser Ser Pro
   785                 790                 795                 800
   Tyr Val Gln Thr Pro Phe Glu Lys Cys Ala Lys Ser Leu Lys Ala Cys
                   805                 810                 815
   Ser Val His Gly Ser Gly His Ala Ile Gly Ile Gln Asn Phe Lys Ser
               820                 825                 830
   Leu Phe Ile Tyr Met Leu Cys Lys Asn Asn Lys
           835                 840
<210> 53
   <211> 842
   <212> PRT
   <213> Plasmodium falciparum
<220>
   <221> misc_feature
   <223> SPECT2
<400> 53
   Met Lys Leu Arg Ile Leu Lys Lys His Tyr Tyr Val Val Phe Ile Leu
   1               5                   10                  15
   Leu Tyr Leu Tyr Asp Ile Ser Cys Phe Lys Cys Ile Arg Leu Asn Asn
               20                  25                  30
   Arg Ser Ile Tyr Lys Asn Lys Tyr Lys Asn Asn Val His Ile Gly Thr
   35 40 45
   Asn Glu Asn Ile Arg Ser Ile Glu Lys Tyr Ser Asn Val Leu Cys Asn
   50 55 60
   Ser Ile Leu Cys Lys Asn Asp Lys Ile Ser Ser Phe Ile Asn Gln Arg
   65 70 75 80
   Lys Asn Val Asp Asp Asp Asp Glu Ser Glu Asn Asp Asp Met Tyr Glu
   85 90 95
   Ser Thr Thr Ala Gly Ser Ser Ser Glu Thr Asp Asn Glu Ser Asp Glu
   100 105 110
   Glu Glu Asn Asp Ser Ser Asp Asn Asn Asn Ser Asp Glu Glu Gln Ile
   115 120 125
   Glu Asn Ser Asn Asn Asn Asn Ser Asp Glu Glu Gln Asn Asp Ser Ser
   130 135 140
   Ser Asn Asp Asn Asn Asp Glu Glu Asn Glu Glu Gln Asp Asp Val Met
   145 150 155 160
   Asp Asn Asp Gln Asn Asp Lys Lys Ile Lys His Ser Phe Asn Leu Ala
   165 170 175
   Asn Glu Ser Lys His Thr Lys Glu Glu Arg Val Lys Glu Glu Lys Lys
   180 185 190
   Leu Lys Ile Tyr Asp Phe Ile Asn Asp Lys Glu Lys Arg Leu Asn Phe
   195 200 205
   Asn Gly Asp Gln Lys Asp Glu Asp Asn Glu Glu Asn Asp Asp Lys Asp
   210 215 220
   Glu Asn Thr Leu Glu Asn Arg Asn Ile Ile Ser Lys His Thr Ser Val
   225 230 235 240
   Phe Pro Gly Leu Tyr Phe Ile Gly Ile Gly Tyr Asn Leu Leu Phe Gly
   245 250 255
   Asn Pro Leu Gly Glu Ala Asp Ser Leu Ile Asp Pro Gly Tyr Arg Ala
   260 265 270
   Gln Ile Tyr Leu Met Glu Trp Ala Leu Ser Lys Glu Gly Ile Ala Asn
   275 280 285
   Asp Leu Ser Thr Leu Gln Pro Val Asn Gly Trp Ile Arg Lys Glu Asn
       290                 295                 300
   Ala Cys Ser Arg Val Glu Ser Ile Thr Glu Cys Ser Ser Ile Ser Asp
   305                 310                 315                 320
   Tyr Thr Lys Ser Leu Ser Ala Glu Ala Lys Val Ser Gly Ser Tyr Trp
                   325                 330                 335
   Gly Ile Ala Ser Phe Ser Ala Ser Thr Gly Tyr Ser Ser Phe Leu His
               340                 345                 350
   Glu Val Thr Lys Arg Ser Lys Lys Thr Phe Leu Val Lys Ser Asn Cys
           355                 360                 365
   Val Lys Tyr Thr Ile Gly Leu Pro Pro Tyr Ile Pro Trp Asp Lys Thr
       370                 375                 380
   Thr Ala Tyr Lys Asn Ala Val Asn Glu Leu Pro Ala Val Phe Thr Gly
   385                 390                 395                 400
   Leu Asp Lys Glu Ser Glu Cys Pro Ser Asp Val Tyr Glu Glu Asn Lys
                   405                 410                 415
   Thr Lys Ser Asn Cys Glu Asn Val Ser Leu Trp Met Lys Phe Phe Asp
               420                 425                 430
   Ile Tyr Gly Thr His Ile Ile Tyr Glu Ser Gln Leu Gly Gly Lys Ile
           435                 440                 445
   Thr Lys Ile Ile Asn Val Ser Thr Ser Ser Ile Glu Gln Met Lys Lys
       450                 455                 460
   Asn Gly Val Ser Val Lys Ala Lys Ile Gln Ala Gln Phe Gly Phe Gly
   465                 470                 475                 480
   Ser Ala Gly Gly Ser Thr Asp Val Asn Ser Ser Asn Ser Ser Ala Asn
                   485                 490                 495
   Asp Glu Gln Ser Tyr Asp Met Asn Glu Gln Leu Ile Val Ile Gly Gly
               500                 505                 510
   Asn Pro Ile Lys Asp Val Thr Lys Glu Glu Asn Leu Phe Glu Trp Ser
           515                 520                 525
   Lys Thr Val Thr Asn His Pro Met Pro Ile Asn Ile Lys Leu Thr Pro
   530 535 540
   Ile Ser Asp Ser Phe Asp Ser Asp Asp Leu Lys Glu Ser Tyr Asp Lys
   545 550 555 560
   Ala Ile Ile Tyr Tyr Ser Arg Leu Tyr Gly Leu Ser Pro His Asp Thr
   565 570 575
   Met Gln Lys Asp Asp Lys Asp Ile Ile Lys Ile Leu Thr Asn Ala Asp
   580 585 590
   Thr Val Thr Lys Asn Ser Ala Pro Pro Ile Asn Ala Gln Cys Pro His
   595 600 605
   Gly Lys Val Val Met Phe Gly Phe Ser Leu Lys Gln Asn Phe Trp Asp
   610 615 620
   Asn Thr Asn Ala Leu Lys Gly Tyr Asn Ile Glu Val Cys Glu Ala Gly
   625 630 635 640
   Ser Asn Ser Cys Thr Ser Lys Gln Gly Ser Ser Asn Lys Tyr Asp Thr
   645 650 655
   Ser Tyr Leu Tyr Met Glu Cys Gly Asp Gln Pro Leu Pro Phe Ser Glu
   660 665 670
   Gln Val Ile Ser Glu Ser Thr Ser Thr Tyr Asn Thr Val Lys Cys Pro
   675 680 685
   Asn Asp Tyr Ser Ile Leu Leu Gly Phe Gly Ile Ser Ser Ser Ser Gly
   690 695 700
   Arg Ile Asn Ser Ala Glu Tyr Val Tyr Ser Thr Pro Cys Ile Pro Gly
   705 710 715 720
   Met Lys Ser Cys Ser Leu Asn Met Asn Asn Asp Asn Gln Lys Ser Tyr
   725 730 735
   Ile Tyr Val Leu Cys Val Asp Thr Thr Ile Trp Ser Gly Val Asn Asn
   740 745 750
   Leu Ser Leu Val Ala Leu Asp Gly Ala His Gly Lys Val Asn Arg Ser
   755 760 765
   Lys Lys Tyr Ser Asp Gly Glu Leu Val Gly Thr Cys Pro Leu Asp Gly
   770 775 780
   Thr Val Leu Thr Gly Phe Lys Val Glu Phe His Thr Ser Ser Pro Tyr
   785                 790                 795                 800
   Val Gln Thr Pro Phe Glu Lys Cys Ala Lys Ser Leu Lys Ala Cys Ser
                   805                 810                 815
   Val His Gly Ser Gly His Ala Ile Gly Ile Gln Asn Phe Lys Ser Leu
               820                 825                 830
   Phe Ile Tyr Met Leu Cys Lys Asn Asn Lys
           835                 840
<210> 54
   <211> 2541
   <212> DNA
   <213> Artificial sequence
<220>
   <223> Synthetic sequence
<220>
   <221> misc_feature
   <223> PvSPECT2 human CO
<400> 54
   gccaccatgg ctaagccaag aaatataaac tctctgttgg ccatatggtg tattctcttc 60
   tcaatctgtg agtacggcta tgtgggatct ttgagaatag gtcttcggag aaactatagt 120
   caaggggact cctctgatcg gctcgggggt agtgacgtgg acgtgagacg gtccggcgag 180
   cttatggacc tcggtaagaa cgcaaacatc ctctgcaata acatcagctg taacagtaaa 240
   aaagaggctt cttttctgag tcagaaaaaa caacttgagg atgacgatga cgacgagctg 300
   gccgcgctgt acaatgatga tgatgacgat gctagcacaa ccactggggg gggttcagaa 360
   actagcgatg acgacgagct tgaactccct gatcaggacg aaggtgcgga cgagggtgaa 420
   gcagaggacc aactcgcgac acaggaggat tccgacaact cagggaccga ccaaggtctg 480
   aaaaaaaaag taaaccttag ccgccacgaa aagttgatag aagacaaaaa acaacagaca 540
   gagaatactt ttaagaagta caggtttggg gatgaggagg aagagtcaga ggagaaatcc 600
   ccggggaaat ctaagtcact cgatccaagc agcctcgatg atgacgacgg cgaaggtgat 660
   gatgatgatg atggcgacga gagagaaaag aagcaaagta atacaaggaa agccatgaag 720
   aaggacttgg atgttttccc agggctttac tttgcaggca tcggatacga tagcctcttc 780
   ggaaaccccc tgggcgaggc tgacagcttg accgatcccg ggtaccgggg ccaaatcata 840
   ctcatgaact gggaactctc aaataaggga gttgcgaatg atcttgcgac attgcaacct 900
   ctgaatggat ggatccgcaa ggaaaatgcg tgcagtcgag cagaatctat aaaagagtgt 960
   tcttcagtat cagactacac gaaaaatttg accgcagagg catctgtatc tggctcctac 1020
   atgggctttg gagcttttag tgctagtact ggttataaga aatttttgca agaagccagt 1080
   aaacgcacgt ccaaaactta tctggtaaaa agcaattgcg tgaaatacac ggtcgggctc 1140
   cctccttatg tgcgctggga gcagacgacc gcctttaaga acgctgtgaa cgggttgcca 1200
   ccgcatttca ccggactgga ggccgactct gaatgcgcct ctgatgtcta cgaacaaaag 1260
   aagacatctg aagaatgcga aaccgtacat gcctggatac gcttttttaa gacttatgga 1320
   acacacgtga tcatggaagc acagcttggc ggcaaaataa caaagattat ccgggtcgag 1380
   aatagctccg tcaatcaaat gaagaaagac ggagtcagcg tgaaagccca gatcaaagcc 1440
   caattcggct ttgcatctgt ggggggaagc acaaacgtct ctagtgacca tagttcaaaa 1500
   aagaacgagg ataattatga gatgtctgag cagctggtag tgattggagg caacccaatc 1560
   aaggatgtga cgaaagaaga aaatctctat gagtggtcta aaacggtctc aacaaatccg 1620
   atgcctatca atatcaagct cctgccgatt agcactatct ttgactccga cgatctgaaa 1680
   aatagttacg aaaaggcatt gatctactac actcgcttgt atggcttttc accccatgat 1740
   acaatgcaaa aagatgaaaa ggatattgtt aagatcctga cggccagcac cacagtcaca 1800
   aagacaggcc cgccgcctat atctgcagag tgtccgcata atatggtggt cttgttcggt 1860
   ttcgtagtca aacagaattt ttgggatcat acgaacaaac tccagagtta tgaaatggag 1920
   atttgtgaaa gcggtgccag ctcatgcacg tctaagcagg gaaatacaaa taagtatgac 1980
   gtatcctata cgtatattga gtgcggacca caggcattgc cgttcactga gcaggttgta 2040
   tctgtatccg gtacaacata taactctgtt aaatgcccga atgactacag cgtgttgttt 2100
   ggctttggaa tggctacgag ttctggcagg caccaaagtg cgctttatag ctatttcaca 2160
   ccatgtcgac cagggctcaa aagctgtagc ttgaatatga acgaacatga tgataagtct 2220
   tacatttacc tggtctgcgt cgacgcgact atctggacgg gacttaacgc gttgagcatg 2280
   atcgcgaaag acgatttgca cagtgccgtg aaccggtacc aacaattcaa tgatggagag 2340
   ctggttgtga cgtgtcctag cgagggcact atactgactg gattctatgg ggagacccat 2400
   acctccagtc catatgttac tgtacctttt gggaaatgcg caaagtcatt gaaagcctgt 2460
   tctgttcatg ggtccggcca agccataggc attcacaatt atagaacttt gttcacagtc 2520
   gcactgtgta agaataatta g 2541
<210> 55
   <211> 844
   <212> PRT
   <213> Plasmodium vivax
<220>
   <221> misc_feature
   <223> PvSPECT2
<400> 55
   Met Ala Lys Pro Arg Asn Ile Asn Ser Leu Leu Ala Ile Trp Cys Ile
   1               5                   10                  15
   Leu Phe Ser Ile Cys Glu Tyr Gly Tyr Val Gly Ser Leu Arg Ile Gly
               20                  25                  30
   Leu Arg Arg Asn Tyr Ser Gln Gly Asp Ser Ser Asp Arg Leu Gly Gly
           35                  40                  45
   Ser Asp Val Asp Val Arg Arg Ser Gly Glu Leu Met Asp Leu Gly Lys
       50                  55                  60
   Asn Ala Asn Ile Leu Cys Asn Asn Ile Ser Cys Asn Ser Lys Lys Glu
   65                  70                  75                  80
   Ala Ser Phe Leu Ser Gln Lys Lys Gln Leu Glu Asp Asp Asp Asp Asp
                   85                  90                  95
   Glu Leu Ala Ala Leu Tyr Asn Asp Asp Asp Asp Asp Ala Ser Thr Thr
               100                 105                 110
   Thr Gly Gly Gly Ser Glu Thr Ser Asp Asp Asp Glu Leu Glu Leu Pro
           115                 120                 125
   Asp Gln Asp Glu Gly Ala Asp Glu Gly Glu Ala Glu Asp Gln Leu Ala
       130                 135                 140
   Thr Gln Glu Asp Ser Asp Asn Ser Gly Thr Asp Gln Gly Leu Lys Lys
   145                 150                 155                 160
   Lys Val Asn Leu Ser Arg His Glu Lys Leu Ile Glu Asp Lys Lys Gln
                   165                 170                 175
   Gln Thr Glu Asn Thr Phe Lys Lys Tyr Arg Phe Gly Asp Glu Glu Glu
               180                 185                 190
   Glu Ser Glu Glu Lys Ser Pro Gly Lys Ser Lys Ser Leu Asp Pro Ser
           195                 200                 205
   Ser Leu Asp Asp Asp Asp Gly Glu Gly Asp Asp Asp Asp Asp Gly Asp
       210                 215                 220
   Glu Arg Glu Lys Lys Gln Ser Asn Thr Arg Lys Ala Met Lys Lys Asp
   225                 230                 235                 240
   Leu Asp Val Phe Pro Gly Leu Tyr Phe Ala Gly Ile Gly Tyr Asp Ser
   245 250 255
   Leu Phe Gly Asn Pro Leu Gly Glu Ala Asp Ser Leu Thr Asp Pro Gly
   260 265 270
   Tyr Arg Gly Gln Ile Ile Leu Met Asn Trp Glu Leu Ser Asn Lys Gly
   275 280 285
   Val Ala Asn Asp Leu Ala Thr Leu Gln Pro Leu Asn Gly Trp Ile Arg
   290 295 300
   Lys Glu Asn Ala Cys Ser Arg Ala Glu Ser Ile Lys Glu Cys Ser Ser
   305 310 315 320
   Val Ser Asp Tyr Thr Lys Asn Leu Thr Ala Glu Ala Ser Val Ser Gly
   325 330 335
   Ser Tyr Met Gly Phe Gly Ala Phe Ser Ala Ser Thr Gly Tyr Lys Lys
   340 345 350
   Phe Leu Gln Glu Ala Ser Lys Arg Thr Ser Lys Thr Tyr Leu Val Lys
   355 360 365
   Ser Asn Cys Val Lys Tyr Thr Val Gly Leu Pro Pro Tyr Val Arg Trp
   370 375 380
   Glu Gln Thr Thr Ala Phe Lys Asn Ala Val Asn Gly Leu Pro Pro His
   385 390 395 400
   Phe Thr Gly Leu Glu Ala Asp Ser Glu Cys Ala Ser Asp Val Tyr Glu
   405 410 415
   Gln Lys Lys Thr Ser Glu Glu Cys Glu Thr Val His Ala Trp Ile Arg
   420 425 430
   Phe Phe Lys Thr Tyr Gly Thr His Val Ile Met Glu Ala Gln Leu Gly
   435 440 445
   Gly Lys Ile Thr Lys Ile Ile Arg Val Glu Asn Ser Ser Val Asn Gln
   450 455 460
   Met Lys Lys Asp Gly Val Ser Val Lys Ala Gln Ile Lys Ala Gln Phe
   465 470 475 480
   Gly Phe Ala Ser Val Gly Gly Ser Thr Asn Val Ser Ser Asp His Ser
   485 490 495
   Ser Lys Lys Asn Glu Asp Asn Tyr Glu Met Ser Glu Gln Leu Val Val
   500 505 510
   Ile Gly Gly Asn Pro Ile Lys Asp Val Thr Lys Glu Glu Asn Leu Tyr
   515 520 525
   Glu Trp Ser Lys Thr Val Ser Thr Asn Pro Met Pro Ile Asn Ile Lys
   530 535 540
   Leu Leu Pro Ile Ser Thr Ile Phe Asp Ser Asp Asp Leu Lys Asn Ser
   545 550 555 560
   Tyr Glu Lys Ala Leu Ile Tyr Tyr Thr Arg Leu Tyr Gly Phe Ser Pro
   565 570 575
   His Asp Thr Met Gln Lys Asp Glu Lys Asp Ile Val Lys Ile Leu Thr
   580 585 590
   Ala Ser Thr Thr Val Thr Lys Thr Gly Pro Pro Pro Ile Ser Ala Glu
   595 600 605
   Cys Pro His Asn Met Val Val Leu Phe Gly Phe Val Val Lys Gln Asn
   610 615 620
   Phe Trp Asp His Thr Asn Lys Leu Gln Ser Tyr Glu Met Glu Ile Cys
   625 630 635 640
   Glu Ser Gly Ala Ser Ser Cys Thr Ser Lys Gln Gly Asn Thr Asn Lys
   645 650 655
   Tyr Asp Val Ser Tyr Thr Tyr Ile Glu Cys Gly Pro Gln Ala Leu Pro
   660 665 670
   Phe Thr Glu Gln Val Val Ser Val Ser Gly Thr Thr Tyr Asn Ser Val
   675 680 685
   Lys Cys Pro Asn Asp Tyr Ser Val Leu Phe Gly Phe Gly Met Ala Thr
   690 695 700
   Ser Ser Gly Arg His Gln Ser Ala Leu Tyr Ser Tyr Phe Thr Pro Cys
   705 710 715 720
   Arg Pro Gly Leu Lys Ser Cys Ser Leu Asn Met Asn Glu His Asp Asp
   725 730 735
   Lys Ser Tyr Ile Tyr Leu Val Cys Val Asp Ala Thr Ile Trp Thr Gly
   740 745 750
   Leu Asn Ala Leu Ser Met Ile Ala Lys Asp Asp Leu His Ser Ala Val
   755 760 765
   Asn Arg Tyr Gln Gln Phe Asn Asp Gly Glu Leu Val Val Thr Cys Pro
   770 775 780
   Ser Glu Gly Thr Ile Leu Thr Gly Phe Tyr Gly Glu Thr His Thr Ser
   785 790 795 800
   Ser Pro Tyr Val Thr Val Pro Phe Gly Lys Cys Ala Lys Ser Leu Lys
   805 810 815
   Ala Cys Ser Val His Gly Ser Gly Gln Ala Ile Gly Ile His Asn Tyr
   820 825 830
   Arg Thr Leu Phe Thr Val Ala Leu Cys Lys Asn Asn
   835 840
<210> 56
   <211> 843
   <212> PRT
   <213> Plasmodium vivax
<220>
   <221> misc_feature
   <223> SPECT2
<400> 56
   Met Lys Pro Arg Asn Ile Asn Ser Leu Leu Ala Ile Trp Cys Ile Leu
   1 5 10 15
   Phe Ser Ile Cys Glu Tyr Gly Tyr Val Gly Ser Leu Arg Ile Gly Leu
   20 25 30
   Arg Arg Asn Tyr Ser Gln Gly Asp Ser Ser Asp Arg Leu Gly Gly Ser
   35 40 45
   Asp Val Asp Val Arg Arg Ser Gly Glu Leu Met Asp Phe Gly Lys Asn
   50 55 60
   Ala Asn Ile Leu Cys Asn Asn Ile Ser Cys Asn Ser Lys Lys Glu Ala
   65 70 75 80
   Ser Phe Leu Ser Gln Lys Lys Gln Leu Glu Asp Asp Asp Asp Asp Glu
   85 90 95
   Leu Ala Ala Leu Tyr Asn Asp Asp Asp Asp Asp Ala Ser Thr Thr Thr
               100                 105                 110
   Gly Gly Gly Ser Glu Thr Ser Asp Asp Asp Glu Leu Glu Leu Pro Asp
           115                 120                 125
   Gln Asp Glu Gly Ala Asp Glu Gly Glu Ala Glu Asp Gln Leu Ala Thr
       130                 135                 140
   Gln Glu Asp Ser Asp Asn Ser Gly Thr Asp Gln Gly Leu Lys Lys Lys
   145                 150                 155                 160
   Val Ser Leu Ser Arg His Glu Lys Leu Ile Glu Asp Lys Lys Gln Gln
                   165                 170                 175
   Thr Glu Asn Thr Phe Lys Lys Tyr Arg Phe Gly Asp Glu Glu Glu Glu
               180                 185                 190
   Ser Glu Glu Lys Ser Pro Gly Lys Ser Lys Ser Leu Asp Pro Ser Ser
           195                 200                 205
   Leu Asp Asp Asp Asp Gly Glu Gly Asp Asp Asp Asp Asp Gly Asp Glu
       210                 215                 220
   Arg Glu Lys Lys Gln Ser Asn Thr Arg Lys Ala Met Lys Lys Asp Leu
   225                 230                 235                 240
   Asp Val Phe Pro Gly Leu Tyr Phe Ala Gly Ile Gly Tyr Asp Ser Leu
                   245                 250                 255
   Phe Gly Asn Pro Leu Gly Glu Ala Asp Ser Leu Thr Asp Pro Gly Tyr
               260                 265                 270
   Arg Gly Gln Ile Ile Leu Met Asn Trp Glu Leu Ser Asn Lys Gly Val
           275                 280                 285
   Ala Asn Asp Leu Ala Thr Leu Gln Pro Leu Asn Gly Trp Ile Arg Lys
       290                 295                 300
   Glu Asn Ala Cys Ser Arg Ala Glu Ser Ile Lys Glu Cys Ser Ser Val
   305                 310                 315                 320
   Ser Asp Tyr Thr Lys Asn Leu Thr Ala Glu Ala Ser Val Ser Gly Ser
                   325                 330                 335
   Tyr Met Gly Phe Gly Ala Phe Ser Ala Ser Thr Gly Tyr Lys Lys Phe
               340                 345                 350
   Leu Gln Glu Ala Ser Lys Arg Thr Ser Lys Thr Tyr Leu Val Lys Ser
           355                 360                 365
   Asn Cys Val Lys Tyr Thr Val Gly Leu Pro Pro Tyr Val Arg Trp Glu
       370                 375                 380
   Gln Thr Thr Ala Phe Lys Asn Ala Val Asn Gly Leu Pro Pro His Phe
   385                 390                 395                 400
   Thr Gly Leu Glu Ala Asp Ser Glu Cys Ala Ser Asp Val Tyr Glu Gln
                   405                 410                 415
   Lys Lys Thr Ser Glu Glu Cys Glu Thr Val His Ala Trp Ile Arg Phe
               420                 425                 430
   Phe Lys Thr Tyr Gly Thr His Val Ile Met Glu Ala Gln Leu Gly Gly
           435                 440                 445
   Lys Ile Thr Lys Ile Ile Arg Val Glu Asn Ser Ser Val Asn Gln Met
       450                 455                 460
   Lys Lys Asp Gly Val Ser Val Lys Ala Gln Ile Lys Ala Gln Phe Gly
   465                 470                 475                 480
   Phe Ala Ser Val Gly Gly Ser Thr Asn Val Ser Ser Asp His Ser Ser
                   485                 490                 495
   Lys Lys Asn Glu Asp Asn Tyr Glu Met Ser Glu Gln Leu Val Val Ile
               500                 505                 510
   Gly Gly Asn Pro Ile Lys Asp Val Thr Lys Glu Glu Asn Leu Tyr Glu
           515                 520                 525
   Trp Ser Lys Thr Val Ser Thr Asn Pro Met Pro Ile Asn Ile Lys Leu
       530                 535                 540
   Leu Pro Ile Ser Thr Ile Phe Asp Ser Asp Asp Leu Lys Asn Ser Tyr
   545                 550                 555                 560
   Glu Lys Ala Leu Ile Tyr Tyr Thr Arg Leu Tyr Gly Phe Ser Pro His
                   565                 570                 575
   Asp Thr Met Gln Lys Asp Glu Lys Asp Ile Val Lys Ile Leu Thr Ala
               580                 585                 590
   Ser Thr Thr Val Thr Lys Thr Gly Pro Pro Pro Ile Ser Ala Glu Cys
           595                 600                 605
   Pro His Asn Met Val Val Leu Phe Gly Phe Val Val Lys Gln Asn Phe
   610 615 620
   Trp Asp His Thr Asn Lys Leu Gln Ser Tyr Glu Met Glu Ile Cys Glu
   625 630 635 640
   Ser Gly Ala Ser Ser Cys Thr Ser Lys Gln Gly Asn Thr Asn Lys Tyr
   645 650 655
   Asp Val Ser Tyr Thr Tyr Ile Glu Cys Gly Pro Gln Ala Leu Pro Phe
   660 665 670
   Thr Glu Gln Val Val Ser Val Ser Gly Thr Thr Tyr Asn Ser Val Lys
   675 680 685
   Cys Pro Asn Asp Tyr Ser Val Leu Phe Gly Phe Gly Met Ala Thr Ser
   690 695 700
   Ser Gly Arg His Gln Ser Ala Leu Tyr Ser Tyr Phe Thr Pro Cys Arg
   705 710 715 720
   Pro Gly Leu Lys Ser Cys Ser Leu Asn Met Asn Glu His Asp Asp Lys
   725 730 735
   Ser Tyr Ile Tyr Leu Val Cys Val Asp Ala Thr Ile Trp Thr Gly Leu
   740 745 750
   Asn Ala Leu Ser Met Ile Ala Lys Asp Asp Leu His Ser Ala Val Asn
   755 760 765
   Arg Tyr Gln Gln Phe Asn Asp Gly Glu Leu Val Val Thr Cys Pro Ser
   770 775 780
   Glu Gly Thr Ile Leu Thr Gly Phe Tyr Gly Glu Thr His Thr Ser Ser
   785 790 795 800
   Pro Tyr Val Thr Val Pro Phe Gly Lys Cys Ala Lys Ser Leu Lys Ala
   805 810 815
   Cys Ser Val His Gly Ser Gly Gln Ala Ile Gly Ile His Asn Tyr Arg
   820 825 830
   Thr Leu Phe Thr Val Ala Leu Cys Lys Asn Asn
   835 840
<210> 57
   <211> 2478
   <212> DNA
   <213> Plasmodium berghei
<220>
   <221> misc_feature
   <223> GPI_P113
<400> 57
   ggatccgcca ccatggctaa gatcttcctg tttagtttct tcttcgtatg gttccagtat      60
   tgctattcta agaacccctc agactacgct catagcattg tctctaattt tgagtccgag     120
   aatacgttga agtgtttgaa aggcaacgtg tatatacttc aatgtcagat caagtgcatg     180
   aactcaaaca acgagatcat atacaaggag tgtctcaacg atatcgaaaa gatttgcaaa     240
   gataagaaaa catgcagtta ttacttcgac tacatcttca aaaccaagaa tcacaagctt     300
   agaaacaaca acaataacaa catctacatc gacaattgca tcgattccga caagaatgag     360
   atcaaatcta ccttcacctg tgtcctcaat ccattgctgg aatttgacaa taacaaccac     420
   gtcatttata acttcctgct gaacaacaag aacaatgaca agatcgtctg taaaaatagc     480
   aacatatata ttaacaacgc tactattcac tataccttct ccgacattaa gtttaaagac     540
   gtgactagct acataaaaga gaaatgtaac gagaaaacta attgcgtgat aaacccctat     600
   tctatccaaa ccgatatcct gaatgagaag aacgacgcat atctgctgaa ctcatacatc     660
   agcatatcct tcgcatgtgt gaaaattaac ctggaaagct atctgtacgg aggtgatatc     720
   gatgaatttg atcagataaa cgatgaagaa aatgaggaca ataagtacct ggatcacaat     780
   gatctcgatg aaaaaaatga ggaaattatt tccctgaaga acgaaatcaa cgacatcctg     840
   aatgacgaga agatcgataa catcgccgag aagctgaaga ttgcgaaatt tacaataagc     900
   aagaaaatca acgaggagat caagaagaag aacgacatct ttaacaacct ggccaacgac     960
   atctaccagt tcatcggtaa cgagtactac tttacttccg acatcaaaga catgattgaa    1020
   gataggtaca atgaactgaa caaaacatct cagtctgact tgtattacat ttaccttctg    1080
   aatgtgtttg acattgaaaa gatttacggg atctacctgt ctagttatca ggagcgactg    1140
   cagcagatcc tgcaaaccaa catgacaaat ctggactatg ttgagaagaa gattggaagc    1200
   ttgcggaaca tttacatgtt cctgtataaa aagagtaaga agtataacgc gctggatatc    1260
   ttcgacgaat actacgatta tgtgctcaat tacaatgact tcgctaaaga caatgagata    1320
   atcagcgccg acattttcat taagtcaaaa cctgatatcc ctcaactcaa tttcgagatc    1380
   aacaatgaga acaagaacgt gaagtataaa gacgttaccg atcttgacga gctggataac    1440
   ctcaacagga taaatagaat tatcaatatc cggaatgtgc tggtcaagca gctcaagatc    1500
   ctttacaacc agagaaataa catatttatc aaacaggcca tgctggtgaa atcatactgc    1560
   tacaaaaatc cactggatat tacggatttc agcagcatct tcaagaataa ctacaataag    1620
   ttgaaatacg acgcctataa ggagggcaat ggacacatta acgtggccga caaaatcaat 1680
   ccaaactttg tcgtgaaata cctgaacaat ctttataaac agcatgttaa caagaattat 1740
   atcctgaaca gctgggaccc taaatacaat cgcatgaata agaagattaa gatcattctc 1800
   attttggggt atggccaggt aatccagatc gagaaacaga ttaaccgtca tataggcaag 1860
   tacaacgccc tgcttgaaaa ggcaaccctc tataacgtgg gaaatctctt tacacagact 1920
   acgaatattt tgaacgacat ctcaggctca ctgaacgatg ggcttgaccc gaacatccat 1980
   gatcaggagg atgtcactgt cgttgaatcc tctgaaagta acaagctcgc agaacctgag 2040
   gaacccattg agaaggtaga ggttgatagg gtagagaaat ccgatgatgc caataatgcc 2100
   actcaacaag tgacaggaac agacgaggcc aattacgata cagctagcgg gaatagtacc 2160
   aacatcaaca ttgacaacat agactatggt gtggacgaat ctatccgaat tattaagtac 2220
   tccaaggctg aggaggatga atataatgag agcggcaata acgaaaatga aaacaacgag 2280
   aataacgaga acgagaacaa tgaaaatgag aacaacgaga atgagaataa tgagaatgaa 2340
   aataacgaga acgaaaatat agaactgaag aatatagaac acgaaaacaa atccaacgca 2400
   tctagcgctt ccctcagtaa cattttcttt acctttatca ttgcagctct gtttatccgc 2460
   ccctttctgt gactcgag 2478
<210> 58
   <211> 819
   <212> PRT
   <213> Plasmodium berghei
<220>
   <221> misc_feature
   <223> GPI_P113
<400> 58
   Met Ala Lys Ile Phe Leu Phe Ser Phe Phe Phe Val Trp Phe Gln Tyr
   1               5                   10                  15
   Cys Tyr Ser Lys Asn Pro Ser Asp Tyr Ala His Ser Ile Val Ser Asn
               20                  25                  30
   Phe Glu Ser Glu Asn Thr Leu Lys Cys Leu Lys Gly Asn Val Tyr Ile
           35                  40                  45
   Leu Gln Cys Gln Ile Lys Cys Met Asn Ser Asn Asn Glu Ile Ile Tyr
       50                  55                  60
   Lys Glu Cys Leu Asn Asp Ile Glu Lys Ile Cys Lys Asp Lys Lys Thr
   65                  70                  75                  80
   Cys Ser Tyr Tyr Phe Asp Tyr Ile Phe Lys Thr Lys Asn His Lys Leu
                   85                  90                  95
   Arg Asn Asn Asn Asn Asn Asn Ile Tyr Ile Asp Asn Cys Ile Asp Ser
               100                 105                 110
   Asp Lys Asn Glu Ile Lys Ser Thr Phe Thr Cys Val Leu Asn Pro Leu
           115                 120                 125
   Leu Glu Phe Asp Asn Asn Asn His Val Ile Tyr Asn Phe Leu Leu Asn
       130                 135                 140
   Asn Lys Asn Asn Asp Lys Ile Val Cys Lys Asn Ser Asn Ile Tyr Ile
   145                 150                 155                 160
   Asn Asn Ala Thr Ile His Tyr Thr Phe Ser Asp Ile Lys Phe Lys Asp
                   165                 170                 175
   Val Thr Ser Tyr Ile Lys Glu Lys Cys Asn Glu Lys Thr Asn Cys Val
               180                 185                 190
   Ile Asn Pro Tyr Ser Ile Gln Thr Asp Ile Leu Asn Glu Lys Asn Asp
           195                 200                 205
   Ala Tyr Leu Leu Asn Ser Tyr Ile Ser Ile Ser Phe Ala Cys Val Lys
       210                 215                 220
   Ile Asn Leu Glu Ser Tyr Leu Tyr Gly Gly Asp Ile Asp Glu Phe Asp
   225                 230                 235                 240
   Gln Ile Asn Asp Glu Glu Asn Glu Asp Asn Lys Tyr Leu Asp His Asn
                   245                 250                 255
   Asp Leu Asp Glu Lys Asn Glu Glu Ile Ile Ser Leu Lys Asn Glu Ile
               260                 265                 270
   Asn Asp Ile Leu Asn Asp Glu Lys Ile Asp Asn Ile Ala Glu Lys Leu
           275                 280                 285
   Lys Ile Ala Lys Phe Thr Ile Ser Lys Lys Ile Asn Glu Glu Ile Lys
       290                 295                 300
   Lys Lys Asn Asp Ile Phe Asn Asn Leu Ala Asn Asp Ile Tyr Gln Phe
   305                 310                 315                 320
   Ile Gly Asn Glu Tyr Tyr Phe Thr Ser Asp Ile Lys Asp Met Ile Glu
                   325                 330                 335
   Asp Arg Tyr Asn Glu Leu Asn Lys Thr Ser Gln Ser Asp Leu Tyr Tyr
               340                 345                 350
   Ile Tyr Leu Leu Asn Val Phe Asp Ile Glu Lys Ile Tyr Gly Ile Tyr
           355                 360                 365
   Leu Ser Ser Tyr Gln Glu Arg Leu Gln Gln Ile Leu Gln Thr Asn Met
       370                 375                 380
   Thr Asn Leu Asp Tyr Val Glu Lys Lys Ile Gly Ser Leu Arg Asn Ile
   385                 390                 395                 400
   Tyr Met Phe Leu Tyr Lys Lys Ser Lys Lys Tyr Asn Ala Leu Asp Ile
                   405                 410                 415
   Phe Asp Glu Tyr Tyr Asp Tyr Val Leu Asn Tyr Asn Asp Phe Ala Lys
               420                 425                 430
   Asp Asn Glu Ile Ile Ser Ala Asp Ile Phe Ile Lys Ser Lys Pro Asp
           435                 440                 445
   Ile Pro Gln Leu Asn Phe Glu Ile Asn Asn Glu Asn Lys Asn Val Lys
       450                 455                 460
   Tyr Lys Asp Val Thr Asp Leu Asp Glu Leu Asp Asn Leu Asn Arg Ile
   465                 470                 475                 480
   Asn Arg Ile Ile Asn Ile Arg Asn Val Leu Val Lys Gln Leu Lys Ile
                   485                 490                 495
   Leu Tyr Asn Gln Arg Asn Asn Ile Phe Ile Lys Gln Ala Met Leu Val
               500                 505                 510
   Lys Ser Tyr Cys Tyr Lys Asn Pro Leu Asp Ile Thr Asp Phe Ser Ser
           515                 520                 525
   Ile Phe Lys Asn Asn Tyr Asn Lys Leu Lys Tyr Asp Ala Tyr Lys Glu
       530                 535                 540
   Gly Asn Gly His Ile Asn Val Ala Asp Lys Ile Asn Pro Asn Phe Val
   545                 550                 555                 560
   Val Lys Tyr Leu Asn Asn Leu Tyr Lys Gln His Val Asn Lys Asn Tyr
                   565                 570                 575
   Ile Leu Asn Ser Trp Asp Pro Lys Tyr Asn Arg Met Asn Lys Lys Ile
               580                 585                 590
   Lys Ile Ile Leu Ile Leu Gly Tyr Gly Gln Val Ile Gln Ile Glu Lys
             595                 600                 605
   Gln Ile Asn Arg His Ile Gly Lys Tyr Asn Ala Leu Leu Glu Lys Ala
       610                 615                 620
   Thr Leu Tyr Asn Val Gly Asn Leu Phe Thr Gln Thr Thr Asn Ile Leu
   625                 630                 635                 640
   Asn Asp Ile Ser Gly Ser Leu Asn Asp Gly Leu Asp Pro Asn Ile His
                   645                 650                 655
   Asp Gln Glu Asp Val Thr Val Val Glu Ser Ser Glu Ser Asn Lys Leu
               660                 665                 670
   Ala Glu Pro Glu Glu Pro Ile Glu Lys Val Glu Val Asp Arg Val Glu
           675                 680                 685
   Lys Ser Asp Asp Ala Asn Asn Ala Thr Gln Gln Val Thr Gly Thr Asp
       690                 695                 700
   Glu Ala Asn Tyr Asp Thr Ala Ser Gly Asn Ser Thr Asn Ile Asn Ile
   705                 710                 715                 720
   Asp Asn Ile Asp Tyr Gly Val Asp Glu Ser Ile Arg Ile Ile Lys Tyr
                   725                 730                 735
   Ser Lys Ala Glu Glu Asp Glu Tyr Asn Glu Ser Gly Asn Asn Glu Asn
               740                 745                 750
   Glu Asn Asn Glu Asn Asn Glu Asn Glu Asn Asn Glu Asn Glu Asn Asn
           755                 760                 765
   Glu Asn Glu Asn Asn Glu Asn Glu Asn Asn Glu Asn Glu Asn Ile Glu
       770                 775                 780
   Leu Lys Asn Ile Glu His Glu Asn Lys Ser Asn Ala Ser Ser Ala Ser
   785                 790                 795                 800
   Leu Ser Asn Ile Phe Phe Thr Phe Ile Ile Ala Ala Leu Phe Ile Arg
                   805                 810                 815
   Pro Phe Leu
<210> 59
   <211> 818
   <212> PRT
   <213> Plasmodium berghei
<220>
   <221> misc_feature
   <223> P113
<400> 59
   Met Lys Ile Phe Leu Phe Ser Phe Phe Phe Val Trp Phe Gln Tyr Cys
   1               5                   10                  15
   Tyr Ser Lys Asn Pro Ser Asp Tyr Ala His Ser Ile Val Ser Asn Phe
               20                  25                  30
   Glu Ser Glu Asn Thr Leu Lys Cys Leu Lys Gly Asn Val Tyr Ile Leu
           35                  40                  45
   Gln Cys Gln Ile Lys Cys Met Asn Ser Asn Asn Glu Ile Ile Tyr Lys
       50                  55                  60
   Glu Cys Leu Asn Asp Ile Glu Lys Ile Cys Lys Asp Lys Lys Thr Cys
   65                  70                  75                  80
   Ser Tyr Tyr Phe Asp Tyr Ile Phe Lys Thr Lys Asn His Lys Leu Arg
                   85                  90                  95
   Asn Asn Asn Asn Asn Asn Ile Tyr Ile Asp Asn Cys Ile Asp Ser Asp
               100                 105                 110
   Lys Asn Glu Ile Lys Ser Thr Phe Thr Cys Val Leu Asn Pro Leu Leu
           115                 120                 125
   Glu Phe Asp Asn Asn Asn His Val Ile Tyr Asn Phe Leu Leu Asn Asn
       130                 135                 140
   Lys Asn Asn Asp Lys Ile Val Cys Lys Asn Ser Asn Ile Tyr Ile Asn
   145                 150                 155                 160
   Asn Ala Thr Ile His Tyr Thr Phe Ser Asp Ile Lys Phe Lys Asp Val
                   165                 170                 175
   Thr Ser Tyr Ile Lys Glu Lys Cys Asn Glu Lys Thr Asn Cys Val Ile
               180                 185                 190
   Asn Pro Tyr Ser Ile Gln Thr Asp Ile Leu Asn Glu Lys Asn Asp Ala
           195                 200                 205
   Tyr Leu Leu Asn Ser Tyr Ile Ser Ile Ser Phe Ala Cys Val Lys Ile
       210                 215                 220
   Asn Leu Glu Ser Tyr Leu Tyr Gly Gly Asp Ile Asp Glu Phe Asp Gln
   225                 230                 235                 240
   Ile Asn Asp Glu Glu Asn Glu Asp Asn Lys Tyr Leu Asp His Asn Asp
                   245                 250                 255
   Leu Asp Glu Lys Asn Glu Glu Ile Ile Ser Leu Lys Asn Glu Ile Asn
               260                 265                 270
   Asp Ile Leu Asn Asp Glu Lys Ile Asp Asn Ile Ala Glu Lys Leu Lys
           275                 280                 285
   Ile Ala Lys Phe Thr Ile Ser Lys Lys Ile Asn Glu Glu Ile Lys Lys
       290                 295                 300
   Lys Asn Asp Ile Phe Asn Asn Leu Ala Asn Asp Ile Tyr Gln Phe Ile
   305                 310                 315                 320
   Gly Asn Glu Tyr Tyr Phe Thr Ser Asp Ile Lys Asp Met Ile Glu Asp
                   325                 330                 335
   Arg Tyr Asn Glu Leu Asn Lys Thr Ser Gln Ser Asp Leu Tyr Tyr Ile
               340                 345                 350
   Tyr Leu Leu Asn Val Phe Asp Ile Glu Lys Ile Tyr Gly Ile Tyr Leu
           355                 360                 365
   Ser Ser Tyr Gln Glu Arg Leu Gln Gln Ile Leu Gln Thr Asn Met Thr
       370                 375                 380
   Asn Leu Asp Tyr Val Glu Lys Lys Ile Gly Ser Leu Arg Asn Ile Tyr
   385                 390                 395                 400
   Met Phe Leu Tyr Lys Lys Ser Lys Lys Tyr Asn Ala Leu Asp Ile Phe
                   405                 410                 415
   Asp Glu Tyr Tyr Asp Tyr Val Leu Asn Tyr Asn Asp Phe Ala Lys Asp
               420                 425                 430
   Asn Glu Ile Ile Ser Ala Asp Ile Phe Ile Lys Ser Lys Pro Asp Ile
           435                 440                 445
   Pro Gln Leu Asn Phe Glu Ile Asn Asn Glu Asn Lys Asn Val Lys Tyr
       450                 455                 460
   Lys Asp Val Thr Asp Leu Asp Glu Leu Asp Asn Leu Asn Arg Ile Asn
   465                 470                 475                 480
   Arg Ile Ile Asn Ile Arg Asn Val Leu Val Lys Gln Leu Lys Ile Leu
                   485                 490                 495
   Tyr Asn Gln Arg Asn Asn Ile Phe Ile Lys Gln Ala Met Leu Val Lys
               500                 505                 510
   Ser Tyr Cys Tyr Lys Asn Pro Leu Asp Ile Thr Asp Phe Ser Ser Ile
           515                 520                 525
   Phe Lys Asn Asn Tyr Asn Lys Leu Lys Tyr Asp Ala Tyr Lys Glu Gly
       530                 535                 540
   Asn Gly His Ile Asn Val Ala Asp Lys Ile Asn Pro Asn Phe Val Val
   545                 550                 555                 560
   Lys Tyr Leu Asn Asn Leu Tyr Lys Gln His Val Asn Lys Asn Tyr Ile
                   565                 570                 575
   Leu Asn Ser Trp Asp Pro Lys Tyr Asn Arg Met Asn Lys Lys Ile Lys
               580                 585                 590
   Ile Ile Leu Ile Leu Gly Tyr Gly Gln Val Ile Gln Ile Glu Lys Gln
           595                 600                 605
   Ile Asn Arg His Ile Gly Lys Tyr Asn Ala Leu Leu Glu Lys Ala Thr
       610                 615                 620
   Leu Tyr Asn Val Gly Asn Leu Phe Thr Gln Thr Thr Asn Ile Leu Asn
   625                 630                 635                 640
   Asp Ile Ser Gly Ser Leu Asn Asp Gly Leu Asp Pro Asn Ile His Asp
                   645                 650                 655
   Gln Glu Asp Val Thr Val Val Glu Ser Ser Glu Ser Asn Lys Leu Ala
               660                 665                 670
   Glu Pro Glu Glu Pro Ile Glu Lys Val Glu Val Asp Arg Val Glu Lys
           675                 680                 685
   Ser Asp Asp Ala Asn Asn Ala Thr Gln Gln Val Thr Gly Thr Asp Glu
       690                 695                 700
   Ala Asn Tyr Asp Thr Ala Ser Gly Asn Ser Thr Asn Ile Asn Ile Asp
   705                 710                 715                 720
   Asn Ile Asp Tyr Gly Val Asp Glu Ser Ile Arg Ile Ile Lys Tyr Ser
                   725                 730                 735
   Lys Ala Glu Glu Asp Glu Tyr Asn Glu Ser Gly Asn Asn Glu Asn Glu
               740                 745                 750
   Asn Asn Glu Asn Asn Glu Asn Glu Asn Asn Glu Asn Glu Asn Asn Glu
           755                 760                 765
   Asn Glu Asn Asn Glu Asn Glu Asn Asn Glu Asn Glu Asn Ile Glu Leu
       770                 775                 780
   Lys Asn Ile Glu His Glu Asn Lys Ser Asn Ala Ser Ser Ala Ser Leu
   785                 790                 795                 800
   Ser Asn Ile Phe Phe Thr Phe Ile Ile Ala Ala Leu Phe Ile Arg Pro
                   805                 810                 815
   Phe Leu
<210> 60
   <211> 2919
   <212> DNA
   <213> Artificial Sequence
<220>
   <223> PfP113 human CO
<400> 60
   gccaccatgg ctaagatccc gttctttatt ctgcacatcc tcctcctgca gtttctgctt      60
   tgtctgatac gctgttatgt gcacaatgat gtgattaagt tcggtgagga gaatagcctg     120
   aagtgctctc agggaaactt gtatgtgttg cactgtgagg tgcagtgcct taatggcaat     180
   aatgagatta tccacaagag gtgtaatgac gacattgaga aaaagtgcaa cggcaataat     240
   aaatgcatat acttctttga gtacgaactg cggaagaaaa cacaaagctt tcgaaataag     300
   aattctatcg agatttccga gtgtgtcgaa agcgagcaga acgaagtgaa aacctcaact     360
   acctgtctcc tgagcaattc ctttattctt gacgaggcct ttatacagta tttcttcttc     420
   ataaagaaca agaacgaaga gcctgtgatt tgtaaggatg ggaatatcaa cattaagagt     480
   gcactcctgc actctccgtt ctgtgaaatc aaactcaagg acatttccga atatatacgc     540
   aaaaagtgtg acaacaacaa ggaatgcctt atagatcctc tcgatgttca gaagaatttg     600
   ctgaacgaag aagatccctg ctacatcaat aacagttacg tatctgtgaa tgtggtctgc     660
   aacaaagagg aggagatagg ggatgagagc actgacagct catcaatgga gatccaggac     720
   tcaacatcaa atgagcaaga cgagaatgtt aaaggaatgt caagcagcca agagatgaac 780
   tcaaacaacg atgaaaacaa aaaccaagac aacgaaagcg acgatgacgt caataataat 840
   aacaacaata acaatgatga ccaggacgag caaggaaacg atggcgatgt caccagctct 900
   atgaacaaga atgaggacaa caaggatttg gagcatggtt cctccaatga tgtcaataac 960
   aacactgaca ccttggttaa caacaaagag aataaggagt tcgtccttaa agagaagtct 1020
   agccttactt ctaaaattaa caaagagctg gctcatagaa ctgccctgtt taacaaactt 1080
   gcagacaaca tatcacttct gcttaacaag aaatacgatt ccttcgaaat taaggatgtg 1140
   ctggaagata ggtacaacga gatgaagagg gacgcaaacc ccgatgtcta ctacatatac 1200
   ctgatggata ctctggatat tgaaaagatc gaagatatca acctggaaga ggttaagatg 1260
   tctctgctgg catcactgaa agaaacgatg aacaaaattg atacgatcga aaagaaaatc 1320
   gaagaattta agaacaagta catctccttg tataacaagg tgaaaaccac aatgcccgaa 1380
   ctctttgacc tgaatgagga tcttgtactg ctctacaacg attttccctt tgacaacggc 1440
   atgatcagct ccgacatctt ctttaagtac aatccttccg agaacatcat ggatcatcag 1500
   gaaatggtga agaaagggag tatcaccgaa gatgaactca ggattgttaa cgatcttgag 1560
   ccactggata actatagacg tcgtaaacga attacagagc tgagaaagat tctggtggag 1620
   aagctgcgga ttctgtacct ggagaagaac aatctcttca atacacaggc gagttgcatc 1680
   aagagctatt gctataagaa tcctctgaac ctcaaaacct tggaagtgct cttgaagaag 1740
   aactactata gactgaagga gaataaagat tacgatgttg tatccagcat tatccagcat 1800
   ctcgacaatg tagacgccaa caagaagaag aaatggctga cccatgaacg gatactgaag 1860
   aagctccaag ttctgattgc cgaaggctat aagcggatca acgagaaaga aaaggacatc 1920
   gaccgaagaa tggctgtcta caatgccctc tatgagaaag cacagtctta caacctgcag 1980
   aagcttttca acgactccaa cgattttctg aagaaatatg ccataatggg aaacagtttc 2040
   gacgacggcg atgaggtttt cggttcccaa agctcaaact ttaacatctt cgatagcaat 2100
   aacaccgacc agaacaatga gcaagagcag ccaaagcaag atgaccagct tttgaacaat 2160
   aataacgatg acgtgctctc agagtcaaac aatgagaata aagagaaaac aagtgatgac 2220
   gctactcata aggagactca ggagaaaagc gaccaggaac cttcccagaa cattcaggag 2280
   gacaactccg atgagaaaca tgccgaaaac gaggagaacg tagaacagat cgaaactgat 2340
   agtaatgtca gcgaagaagc caatgacgag aataaggata acatgcagac aaccactgac 2400
   gaaggaaccg aagaacttca gcagaatgac gaagatgcgg agagtctgac caaggagaat 2460
   tccaaatctg aggagcagga gaatgaagat tctactgacg ccgaggcgat tgataaagag 2520
   gaagttgaaa cggaagagaa gggaaaggac gaacagaaga aagatgagca gaaggagcag 2580
   gatgaggaag aggatggaga gaaagaaaat aagcacaaga gctccgaaac taccaatgag 2640
   actgtgaccg acatcgagga aaataagaac gaggtcaaag gtgaggagca tcttcagggg 2700
   tctgagcaga gcattgaggc ttccgaatca tcccagaaag atgagactaa agaaacagag 2760
   gacaaggagg aatacgtgaa cgctaatgat gacgaatctt ctgaggagga cacgacgcct 2820
   aacgagacaa ataaaaccga caacgggtca agtttcttct tcgcgatgag caatgcactt 2880
   ctcgtgatct tgctgttgct tttcatcgaa ttcctgtaa 2919
<210> 61
   <211> 970
   <212> PRT
   <213> Plasmodium falciparum
<220>
   <221> misc_feature
   <223> PfP113
<400> 61
   Met Ala Lys Ile Pro Phe Phe Ile Leu His Ile Leu Leu Leu Gln Phe
   1 5 10 15
   Leu Leu Cys Leu Ile Arg Cys Tyr Val His Asn Asp Val Ile Lys Phe
   20 25 30
   Gly Glu Glu Asn Ser Leu Lys Cys Ser Gln Gly Asn Leu Tyr Val Leu
   35 40 45
   His Cys Glu Val Gln Cys Leu Asn Gly Asn Asn Glu Ile Ile His Lys
   50 55 60
   Arg Cys Asn Asp Asp Ile Glu Lys Lys Cys Asn Gly Asn Asn Lys Cys
   65 70 75 80
   Ile Tyr Phe Phe Glu Tyr Glu Leu Arg Lys Lys Thr Gln Ser Phe Arg
   85 90 95
   Asn Lys Asn Ser Ile Glu Ile Ser Glu Cys Val Glu Ser Glu Gln Asn
   100 105 110
   Glu Val Lys Thr Ser Thr Thr Cys Leu Leu Ser Asn Ser Phe Ile Leu
   115 120 125
   Asp Glu Ala Phe Ile Gln Tyr Phe Phe Phe Ile Lys Asn Lys Asn Glu
   130 135 140
   Glu Pro Val Ile Cys Lys Asp Gly Asn Ile Asn Ile Lys Ser Ala Leu
   145 150 155 160
   Leu His Ser Pro Phe Cys Glu Ile Lys Leu Lys Asp Ile Ser Glu Tyr
                   165                 170                 175
   Ile Arg Lys Lys Cys Asp Asn Asn Lys Glu Cys Leu Ile Asp Pro Leu
               180                 185                 190
   Asp Val Gln Lys Asn Leu Leu Asn Glu Glu Asp Pro Cys Tyr Ile Asn
           195                 200                 205
   Asn Ser Tyr Val Ser Val Asn Val Val Cys Asn Lys Glu Glu Glu Ile
       210                 215                 220
   Gly Asp Glu Ser Thr Asp Ser Ser Ser Met Glu Ile Gln Asp Ser Thr
   225                 230                 235                 240
   Ser Asn Glu Gln Asp Glu Asn Val Lys Gly Met Ser Ser Ser Gln Glu
                   245                 250                 255
   Met Asn Ser Asn Asn Asp Glu Asn Lys Asn Gln Asp Asn Glu Ser Asp
               260                 265                 270
   Asp Asp Val Asn Asn Asn Asn Asn Asn Asn Asn Asp Asp Gln Asp Glu
           275                 280                 285
   Gln Gly Asn Asp Gly Asp Val Thr Ser Ser Met Asn Lys Asn Glu Asp
       290                 295                 300
   Asn Lys Asp Leu Glu His Gly Ser Ser Asn Asp Val Asn Asn Asn Thr
   305                 310                 315                 320
   Asp Thr Leu Val Asn Asn Lys Glu Asn Lys Glu Phe Val Leu Lys Glu
                   325                 330                 335
   Lys Ser Ser Leu Thr Ser Lys Ile Asn Lys Glu Leu Ala His Arg Thr
               340                 345                 350
   Ala Leu Phe Asn Lys Leu Ala Asp Asn Ile Ser Leu Leu Leu Asn Lys
           355                 360                 365
   Lys Tyr Asp Ser Phe Glu Ile Lys Asp Val Leu Glu Asp Arg Tyr Asn
       370                 375                 380
   Glu Met Lys Arg Asp Ala Asn Pro Asp Val Tyr Tyr Ile Tyr Leu Met
   385                 390                 395                 400
   Asp Thr Leu Asp Ile Glu Lys Ile Glu Asp Ile Asn Leu Glu Glu Val
                   405                 410                 415
   Lys Met Ser Leu Leu Ala Ser Leu Lys Glu Thr Met Asn Lys Ile Asp
   420 425 430
   Thr Ile Glu Lys Lys Ile Glu Glu Phe Lys Asn Lys Tyr Ile Ser Leu
   435 440 445
   Tyr Asn Lys Val Lys Thr Thr Met Pro Glu Leu Phe Asp Leu Asn Glu
   450 455 460
   Asp Leu Val Leu Leu Tyr Asn Asp Phe Pro Phe Asp Asn Gly Met Ile
   465 470 475 480
   Ser Ser Asp Ile Phe Phe Lys Tyr Asn Pro Ser Glu Asn Ile Met Asp
   485 490 495
   His Gln Glu Met Val Lys Lys Gly Ser Ile Thr Glu Asp Glu Leu Arg
   500 505 510
   Ile Val Asn Asp Leu Glu Pro Leu Asp Asn Tyr Arg Arg Arg Lys Arg
   515 520 525
   Ile Thr Glu Leu Arg Lys Ile Leu Val Glu Lys Leu Arg Ile Leu Tyr
   530 535 540
   Leu Glu Lys Asn Asn Leu Phe Asn Thr Gln Ala Ser Cys Ile Lys Ser
   545 550 555 560
   Tyr Cys Tyr Lys Asn Pro Leu Asn Leu Lys Thr Leu Glu Val Leu Leu
   565 570 575
   Lys Lys Asn Tyr Tyr Arg Leu Lys Glu Asn Lys Asp Tyr Asp Val Val
   580 585 590
   Ser Ser Ile Ile Gln His Leu Asp Asn Val Asp Ala Asn Lys Lys Lys
   595 600 605
   Lys Trp Leu Thr His Glu Arg Ile Leu Lys Lys Leu Gln Val Leu Ile
   610 615 620
   Ala Glu Gly Tyr Lys Arg Ile Asn Glu Lys Glu Lys Asp Ile Asp Arg
   625 630 635 640
   Arg Met Ala Val Tyr Asn Ala Leu Tyr Glu Lys Ala Gln Ser Tyr Asn
   645 650 655
   Leu Gln Lys Leu Phe Asn Asp Ser Asn Asp Phe Leu Lys Lys Tyr Ala
   660 665 670
   Ile Met Gly Asn Ser Phe Asp Asp Gly Asp Glu Val Phe Gly Ser Gln
   675 680 685
   Ser Ser Asn Phe Asn Ile Phe Asp Ser Asn Asn Thr Asp Gln Asn Asn
   690 695 700
   Glu Gln Glu Gln Pro Lys Gln Asp Asp Gln Leu Leu Asn Asn Asn Asn
   705 710 715 720
   Asp Asp Val Leu Ser Glu Ser Asn Asn Glu Asn Lys Glu Lys Thr Ser
   725 730 735
   Asp Asp Ala Thr His Lys Glu Thr Gln Glu Lys Ser Asp Gln Glu Pro
   740 745 750
   Ser Gln Asn Ile Gln Glu Asp Asn Ser Asp Glu Lys His Ala Glu Asn
   755 760 765
   Glu Glu Asn Val Glu Gln Ile Glu Thr Asp Ser Asn Val Ser Glu Glu
   770 775 780
   Ala Asn Asp Glu Asn Lys Asp Asn Met Gln Thr Thr Thr Asp Glu Gly
   785 790 795 800
   Thr Glu Glu Leu Gln Gln Asn Asp Glu Asp Ala Glu Ser Leu Thr Lys
   805 810 815
   Glu Asn Ser Lys Ser Glu Glu Gln Glu Asn Glu Asp Ser Thr Asp Ala
   820 825 830
   Glu Ala Ile Asp Lys Glu Glu Val Glu Thr Glu Glu Lys Gly Lys Asp
   835 840 845
   Glu Gln Lys Lys Asp Glu Gln Lys Glu Gln Asp Glu Glu Glu Asp Gly
   850 855 860
   Glu Lys Glu Asn Lys His Lys Ser Ser Glu Thr Thr Asn Glu Thr Val
   865 870 875 880
   Thr Asp Ile Glu Glu Asn Lys Asn Glu Val Lys Gly Glu Glu His Leu
   885 890 895
   Gln Gly Ser Glu Gln Ser Ile Glu Ala Ser Glu Ser Ser Gln Lys Asp
   900 905 910
   Glu Thr Lys Glu Thr Glu Asp Lys Glu Glu Tyr Val Asn Ala Asn Asp
   915 920 925
   Asp Glu Ser Ser Glu Glu Asp Thr Thr Pro Asn Glu Thr Asn Lys Thr
   930 935 940
   Asp Asn Gly Ser Ser Phe Phe Phe Ala Met Ser Asn Ala Leu Leu Val
   945 950 955 960
   Ile Leu Leu Leu Leu Phe Ile Glu Phe Leu
   965 970
<210> 62
   <211> 969
   <212> PRT
   <213> Plasmodium falciparum
<220>
   <221> misc_feature
   <223> PfP113
<400> 62
   Met Lys Ile Pro Phe Phe Ile Leu His Ile Leu Leu Leu Gln Phe Leu
   1 5 10 15
   Leu Cys Leu Ile Arg Cys Tyr Val His Asn Asp Val Ile Lys Phe Gly
   20 25 30
   Glu Glu Asn Ser Leu Lys Cys Ser Gln Gly Asn Leu Tyr Val Leu His
   35 40 45
   Cys Glu Val Gln Cys Leu Asn Gly Asn Asn Glu Ile Ile His Lys Arg
   50 55 60
   Cys Asn Asp Asp Ile Glu Lys Lys Cys Asn Gly Asn Asn Lys Cys Ile
   65 70 75 80
   Tyr Phe Phe Glu Tyr Glu Leu Arg Lys Lys Thr Gln Ser Phe Arg Asn
   85 90 95
   Lys Asn Ser Ile Glu Ile Ser Glu Cys Val Glu Ser Glu Gln Asn Glu
   100 105 110
   Val Lys Thr Ser Thr Thr Cys Leu Leu Ser Asn Ser Phe Ile Leu Asp
   115 120 125
   Glu Ala Phe Ile Gln Tyr Phe Phe Phe Ile Lys Asn Lys Asn Glu Glu
   130 135 140
   Pro Val Ile Cys Lys Asp Gly Asn Ile Asn Ile Lys Ser Ala Leu Leu
   145                 150                 155                 160
   His Ser Pro Phe Cys Glu Ile Lys Leu Lys Asp Ile Ser Glu Tyr Ile
                   165                 170                 175
   Arg Lys Lys Cys Asp Asn Asn Lys Glu Cys Leu Ile Asp Pro Leu Asp
               180                 185                 190
   Val Gln Lys Asn Leu Leu Asn Glu Glu Asp Pro Cys Tyr Ile Asn Asn
           195                 200                 205
   Ser Tyr Val Ser Val Asn Val Val Cys Asn Lys Glu Glu Glu Ile Gly
       210                 215                 220
   Asp Glu Ser Thr Asp Ser Ser Ser Met Glu Ile Gln Asp Ser Thr Ser
   225                 230                 235                 240
   Asn Glu Gln Asp Glu Asn Val Lys Gly Met Ser Ser Ser Gln Glu Met
                   245                 250                 255
   Asn Ser Asn Asn Asp Glu Asn Lys Asn Gln Asp Asn Glu Ser Asp Asp
               260                 265                 270
   Asp Val Asn Asn Asn Asn Asn Asn Asn Asn Asp Asp Gln Asp Glu Gln
           275                 280                 285
   Gly Asn Asp Gly Asp Val Thr Ser Ser Met Asn Lys Asn Glu Asp Asn
       290                 295                 300
   Lys Asp Leu Glu His Gly Ser Ser Asn Asp Val Asn Asn Asn Thr Asp
   305                 310                 315                 320
   Thr Leu Val Asn Asn Lys Glu Asn Lys Glu Phe Val Leu Lys Glu Lys
                   325                 330                 335
   Ser Ser Leu Thr Ser Lys Ile Asn Lys Glu Leu Ala His Arg Thr Ala
               340                 345                 350
   Leu Phe Asn Lys Leu Ala Asp Asn Ile Ser Leu Leu Leu Asn Lys Lys
           355                 360                 365
   Tyr Asp Ser Phe Glu Ile Lys Asp Val Leu Glu Asp Arg Tyr Asn Glu
       370                 375                 380
   Met Lys Arg Asp Ala Asn Pro Asp Val Tyr Tyr Ile Tyr Leu Met Asp
   385                 390                 395                 400
   Thr Leu Asp Ile Glu Lys Ile Glu Asp Ile Asn Leu Glu Glu Val Lys
   405 410 415
   Met Ser Leu Leu Ala Ser Leu Lys Glu Thr Met Asn Lys Ile Asp Thr
   420 425 430
   Ile Glu Lys Lys Ile Glu Glu Phe Lys Asn Lys Tyr Ile Ser Leu Tyr
   435 440 445
   Asn Lys Val Lys Thr Thr Met Pro Glu Leu Phe Asp Leu Asn Glu Asp
   450 455 460
   Leu Val Leu Leu Tyr Asn Asp Phe Pro Phe Asp Asn Gly Met Ile Ser
   465 470 475 480
   Ser Asp Ile Phe Phe Lys Tyr Asn Pro Ser Glu Asn Ile Met Asp His
   485 490 495
   Gln Glu Met Val Lys Lys Gly Ser Ile Thr Glu Asp Glu Leu Arg Ile
   500 505 510
   Val Asn Asp Leu Glu Pro Leu Asp Asn Tyr Arg Arg Arg Lys Arg Ile
   515 520 525
   Thr Glu Leu Arg Lys Ile Leu Val Glu Lys Leu Arg Ile Leu Tyr Leu
   530 535 540
   Glu Lys Asn Asn Leu Phe Asn Thr Gln Ala Ser Cys Ile Lys Ser Tyr
   545 550 555 560
   Cys Tyr Lys Asn Pro Leu Asn Leu Lys Thr Leu Glu Val Leu Leu Lys
   565 570 575
   Lys Asn Tyr Tyr Arg Leu Lys Glu Asn Lys Asp Tyr Asp Val Val Ser
   580 585 590
   Ser Ile Ile Gln His Leu Asp Asn Val Asp Ala Asn Lys Lys Lys Lys
   595 600 605
   Trp Leu Thr His Glu Arg Ile Leu Lys Lys Leu Gln Val Leu Ile Ala
   610 615 620
   Glu Gly Tyr Lys Arg Ile Asn Glu Lys Glu Lys Asp Ile Asp Arg Arg
   625 630 635 640
   Met Ala Val Tyr Asn Ala Leu Tyr Glu Lys Ala Gln Ser Tyr Asn Leu
   645 650 655
   Gln Lys Leu Phe Asn Asp Ser Asn Asp Phe Leu Lys Lys Tyr Ala Ile
   660 665 670
   Met Gly Asn Ser Phe Asp Asp Gly Asp Glu Val Phe Gly Ser Gln Ser
   675 680 685
   Ser Asn Phe Asn Ile Phe Asp Ser Asn Asn Thr Asp Gln Asn Asn Glu
   690 695 700
   Gln Glu Gln Pro Lys Gln Asp Asp Gln Leu Leu Asn Asn Asn Asn Asp
   705 710 715 720
   Asp Val Leu Ser Glu Ser Asn Asn Glu Asn Lys Glu Lys Thr Ser Asp
   725 730 735
   Asp Ala Thr His Lys Glu Thr Gln Glu Lys Ser Asp Gln Glu Pro Ser
   740 745 750
   Gln Asn Ile Gln Glu Asp Asn Ser Asp Glu Lys His Ala Glu Asn Glu
   755 760 765
   Glu Asn Val Glu Gln Ile Glu Thr Asp Ser Asn Val Ser Glu Glu Ala
   770 775 780
   Asn Asp Glu Asn Lys Asp Asn Met Gln Thr Thr Thr Asp Glu Gly Thr
   785 790 795 800
   Glu Glu Leu Gln Gln Asn Asp Glu Asp Ala Glu Ser Leu Thr Lys Glu
   805 810 815
   Asn Ser Lys Ser Glu Glu Gln Glu Asn Glu Asp Ser Thr Asp Ala Glu
   820 825 830
   Ala Ile Asp Lys Glu Glu Val Glu Thr Glu Glu Lys Gly Lys Asp Glu
   835 840 845
   Gln Lys Lys Asp Glu Gln Lys Glu Gln Asp Glu Glu Glu Asp Gly Glu
   850 855 860
   Lys Glu Asn Lys His Lys Ser Ser Glu Thr Thr Asn Glu Thr Val Thr
   865 870 875 880
   Asp Ile Glu Glu Asn Lys Asn Glu Val Lys Gly Glu Glu His Leu Gln
   885 890 895
   Gly Ser Glu Gln Ser Ile Glu Ala Ser Glu Ser Ser Gln Lys Asp Glu
   900 905 910
   Thr Lys Glu Thr Glu Asp Lys Glu Glu Tyr Val Asn Ala Asn Asp Asp
   915 920 925
   Glu Ser Ser Glu Glu Asp Thr Thr Pro Asn Glu Thr Asn Lys Thr Asp
   930 935 940
   Asn Gly Ser Ser Phe Phe Phe Ala Met Ser Asn Ala Leu Leu Val Ile
   945 950 955 960
   Leu Leu Leu Leu Phe Ile Glu Phe Leu
   965
<210> 63
   <211> 3141
   <212> DNA
   <213> Artificial Sequence
<220>
   <223> PvP113 human CO
<400> 63
   gccaccatgg ctaagctccc gcccctctgc aggttgccac ttgcgctggt gcttctttgt      60
   ctgacatcca gagcgcgctg ttatgtacac aacgacgtga tgaaatttgg tgaagaaaac     120
   tctctcaagt gttcacaagg gtctctctac atccttcact gcgaggtcaa gtgtgtgaat     180
   gcaaagaaca gaatcatcca ccgaagttgc atcgaccagg ttgaggcgaa gtgcatgggc     240
   aacgccaaat gtaagtacta ttttgactat gtcgtgaaat cccgcggaca gagtttgcgg     300
   aacaaaaatg aaatcgaaat agaagaatgc gtggaatccg aacggaatga gattaagacg     360
   tccacaactt gccttctgtc aaattcattt ttgctggatg agacctacat tcaatatttc     420
   tttttcatca aaaacaaaaa tgaagaaccc ataacgtgtc gagacggtag actgagcgta     480
   aagagtgcga tacttcatag tcccttttgc aaaataaatc ttaaggacat cactgagatt     540
   ctcaagagac aatgcgatca cagtaaggag tgcgtcatta atccgtatgt cctccaaaaa     600
   gatgcattga atgagagaga ccagtgttac attaacaact catatgtctc actcaatgtc     660
   gtttgcacca aagaagggga ggagcaacca gaagagagtg gacataaaca gaagagggat     720
   gacgacgtgg acgaaaccga ggaaggctca tatgacgtct ctgcggatca gaacaaatca     780
   gcaatagtcg gcgagggaaa cgatgatccg gaatccctgg gtgaagaaga tgagctttca     840
   gagactaacg aggcagttga tctcattatg aactccaagg aaagctttga aaacaaaatt     900
   cggaaggcga agtcaatact tctctcacag atgaacgagc aggaggtgaa aaagaatgca     960
   atattcaaga aactcgggga agagctttcc aaaatggtcc tccagaaata tgaaccaagt    1020
   gatctgaaag acttgatcga ggacaggtat aatgaaatga gacggtcccc tgaccaagac 1080
   ctttattatc tctaccttat agatacactc gaaataaata aaatggaaga tcttgacgtg 1140
   actgcactgc aagaccagct cgctatattg ttggaagagc agatgggcaa gatgaatcgc 1200
   attgagaaaa cgattaaccg attgaggaaa aaataccttt ctatttacaa caaagcgaag 1260
   aacaagaaag ttaaagacat ttacgatgaa ggagttgatc ctgtactcac gtacgacgac 1320
   ttcgcgcacg gcaacggcat tattacggca gatattttct ttaagtataa gcctgccatt 1380
   aagccgttga cttttagcaa atctaatgcc tctgaggagc gggggtcatc caaaaaaaaa 1440
   gagtacaagg accttcttga gatggacgcg ctggatgagt acaaccggaa gaaacggata 1500
   actgacatgc gaaacggtct gatggagact ttgaagaaaa tgtattacgc aaaaaatggg 1560
   atattcaata atctggcgag ttgcattaag tcatattgct ataaaaggcc tctcaacctt 1620
   aatgcacttt cctccgttct taaaagaaat tttgaaaatc ttcgagagaa aaaatcaacc 1680
   gatccagtgg cccccatagt cagatatttg caaaaggtca gtggcgaggt tggcggggaa 1740
   gtgggaggcg cggctggcgg tgctgcgggg ggtgctgcaa gtggtgccgc atcaggagct 1800
   gtcagtgaag cagtaggagg cgccataggt ggggctgtgg gcgaggcatc tatcgcagta 1860
   aatccgcccc gatgggaaaa atcacgacga attcttcaaa agctcaaggc tttgctgcac 1920
   ctgggttatc aacaggcact ggacaaagag ttggagatcg acgagaggac tgacaaatac 1980
   aacgctctga atgagaaagc aaaagaatat aaccttcagc ggcttttttc cgagtcagac 2040
   aagttgctta agaaggtcgc aatgcttacg tcagccagtg aaagtgcaga tgaagtattc 2100
   ggtaaccaag cgagcttttt cgatgtttac agaggagagg cagcgtcaca aaaaggagta 2160
   gccgccagtg aaaagggcgt agctgcaaga gagaaaggag tcgccgcgag tgagaagggg 2220
   gtcgcggcat cagaaaaggg tgtggcggcc agcgagaagg gtgtcgcggc atcagaaaaa 2280
   ggtgttgccg caagccaaaa aggcggagaa acgtctgagg aaggggaggc tgccagtcaa 2340
   aaggatgttg ctacgtccga ggagggtgga gctagcagcg agaaacgcga tgacgacgag 2400
   atgaatccgc ccgacgaagg gtatgagtcc gctaaggaag acggggagaa tgcacaagat 2460
   gacgacagcg gcggaaatgc tgaacccgta gagggaaaag caggcgaaag tgaagacgct 2520
   gatggggtaa atgccggctc caataaagaa ggtgaggatg gggagagcgt tgaagaagaa 2580
   gctgcggaag gggaagcggc acccaaggag gaggctgcag acggagaaga tgctccggag 2640
   gaggaagccg agggtgagga tgccccagaa gaagaggccg atggcgaaga cgccccgaag 2700
   gaggaggcta ctgacggaga ggacgcccct aaagaggaag aggccgaagg tgaggatgca 2760
   agcaaggacg aggaggccga cgaaggctca actgacgagg aagaagccgc cgacggcggg 2820
   agtactgatg ctacagcagc cgatgaagct gcaggcggag tcgcggacca aaacgacgtg 2880
   cccgttaagg gagaagactc agatggcgcg gaatctgatg gagcagaaga tgccgccact 2940
   gaaattaggg gtgaggctga agccggtgaa gaggccgcgg agcaacctac tggagaggcc     3000
   gtagtcaaag gggattccga gggcggggct tctgggctgg aaaccgagaa gaagggagac     3060
   gacgggggct ccttcttcca aggtctttcc cgagtgttgc ttactgttct tgcaatactt     3120
   tccttggaat ttctcctttg a                                               3141
<210> 64
   <211> 1044
   <212> PRT
   <213> Plasmodium vivax
<220>
   <221> misc_feature
   <223> PvP113
<400> 64
   Met Ala Lys Leu Pro Pro Leu Cys Arg Leu Pro Leu Ala Leu Val Leu
   1               5                   10                  15
   Leu Cys Leu Thr Ser Arg Ala Arg Cys Tyr Val His Asn Asp Val Met
               20                  25                  30
   Lys Phe Gly Glu Glu Asn Ser Leu Lys Cys Ser Gln Gly Ser Leu Tyr
           35                  40                  45
   Ile Leu His Cys Glu Val Lys Cys Val Asn Ala Lys Asn Arg Ile Ile
       50                  55                  60
   His Arg Ser Cys Ile Asp Gln Val Glu Ala Lys Cys Met Gly Asn Ala
   65                  70                  75                  80
   Lys Cys Lys Tyr Tyr Phe Asp Tyr Val Val Lys Ser Arg Gly Gln Ser
                   85                  90                  95
   Leu Arg Asn Lys Asn Glu Ile Glu Ile Glu Glu Cys Val Glu Ser Glu
               100                 105                 110
   Arg Asn Glu Ile Lys Thr Ser Thr Thr Cys Leu Leu Ser Asn Ser Phe
           115                 120                 125
   Leu Leu Asp Glu Thr Tyr Ile Gln Tyr Phe Phe Phe Ile Lys Asn Lys
       130                 135                 140
   Asn Glu Glu Pro Ile Thr Cys Arg Asp Gly Arg Leu Ser Val Lys Ser
   145                 150                 155                 160
   Ala Ile Leu His Ser Pro Phe Cys Lys Ile Asn Leu Lys Asp Ile Thr
                   165                 170                 175
   Glu Ile Leu Lys Arg Gln Cys Asp His Ser Lys Glu Cys Val Ile Asn
               180                 185                 190
   Pro Tyr Val Leu Gln Lys Asp Ala Leu Asn Glu Arg Asp Gln Cys Tyr
           195                 200                 205
   Ile Asn Asn Ser Tyr Val Ser Leu Asn Val Val Cys Thr Lys Glu Gly
       210                 215                 220
   Glu Glu Gln Pro Glu Glu Ser Gly His Lys Gln Lys Arg Asp Asp Asp
   225                 230                 235                 240
   Val Asp Glu Thr Glu Glu Gly Ser Tyr Asp Val Ser Ala Asp Gln Asn
                   245                 250                 255
   Lys Ser Ala Ile Val Gly Glu Gly Asn Asp Asp Pro Glu Ser Leu Gly
               260                 265                 270
   Glu Glu Asp Glu Leu Ser Glu Thr Asn Glu Ala Val Asp Leu Ile Met
           275                 280                 285
   Asn Ser Lys Glu Ser Phe Glu Asn Lys Ile Arg Lys Ala Lys Ser Ile
       290                 295                 300
   Leu Leu Ser Gln Met Asn Glu Gln Glu Val Lys Lys Asn Ala Ile Phe
   305                 310                 315                 320
   Lys Lys Leu Gly Glu Glu Leu Ser Lys Met Val Leu Gln Lys Tyr Glu
                   325                 330                 335
   Pro Ser Asp Leu Lys Asp Leu Ile Glu Asp Arg Tyr Asn Glu Met Arg
               340                 345                 350
   Arg Ser Pro Asp Gln Asp Leu Tyr Tyr Leu Tyr Leu Ile Asp Thr Leu
           355                 360                 365
   Glu Ile Asn Lys Met Glu Asp Leu Asp Val Thr Ala Leu Gln Asp Gln
       370                 375                 380
   Leu Ala Ile Leu Leu Glu Glu Gln Met Gly Lys Met Asn Arg Ile Glu
   385                 390                 395                 400
   Lys Thr Ile Asn Arg Leu Arg Lys Lys Tyr Leu Ser Ile Tyr Asn Lys
                   405                 410                 415
   Ala Lys Asn Lys Lys Val Lys Asp Ile Tyr Asp Glu Gly Val Asp Pro
               420                 425                 430
   Val Leu Thr Tyr Asp Asp Phe Ala His Gly Asn Gly Ile Ile Thr Ala
           435                 440                 445
   Asp Ile Phe Phe Lys Tyr Lys Pro Ala Ile Lys Pro Leu Thr Phe Ser
       450                 455                 460
   Lys Ser Asn Ala Ser Glu Glu Arg Gly Ser Ser Lys Lys Lys Glu Tyr
   465                 470                 475                 480
   Lys Asp Leu Leu Glu Met Asp Ala Leu Asp Glu Tyr Asn Arg Lys Lys
                   485                 490                 495
   Arg Ile Thr Asp Met Arg Asn Gly Leu Met Glu Thr Leu Lys Lys Met
               500                 505                 510
   Tyr Tyr Ala Lys Asn Gly Ile Phe Asn Asn Leu Ala Ser Cys Ile Lys
           515                 520                 525
   Ser Tyr Cys Tyr Lys Arg Pro Leu Asn Leu Asn Ala Leu Ser Ser Val
       530                 535                 540
   Leu Lys Arg Asn Phe Glu Asn Leu Arg Glu Lys Lys Ser Thr Asp Pro
   545                 550                 555                 560
   Val Ala Pro Ile Val Arg Tyr Leu Gln Lys Val Ser Gly Glu Val Gly
                   565                 570                 575
   Gly Glu Val Gly Gly Ala Ala Gly Gly Ala Ala Gly Gly Ala Ala Ser
               580                 585                 590
   Gly Ala Ala Ser Gly Ala Val Ser Glu Ala Val Gly Gly Ala Ile Gly
           595                 600                 605
   Gly Ala Val Gly Glu Ala Ser Ile Ala Val Asn Pro Pro Arg Trp Glu
       610                 615                 620
   Lys Ser Arg Arg Ile Leu Gln Lys Leu Lys Ala Leu Leu His Leu Gly
   625                 630                 635                 640
   Tyr Gln Gln Ala Leu Asp Lys Glu Leu Glu Ile Asp Glu Arg Thr Asp
                   645                 650                 655
   Lys Tyr Asn Ala Leu Asn Glu Lys Ala Lys Glu Tyr Asn Leu Gln Arg
               660                 665                 670
   Leu Phe Ser Glu Ser Asp Lys Leu Leu Lys Lys Val Ala Met Leu Thr
           675                 680                 685
   Ser Ala Ser Glu Ser Ala Asp Glu Val Phe Gly Asn Gln Ala Ser Phe
       690                 695                 700
   Phe Asp Val Tyr Arg Gly Glu Ala Ala Ser Gln Lys Gly Val Ala Ala
   705                 710                 715                 720
   Ser Glu Lys Gly Val Ala Ala Arg Glu Lys Gly Val Ala Ala Ser Glu
                   725                 730                 735
   Lys Gly Val Ala Ala Ser Glu Lys Gly Val Ala Ala Ser Glu Lys Gly
               740                 745                 750
   Val Ala Ala Ser Glu Lys Gly Val Ala Ala Ser Gln Lys Gly Gly Glu
           755                 760                 765
   Thr Ser Glu Glu Gly Glu Ala Ala Ser Gln Lys Asp Val Ala Thr Ser
       770                 775                 780
   Glu Glu Gly Gly Ala Ser Ser Glu Lys Arg Asp Asp Asp Glu Met Asn
   785                 790                 795                 800
   Pro Pro Asp Glu Gly Tyr Glu Ser Ala Lys Glu Asp Gly Glu Asn Ala
                   805                 810                 815
   Gln Asp Asp Asp Ser Gly Gly Asn Ala Glu Pro Val Glu Gly Lys Ala
               820                 825                 830
   Gly Glu Ser Glu Asp Ala Asp Gly Val Asn Ala Gly Ser Asn Lys Glu
           835                 840                 845
   Gly Glu Asp Gly Glu Ser Val Glu Glu Glu Ala Ala Glu Gly Glu Ala
       850                 855                 860
   Ala Pro Lys Glu Glu Ala Ala Asp Gly Glu Asp Ala Pro Glu Glu Glu
   865                 870                 875                 880
   Ala Glu Gly Glu Asp Ala Pro Glu Glu Glu Ala Asp Gly Glu Asp Ala
                   885                 890                 895
   Pro Lys Glu Glu Ala Thr Asp Gly Glu Asp Ala Pro Lys Glu Glu Glu
               900                 905                 910
   Ala Glu Gly Glu Asp Ala Ser Lys Asp Glu Glu Ala Asp Glu Gly Ser
           915                 920                 925
   Thr Asp Glu Glu Glu Ala Ala Asp Gly Gly Ser Thr Asp Ala Thr Ala
   930 935 940
   Ala Asp Glu Ala Ala Gly Gly Val Ala Asp Gln Asn Asp Val Pro Val
   945 950 955 960
   Lys Gly Glu Asp Ser Asp Gly Ala Glu Ser Asp Gly Ala Glu Asp Ala
   965 970 975
   Ala Thr Glu Ile Arg Gly Glu Ala Glu Ala Gly Glu Glu Ala Ala Glu
   980 985 990
   Gln Pro Thr Gly Glu Ala Val Val Lys Gly Asp Ser Glu Gly Gly Ala
   995 1000 1005
   Ser Gly Leu Glu Thr Glu Lys Lys Gly Asp Asp Gly Gly Ser Phe
   1010 1015 1020
   Phe Gln Gly Leu Ser Arg Val Leu Leu Thr Val Leu Ala Ile Leu
   1025 1030 1035
   Ser Leu Glu Phe Leu Leu
   1040
<210> 65
   <211> 969
   <212> PRT
   <213> Plasmodium vivax
<220>
   <221> misc_feature
   <223> P113
<400> 65
   Met Lys Leu Pro Pro Leu Cys Arg Leu Pro Leu Ala Leu Val Leu Leu
   1 5 10 15
   Cys Leu Thr Ser Arg Ala Arg Cys Tyr Val His Asn Asp Val Met Lys
   20 25 30
   Phe Gly Glu Glu Asn Ser Leu Lys Cys Ser Gln Gly Ser Leu Tyr Ile
   35 40 45
   Leu His Cys Glu Val Lys Cys Val Asn Ala Lys Asn Arg Ile Ile His
   50 55 60
   Arg Ser Cys Ile Asp Gln Val Glu Ala Lys Cys Met Gly Asn Ala Lys
   65                  70                  75                  80
   Cys Lys Tyr Tyr Phe Asp Tyr Val Val Lys Ser Arg Gly Gln Ser Leu
                   85                  90                  95
   Arg Asn Lys Asn Glu Ile Glu Ile Glu Glu Cys Val Glu Ser Glu Arg
               100                 105                 110
   Asn Glu Ile Lys Thr Ser Thr Thr Cys Leu Leu Ser Asn Ser Phe Leu
           115                 120                 125
   Leu Asp Glu Thr Tyr Ile Gln Tyr Phe Phe Phe Ile Lys Asn Lys Asn
       130                 135                 140
   Glu Glu Pro Ile Thr Cys Arg Asp Gly Arg Leu Ser Val Lys Ser Ala
   145                 150                 155                 160
   Ile Leu His Ser Pro Phe Cys Lys Ile Asn Leu Lys Asp Ile Thr Glu
                   165                 170                 175
   Ile Leu Lys Arg Gln Cys Asp His Ser Lys Glu Cys Val Ile Asn Pro
               180                 185                 190
   Tyr Val Leu Gln Lys Asp Ala Leu Asn Glu Arg Asp Gln Cys Tyr Ile
           195                 200                 205
   Asn Asn Ser Tyr Val Ser Leu Asn Val Val Cys Thr Lys Glu Gly Glu
       210                 215                 220
   Glu Gln Pro Glu Glu Ser Gly His Lys Gln Lys Arg Asp Asp Asp Val
   225                 230                 235                 240
   Asp Glu Thr Glu Glu Gly Ser Tyr Asp Val Ser Ala Asp Gln Asn Lys
                   245                 250                 255
   Ser Ala Ile Val Gly Glu Gly Asn Asp Asp Pro Glu Ser Leu Gly Glu
               260                 265                 270
   Glu Asp Glu Leu Ser Glu Thr Asn Glu Ala Val Asp Leu Ile Met Asn
           275                 280                 285
   Ser Lys Glu Ser Phe Glu Asn Lys Ile Arg Lys Ala Lys Ser Ile Leu
       290                 295                 300
   Leu Ser Gln Met Asn Glu Gln Glu Val Lys Lys Asn Ala Ile Phe Lys
   305                 310                 315                 320
   Lys Leu Gly Glu Glu Leu Ser Lys Met Val Leu Gln Lys Tyr Glu Pro
   325 330 335
   Ser Asp Leu Lys Asp Leu Ile Glu Asp Arg Tyr Asn Glu Met Arg Arg
   340 345 350
   Ser Pro Asp Gln Asp Leu Tyr Tyr Leu Tyr Leu Ile Asp Thr Leu Glu
   355 360 365
   Ile Asn Lys Met Glu Asn Leu Asp Val Thr Ala Leu Gln Asp Gln Leu
   370 375 380
   Ala Ile Leu Leu Glu Glu Gln Met Gly Lys Met Asn Arg Ile Glu Lys
   385 390 395 400
   Thr Ile Asn Arg Leu Arg Lys Lys Tyr Leu Ser Ile Tyr Asn Lys Ala
   405 410 415
   Lys Asn Lys Lys Val Lys Asp Ile Tyr Asp Glu Gly Val Asp Pro Val
   420 425 430
   Leu Thr Tyr Asp Asp Phe Ala His Gly Asn Gly Ile Ile Thr Ala Asp
   435 440 445
   Ile Phe Phe Lys Tyr Lys Pro Ala Ile Lys Pro Leu Thr Phe Ser Lys
   450 455 460
   Ser Asn Ala Ser Glu Glu Arg Gly Ser Ser Lys Lys Lys Glu Tyr Lys
   465 470 475 480
   Asp Leu Leu Glu Met Asp Ala Leu Asp Glu Tyr Asn Arg Lys Lys Arg
   485 490 495
   Ile Thr Asp Met Arg Asn Gly Leu Met Glu Thr Leu Lys Lys Met Tyr
   500 505 510
   Tyr Ala Lys Asn Gly Ile Phe Asn Asn Leu Ala Ser Cys Ile Lys Ser
   515 520 525
   Tyr Cys Tyr Lys Arg Pro Leu Asn Leu Asn Ala Leu Ser Ser Val Leu
   530 535 540
   Lys Arg Asn Phe Glu Asn Leu Arg Glu Lys Lys Ser Thr Asp Pro Val
   545 550 555 560
   Ala Pro Ile Val Arg Tyr Leu Gln Lys Lys Ser Arg Arg Ile Leu Gln
   565 570 575
   Lys Leu Lys Ala Leu Leu His Leu Gly Tyr Gln Gln Ala Leu Asp Lys
               580                 585                 590
   Glu Leu Glu Ile Asp Glu Arg Thr Asp Lys Tyr Asn Ala Leu Asn Glu
           595                 600                 605
   Lys Ala Lys Glu Tyr Asn Leu Gln Arg Leu Phe Ser Glu Ser Asp Lys
       610                 615                 620
   Leu Leu Lys Lys Val Ala Met Leu Thr Ser Ala Ser Glu Ser Ala Asp
   625                 630                 635                 640
   Glu Val Phe Gly Asn Gln Ala Ser Phe Phe Asp Val Tyr Arg Gly Glu
                   645                 650                 655
   Ala Ala Ser Glu Lys Gly Val Ala Ala Ser Gln Lys Gly Gly Glu Thr
               660                 665                 670
   Ser Glu Glu Gly Glu Ala Ala Ser Gln Lys Asp Val Ala Thr Ser Glu
           675                 680                 685
   Glu Gly Gly Ala Ser Ser Glu Lys Arg Asp Asp Asp Glu Met Asn Pro
       690                 695                 700
   Pro Asp Glu Gly Tyr Glu Ser Ala Lys Glu Asp Gly Glu Asn Ala Gln
   705                 710                 715                 720
   Asp Asp Asp Ser Gly Gly Asn Ala Glu Pro Val Glu Gly Lys Ala Gly
                   725                 730                 735
   Glu Ser Glu Asp Ala Asp Gly Val Asn Ala Gly Ser Asn Lys Glu Gly
               740                 745                 750
   Glu Asp Gly Glu Ser Val Glu Glu Glu Ala Ala Glu Gly Glu Ala Ala
           755                 760                 765
   Pro Lys Glu Glu Ala Ala Asp Gly Glu Asp Ala Pro Glu Glu Glu Ala
       770                 775                 780
   Glu Gly Glu Asp Ala Pro Lys Glu Glu Ala Ala Asp Gly Glu Ala Ser
   785                 790                 795                 800
   Ser Lys Glu Glu Glu Thr Asp Gly Glu Val Ala Pro Glu Glu Ala Ala
                   805                 810                 815
   Asp Gly Glu Asp Ala Pro Lys Glu Glu Ala Thr Asp Gly Glu Asp Ala
               820                 825                 830
   Pro Lys Glu Glu Glu Ala Glu Gly Glu Asp Ala Ser Lys Asp Glu Glu
   835 840 845
   Ala Asp Glu Gly Ser Thr Asp Glu Glu Glu Ala Ala Asp Gly Gly Ser
   850 855 860
   Thr Asp Ala Thr Ala Ala Asp Glu Ala Ala Gly Gly Val Ala Asp Gln
   865 870 875 880
   Asn Asp Val Pro Val Lys Gly Glu Asp Ser Asp Gly Ala Glu Ser Asp
   885 890 895
   Gly Ala Glu Asp Ala Ala Thr Glu Ile Arg Gly Glu Ala Glu Ala Gly
   900 905 910
   Glu Glu Ala Ala Glu Gln Pro Thr Gly Glu Ala Val Val Lys Gly Asp
   915 920 925
   Ser Glu Gly Gly Ala Ser Gly Leu Glu Thr Glu Lys Lys Gly Asp Asp
   930 935 940
   Gly Gly Ser Phe Phe Gln Gly Leu Ser Arg Val Leu Leu Thr Val Leu
   945 950 955 960
   Ala Ile Leu Ser Leu Glu Phe Leu Leu
   965
<210> 66
   <211> 654
   <212> DNA
   <213> Plasmodium berghei
<220>
   <221> misc_feature
   <223> Ag40
<400> 66
   ggatccgcca ccatggctgg ggcgagtatg agccacttgc agtgtctgac atctgttgct 60
   ggtctgtcct ctatcgtcat gtcaatgttc cccaaactca ttgccaataa tccttccctg 120
   tttagaccac tgctcaacat ttcctgggga tatctgttcg gaagcactgt atggctgtgc 180
   ttcttcagtg agattgggtt ggtcaggaga atcaatgctc ctaaacggaa gaatctgcca 240
   gagaatgcag aacaagccaa agaacagctg aaggagatca agaacaacga aggcgatttt 300
   aaccgacgca atatcgactt caagtacttc tttagccttt ccacaatctt ctctagcata 360
   ctgctgctta gcacagtgaa actcgccaac aacaatctgc agttgaggat ctgttccacc 420
   attgtgtcac tgagttgcat actgaacaat atgtactttc agaacaagat acactcactt      480
   gcactgaaga aagagagtct ctttaaggac atgatcgatc gtccgaaaga taccactatt      540
   ctggtgaacc tgaagaagaa caagaccgac tttcacatcc atcatggcct ttctctgctc      600
   ttgctctata gcagcttctt tggcctcact ccctacattt tcacgtgact cgag            654
<210> 67
   <211> 211
   <212> PRT
   <213> Plasmodium berghei
<220>
   <221> misc_feature
   <223> Ag40
<400> 67
   Met Ala Gly Ala Ser Met Ser His Leu Gln Cys Leu Thr Ser Val Ala
   1               5                   10                  15
   Gly Leu Ser Ser Ile Val Met Ser Met Phe Pro Lys Leu Ile Ala Asn
               20                  25                  30
   Asn Pro Ser Leu Phe Arg Pro Leu Leu Asn Ile Ser Trp Gly Tyr Leu
           35                  40                  45
   Phe Gly Ser Thr Val Trp Leu Cys Phe Phe Ser Glu Ile Gly Leu Val
       50                  55                  60
   Arg Arg Ile Asn Ala Pro Lys Arg Lys Asn Leu Pro Glu Asn Ala Glu
   65                  70                  75                  80
   Gln Ala Lys Glu Gln Leu Lys Glu Ile Lys Asn Asn Glu Gly Asp Phe
                   85                  90                  95
   Asn Arg Arg Asn Ile Asp Phe Lys Tyr Phe Phe Ser Leu Ser Thr Ile
               100                 105                 110
   Phe Ser Ser Ile Leu Leu Leu Ser Thr Val Lys Leu Ala Asn Asn Asn
           115                 120                 125
   Leu Gln Leu Arg Ile Cys Ser Thr Ile Val Ser Leu Ser Cys Ile Leu
       130                 135                 140
   Asn Asn Met Tyr Phe Gln Asn Lys Ile His Ser Leu Ala Leu Lys Lys
   145                 150                 155                 160
   Glu Ser Leu Phe Lys Asp Met Ile Asp Arg Pro Lys Asp Thr Thr Ile
                   165                 170                 175
   Leu Val Asn Leu Lys Lys Asn Lys Thr Asp Phe His Ile His His Gly
               180                 185                 190
   Leu Ser Leu Leu Leu Leu Tyr Ser Ser Phe Phe Gly Leu Thr Pro Tyr
           195                 200                 205
   Ile Phe Thr
       210
<210> 68
   <211> 210
   <212> PRT
   <213> Plasmodium berghei
<220>
   <221> misc_feature
   <223> Ag40
<400> 68
   Met Gly Ala Ser Met Ser His Leu Gln Cys Leu Thr Ser Val Ala Gly
   1               5                   10                  15
   Leu Ser Ser Ile Val Met Ser Met Phe Pro Lys Leu Ile Ala Asn Asn
               20                  25                  30
   Pro Ser Leu Phe Arg Pro Leu Leu Asn Ile Ser Trp Gly Tyr Leu Phe
           35                  40                  45
   Gly Ser Thr Val Trp Leu Cys Phe Phe Ser Glu Ile Gly Leu Val Arg
       50                  55                  60
   Arg Ile Asn Ala Pro Lys Arg Lys Asn Leu Pro Glu Asn Ala Glu Gln
   65                  70                  75                  80
   Ala Lys Glu Gln Leu Lys Glu Ile Lys Asn Asn Glu Gly Asp Phe Asn
                   85                  90                  95
   Arg Arg Asn Ile Asp Phe Lys Tyr Phe Phe Ser Leu Ser Thr Ile Phe
               100                 105                 110
   Ser Ser Ile Leu Leu Leu Ser Thr Val Lys Leu Ala Asn Asn Asn Leu
           115                 120                 125
   Gln Leu Arg Ile Cys Ser Thr Ile Val Ser Leu Ser Cys Ile Leu Asn
       130                 135                 140
   Asn Met Tyr Phe Gln Asn Lys Ile His Ser Leu Ala Leu Lys Lys Glu
   145                 150                 155                 160
   Ser Leu Phe Lys Asp Met Ile Asp Arg Pro Lys Asp Thr Thr Ile Leu
                   165                 170                 175
   Val Asn Leu Lys Lys Asn Lys Thr Asp Phe His Ile His His Gly Leu
               180                 185                 190
   Ser Leu Leu Leu Leu Tyr Ser Ser Phe Phe Gly Leu Thr Pro Tyr Ile
           195                 200                 205
   Phe Thr
       210
<210> 69
   <211> 642
   <212> DNA
   <213> Artificial Sequence
<220>
   <223> PfAg40 human CO
<400> 69
   gccaccatgg ctgggtgtac agtctctaat ctcaaatgcg tgaccaatgt ggcaggactg        60
   gcaagtctgg ttatcagtct gtttccgaaa ctcatcataa agaacccaca agtgcttcga       120
   ccactgctga atgtgtcctg gggttatctg tttggtagca ccttttggct gtgcttcttc       180
   tccgaagtag gactgcttcg cagcctgaag aacatgaaag gggtaccttt gcctgaatca       240
   gccagtgagg cgaagaagct tctcgaagag atgaagaact ctgagggcga tttcaatcgg       300
   agatcactgg acttccagta cttcttttcc ctcgctacgt tgttctcagg cattctgttg       360
   ctgagcacag tgaagttggc caaccataac ctgcagctta ggcttagtag ctctgtggtc       420
   gtcatcacat cactgctgaa tagcctgtat ctgcacaata aagtgcataa tctgaaaagc       480
   aagaaagaaa gcctctataa cgactttatt gccaatccca agaacgagaa aactgtcgct       540
   gatctgaaga agaacaagaa agagtttcac atctttcacg gattgtccgt tctctctctc       600
   tacgtttcct tcttcggcct gactccctac attttcacct aa                          642
<210> 70
   <211> 211
   <212> PRT
   <213> Plasmodium falciparum
<220>
   <221> misc_feature
   <223> PfAg40
<400> 70
   Met Ala Gly Cys Thr Val Ser Asn Leu Lys Cys Val Thr Asn Val Ala
   1               5                   10                  15
   Gly Leu Ala Ser Leu Val Ile Ser Leu Phe Pro Lys Leu Ile Ile Lys
               20                  25                  30
   Asn Pro Gln Val Leu Arg Pro Leu Leu Asn Val Ser Trp Gly Tyr Leu
           35                  40                  45
   Phe Gly Ser Thr Phe Trp Leu Cys Phe Phe Ser Glu Val Gly Leu Leu
       50                  55                  60
   Arg Ser Leu Lys Asn Met Lys Gly Val Pro Leu Pro Glu Ser Ala Ser
   65                  70                  75                  80
   Glu Ala Lys Lys Leu Leu Glu Glu Met Lys Asn Ser Glu Gly Asp Phe
                   85                  90                  95
   Asn Arg Arg Ser Leu Asp Phe Gln Tyr Phe Phe Ser Leu Ala Thr Leu
               100                 105                 110
   Phe Ser Gly Ile Leu Leu Leu Ser Thr Val Lys Leu Ala Asn His Asn
           115                 120                 125
   Leu Gln Leu Arg Leu Ser Ser Ser Val Val Val Ile Thr Ser Leu Leu
       130                 135                 140
   Asn Ser Leu Tyr Leu His Asn Lys Val His Asn Leu Lys Ser Lys Lys
   145                 150                 155                 160
   Glu Ser Leu Tyr Asn Asp Phe Ile Ala Asn Pro Lys Asn Glu Lys Thr
                   165                 170                 175
   Val Ala Asp Leu Lys Lys Asn Lys Lys Glu Phe His Ile Phe His Gly
               180                 185                 190
   Leu Ser Val Leu Ser Leu Tyr Val Ser Phe Phe Gly Leu Thr Pro Tyr
           195                 200                 205
   Ile Phe Thr
       210
<210> 71
   <211> 210
   <212> PRT
   <213> Plasmodium falciparum
<220>
   <221> misc_feature
   <223> Ag40
<400> 71
   Met Gly Cys Thr Val Ser Asn Leu Lys Cys Val Thr Asn Val Ala Gly
   1               5                   10                  15
   Leu Ala Ser Leu Val Ile Ser Leu Phe Pro Lys Leu Ile Ile Lys Asn
               20                  25                  30
   Pro Gln Val Leu Arg Pro Leu Leu Asn Val Ser Trp Gly Tyr Leu Phe
           35                  40                  45
   Gly Ser Thr Phe Trp Leu Cys Phe Phe Ser Glu Val Gly Leu Leu Arg
       50                  55                  60
   Ser Leu Lys Asn Met Lys Gly Val Pro Leu Pro Glu Ser Ala Ser Glu
   65                  70                  75                  80
   Ala Lys Lys Leu Leu Glu Glu Met Lys Asn Ser Glu Gly Asp Phe Asn
                   85                  90                  95
   Arg Arg Ser Leu Asp Phe Gln Tyr Phe Phe Ser Leu Ala Thr Leu Phe
               100                 105                 110
   Ser Gly Ile Leu Leu Leu Ser Thr Val Lys Leu Ala Asn His Asn Leu
           115                 120                 125
   Gln Leu Arg Leu Ser Ser Ser Val Val Val Ile Thr Ser Leu Leu Asn
       130                 135                 140
   Ser Leu Tyr Leu His Asn Lys Val His Asn Leu Lys Ser Lys Lys Glu
   145                 150                 155                 160
   Ser Leu Tyr Asn Asp Phe Ile Ala Asn Pro Lys Asn Glu Lys Thr Val
                   165                 170                 175
   Ala Asp Leu Lys Lys Asn Lys Lys Glu Phe His Ile Phe His Gly Leu
               180                 185                 190
   Ser Val Leu Ser Leu Tyr Val Ser Phe Phe Gly Leu Thr Pro Tyr Ile
           195                 200                 205
   Phe Thr
       210
<210> 72
   <211> 639
   <212> DNA
   <213> Artificial Sequence
<220>
   <223> PvAg40 human CO + kozac
<400> 72
   gccaccatgg gggcgacggt atcatatctt agatgcgtga ccagtatagc agggctgagc      60
   agcctcgtgc tgtctttgtt tcccaagctg attatgaaaa accctcaggt actccgacca     120
   ctccttaaca ttagctgggg ttatttgttt ggctcaacct tttggttgtg tttgttctcc     180
   gaagtaggac ttttccggtc cctgaaaaat atgaagcgca taccaatccc tgaaaacgca     240
   gaagaggcta agaagcaatt ggaggagatg aaaagcatgg aaggggattt taccaggcgc     300
   agggaagatt tccaatattt ttttggtttt tccaccttgt tttctggtat tcttcttctc     360
   agtacggtaa gacttgcgaa tcacaacatg caactgagga tttccagtac catcgttgcc     420
   cttagctgcc tgctcaataa cttgtacctt cagaataagg tacattctct taaaatccaa     480
   aaagaaaacc tgtacaacga actcatccgc aatcctaagt cagagacgac tatagcggag     540
   attaagaaaa acaaaaaaga tttccatata taccacggct tgtccctgtt gtccctttac     600
   ataagcttcc tcggccttac tccatatata tttacctag                            639
<210> 73
   <211> 210
   <212> PRT
   <213> Plasmodium vivax
<220>
   <221> misc_feature
   <223> PvAg40
<400> 73
   Met Gly Ala Thr Val Ser Tyr Leu Arg Cys Val Thr Ser Ile Ala Gly
   1               5                   10                  15
   Leu Ser Ser Leu Val Leu Ser Leu Phe Pro Lys Leu Ile Met Lys Asn
               20                  25                  30
   Pro Gln Val Leu Arg Pro Leu Leu Asn Ile Ser Trp Gly Tyr Leu Phe
           35                  40                  45
   Gly Ser Thr Phe Trp Leu Cys Leu Phe Ser Glu Val Gly Leu Phe Arg
       50                  55                  60
   Ser Leu Lys Asn Met Lys Arg Ile Pro Ile Pro Glu Asn Ala Glu Glu
   65                  70                  75                  80
   Ala Lys Lys Gln Leu Glu Glu Met Lys Ser Met Glu Gly Asp Phe Thr
                   85                  90                  95
   Arg Arg Arg Glu Asp Phe Gln Tyr Phe Phe Gly Phe Ser Thr Leu Phe
               100                 105                 110
   Ser Gly Ile Leu Leu Leu Ser Thr Val Arg Leu Ala Asn His Asn Met
           115                 120                 125
   Gln Leu Arg Ile Ser Ser Thr Ile Val Ala Leu Ser Cys Leu Leu Asn
       130                 135                 140
   Asn Leu Tyr Leu Gln Asn Lys Val His Ser Leu Lys Ile Gln Lys Glu
   145                 150                 155                 160
   Asn Leu Tyr Asn Glu Leu Ile Arg Asn Pro Lys Ser Glu Thr Thr Ile
                   165                 170                 175
   Ala Glu Ile Lys Lys Asn Lys Lys Asp Phe His Ile Tyr His Gly Leu
               180                 185                 190
   Ser Leu Leu Ser Leu Tyr Ile Ser Phe Leu Gly Leu Thr Pro Tyr Ile
           195                 200                 205
   Phe Thr
       210
<210> 74
   <211> 210
   <212> PRT
   <213> Plasmodium vivax
<220>
   <221> misc_feature
   <223> Ag40
<400> 74
   Met Gly Ala Thr Val Ser Tyr Leu Arg Cys Val Thr Ser Ile Ala Gly
   1               5                   10                  15
   Leu Ser Ser Leu Val Leu Ser Leu Phe Pro Lys Leu Ile Met Lys Asn
               20                  25                  30
   Pro Gln Val Leu Arg Pro Leu Leu Asn Ile Ser Trp Gly Tyr Leu Phe
           35                  40                  45
   Gly Ser Thr Phe Trp Leu Cys Leu Phe Ser Glu Val Gly Leu Phe Arg
       50                  55                  60
   Ser Leu Lys Asn Met Lys Arg Ile Pro Ile Pro Glu Asn Ala Glu Glu
   65                  70                  75                  80
   Ala Lys Lys Gln Leu Glu Glu Met Lys Ser Met Glu Gly Asp Phe Thr
                   85                  90                  95
   Arg Arg Arg Glu Asp Phe Gln Tyr Phe Phe Gly Phe Ser Thr Leu Phe
               100                 105                 110
   Ser Gly Ile Leu Leu Leu Ser Thr Val Arg Leu Ala Asn His Asn Met
           115                 120                 125
   Gln Leu Arg Ile Ser Ser Thr Ile Val Ala Leu Ser Cys Leu Leu Asn
       130                 135                 140
   Asn Leu Tyr Leu Gln Asn Lys Val His Ser Leu Lys Ile Gln Lys Glu
   145                 150                 155                 160
   Asn Leu Tyr Asn Glu Leu Ile Arg Asn Pro Lys Ser Glu Thr Thr Ile
                   165                 170                 175
   Ala Glu Ile Lys Lys Asn Lys Lys Asp Phe His Ile Tyr His Gly Leu
               180                 185                 190
   Ser Leu Leu Ser Leu Tyr Ile Ser Phe Leu Gly Leu Thr Pro Tyr Ile
           195                 200                 205
   Phe Thr
       210
<210> 75
   <211> 1077
   <212> DNA
   <213> Plasmodium berghei
<220>
   <221> misc_feature
   <223> Ag45
<400> 75
   ggatccgcca ccatggcttc ttactcaaac tcttccatta agcagaaatc cgatagtgtg      60
   agtgtctaca atactcggac tggaaatgtc agtaaaactc gcttgatccg tctgcaaaat     120
   gggcattacc gtagagtggt cgacattagc aataaggacg agaaggagat tctcttcagg     180
   acatgtgctt gcgcttgtcc aacacctcga aatgaggaga cacgcaaaac ctatatgcca     240
   cctctgaaca atgtgtctac cgtagcgtat agaaagcgga tctattcttc ctttgggaat     300
   aaggacggta acgatacagg caacaacgag agcataacag aacatgagga cccgattagg     360
   accttttccg aaacgacaag taggcaggaa agtaccatcg acgacaaaac ggagactagc    420
   atcaatagca aggaaacaga tgatggcaac cagtttggaa ggttgtttga agaactggag     480
   gagaaagagg atgaactgat tgaggaaaag gaggaggagc tgatagaaga gaaggaggag     540
   gaacttatag aggagaaaga agaagagctg atcgaagaga aagaggaaat cacccctgag     600
   aacaaaaccc tcataatgcc ctctaaaact ctgatgaagg gcattaagac caacatttac     660
   ttcctgtcaa acaaggaaaa gatccaagtg cttatgtgct ataactacaa gtgtgatgcc     720
   gttgtgttcg agaaagacac ctttctgcgc tatctctaca tcaagagcat caataatatc     780
   atcctgaacg aaagaatgat tgaacagttg tgcaagaacg aaaacctgaa gtacatcctt     840
   gcctgcaaca gcatagtggt tgaatcaagc gacttcatca aacccctgat cattgagttt     900
   gagtcatcca cttccaagaa catcttcgta aagcacatta agcacaatag ccagaaagaa     960
   atggacatca acaagttcaa cgagtatatg cgggatctca aaagcaatga gaagctcaga    1020
   ctgaagaaag tcgagcgatt ccactctatt aatctggcag ccaagaaatg actcgag       1077
<210> 76
   <211> 352
   <212> PRT
   <213> Plasmodium berghei
<220>
   <221> misc_feature
   <223> Ag45
<400> 76
   Met Ala Ser Tyr Ser Asn Ser Ser Ile Lys Gln Lys Ser Asp Ser Val
   1               5                   10                  15
   Ser Val Tyr Asn Thr Arg Thr Gly Asn Val Ser Lys Thr Arg Leu Ile
               20                  25                  30
   Arg Leu Gln Asn Gly His Tyr Arg Arg Val Val Asp Ile Ser Asn Lys
           35                  40                  45
   Asp Glu Lys Glu Ile Leu Phe Arg Thr Cys Ala Cys Ala Cys Pro Thr
       50                  55                  60
   Pro Arg Asn Glu Glu Thr Arg Lys Thr Tyr Met Pro Pro Leu Asn Asn
   65                  70                  75                  80
   Val Ser Thr Val Ala Tyr Arg Lys Arg Ile Tyr Ser Ser Phe Gly Asn
                   85                  90                  95
   Lys Asp Gly Asn Asp Thr Gly Asn Asn Glu Ser Ile Thr Glu His Glu
               100                 105                 110
   Asp Pro Ile Arg Thr Phe Ser Glu Thr Thr Ser Arg Gln Glu Ser Thr
           115                 120                 125
   Ile Asp Asp Lys Thr Glu Thr Ser Ile Asn Ser Lys Glu Thr Asp Asp
       130                 135                 140
   Gly Asn Gln Phe Gly Arg Leu Phe Glu Glu Leu Glu Glu Lys Glu Asp
   145                 150                 155                 160
   Glu Leu Ile Glu Glu Lys Glu Glu Glu Leu Ile Glu Glu Lys Glu Glu
                   165                 170                 175
   Glu Leu Ile Glu Glu Lys Glu Glu Glu Leu Ile Glu Glu Lys Glu Glu
               180                 185                 190
   Ile Thr Pro Glu Asn Lys Thr Leu Ile Met Pro Ser Lys Thr Leu Met
           195                 200                 205
   Lys Gly Ile Lys Thr Asn Ile Tyr Phe Leu Ser Asn Lys Glu Lys Ile
       210                 215                 220
   Gln Val Leu Met Cys Tyr Asn Tyr Lys Cys Asp Ala Val Val Phe Glu
   225                 230                 235                 240
   Lys Asp Thr Phe Leu Arg Tyr Leu Tyr Ile Lys Ser Ile Asn Asn Ile
                   245                 250                 255
   Ile Leu Asn Glu Arg Met Ile Glu Gln Leu Cys Lys Asn Glu Asn Leu
               260                 265                 270
   Lys Tyr Ile Leu Ala Cys Asn Ser Ile Val Val Glu Ser Ser Asp Phe
           275                 280                 285
   Ile Lys Pro Leu Ile Ile Glu Phe Glu Ser Ser Thr Ser Lys Asn Ile
       290                 295                 300
   Phe Val Lys His Ile Lys His Asn Ser Gln Lys Glu Met Asp Ile Asn
   305                 310                 315                 320
   Lys Phe Asn Glu Tyr Met Arg Asp Leu Lys Ser Asn Glu Lys Leu Arg
                   325                 330                 335
   Leu Lys Lys Val Glu Arg Phe His Ser Ile Asn Leu Ala Ala Lys Lys
               340                 345                 350
<210> 77
   <211> 351
   <212> PRT
   <213> Plasmodium berghei
<220>
   <221> misc_feature
   <223> Ag45
<400> 77
   Met Ser Tyr Ser Asn Ser Ser Ile Lys Gln Lys Ser Asp Ser Val Ser
   1               5                   10                  15
   Val Tyr Asn Thr Arg Thr Gly Asn Val Ser Lys Thr Arg Leu Ile Arg
               20                  25                  30
   Leu Gln Asn Gly His Tyr Arg Arg Val Val Asp Ile Ser Asn Lys Asp
           35                  40                  45
   Glu Lys Glu Ile Leu Phe Arg Thr Cys Ala Cys Ala Cys Pro Thr Pro
       50                  55                  60
   Arg Asn Glu Glu Thr Arg Lys Thr Tyr Met Pro Pro Leu Asn Asn Val
   65                  70                  75                  80
   Ser Thr Val Ala Tyr Arg Lys Arg Ile Tyr Ser Ser Phe Gly Asn Lys
                   85                  90                  95
   Asp Gly Asn Asp Thr Gly Asn Asn Glu Ser Ile Thr Glu His Glu Asp
               100                 105                 110
   Pro Ile Arg Thr Phe Ser Glu Thr Thr Ser Arg Gln Glu Ser Thr Ile
           115                 120                 125
   Asp Asp Lys Thr Glu Thr Ser Ile Asn Ser Lys Glu Thr Asp Asp Gly
       130                 135                 140
   Asn Gln Phe Gly Arg Leu Phe Glu Glu Leu Glu Glu Lys Glu Asp Glu
   145                 150                 155                 160
   Leu Ile Glu Glu Lys Glu Glu Glu Leu Ile Glu Glu Lys Glu Glu Glu
                   165                 170                 175
   Leu Ile Glu Glu Lys Glu Glu Glu Leu Ile Glu Glu Lys Glu Glu Ile
               180                 185                 190
   Thr Pro Glu Asn Lys Thr Leu Ile Met Pro Ser Lys Thr Leu Met Lys
           195                 200                 205
   Gly Ile Lys Thr Asn Ile Tyr Phe Leu Ser Asn Lys Glu Lys Ile Gln
       210                 215                 220
   Val Leu Met Cys Tyr Asn Tyr Lys Cys Asp Ala Val Val Phe Glu Lys
   225                 230                 235                 240
   Asp Thr Phe Leu Arg Tyr Leu Tyr Ile Lys Ser Ile Asn Asn Ile Ile
                   245                 250                 255
   Leu Asn Glu Arg Met Ile Glu Gln Leu Cys Lys Asn Glu Asn Leu Lys
               260                 265                 270
   Tyr Ile Leu Ala Cys Asn Ser Ile Val Val Glu Ser Ser Asp Phe Ile
           275                 280                 285
   Lys Pro Leu Ile Ile Glu Phe Glu Ser Ser Thr Ser Lys Asn Ile Phe
       290                 295                 300
   Val Lys His Ile Lys His Asn Ser Gln Lys Glu Met Asp Ile Asn Lys
   305                 310                 315                 320
   Phe Asn Glu Tyr Met Arg Asp Leu Lys Ser Asn Glu Lys Leu Arg Leu
                   325                 330                 335
   Lys Lys Val Glu Arg Phe His Ser Ile Asn Leu Ala Ala Lys Lys
               340                 345                 350
<210> 78
   <211> 1197
   <212> DNA
   <213> Artificial Sequence
<220>
   <223> PfAg45 human CO
<400> 78
   gccaccatgg cttcagatta ctttacaatt ctgtccaata ttttcacaag cactagcctg      60
   aagaagaaat acagttcccg gttgagcaca aaatccaaga agaaccaaaa gcgagtcaaa     120
   ctgataagac tgcggaatgg acattttcgc cgaattgtgg atatttccaa cattgacgag     180
   aagagcatct tccccagaag ctgtactttt gcgtcaatta gcagtgctag caaagaaaac     240
   gagaggaaga attcaagcga ggacacaaaa gaacctcagg agaatctgta tggcaaatca     300
   aacacttcaa gctctatcac gataaagatc aatttcgacg aaagcgatga gaacaagagt     360
   gatcaggata accactctat cgataccatt agcgacatct cttttaccca gacttcacgc     420
   aaatctcttg aaattgaaag taatacctat gagagttatc gcgaagtgga gaaggaggac     480
   attgaggagg aggaggaaga ggagaaagaa gaagaatatg aggaggaaga agaagaagag     540
   gaatacgaag aggaagagga agaagaggag gaagagtatg aggaggaggg tctgaaaacc     600
   gaagaggaga aggaagaaga taataaggag gtagagccag aagaggagct taaagaagaa     660
   gatgacaagg aggttgagcc tgaggaggag aaggagaatg agcagaagaa agaagaacaa     720
   gaggagaata acctcgaagc tcccagcaaa acactgatga aaggggttaa gaccaacata     780
   tacttcctgt ctaccaaaga gcggatagaa gcactcatgt gctacaacta catatccaac     840
   gccattattt tcgaaaaggg caagtttctc cgttatatct tcatgaacaa tgtcaacaat     900
   atcatcgtga acgagcacat gatcaatatg ttgtgcaaga aggaaaagat caaatacatc     960
   ctgtcatcta actccatcat cattgaaagc aacgacttca tcaaaccgct catcattgag    1020
   tttgacagta acatctctaa gaagatcttt gtcaaacact tgaaaatggt ggactccttc    1080
   aaactggatg acaagctgta cagggagtac ctgaatgacc tttctgaaca tgagagggat    1140
   agactgaaac atgtggagtc cttctattcc aatgccataa aggtgcacaa tacgtaa       1197
<210> 79
   <211> 396
   <212> PRT
   <213> Plasmodium falciparum
<220>
   <221> misc_feature
   <223> PfAg45
<400> 79
   Met Ala Ser Asp Tyr Phe Thr Ile Leu Ser Asn Ile Phe Thr Ser Thr
   1               5                   10                  15
   Ser Leu Lys Lys Lys Tyr Ser Ser Arg Leu Ser Thr Lys Ser Lys Lys
               20                  25                  30
   Asn Gln Lys Arg Val Lys Leu Ile Arg Leu Arg Asn Gly His Phe Arg
           35                  40                  45
   Arg Ile Val Asp Ile Ser Asn Ile Asp Glu Lys Ser Ile Phe Pro Arg
       50                  55                  60
   Ser Cys Thr Phe Ala Ser Ile Ser Ser Ala Ser Lys Glu Asn Glu Arg
   65                  70                  75                  80
   Lys Asn Ser Ser Glu Asp Thr Lys Glu Pro Gln Glu Asn Leu Tyr Gly
                   85                  90                  95
   Lys Ser Asn Thr Ser Ser Ser Ile Thr Ile Lys Ile Asn Phe Asp Glu
               100                 105                 110
   Ser Asp Glu Asn Lys Ser Asp Gln Asp Asn His Ser Ile Asp Thr Ile
           115                 120                 125
   Ser Asp Ile Ser Phe Thr Gln Thr Ser Arg Lys Ser Leu Glu Ile Glu
       130                 135                 140
   Ser Asn Thr Tyr Glu Ser Tyr Arg Glu Val Glu Lys Glu Asp Ile Glu
   145                 150                 155                 160
   Glu Glu Glu Glu Glu Glu Lys Glu Glu Glu Tyr Glu Glu Glu Glu Glu
                   165                 170                 175
   Glu Glu Glu Tyr Glu Glu Glu Glu Glu Glu Glu Glu Glu Glu Tyr Glu
               180                 185                 190
   Glu Glu Gly Leu Lys Thr Glu Glu Glu Lys Glu Glu Asp Asn Lys Glu
           195                 200                 205
   Val Glu Pro Glu Glu Glu Leu Lys Glu Glu Asp Asp Lys Glu Val Glu
       210                 215                 220
   Pro Glu Glu Glu Lys Glu Asn Glu Gln Lys Lys Glu Glu Gln Glu Glu
   225                 230                 235                 240
   Asn Asn Leu Glu Ala Pro Ser Lys Thr Leu Met Lys Gly Val Lys Thr
                   245                 250                 255
   Asn Ile Tyr Phe Leu Ser Thr Lys Glu Arg Ile Glu Ala Leu Met Cys
               260                 265                 270
   Tyr Asn Tyr Ile Ser Asn Ala Ile Ile Phe Glu Lys Gly Lys Phe Leu
           275                 280                 285
   Arg Tyr Ile Phe Met Asn Asn Val Asn Asn Ile Ile Val Asn Glu His
       290                 295                 300
   Met Ile Asn Met Leu Cys Lys Lys Glu Lys Ile Lys Tyr Ile Leu Ser
   305                 310                 315                 320
   Ser Asn Ser Ile Ile Ile Glu Ser Asn Asp Phe Ile Lys Pro Leu Ile
                   325                 330                 335
   Ile Glu Phe Asp Ser Asn Ile Ser Lys Lys Ile Phe Val Lys His Leu
               340                 345                 350
   Lys Met Val Asp Ser Phe Lys Leu Asp Asp Lys Leu Tyr Arg Glu Tyr
           355                 360                 365
   Leu Asn Asp Leu Ser Glu His Glu Arg Asp Arg Leu Lys His Val Glu
   370 375 380
   Ser Phe Tyr Ser Asn Ala Ile Lys Val His Asn Thr
   385 390 395
<210> 80
   <211> 395
   <212> PRT
   <213> Plasmodium falciparum
<220>
   <221> misc_feature
   <223> Ag45
<400> 80
   Met Ser Asp Tyr Phe Thr Ile Leu Ser Asn Ile Phe Thr Ser Thr Ser
   1 5 10 15
   Leu Lys Lys Lys Tyr Ser Ser Arg Leu Ser Thr Lys Ser Lys Lys Asn
   20 25 30
   Gln Lys Arg Val Lys Leu Ile Arg Leu Arg Asn Gly His Phe Arg Arg
   35 40 45
   Ile Val Asp Ile Ser Asn Ile Asp Glu Lys Ser Ile Phe Pro Arg Ser
   50 55 60
   Cys Thr Phe Ala Ser Ile Ser Ser Ala Ser Lys Glu Asn Glu Arg Lys
   65 70 75 80
   Asn Ser Ser Glu Asp Thr Lys Glu Pro Gln Glu Asn Leu Tyr Gly Lys
   85 90 95
   Ser Asn Thr Ser Ser Ser Ile Thr Ile Lys Ile Asn Phe Asp Glu Ser
   100 105 110
   Asp Glu Asn Lys Ser Asp Gln Asp Asn His Ser Ile Asp Thr Ile Ser
   115 120 125
   Asp Ile Ser Phe Thr Gln Thr Ser Arg Lys Ser Leu Glu Ile Glu Ser
   130 135 140
   Asn Thr Tyr Glu Ser Tyr Arg Glu Val Glu Lys Glu Asp Ile Glu Glu
   145 150 155 160
   Glu Glu Glu Glu Glu Lys Glu Glu Glu Tyr Glu Glu Glu Glu Glu Glu
   165 170 175
   Glu Glu Tyr Glu Glu Glu Glu Glu Glu Glu Glu Glu Glu Tyr Glu Glu
               180                 185                 190
   Glu Gly Leu Lys Thr Glu Glu Glu Lys Glu Glu Asp Asn Lys Glu Val
           195                 200                 205
   Glu Pro Glu Glu Glu Leu Lys Glu Glu Asp Asp Lys Glu Val Glu Pro
       210                 215                 220
   Glu Glu Glu Lys Glu Asn Glu Gln Lys Lys Glu Glu Gln Glu Glu Asn
   225                 230                 235                 240
   Asn Leu Glu Ala Pro Ser Lys Thr Leu Met Lys Gly Val Lys Thr Asn
                   245                 250                 255
   Ile Tyr Phe Leu Ser Thr Lys Glu Arg Ile Glu Ala Leu Met Cys Tyr
               260                 265                 270
   Asn Tyr Ile Ser Asn Ala Ile Ile Phe Glu Lys Gly Lys Phe Leu Arg
           275                 280                 285
   Tyr Ile Phe Met Asn Asn Val Asn Asn Ile Ile Val Asn Glu His Met
       290                 295                 300
   Ile Asn Met Leu Cys Lys Lys Glu Lys Ile Lys Tyr Ile Leu Ser Ser
   305                 310                 315                 320
   Asn Ser Ile Ile Ile Glu Ser Asn Asp Phe Ile Lys Pro Leu Ile Ile
                   325                 330                 335
   Glu Phe Asp Ser Asn Ile Ser Lys Lys Ile Phe Val Lys His Leu Lys
               340                 345                 350
   Met Val Asp Ser Phe Lys Leu Asp Asp Lys Leu Tyr Arg Glu Tyr Leu
           355                 360                 365
   Asn Asp Leu Ser Glu His Glu Arg Asp Arg Leu Lys His Val Glu Ser
       370                 375                 380
   Phe Tyr Ser Asn Ala Ile Lys Val His Asn Thr
   385                 390                 395
<210> 81
   <211> 1122
   <212> DNA
   <213> Artificial Sequence
<220>
   <223> PvAg45 human CO + kozac
<400> 81
   gccaccatgg ctaatttgag ttctcccctc ctggccttgc ccgaggaggg taagaagcga      60
   agaacaaaac tcatccgact gaggaacggt cactataggc ggatagtgga catttcaaat     120
   accgacgaac ggaagctcat tccttctatg tgccgctgcg cgtgtgtcac tcccagaaaa     180
   gacgaagtgg agaatgaggg taagtgggaa gacgctaaga aagcaaaatc aagccaggaa     240
   tatgatgaaa cttctgatta tgttgaatct gaaaagaaag agagctatat gctcgcagtc     300
   aatgaggagg atcagacgga ggatatgtac tcaaagacga tcagctttac ttctataacc     360
   cctacatcta taagatccga agagccagag ccaaggcgga aactctccct tctggatgtt     420
   aaagaagaag aggaagagga ggaggaggaa gaagaagagg aagaagagga agaggaggaa     480
   gaggaagagg aagaagagga agagaaagaa aaagaaaaag agaaggagga agaggaagaa     540
   gaggaggagg aagaggaaga ggaagaggag gaggaagaag aagaggaaga tgaaatagaa     600
   tctaccgcag aggaaaagga agaagagaag aagcaagtcc caccggaagg taagaaattg     660
   atcgaaccct caaagaccct tatgagaggg actaagacca acatttattt tttgagtaat     720
   aaggagatgg ttcaaactct gatgtgttat aattataatt gcaacgcagt ggtattcgaa     780
   aaagacactt ttttgaggta tctgtacatg aagagcatca gcaacatcat cctcaacgaa     840
   cgaatgatag atgaactgtg caaacaggaa gatcttaaat acgtgcttac aagcaatgct     900
   atcgtgttgg aatctactga ctttcttaag cctcttataa ttgagtttga atccagtatt     960
   agtaaaagag ttttcgtgcg ccacctgaag cataacgctc gcaaggaaat cgacatgaaa    1020
   aaatatcatg attacatggg cgaacttaac gctaacgaga agattaggct gatgaaaatt    1080
   gagcgatttc atagtttcaa caagatgatc caatgcaact ag                       1122
<210> 82
   <211> 371
   <212> PRT
   <213> Plasmodium vivax
<220>
   <221> misc_feature
   <223> PvAg45
<400> 82
   Met Ala Asn Leu Ser Ser Pro Leu Leu Ala Leu Pro Glu Glu Gly Lys
   1               5                   10                  15
   Lys Arg Arg Thr Lys Leu Ile Arg Leu Arg Asn Gly His Tyr Arg Arg
               20                  25                  30
   Ile Val Asp Ile Ser Asn Thr Asp Glu Arg Lys Leu Ile Pro Ser Met
   35 40 45
   Cys Arg Cys Ala Cys Val Thr Pro Arg Lys Asp Glu Val Glu Asn Glu
   50 55 60
   Gly Lys Trp Glu Asp Ala Lys Lys Ala Lys Ser Ser Gln Glu Tyr Asp
   65 70 75 80
   Glu Thr Ser Asp Tyr Val Glu Ser Glu Lys Lys Glu Ser Tyr Met Leu
   85 90 95
   Ala Val Asn Glu Glu Asp Gln Thr Glu Asp Met Tyr Ser Lys Thr Ile
   100 105 110
   Ser Phe Thr Ser Ile Thr Pro Thr Ser Ile Arg Ser Glu Glu Pro Glu
   115 120 125
   Pro Arg Arg Lys Leu Ser Leu Leu Asp Val Lys Glu Glu Glu Glu Glu
   130 135 140
   Glu Glu Glu Glu Glu Glu Glu Glu Glu Glu Glu Glu Glu Glu Glu Glu
   145 150 155 160
   Glu Glu Glu Glu Glu Glu Lys Glu Lys Glu Lys Glu Lys Glu Glu Glu
   165 170 175
   Glu Glu Glu Glu Glu Glu Glu Glu Glu Glu Glu Glu Glu Glu Glu Glu
   180 185 190
   Glu Glu Asp Glu Ile Glu Ser Thr Ala Glu Glu Lys Glu Glu Glu Lys
   195 200 205
   Lys Gln Val Pro Pro Glu Gly Lys Lys Leu Ile Glu Pro Ser Lys Thr
   210 215 220
   Leu Met Arg Gly Thr Lys Thr Asn Ile Tyr Phe Leu Ser Asn Lys Glu
   225 230 235 240
   Met Val Gln Thr Leu Met Cys Tyr Asn Tyr Asn Cys Asn Ala Val Val
   245 250 255
   Phe Glu Lys Asp Thr Phe Leu Arg Tyr Leu Tyr Met Lys Ser Ile Ser
   260 265 270
   Asn Ile Ile Leu Asn Glu Arg Met Ile Asp Glu Leu Cys Lys Gln Glu
   275 280 285
   Asp Leu Lys Tyr Val Leu Thr Ser Asn Ala Ile Val Leu Glu Ser Thr
       290                 295                 300
   Asp Phe Leu Lys Pro Leu Ile Ile Glu Phe Glu Ser Ser Ile Ser Lys
   305                 310                 315                 320
   Arg Val Phe Val Arg His Leu Lys His Asn Ala Arg Lys Glu Ile Asp
                   325                 330                 335
   Met Lys Lys Tyr His Asp Tyr Met Gly Glu Leu Asn Ala Asn Glu Lys
               340                 345                 350
   Ile Arg Leu Met Lys Ile Glu Arg Phe His Ser Phe Asn Lys Met Ile
           355                 360                 365
   Gln Cys Asn
       370
<210> 83
   <211> 345
   <212> PRT
   <213> Plasmodium vivax
<220>
   <221> misc_feature
   <223> Ag45
<400> 83
   Met Asn Leu Ser Ser Pro Leu Leu Ala Leu Pro Glu Glu Gly Lys Lys
   1               5                   10                  15
   Arg Arg Thr Lys Leu Ile Arg Leu Arg Asn Gly His Tyr Arg Arg Ile
               20                  25                  30
   Val Asp Ile Ser Asn Thr Asp Glu Arg Lys Leu Ile Pro Ser Met Cys
           35                  40                  45
   Arg Cys Ala Cys Val Thr Pro Arg Lys Asp Glu Val Glu Asn Glu Gly
       50                  55                  60
   Lys Trp Glu Asp Ala Lys Lys Ala Lys Ser Ser Gln Glu Tyr Asp Glu
   65                  70                  75                  80
   Thr Ser Asp Tyr Val Glu Ser Glu Lys Lys Glu Ser Tyr Met Leu Ala
                   85                  90                  95
   Val Asn Glu Glu Asp Gln Thr Glu Asp Met Tyr Ser Lys Thr Ile Ser
               100                 105                 110
   Phe Thr Ser Ile Thr Pro Thr Ser Ile Arg Ser Glu Glu Pro Glu Pro
           115                 120                 125
   Arg Arg Lys Leu Ser Leu Leu Asp Val Lys Glu Glu Glu Glu Glu Glu
       130                 135                 140
   Glu Glu Glu Glu Glu Glu Glu Glu Glu Glu Glu Glu Glu Glu Glu Glu
   145                 150                 155                 160
   Glu Lys Glu Glu Glu Glu Glu Glu Asp Glu Ile Glu Ser Thr Ala Glu
                   165                 170                 175
   Glu Lys Glu Glu Glu Lys Lys Gln Val Pro Pro Glu Gly Lys Lys Leu
               180                 185                 190
   Ile Glu Pro Ser Lys Thr Leu Met Arg Gly Thr Lys Thr Asn Ile Tyr
           195                 200                 205
   Phe Leu Ser Asn Lys Glu Met Val Gln Thr Leu Met Cys Tyr Asn Tyr
       210                 215                 220
   Asn Cys Asn Ala Val Val Phe Glu Lys Asp Thr Phe Leu Arg Tyr Leu
   225                 230                 235                 240
   Tyr Met Lys Ser Ile Ser Asn Ile Ile Leu Asn Glu Arg Met Ile Asp
                   245                 250                 255
   Glu Leu Cys Lys Gln Glu Asp Leu Lys Tyr Val Leu Thr Ser Asn Ala
               260                 265                 270
   Ile Val Leu Glu Ser Thr Asp Phe Leu Lys Pro Leu Ile Ile Glu Phe
           275                 280                 285
   Glu Ser Ser Ile Ser Lys Arg Val Phe Val Arg His Leu Lys His Asn
       290                 295                 300
   Ala Arg Lys Glu Ile Asp Met Lys Lys Tyr His Asp Tyr Met Gly Glu
   305                 310                 315                 320
   Leu Asn Ala Asn Glu Lys Ile Arg Leu Met Lys Ile Glu Arg Phe His
                   325                 330                 335
   Ser Phe Asn Lys Met Ile Gln Cys Asn
               340                 345
<210> 84
   <211> 12
   <212> DNA
   <213> Artificial Sequence
<220>
   <223> Kozac consensus sequence
<400> 84
   gccaccatgg ct 12
<210> 85
   <211> 12
   <212> DNA
   <213> Artificial Sequence
<220>
   <223> Kozac consensus sequence
<400> 85
   gccaccatgg cc 12
<210> 86
   <211> 6
   <212> DNA
   <213> Artificial Sequence
<220>
   <223> BamHI site
<400> 86
   ggatcc 6
<210> 87
   <211> 6
   <212> DNA
   <213> Artificial Sequence
<220>
   <223> Xhol site
<400> 87
   ctcgag 6
<210> 88
   <211> 9
   <212> PRT
   <213> Artificial Sequence
<220>
   <223> CD8 T cell epitope
<400> 88
   Gln Ala Gln Arg Asn Leu Asn Glu Leu
   1               5
<210> 89
   <211> 9
   <212> PRT
   <213> Artificial Sequence
<220>
   <223> CD8 T cell epitope
<400> 89
   Ser Ala Leu Leu Asn Val Asp Asn Leu
   1 5
<210> 90
   <211> 9
   <212> PRT
   <213> Artificial Sequence
<220>
   <223> CD8 T cell epitope
<400> 90
   Lys Ser Pro Ser Asn Phe Thr Ile Ile
   1 5
<210> 91
   <211> 9
   <212> PRT
   <213> Artificial Sequence
<220>
   <223> CD8 T cell epitope
<400> 91
   Ser Asn Gln Thr Asn Gln Glu Thr Ile
   1 5
<210> 92
   <211> 9
   <212> PRT
   <213> Artificial Sequence
<220>
   <223> CD8 T cell epitope
<400> 92
   Ile Thr Pro Glu Asn Lys Thr Leu Ile
   1 5
<210> 93
   <211> 9
   <212> PRT
   <213> Artificial Sequence
<220>
   <223> CD8 T cell epitope
<400> 93
   Lys Leu Ile Ala Asn Asn Pro Ser Leu
   1 5
<210> 94
   <211> 9
   <212> PRT
   <213> Artificial Sequence
<220>
   <223> CD8 T cell epitope
<400> 94
   Ser Cys Ile Leu Asn Asn Met Tyr Phe
   1 5